# EUROPEAN PATENT APPLICATION

(11) **EP 4 600 246 A1**
(43) Date of publication of application: **13.08.2025**
(21) Application number: 23874823.0
(22) Date of filing: 02.10.2023
(51) Int. Cl.: C07D 471/22, A61K 31/395, A61K 31/4375, A61K 31/444, A61K 31/4545, A61K 31/496, A61K 31/506, A61K 31/519, A61K 31/5365, A61K 31/5377, A61K 31/5386, A61K 31/5395, A61K 31/55, A61K 31/551, A61K 39/395, A61K 45/00, A61P 35/00, A61P 35/02, A61P 43/00, C07D 498/22, C07D 513/22

(54) **IMIDAZOPYRIDINE DERIVATIVES WITH BICYCLIC STRUCTURE**

(30) Priority: 03.10.2022 JP 2022159626; 20.04.2023 JP 2023069305
(71) Applicant: Public University Corporation Yokohama City University, Yokohama-shi, Kanagawa 236-0027 (JP); Daiichi Sankyo Company, Limited, Tokyo 103-8426 (JP)
(72) Inventor: NAMIKI, Hidenori, Tokyo 103-8426 (JP); TAKEDA, Yasuyuki, Tokyo 103-8426 (JP); YOSHIKAWA, Kenji, Tokyo 103-8426 (JP); AKIU, Mayuko, Tokyo 103-8426 (JP); KAWAMOTO, Yoshito, Tokyo 103-8426 (JP); MOTOYAMA, Keisuke, Tokyo 103-8426 (JP); YOSHIOKA, Shun, Tokyo 103-8426 (JP); MINAGAWA, Kosuke, Tokyo 103-8426 (JP); KADOSHIMA, Kumiko, Tokyo 103-8426 (JP); YOSHIHAMA, Yohei, Tokyo 103-8426 (JP); TSUNEMATSU, Hiroki, Tokyo 103-8426 (JP); ONO, Shigeo, Yokohama-shi, Kanagawa 236-0004 (JP); YAMASHITA, Akio, Yokohama-shi, Kanagawa 236-0004 (JP)
(74) Representative: Marks & Clerk LLP
(86) International application number: PCT/JP2023/035927
(87) International publication number: WO 2024/075696

(57) **Abstract**

The present invention addresses the problem of providing a novel compound having an SMG1 inhibitory activity and an anticancer effect, or a pharmaceutically acceptable salt thereof etc. A compound represented by formula (1) or a pharmaceutically acceptable salt thereof. 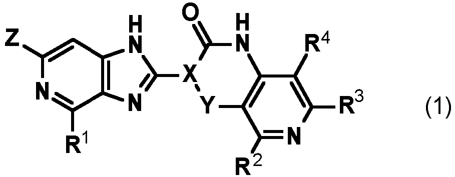 (Here, in formula (1), R¹, R², R³, R⁴, X, Y, and Z are each as defined in the specification.)

## Description

### Technical Field

The present invention relates to compounds having a specific chemical structure with SMG1 inhibitory activity, or pharmaceutically acceptable salts thereof.

### Background Art

SMG1 is a serine/threonine kinase identified as a human ortholog of *C*. *elegans* smg-1, and is classified as a PI3K-related protein kinase (PIKK) family member, similar to mTOR. SMG1 plays an essential role in the nonsense-mediated mRNA decay (NMD) system and contributes to the degradation of transcripts with premature termination codons. It has also been reported that SMG1 kinase activity is essential for its function (Non Patent Literature 1).

It has been reported that suppression of SMG1 reduces the responsiveness of cancer cells to ER stress and DNA damage, and increases their sensitivity and vulnerability to these stresses (Non Patent Literatures 2-4). Therefore, compounds that inhibit SMG1 are expected to be pharmaceuticals that exhibit efficacy against malignant tumors. SMG1 inhibitors are also expected to be pharmaceuticals for hereditary diseases such as cystic fibrosis and congenital muscular dystrophy (Non Patent Literatures 5 and 6).

In recent years, it has been suggested that the mechanism of increasing tumor neoantigens by SMG1 inhibition may be effective as a treatment for malignant tumors. For example, it has been demonstrated that suppressing the expression of SMG1 or other NMD-related factors in tumors has an antitumor effect in mouse synegeneic models, and that this effect is mediated by T cell-mediated cellular immunity (Non Patent Literature 7). The mechanism of action is thought to be that SMG1 inhibition increases and accumulates transcripts derived from somatic mutations in tumors and transcripts derived from aberrant splicing isoforms, resulting in neoantigens and activating tumor immunity (Non Patent Literatures 7 and 8).

In addition, there have been reports on the regulation of immune cell activation mechanisms by SMG1 inhibition (Non Patent Literature 9). Furthermore, papers have suggested the importance of NMD in clinical tumor immunity (Non Patent Literature 10), the importance of neoantigens in the therapeutic effect of immune checkpoint inhibitors, and the involvement of NMD (Non Patent Literatures 11-13). Compounds which inhibit NMD and SMG1 activity, such as those shown below, are known (Non Patent Literatures 14-17; Patent Literatures 1 and 2).

### Citation List

### Patent Literatures

Patent Literature 1: International Publication No. WO2018/152095
Patent Literature 2: International Publication No. WO2017/112954

### Non Patent Literatures

Non Patent Literature 1: A Yamashita, et al., Genes & Dev. 2001, 15, 2215-2228
Non Patent Literature 2: K M Brumbaugh, et al., Mol Cell. 2004, 14(5), 585-98
Non Patent Literature 3: S C Gehen, et al., Oncogene 2008, 27(29), 4065-4074
Non Patent Literature 4: E Gubanova, et al., Clin Cancer Res. 2012, 18(5), 1257-1267
Non Patent Literature 5: F Usuki, et al., Ann Neurol. 2004, 55, 740-744
Non Patent Literature 6: D R McHugh, et al., Int J Mol Sci. 2021, 22(1), 344
Non Patent Literature 7: F Pastor, et al., Nature 2010, 465(7295), 227-30
Non Patent Literature 8: J El-Bchiri, et al., PLoS ONE 2008, 3(7), e2583
Non Patent Literature 9: T Mino, et al., Nucleic Acids Res. 2019, 47(16), 8838-8859
Non Patent Literature 10: B Zhao, et al., PLoS Comput Biol. 2019, 15(10), e1007467
Non Patent Literature 11: R G H Lindeboom, et al., Nat Genetics 2019, 51(11), 1645-1651
Non Patent Literature 12: K Litchfield, et al., Nat Commun. 2020, 11, 3800
Non Patent Literature 13: K Litchfield, et al., Cell 2021, 184(3), 596-614 e14
Non Patent Literature 14: A Gopalsamy, et al., Bioorg. Med. Chem. Lett. 2012, 22 (21), 6636-6641
Non Patent Literature 15: A Nickless, et al., Cell Biosci. 2017, 7, 26
Non Patent Literature 16: Cheruiyot, et al., Cancer Res. 2021, 81(17), 4499-4513
Non Patent Literature 17: J P Becker, et al., iScience 2021, 24(4), 102389

### Summary of Invention

### Problems to be solved by the invention

An object of the present invention is to provide novel compounds having SMG1 inhibitory activity and anticancer activity, or pharmaceutically acceptable salts thereof, and the like.

### Means for solution of the problems

To solve the above object, the present inventors synthesized compounds having various structures to search for compounds having an SMG1 inhibitory effect and exhibiting an anticancer effect. As a result, the present inventors found the compounds of the present invention and pharmaceutically acceptable salts thereof, thus completing the present invention. That is, the present invention is as described below.

That is, the present invention includes the following aspects.
[1] A compound represented by formula (1), or a pharmaceutically acceptable salt thereof: 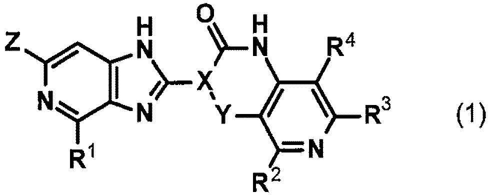 where in formula (1),
   R¹ represents a hydrogen atom, a C1-C6 alkyl group, where the C1-C6 alkyl group may be substituted with a hydroxyl group or a halogen atom, a C3-C6 cycloalkyl group, a phenyl group, where the phenyl group may be substituted with an amino group or a halogen atom, a 2-tert butylamino-2-oxoethyl group, a [dimethyl(oxido)-λ6-sulfanylidene]amino group, a group represented by the following formula (2): 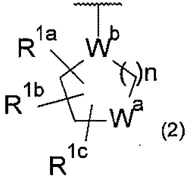
   where in formula (2), W^{a} represents CH₂, an oxygen atom, or N-R^{1f}, W^{b} represents CH, a nitrogen atom, or C-OH, R^{1a}, R¹⁶, and R^{1c} each independently represent the same or different hydrogen atom, halogen atom, hydroxyl group, C1-C6 alkyl group optionally substituted with a halogen atom, amino group, or oxo group, and n represents 0, 1, or 2,
   or -NR^{1d}R^{1e};
   R^{1d} and R^{1e} each independently represent the same or different hydrogen atom, C1-C6 alkyl group, where the C1-C6 alkyl group may be substituted with a halogen atom or a 4- to 7-membered heterocyclic group, a 4- to 7-membered heterocyclic group, or a C3-C6 cycloalkyl group optionally substituted with a hydroxyl group;
   R^{1f} represents a hydrogen atom, a C1-C6 alkyl group, or a C3-C6 cycloalkyl group;
   R² represents a hydrogen atom, a C1-C6 alkyl group, a C3-C6 cycloalkyl group optionally substituted with a C1-C6 alkyl group, a 2,4-dimethylpyrazolyl group, or any group selected from the following formula (3): 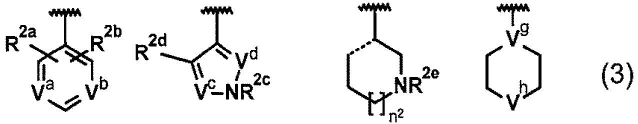
   where in formula (3), the broken line represents a single bond or a double bond, V^{a} represents C-R^{2f} or a nitrogen atom, V^{b} represents C-R^{2g} or a nitrogen atom, V^{c} represents C-R^{2j} or a nitrogen atom, V^{d} represents C-R^{2k} or a nitrogen atom, V^{g} represents CH or a nitrogen atom, V^{h} represents CHN(CH₃)₂, N-R^{2l}, or an oxygen atom, and n² represents 1 or 2,
   R^{2a} and R^{2b} each independently represent the same or different hydrogen atom, halogen atom, hydroxyl group, cyano group, C1-C6 alkyl group, where the C1-C6 alkyl group may be substituted with a hydroxyl group, a halogen atom, or a 4- to 7-membered heterocyclic group, a C1-C6 alkoxy group, where the C1-C6 alkoxy group may be substituted with a halogen atom or deuterium, or a C3-C6 cycloalkyl group,
   R^{2c} represents a C3-C6 cycloalkyl group or a C1-C6 alkyl group, where the C1-C6 alkyl group may be substituted with a C3-C6 cycloalkyl group or a halogen atom,
   R^{2d} represents a hydrogen atom, a C1-C6 alkyl group optionally substituted with a halogen atom, a C1-C6 alkoxy group, or a C3-C6 cycloalkyl group,
   R^{2e} represents a C1-C6 alkylsulfonyl group, a C3-C6 cycloalkylsulfonyl group, a benzenesulfonyl group, where the benzenesulfonyl group may be substituted with a C1-C6 alkyl group or a halogen atom, a C3-C6 cycloalkylcarbonyl group, a C1-C6 alkylcarbonyl group, or a benzoyl group, where the benzoyl group may be substituted with a C1-C6 alkyl group or a halogen atom,
   R^{2f} and R^{2g} each independently represent the same or different hydrogen atom, halogen atom, C1-C6 alkoxy group optionally substituted with a halogen atom, or C1-C6 alkyl group optionally substituted with a hydroxyl group, or the following formula (4) 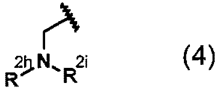
   where in formula (4), R^{2h} and R²ⁱ each independently represent the same or different C1-C6 alkyl group, or R^{2h} and R²ⁱ may be bonded to each other to form a 4- to 7-membered heterocyclic group optionally substituted with a C1-C6 alkyl group,
   R^{2j} and R^{2k} each independently represent the same or different hydrogen atom, C1-C6 alkyl group optionally substituted with a halogen atom, or C3-C6 cycloalkyl group, or
   R^{2c} and R^{2k} may be bonded to each other to form a group shown below, 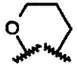
   R^{2l} represents a C1-C6 alkyl group or a C3-C6 cycloalkyl group;
   or alternatively, R¹ and R² may be bonded to each other to form a group represented by the following formula (5),
   where the leftmost bond of each group represents the bond between the aromatic ring and R¹ of the compound of formula (1), and the rightmost bond represents the bond between the aromatic ring and R² of the compound of formula (1), 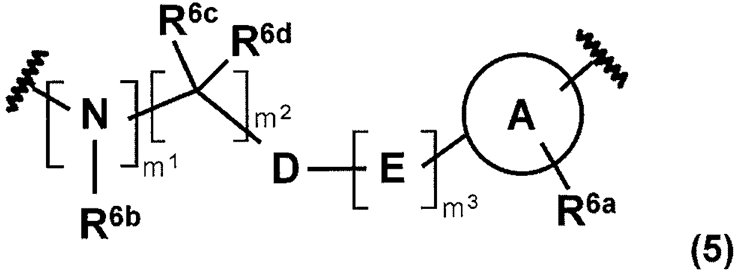
   where in formula (5), A represents an aryl group, a heteroaryl group, or a 4- to 7-membered heterocyclic group, R^{6a} represents a hydrogen atom, a halogen atom, a C1-C6 alkyl group, a C3-C6 cycloalkyl group, or a C1-C6 alkoxy group, D represents -NR^{6e}CO- or -CONR^{6f}-, E represents -CR^{6g}R^{6h}-, -NR⁶ⁱ-, -O-, -S-, -SO-, or -SO₂-, and when there are a plurality of Es, the plurality of Es may be the same or different, R^{6b}, R^{6c}, R^{6d}, R^{6e}, R^{6f}, R^{6g}, R^{6h}, and R⁶ⁱ each independently represent a hydrogen atom, a C1-C6 alkyl group, or a C3-C6 cycloalkyl group, or R^{6b} and R^{6c}, R^{6b} and R^{6e}, or R^{6c} and R^{6d} may be bonded to each other to form a ring, m¹ represents 0 or 1, m² represents 1, 2, 3, 4, or 5, and m³ represents 0, 1, 2, or 3;
   R³ represents a hydrogen atom, a halogen atom, a C1-C6 alkyl group, or a 1-methyl-3,6-dihydro-2H-pyrazin-5-yl group;
   R⁴ represents a hydrogen atom or a halogen atom;
   X-Y represents C=CH or N-CH₂; and
   Z represents a hydrogen atom, a halogen atom, a C1-C6 alkyl group optionally substituted with a halogen atom, a C1-C6 alkoxy group, a cyano group, or a C1-C6 alkoxycarbonyl group.
[2] The compound according to [1], or a pharmaceutically acceptable salt thereof, wherein R¹ represents a hydrogen atom, a C1-C6 alkyl group, -NR^{11d}R^{11e}, or any group selected from the following formula (6): 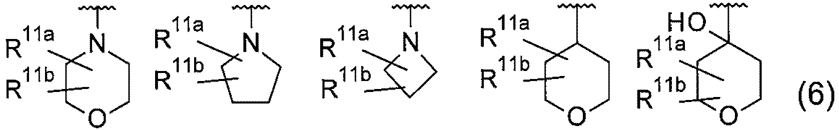 where in formula (6),
   R^{11a} and R^{11b} each independently represent the same or different hydrogen atom, halogen atom, hydroxyl group, or C1-C6 alkyl group; and
   R^{11d} and R^{11e} each independently represent the same or different hydrogen atom or C1-C6 alkyl group optionally substituted with a halogen atom.
[3] The compound according to [1] or [2], or a pharmaceutically acceptable salt thereof, wherein R² represents a hydrogen atom, a C1-C6 alkyl group, a C3-C6 cycloalkyl group optionally substituted with a C1-C6 alkyl group, a 4-(dimethylamino)piperidin-1-yl group, or any group selected from the following formula (7): 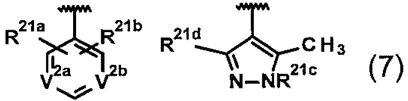 where in formula (7),
   V^{2a} represents C-R^{21e} or a nitrogen atom;
   V^{2b} represents C-R^{21f} or a nitrogen atom;
   R^{21a} and R^{21b} each independently represent the same or different hydrogen atom, halogen atom, hydroxyl group, C1-C6 alkyl group optionally substituted with a halogen atom, C1-C6 alkoxy group, where the C1-C6 alkoxy group may be substituted with a halogen atom or deuterium, or C3-C6 cycloalkyl group;
   R^{21c} represents a C1-C6 alkyl group optionally substituted with a halogen atom;
   R^{21d} represents a hydrogen atom or a C3-C6 cycloalkyl group;
   R^{21e} represents a hydrogen atom or a halogen atom; and
   R^{21f} represents a hydrogen atom or a halogen atom.
[4] The compound according to any one of [1] to [3], or a pharmaceutically acceptable salt thereof, wherein
   R³ represents a hydrogen atom;
   R⁴ represents a hydrogen atom;
   X-Y represents C=CH or N-CH₂; and
   Z represents a halogen atom.
[5] The compound according to [1], or a pharmaceutically acceptable salt thereof, wherein R¹ represents any group selected from an ethyl group, a 2,2-difluoroethylamino group, or a [(2R)-1,1,1-trifluoropropan-2-yl]amino group;
   R² represents a 2-methoxypyridin-3-yl group, a 1,5-dimethyl-1H-pyrazol-4-yl group, or a group represented by the following formula (8): 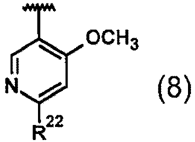
   where in formula (8), R²² represents a C1-C6 alkyl group optionally substituted with a halogen atom, a C1-C6 alkoxy group optionally substituted with a halogen atom, or a C3-C6 cycloalkyl group;
   R³ represents a hydrogen atom;
   R⁴ represents a hydrogen atom;
   X-Y represents C=CH or N-CH₂; and
   Z represents a halogen atom.
[6] The compound according to [1], or a pharmaceutically acceptable salt thereof, which is selected from the following compounds:
   3-(6-chloro-4-ethyl-1H-imidazo[4,5-c]pyridin-2-yl)-5-(6-cyclopropyl-4-methoxypyridin-3-yl)-1,6-naphthyridin-2(1H)-one,
   3-(6-chloro-4-ethyl-1H-imidazo[4,5-c]pyridin-2-yl)-5-[4-methoxy-6-(trifluoromethyl)pyridin-3-yl]-1,6-naphthyridin-2(1H)-one,
   3-(6-chloro-4-{[(2R)-1,1,1-trifluoropropan-2-yl]amino}-1H-imidazo[4,5-c]pyridin-2-yl)-5-(1,5-dimethyl-1H-pyrazol-4-yl)-1,6-naphthyridin-2(1H)-one,
   3-(6-chloro-4-ethyl-1H-imidazo[4,5-c]pyridin-2-yl)-5-[6-(1, 1-difluoroethyl)-4-methoxypyridin-3-yl]-1,6-naphthyridin-2(1H)-one,
   3-(6-chloro-4-ethyl-1H-imidazo[4,5-c]pyridin-2-yl)-5-[6-(difluoromethoxy)-4-methoxypyridin-3-yl]-1,6-naphthyridin-2(1H)-one,
   3-{6-chloro-4-[(2,2-difluoroethyl)amino]-1H-imidazo[4,5-c]pyridin-2-yl}-5-(2-methoxypyridin-3-yl)-1,6-naphthyridin-2(1H)-one, or
   3-(6-chloro-4-ethyl-1H-imidazo[4,5-c]pyridin-2-yl)-5-(6-cyclopropyl-4-methoxypyridin-3-yl)-3,4-dihydropyrido[4,3-d]pyrimidin-2(1H)-one.
[7] 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one, or a pharmaceutically acceptable salt thereof.
[8] 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one 1,2-ethanedisulfonate.
[9] 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one methanesulfonate.
[10] 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one benzenesulfonate.
[11] 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one adipate.
[12] An SMG1 inhibitor comprising the compound according to any one of [1] to [11], or a pharmaceutically acceptable salt thereof, as an active ingredient.
[13] An NMD inhibitor comprising the compound according to any one of [1] to [11], or a pharmaceutically acceptable salt thereof, as an active ingredient.
[14] A pharmaceutical composition comprising the compound according to any one of [1] to [11], or a pharmaceutically acceptable salt thereof, as an active ingredient, and optionally comprising a pharmaceutically acceptable carrier.
[15] The pharmaceutical composition according to [9] for treating cancer.
[16] The pharmaceutical composition according to [10], wherein the compound according to any one of [1] to [11], or a pharmaceutically acceptable salt thereof, is administered in combination with an immune checkpoint inhibitor.
[17] The pharmaceutical composition according to [11], wherein the compound according to any one of [1] to [11], or a pharmaceutically acceptable salt thereof, and the immune checkpoint inhibitor are each contained as an active ingredient in different pharmaceutical formulations, and are administered simultaneously or at different times.
[18] The pharmaceutical composition according to [16], wherein the compound according to any one of [1] to [11], or a pharmaceutically acceptable salt thereof, and the immune checkpoint inhibitor are contained as active ingredients in a single pharmaceutical formulation.
[19] The pharmaceutical composition according to any one of [16] to [18], wherein the immune checkpoint inhibitor is an anti-PD-1 antibody, an anti-PD-L1 antibody, or an anti-CTLA-4 antibody.
[20] The pharmaceutical composition according to any one of [15] to [19], wherein the cancer is hematological cancer selected from the group consisting of leukemia, lymphoma, and multiple myeloma; sarcoma selected from the group consisting of osteosarcoma, chordoma, chondrosarcoma, and Ewing sarcoma; or solid cancer selected from the group consisting of gastric cancer, lung cancer, colorectal cancer, esophageal cancer, head and neck cancer, breast cancer, liver cancer, kidney cancer, tongue cancer, thyroid cancer, uterine cancer, ovarian cancer, prostate cancer, cervical cancer, pancreatic cancer, skin cancer, adrenal cancer, biliary tract cancer, thymic cancer, mesothelioma, bladder cancer, and brain tumor.
[21] An anticancer agent comprising the compound according to any one of [1] to [11], or a pharmaceutically acceptable salt thereof, as an active ingredient.
[22] A method for treating cancer, comprising administering an effective amount of the compound according to any one of [1] to [11], or a pharmaceutically acceptable salt thereof.
[23] A method for treating cancer, wherein the compound according to any one of [1] to [11], or a pharmaceutically acceptable salt thereof, is administered in combination with an immune checkpoint inhibitor.
[24] The treatment method according to [23], wherein the compound according to any one of [1] to [11], or a pharmaceutically acceptable salt thereof, and the immune checkpoint inhibitor are each contained as an active ingredient in different pharmaceutical formulations, and are administered simultaneously or at different times.
[25] The treatment method according to [23], wherein the compound according to any one of [1] to [11], or a pharmaceutically acceptable salt thereof, and the immune checkpoint inhibitor are contained as active ingredients in a single pharmaceutical formulation and administered.
[26] The treatment method according to any one of [23] to [25], wherein the immune checkpoint inhibitor is an anti-PD-1 antibody, an anti-PD-L1 antibody, or an anti-CTLA-4 antibody.
[27] The treatment method according to any one of [22] to [26], wherein the cancer is hematological cancer selected from the group consisting of leukemia, lymphoma, and multiple myeloma; sarcoma selected from the group consisting of osteosarcoma, chordoma, chondrosarcoma, and Ewing sarcoma; or solid cancer selected from the group consisting of gastric cancer, lung cancer, colorectal cancer, esophageal cancer, head and neck cancer, breast cancer, liver cancer, kidney cancer, tongue cancer, thyroid cancer, uterine cancer, ovarian cancer, prostate cancer, cervical cancer, pancreatic cancer, skin cancer, adrenal cancer, biliary tract cancer, thymic cancer, mesothelioma, bladder cancer, and brain tumor.
[28] The compound according to any one of [1] to [11], or a pharmaceutically acceptable salt thereof, for use in treating cancer.
[29] The compound according to any one of [1] to [11], or a pharmaceutically acceptable salt thereof, for use in treating cancer, which is administered in combination with an immune checkpoint inhibitor.
[30] The compound according to [29], or a pharmaceutically acceptable salt thereof, wherein the compound according to any one of [1] to [11], or a pharmaceutically acceptable salt thereof, and the immune checkpoint inhibitor are each contained as an active ingredient in different pharmaceutical formulations, and are administered simultaneously or at different times.
[31] The compound according to [29], or a pharmaceutically acceptable salt thereof, wherein the compound according to any one of [1] to [11], or a pharmaceutically acceptable salt thereof, and the immune checkpoint inhibitor are contained as active ingredients in a single pharmaceutical formulation and are administered.
[32] The compound according to any one of [29] to [31], or a pharmaceutically acceptable salt thereof, wherein the immune checkpoint inhibitor is an anti-PD-1 antibody, an anti-PD-L1 antibody, or an anti-CTLA-4 antibody.
[33] The compound according to any one of [28] to [32], or a pharmaceutically acceptable salt thereof, wherein the cancer is hematological cancer selected from the group consisting of leukemia, lymphoma, and multiple myeloma; sarcoma selected from the group consisting of osteosarcoma, chordoma, chondrosarcoma, and Ewing sarcoma; or solid cancer selected from the group consisting of gastric cancer, lung cancer, colorectal cancer, esophageal cancer, head and neck cancer, breast cancer, liver cancer, kidney cancer, tongue cancer, thyroid cancer, uterine cancer, ovarian cancer, prostate cancer, cervical cancer, pancreatic cancer, skin cancer, adrenal cancer, biliary tract cancer, thymic cancer, mesothelioma, bladder cancer, and brain tumor.
[34] Use of the compound according to any one of [1] to [11], or a pharmaceutically acceptable salt thereof, in the manufacture of a medicament for the treatment of cancer.
[35] The use according to [34], wherein the compound according to any one of [1] to [11], or a pharmaceutically acceptable salt thereof, is administered in combination with an immune checkpoint inhibitor.
[36] The use according to [35], wherein the compound according to any one of [1] to [11], or a pharmaceutically acceptable salt thereof, and the immune checkpoint inhibitor are each contained as an active ingredient in different pharmaceutical formulations, and are administered simultaneously or at different times.
[37] The use according to [35], wherein the compound according to any one of [1] to [11], or a pharmaceutically acceptable salt thereof, and the immune checkpoint inhibitor are contained as active ingredients in a single pharmaceutical formulation and are administered.
[38] The use according to any one of [35] to [37], wherein the immune checkpoint inhibitor is an anti-PD-1 antibody, an anti-PD-L1 antibody, or an anti-CTLA-4 antibody.
[39] The use according to any one of [34] to [38], wherein the cancer is hematological cancer selected from the group consisting of leukemia, lymphoma, and multiple myeloma; sarcoma selected from the group consisting of osteosarcoma, chordoma, chondrosarcoma, and Ewing sarcoma; or solid cancer selected from the group consisting of gastric cancer, lung cancer, colorectal cancer, esophageal cancer, head and neck cancer, breast cancer, liver cancer, kidney cancer, tongue cancer, thyroid cancer, uterine cancer, ovarian cancer, prostate cancer, cervical cancer, pancreatic cancer, skin cancer, adrenal cancer, biliary tract cancer, thymic cancer, mesothelioma, bladder cancer, and brain tumor.

### Advantageous Effects of Invention

The compound of the present invention, or a pharmaceutically acceptable salt thereof, exhibits SMG1 and NMD inhibitory activity, an anticancer effect as a single agent, and an anticancer effect in combination with an immune checkpoint inhibitor.

### Brief Description of Drawings

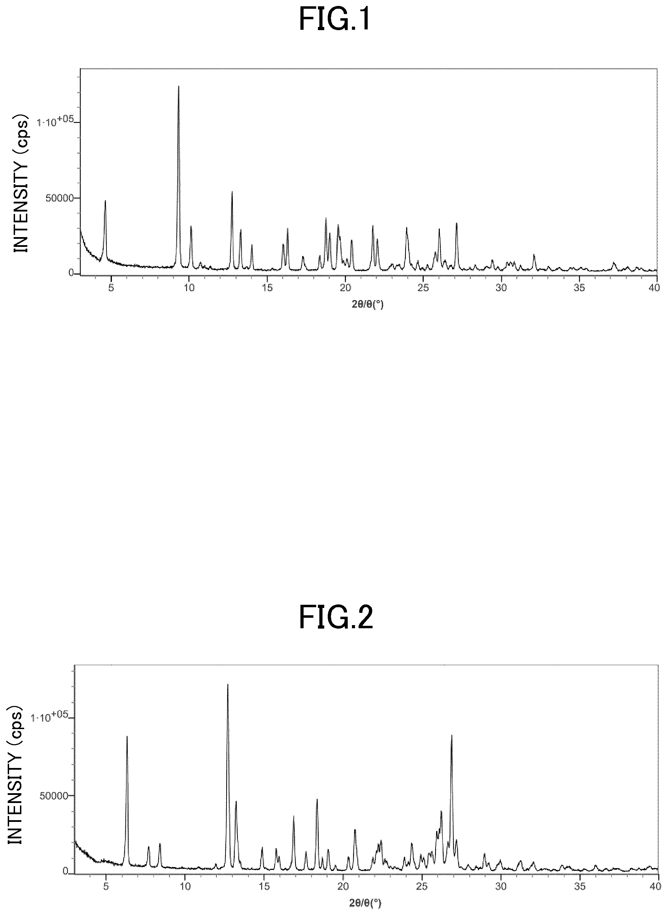

Fig. 1 is a diagram showing the diffraction pattern of powder X-ray diffraction (CuKα, λ = 1.54 angstroms, scan speed = 20°/min) of the salt crystal obtained in Example 138. The vertical axis of the figure indicates the diffraction intensity as relative line intensity, and the horizontal axis indicates the diffraction angle 2θ.
Fig. 2 is a diagram showing the diffraction pattern of powder X-ray diffraction (CuKα, λ = 1.54 angstroms, scan speed = 20°/min) of the salt crystal obtained in Example 155. The vertical axis of the figure indicates the diffraction intensity as relative line intensity, and the horizontal axis indicates the diffraction angle 2θ.

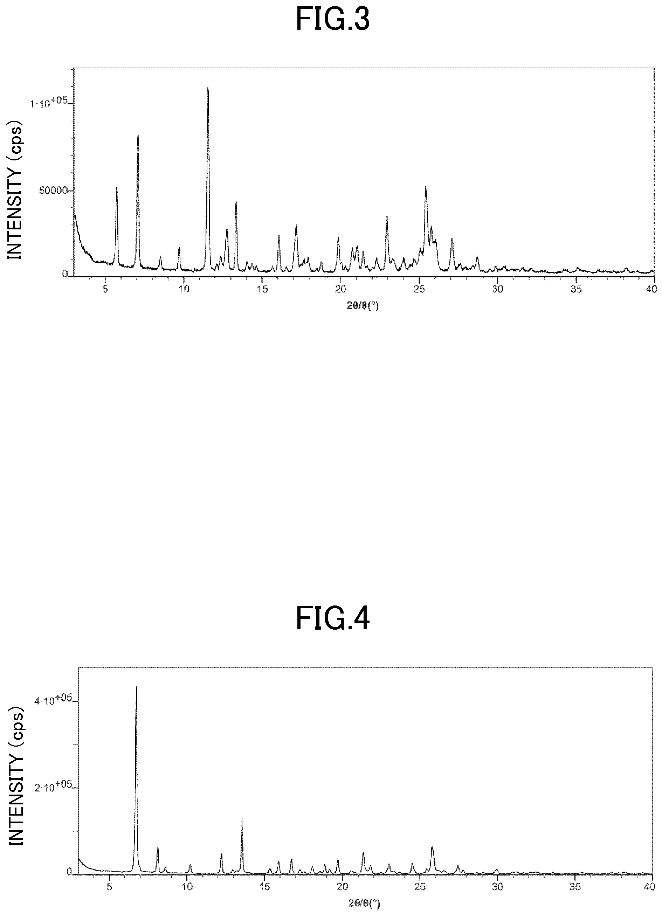

Fig. 3 is a diagram showing the diffraction pattern of powder X-ray diffraction (CuKα, λ = 1.54 angstroms, scan speed = 20°/min) of the salt crystal obtained in Example 156. The vertical axis of the figure indicates the diffraction intensity as relative line intensity, and the horizontal axis indicates the diffraction angle 2θ.
Fig. 4 is a diagram showing the diffraction pattern of powder X-ray diffraction (CuKα, λ = 1.54 angstroms, scan speed = 20°/min) of the salt crystal obtained in Example 157. The vertical axis of the figure indicates the diffraction intensity as relative line intensity, and the horizontal axis indicates the diffraction angle 2θ.

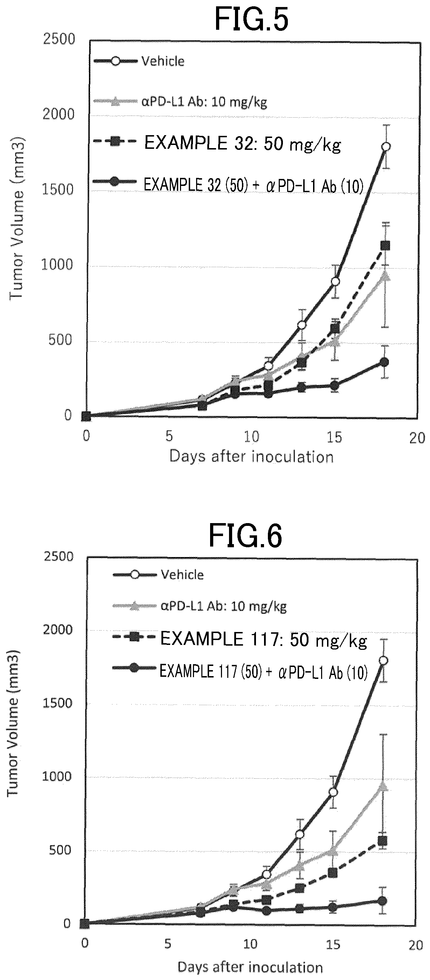

Fig. 5 is a diagram showing the antitumor effect of the compound of Example 32, administered alone and in combination with an immune checkpoint inhibitor (anti-mouse PD-L1 antibody), against subcutaneously implanted MC38 mouse colorectal cancer cell line in mice. In the figure, the horizontal axis indicates the number of days from the first administration, and the vertical axis indicates the tumor volume.
Fig. 6 is a diagram showing the antitumor effect of the compound of Example 117, administered alone and in combination with an immune checkpoint inhibitor (anti-mouse PD-L1 antibody), against subcutaneously implanted MC38 mouse colorectal cancer cell line in mice. In the figure, the horizontal axis indicates the number of days from the first administration, and the vertical axis indicates the tumor volume.

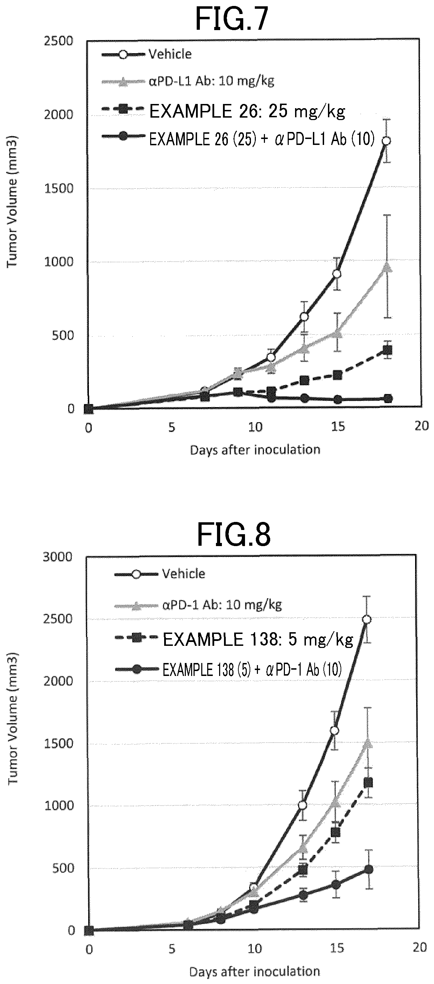

Fig. 7 is a diagram showing the antitumor effect of the compound of Example 26, administered alone and in combination with an immune checkpoint inhibitor (anti-mouse PD-L1 antibody), against subcutaneously implanted MC38 mouse colorectal cancer cell line in mice. In the figure, the horizontal axis indicates the number of days from the first administration, and the vertical axis indicates the tumor volume.
Fig. 8 is a diagram showing the antitumor effect of the compound of Example 138, administered alone and in combination with an immune checkpoint inhibitor (anti-mouse PD-1 antibody), against subcutaneously implanted MC38 mouse colorectal cancer cell line in mice. In the figure, the horizontal axis indicates the number of days from the first administration, and the vertical axis indicates the tumor volume.

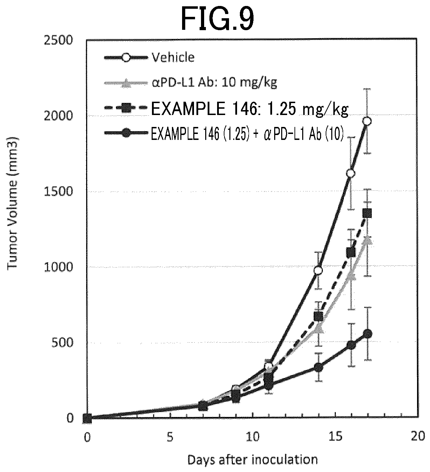

Fig. 9 is a diagram showing the antitumor effect of the compound of Example 146, administered alone and in combination with an immune checkpoint inhibitor (anti-mouse PD-L1 antibody), against subcutaneously implanted MC38 mouse colorectal cancer cell line in mice. In the figure, the horizontal axis indicates the number of days from the first administration, and the vertical axis indicates the tumor volume.

### Description of Embodiments

The substituents and terms used in the present invention are described below.

The "C1-C6 alkyl group" in the present specification is a linear or branched alkyl group having 1 to 6 carbon atoms, such as a methyl group, an ethyl group, a 1-propyl group, an isopropyl group, a 1-butyl group, a 2-butyl group, a 2-methyl-1-propyl group, a 2-methyl-2-propyl group, a 1-pentyl group, a 2-pentyl group, a 3-pentyl group, a 2-methyl-2-butyl group, a 3-methyl-2-butyl group, a 1-hexyl group, a 2-hexyl group, a 3-hexyl group, a 2-methyl-1-pentyl group, a 3-methyl-1-pentyl group, a 2-ethyl-1-butyl group, a 2,2-dimethyl-1-butyl group, or a 2,3-dimethyl-1-butyl group.

The "halogen atom" in the present specification is, for example, a fluorine atom, a chlorine atom, a bromine atom, or an iodine atom.

The "C3-C6 cycloalkyl group" in the present specification is a cyclic alkyl group having 3 to 6 carbon atoms, such as a cyclopropyl group, a cyclobutyl group, a cyclopentyl group, or a cyclohexyl group.

The "C3-C8 cycloalkyl group" in the present specification is a cyclic alkyl group having 3 to 8 carbon atoms, such as a cyclopropyl group, a cyclobutyl group, a cyclopentyl group, or a cyclohexyl group, a cycloheptyl group, or a cyclooctyl group.

The "4- to 7-membered heterocyclic group" in the present specification is a group of a 4- to 7-membered heterocycle containing 1 to 3 atoms selected from the group consisting of a nitrogen atom, an oxygen atom, and a sulfur atom; the ring may contain 1 or 2 unsaturated bonds, and the ring may be bridged by 1 to 3 methylene groups, heteroatoms, or a combination thereof. Examples include an azetidinyl group, a pyrrolidyl group, an imidazolyl group, a pyrazolyl group, an oxazolyl group, a thiazolyl group, a piperidinyl group, an azepanyl group, a piperazinyl group, a hexahydropyrimidinyl group, a morpholyl group, a thiomorpholyl group, an oxetanyl group, a tetrahydrofuryl group, a tetrahydropyranyl group, a dioxanyl group, a thioxanyl group, a dihydropyridyl group, and a 3-oxa-8-azabicyclo[3.2.1]octanyl group.

The "aryl group" in the present specification refers to an aromatic hydrocarbon group, such as phenyl and naphthyl, which is a single benzene ring or a condensed ring of benzene rings.

The "heteroaryl group" in the present specification is a group of a 5- or 6-membered monocyclic heteroaromatic ring containing 1 to 3 atoms selected from the group consisting of a nitrogen atom, an oxygen atom, and a sulfur atom; examples include a thienyl group, a pyridyl group, and a pyrazolyl group.

The "C1-C6 alkylsulfonyl group" in the present specification is a group in which a C1-C6 alkyl group is bonded to a sulfonyl group; examples include a methylsulfonyl group, an ethylsulfonyl group, a 1-propylsulfonyl group, an isopropylsulfonyl group, a 1-butylsulfonyl group, a 2-butylsulfonyl group, a 2-methyl-1-propylsulfonyl group, a 2-methyl-2-propylsulfonyl group, a 1-pentylsulfonyl group, a 2-pentylsulfonyl group, a 3-pentylsulfonyl group, a 2-methyl-2-butylsulfonyl group, a 3-methyl-2-butylsulfonyl group, a 1-hexylsulfonyl group, a 2-hexylsulfonyl group, a 3-hexylsulfonyl group, a 2-methyl-1-pentylsulfonyl group, a 3-methyl-1-pentylsulfonyl group, a 2-ethyl-1-butylsulfonyl group, a 2,2-dimethyl-1-butylsulfonyl group, or a 2,3-dimethyl-1-butylsulfonyl group.

The "C3-C6 cycloalkylsulfonyl group" in the present specification is a group in which a C3-C6 cycloalkyl group is bonded to a sulfonyl group; examples include a cyclopropylsulfonyl group, a cyclobutylsulfonyl group, a cyclopentylsulfonyl group, or a cyclohexylsulfonyl group.

The "C1-C6 alkylcarbonyl group" in the present specification is a group in which a C1-C6 alkyl group is bonded to a carbonyl group; examples include a methylcarbonyl group, an ethylcarbonyl group, a 1-propylcarbonyl group, an isopropylcarbonyl group, a 1-butylcarbonyl group, a 2-butylcarbonyl group, a 2-methyl-1-propylcarbonyl group, a 2-methyl-2-propylcarbonyl group, a 1-pentylcarbonyl group, a 2-pentylcarbonyl group, a 3-pentylcarbonyl group, a 2-methyl-2-butylcarbonyl group, a 3-methyl-2-butylcarbonyl group, a 1-hexylcarbonyl group, a 2-hexylcarbonyl group, a 3-hexylcarbonyl group, a 2-methyl-1-pentylcarbonyl group, a 3-methyl-1-pentylcarbonyl group, a 2-ethyl-1-butylcarbonyl group, a 2,2-dimethyl-1-butylcarbonyl group, or a 2,3-dimethyl-1-butylcarbonyl group.

The "C3-C6 cycloalkylcarbonyl group" in the present specification is a group in which a C3-C6 cycloalkyl group is bonded to a carbonyl group; examples include a cyclopropylcarbonyl group, a cyclobutylcarbonyl group, a cyclopentylcarbonyl group, or a cyclohexylcarbonyl group.

The "pharmaceutically acceptable salt thereof" refers to a salt that can be used as a pharmaceutical. If the compound has an acidic group or a basic group, it can be converted to a basic salt or an acidic salt by reacting with a base or an acid, respectively, so it refers to the salt.

Pharmaceutically acceptable "basic salts" of the compounds are preferably alkali metal salts such as sodium salts, potassium salts, and lithium salts; alkaline earth metal salts such as magnesium salts and calcium salts; organic basic salts such as N-methylmorpholine salts, triethylamine salts, tributylamine salts, diisopropylethylamine salts, dicyclohexylamine salts, N-methylpiperidine salts, pyridine salts, 4-pyrrolidinopyridine salts, and picoline salts; or amino acid salts such as glycine salts, lysine salts, arginine salts, ornithine salts, glutamic acid salts, and aspartic acid salts.

Pharmaceutically acceptable "acid salts" of the compounds are preferably hydrohalide salts such as hydrofluoride, hydrochloride, hydrobromide, and hydroiodide; inorganic acid salts such as nitrate, perchlorate, sulfate, and phosphate; lower alkanesulfonates such as methanesulfonate, trifluoromethanesulfonate, ethanesulfonate, and 1,2-ethanedisulfonate; arylsulfonates such as benzenesulfonate, p-toluenesulfonate, 2-naphthalenesulfonate, and 1,5-naphthalenedisulfonate; organic acid salts such as acetate, malate, fumarate, succinate, citrate, ascorbate, tartrate, oxalate, maleate, and adipate; or amino acid salts such as glycine salts, lysine salts, arginine salts, ornithine salts, glutamic acid salts, and aspartic acid salts.

The compound of the present invention or a pharmaceutically acceptable salt thereof may absorb moisture by being left in the air or by recrystallization, and may be attached with adsorbed water or become a hydrate; the present invention also includes such various hydrates, solvates, and polymorphic compounds.

The compound of the present invention, a pharmaceutically acceptable salt thereof, or a solvate thereof may have various isomers such as geometric isomers such as cis and trans isomers, tautomers, rotational isomers, and optical isomers such as d and l isomers (including enantiomers and diastereomers), depending on the type and combination of substituents. Unless otherwise specified, the compound of the present invention includes all of these isomers, stereoisomers, and mixtures of these isomers and stereoisomers in any ratio. These isomer mixtures can be separated by known resolution means.

The compound of the present invention also includes labeled compounds, that is, compounds in which one or more atoms of the compound are replaced with an isotope (e.g., 2H, 3H, 13C, 14C, 35S, etc.).

The compounds of the present invention are generally named according to the nomenclature of the International Union of Pure and Applied Chemistry (IUPAC).

In the name of the compound of the present invention, when the structure of the compound has an atom as an asymmetric center, the absolute configuration may be indicated by R and S (indicated together with the position number).

The relative configuration may be indicated by adding an asterisk (*) to the configuration designation when the configuration of the asymmetric center described first is R or S (R* and S*) or by placing the prefix (symbol) rel- (meaning relative) before the name.

Racemic mixtures are usually indicated without using R and S for the absolute configuration, but the symbols RS and SR may be used instead of R* and S* or the prefix (symbol) rac- (meaning racemic) may be placed before the name.

The present invention also includes so-called prodrugs. Prodrugs are compounds having a group that can be converted to an amino group, a hydroxyl group, a carboxyl group, etc. of the compound by hydrolysis or under physiological conditions; examples of groups that form such prodrugs are those described in Prog. Med., Vol. 5, pp. 2157-2161, 1985, and the like. More specifically, such prodrugs include the following:
(1) When an amino group is present in the compound,
   a compound in which the amino group is acylated, alkylated, or phosphorylated (e.g., a compound in which the amino group is eicosanoylated, alanylated, pentylaminocarbonylated, (5-methyl-2-oxo-1,3-dioxolen-4-yl)methoxycarbonylated, tetrahydrofurylated, pyrrolidylmethylated, pivaloyloxymethylated, or tert-butylated, etc.);
(2) When a hydroxyl group is present in the compound,
   a compound in which the hydroxyl group is acylated, alkylated, phosphorylated, or boronated (e.g., a compound in which the hydroxyl group is acetylated, palmitoylated, propanoylated, pivaloylated, succinylated, fumarylated, alanylated, or dimethylaminomethylcarbonylated, etc.);
(3) When a carboxyl group is present in the compound,
   a compound in which the carboxyl group is esterified or amidated (e.g., a compound in which the carboxyl group is ethyl esterified, phenyl esterified, carboxymethyl esterified, dimethylaminomethyl esterified, pivaloyloxymethyl esterified, ethoxycarbonyloxyethyl esterified, amidated, or methylamidated, etc.).

The compound of the present invention can also be applied as a partial structure of a degradation inducer for a target protein, generally referred to as TPD (Targeted Protein Degradation), by binding to a ligand of E3 ubiquitin ligase via a linker structure from any site of its chemical structure, utilizing its characteristic ability to bind strongly to SMG1. TPD using the compound of the present invention can be prepared by any known method, for example, the methods described in the following patent documents: WO2015/160845, WO2016/197032, WO2019/199816, WO2017/011371, WO2016/105518, US10849980 B, US10464925 B, WO2013106643, US10730862 B, WO2022/081927, WO2022/081928, WO2017/197051, WO2019/060742, WO2019/060693, WO2019/099868, and WO2018/237026.

Another aspect of the present invention will be described in detail below.

One aspect of the present invention is a compound represented by formula (1), or a pharmaceutically acceptable salt thereof: 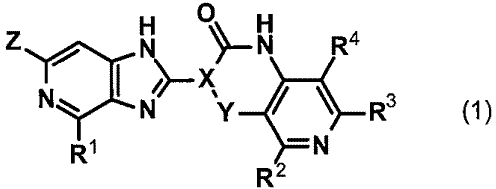 where in formula (1), R¹, R², R³, R⁴, X, Y, and Z have the same meanings as defined above.

Another preferred aspect of the compound represented by formula (1) of the present invention, or a pharmaceutically acceptable salt thereof, is shown below.

R¹ is preferably a hydrogen atom, a C1-C6 alkyl group, -NR^{11d}R^{11e}, or any group selected from the following formula (6): 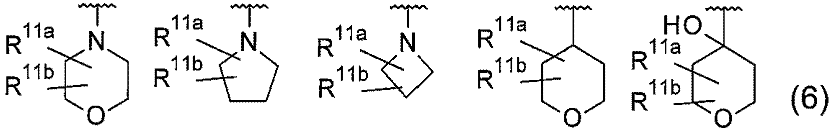 where in formula (6),
R^{11a} and R^{11b} each independently represent the same or different hydrogen atom, halogen atom, hydroxyl group, or C1-C6 alkyl group; and
R^{11d} and R^{11e} each independently represent the same or different hydrogen atom or C1-C6 alkyl group optionally substituted with a halogen atom.

More preferably, R¹ is an ethyl group, a 2,2-difluoroethylamino group, or a [(2R)-1,1,1-trifluoropropan-2-yl]amino group.

R² is preferably a hydrogen atom, a C1-C6 alkyl group, a C3-C6 cycloalkyl group optionally substituted with a C1-C6 alkyl group, a 4-(dimethylamino)piperidin-1-yl group, or any group selected from the following formula (7): 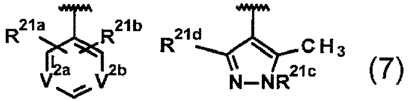 where in formula (7),
V^{2a} represents C-R^{21e} or a nitrogen atom;
V^{2b} represents C-R^{21f} or a nitrogen atom;
R^{21a} and R^{21b} each independently represent the same or different hydrogen atom, halogen atom, hydroxyl group, C1-C6 alkyl group optionally substituted with a halogen atom, C1-C6 alkoxy group, where the C1-C6 alkoxy group may be substituted with a halogen atom or deuterium, or C3-C6 cycloalkyl group;
R^{21c} represents a C1-C6 alkyl group optionally substituted with a halogen atom;
R^{21d} represents a hydrogen atom or a C3-C6 cycloalkyl group;
R^{21e} represents a hydrogen atom or a halogen atom; and
R^{21f} represents a hydrogen atom or a halogen atom.

More preferably, R² is a 2-methoxypyridin-3-yl group, a 1,5-dimethyl-1H-pyrazol-4-yl group, or a group represented by the following formula (8): 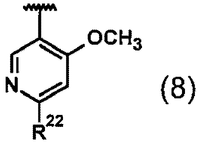 where in formula (8), R²² represents a C1-C6 alkyl group optionally substituted with a halogen atom, a C1-C6 alkoxy group optionally substituted with a halogen atom, or a C3-C6 cycloalkyl group.

In another aspect, R¹ and R² may be bonded to each other to form a group represented by the following formula (5): 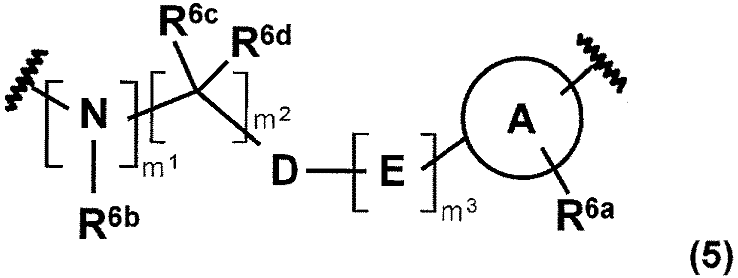 where in formula (5), R^{6a}, R^{6b}, R^{6c}, R^{6d}, A, D, E, m¹, m², and m³ have the same meanings as defined above.

Preferred aspects of the group of formula (5) are as follows:
A is a phenyl group, a 5- to 6-membered heteroaryl group containing 1 to 3 heteroatoms, or a saturated or partially unsaturated 4- to 7-membered heterocyclic group containing 1 to 2 heteroatoms.

Further preferred aspects of the group of formula (5) are any groups selected from the following (The leftmost bond of each group represents the bond between the aromatic ring and R¹ of the compound represented by formula (1), and the rightmost bond represents the bond between the aromatic ring and R² of the compound represented by formula (1).) 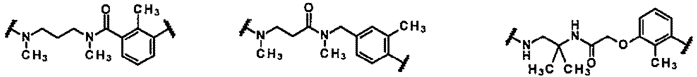 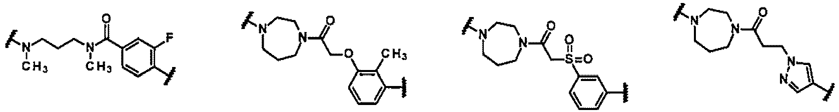 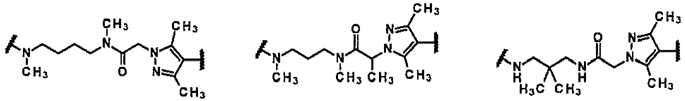
R³ is preferably a hydrogen atom.
R⁴ is preferably a hydrogen atom.
X-Y is preferably C=CH or N-CH₂.
Z is preferably a halogen atom, more preferably a chlorine atom.

In another preferred aspect, R¹ is a hydrogen atom, a methyl group, an ethyl group, an isopropyl group, or any group selected from the following.

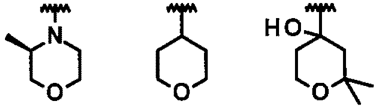

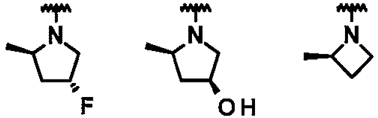

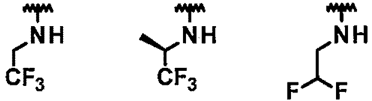

In a further preferred aspect, R¹ is an ethyl group or any group selected from the following.

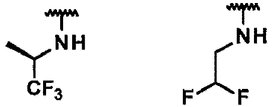

In another preferred aspect, R² is a hydrogen atom, a methyl group, an ethyl group, or any group selected from the following.

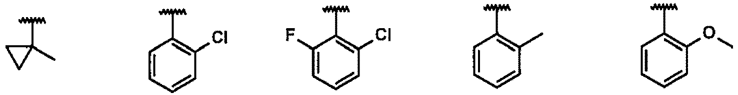

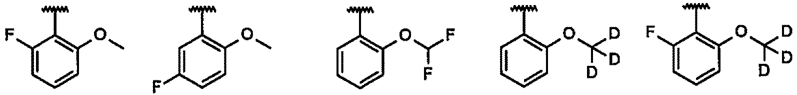

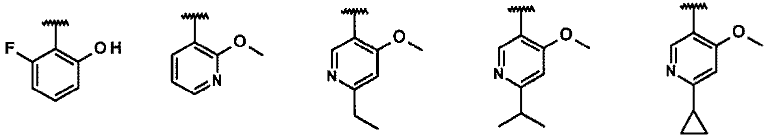

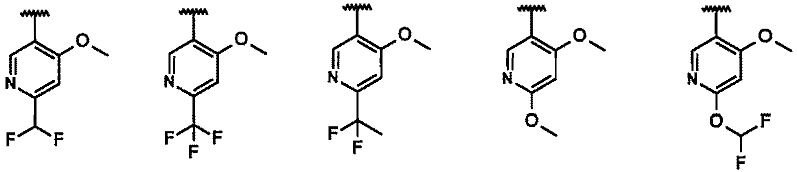

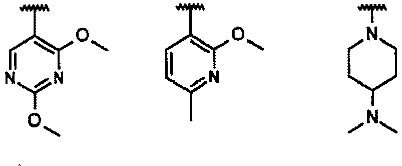

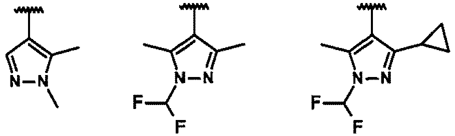

In a further preferred aspect, R² is any group selected from the following.

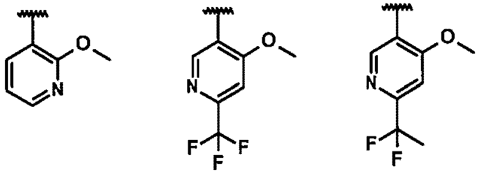

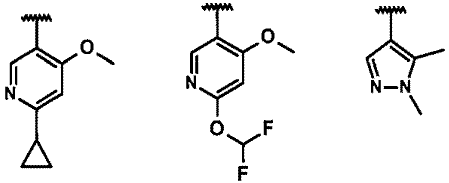

Preferred combinations of substituents in the compound represented by formula (1) in another aspect are shown below.
R¹ represents a hydrogen atom, a C1-C6 alkyl group, -NR^{11d}R^{11e}, or any group selected from the following formula (6): 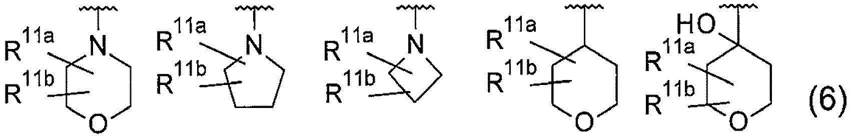 where in formula (6),
R^{11a} and R^{11b} each independently represent the same or different hydrogen atom, halogen atom, hydroxyl group, or C1-C6 alkyl group; and
R^{11d} and R^{11e} each independently represent the same or different hydrogen atom or C1-C6 alkyl group optionally substituted with a halogen atom.
R² represents a hydrogen atom, a C1-C6 alkyl group, a C3-C6 cycloalkyl group optionally substituted with a C1-C6 alkyl group, a 4-(dimethylamino)piperidin-1-yl group, or any group selected from the following formula (7): 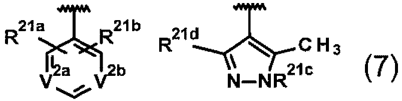 where in formula (7),
   V^{2a} represents C-R^{21e} or a nitrogen atom;
   V^{2b} represents C-R^{21f} or a nitrogen atom;
   R^{21a} and R^{21b} each independently represent the same or different hydrogen atom, halogen atom, hydroxyl group, C1-C6 alkyl group optionally substituted with a halogen atom, C1-C6 alkoxy group, where the C1-C6 alkoxy group may be substituted with a halogen atom or deuterium, or C3-C6 cycloalkyl group;
   R^{21c} represents a C1-C6 alkyl group optionally substituted with a halogen atom;
   R^{21d} represents a hydrogen atom or a C3-C6 cycloalkyl group;
   R^{21e} represents a hydrogen atom or a halogen atom; and
   R^{21f} represents a hydrogen atom or a halogen atom.
   R³ represents a hydrogen atom;
   R⁴ represents a hydrogen atom;
   X-Y represents C=CH or N-CH₂; and
   Z represents a halogen atom.

Another aspect of the present invention is a compound selected from the following, or a pharmaceutically acceptable salt thereof.
3-{6-Chloro-4-[(3R)-3-methylmorpholin-4-yl]-1H-imidazo[4,5-c]pyridin-2-yl}-1,6-naphthyridin-2(1H)-one,
3-{6-chloro-4-[(3R)-3-methylmorpholin-4-yl]-1H-imidazo[4,5-c]pyridin-2-yl}-5-(1-methylcyclopropyl)-1,6-naphthyridin-2(1H)-one,
3-{6-chloro-4-[(3R)-3-methylmorpholin-4-yl]-1H-imidazo[4,5-c]pyridin-2-yl}-7-(1-methyl-1,2,5,6-tetrahydropyridin-3-yl)-1,6-naphthyridin-2(1H)-one,
3-{6-chloro-4-[(3S)-3-(difluoromethyl)morpholin-4-yl]-1H-imidazo[4,5-c]pyridin-2-yl}-1,6-naphthyridin-2(1H)-one,
3-{6-chloro-4-[(trans-4-hydroxycyclohexyl)(methyl)amino]-1H-imidazo[4,5-c]pyridin-2-yl}-1,6-naphthyridin-2(1H)-one,
3-(6-chloro-4-{methyl[1-(tetrahydro-2H-pyran-4-yl)ethyl]amino}-1H-imidazo[4,5-c]pyridin-2-yl)-1,6-naphthyridin-2(1H)-one,
3-(6-chloro-4-{[(2R)-1,1,1-trifluoropropan-2-yl]amino}-1H-imidazo[4,5-c]pyridin-2-yl)-5-(4-methylpiperazin-1-yl)-1,6-naphthyridin-2(1H)-one,
3-(6-chloro-4-{[(2R)-1,1,1-trifluoropropan-2-yl]amino}-1H-imidazo[4,5-c]pyridin-2-yl)-5-[4-(dimethylamino)piperidin-1-yl]-1,6-naphthyridin-2(1H)-one,
3-[4-(5-amino-3,3-difluoropiperidin-1-yl)-6-chloro-1H-imidazo[4,5-c]pyridin-2-yl]-1,6-naphthyridin-2(1H)-one,
3-{6-chloro-4-[(2R,4R)-4-fluoro-2-methylpyrrolidin-1-yl]-1H-imidazo[4,5-c]pyridin-2-yl}-1,6-naphthyridin-2(1H)-one,
3-{6-chloro-4-[(2R,4R)-4-fluoro-2-methylpyrrolidin-1-yl]-1H-imidazo[4,5-c]pyridin-2-yl}-5-(tetrahydro-2H-pyran-4-yl)-1,6-naphthyridin-2(1H)-one,
3-{6-chloro-4-[(2R,4S)-4-hydroxy-2-methylpyrrolidin-1-yl]-1H-imidazo[4,5-c]pyridin-2-yl}-1,6-naphthyridin-2(1H)-one,
3-{6-chloro-4-[(2R,4R)-4-hydroxy-2-methylpyrrolidin-1-yl]-1H-imidazo[4,5-c]pyridin-2-yl}-5-methyl-1,6-naphthyridin-2(1H)-one,
3-{6-chloro-4-[(2R,3S)-3-hydroxy-2-methylpyrrolidin-1-yl]-1H-imidazo[4,5-c]pyridin-2-yl}-5-ethyl-1,6-naphthyridin-2(1H)-one,
3-{6-chloro-4-[(2R)-2-methylazetidin-1-yl]-1H-imidazo[4,5-c]pyridin-2-yl}-5-methyl-1,6-naphthyridin-2(1H)-one,
3-{6-chloro-4-[(3R)-3-methylmorpholin-4-yl]-1H-imidazo[4,5-c]pyridin-2-yl}-5-methyl-1,6-naphthyridin-2(1H)-one,
3-[6-chloro-4-(tetrahydro-2H-pyran-4-yl)-1H-imidazo[4,5-c]pyridin-2-yl]-5-methyl-1,6-naphthyridin-2(1H)-one,
3-[6-chloro-4-(tetrahydro-2H-pyran-4-yl)-1H-imidazo[4,5-c]pyridin-2-yl]-5,7-dimethyl-1,6-naphthyridin-2(1H)-one,
3-[6-chloro-4-(4-hydroxy-2,2-dimethyltetrahydro-2H-pyran-4-yl)-1H-imidazo[4,5-c]pyridin-2-yl]-1,6-naphthyridin-2(1H)-one,
3-(6-chloro-4-propyl-1H-imidazo[4,5-c]pyridin-2-yl)-5-methyl-1,6-naphthyridin-2(1H)-one,
3-[6-chloro-4-(tetrahydro-2H-pyran-4-yl)-1H-imidazo[4,5-c]pyridin-2-yl]-1,6-naphthyridin-2(1H)-one,
3-[6-chloro-4-(tetrahydro-2H-pyran-4-yl)-1H-imidazo[4,5-c]pyridin-2-yl]-5-ethyl-1,6-naphthyridin-2(1H)-one,
3-[6-chloro-4-(tetrahydro-2H-pyran-4-yl)-1H-imidazo[4,5,c]pyridin-2-yl]-5-(1-methylcyclopropyl)-1,6-naphthyridin-2(1H)-one,
3-[4-(3-aminophenyl)-6-chloro-1H-imidazo[4,5-c]pyridin-2-yl]-1H-1,6-naphthyridin-2-one,
3-[6-chloro-4-(2-fluorophenyl)-1H-imidazo[4,5-c]pyridin-2-yl]-1,6-naphthyridin-2(1H)-one,
3-(6-chloro-4-methyl-1H-imidazo[4,5-c]pyridin-2-yl)-5-{2-[(²H₃)methyloxy]phenyl}-1,6-naphthyridin-2(1H)-one,
3-(6-chloro-4-ethyl-1H-imidazo[4,5-c]pyridin-2-yl)-5-(2-fluorophenyl)-1,6-naphthyridin-2(1H)-one,
3-(6-chloro-1H-imidazo[4,5-c]pyridin-2-yl)-5-(2-chlorophenyl)-1,6-naphthyridin-2(1H)-one,
3-(6-chloro-4-methyl-1H-imidazo[4,5-c]pyridin-2-yl)-5-(2-chlorophenyl)-1,6-naphthyridin-2(1H)-one,
5-(2-chloro-6-fluorophenyl)-3-(6-chloro-1H-imidazo[4,5-c]pyridin-2-yl)-1,6-naphthyridin-2(1H)-one,
5-(2-chloro-6-fluorophenyl)-3-(6-chloro-4-methyl-1H-imidazo[4,5-c]pyridin-2-yl)-1,6-naphthyridin-2(1H)-one,
3-(6-chloro-1H-imidazo[4,5-c]pyridin-2-yl)-5-(2-methylphenyl)-1,6-naphthyridin-2(1H)-one,
3-(6-chloro-1H-imidazo[4,5-c]pyridin-2-yl)-5-[3-(difluoromethoxy)-2-methylphenyl]-1,6-naphthyridin-2(1H)-one,
3-(6-chloro-1H-imidazo[4,5-c]pyridin-2-yl)-5-(2-methoxyphenyl)-1,6-naphthyridin-2(1H)-one,
3-(6-chloro-1H-imidazo[4,5-c]pyridin-2-yl)-5-(2-fluoro-6-methoxyphenyl)-1,6-naphthyridin-2(1H)-one,
3-(6-chloro-4-ethyl-1H-imidazo[4,5-c]pyridin-2-yl)-5-(5-fluoro-2-methoxyphenyl)-1,6-naphthyridin-2(1H)-one,
3-(6-chloro-4-ethyl-1H-imidazo[4,5-c]pyridin-2-yl)-5-{2-[(²H₃)methyloxy]phenyl}-1,6-naphthyridin-2(1H)-one,
3-[6-chloro-4-(propan-2-yl)-1H-imidazo[4,5-c]pyridin-2-yl]-5-{2-[(²H₃)methyloxy]phenyl}-1,6-naphthyridin-2(1H)-one,
3-(6-chloro-4-cyclopropyl-1H-imidazo[4,5-c]pyridin-2-yl)-5-{2-[(²H₃)methyloxy]phenyl}-1,6-naphthyridin-2(1H)-one,
3-[6-chloro-4-(1-hydroxyethyl)-1H-imidazo[4,5-c]pyridin-2-yl]-5-{2-[(²H₃)methyloxy]phenyl}-1,6-naphthyridin-2(1H)-one,
3-[6-chloro-4-(1-fluoroethyl)-1H-imidazo[4,5-c]pyridin-2-yl]-5-{2-[(²H₃)methyloxy]phenyl}-1,6-naphthyridin-2(1H)-one,
N-tert-butyl-2-[6-chloro-2-(5-{2-[(²H₃)methyloxy]phenyl}-2-oxo-1,2-dihydro-1,6-naphthyridin-3-yl)-1H-imidazo[4,5-c]pyridin-4-yl]acetamide,
3-[6-chloro-4-(3-oxopiperazin-1-yl)-1H-imidazo[4,5-c]pyridin-2-yl]-5-{2-[(²H₃)methyloxy]phenyl}-1,6-naphthyridin-2(1H)-one,
3-(6-chloro-4-{[dimethyl(oxido)-λ6-sulfanylidene]amino}-1H-imidazo[4,5-c]pyridin-2-yl)-5-{2-[(²H₃)methyloxy]phenyl}-1,6-naphthyridin-2(1H)-one,
2-[3-(6-chloro-4-methyl-1H-imidazo[4,5-c]pyridin-2-yl)-2-oxo-1,2-dihydro-1,6-naphthyridin-5-yl]-3-fluorobenzonitrile,
3-(6-chloro-1H-imidazo[4,5-c]pyridin-2-yl)-5-[3-(2-hydroxypropan-2-yl)phenyl]-1,6-naphthyridin-2(1H)-one,
3-(6-chloro-1H-imidazo[4,5-c]pyridin-2-yl)-5-[3-(3-oxa-8-azabicyclo[3.2.1]octan-8-ylmethyl)phenyl]-1,6-naphthyridin-2(1H)-one,
3-{6-chloro-4-[(2,2,2-trifluoroethyl)amino]-1H-imidazo[4,5-c]pyridin-2-yl}-5-(2-methylphenyl)-1,6-naphthyridin-2(1H)-one,
3-(6-chloro-4-methyl-1H-imidazo[4,5-c]pyridin-2-yl)-5-(2-fluoro-6-methoxyphenyl)-1,6-naphthyridin-2(1H)-one,
3-(6-chloro-4-methyl-1H-imidazo[4,5-c]pyridin-2-yl)-5-(5-fluoro-2-methoxyphenyl)-1,6-naphthyridin-2(1H)-one,
3-(6-chloro-4-methyl-1H-imidazo[4,5-c]pyridin-2-yl)-5-[2-(difluoromethoxy)phenyl]-1,6-naphthyridin-2(1H)-one,
3-(6-chloro-1H-imidazo[4,5-c]pyridin-2-yl)-5-{2-[(²H₃)methyloxy]phenyl}-1,6-naphthyridin-2(1H)-one,
3-{6-chloro-4-[(3S)-tetrahydrofuran-3-ylamino]-1H-imidazo[4,5-c]pyridin-2-yl}-5-{2-[(²H₃)methyloxy]phenyl}-1,6-naphthyridin-2(1H)-one,
3-(6-chloro-1H-imidazo[4,5-c]pyridin-2-yl)-5-{2-fluoro-6-[(²H₃)methyloxy]phenyl}-1,6-naphthyridin-2(1H)-one,
3-(6-chloro-4-ethyl-1H-imidazo[4,5-c]pyridin-2-yl)-5-{2-fluoro-6-[(²H₃)methyloxy]phenyl}-1,6-naphthyridin-2(1H)-one,
3-[4-(3-aminopiperidin-1-yl)-6-chloro-1H-imidazo[4,5-c]pyridin-2-yl]-5-(2-methylphenyl)-1,6-naphthyridin-2(1H)-one,
3-(6-chloro-4-ethyl-1H-imidazo[4,5-c]pyridin-2-yl)-5-(2-fluoro-6-hydroxyphenyl)-1,6-naphthyridin-2(1H)-one,
3-(6-chloro-4-ethyl-1H-imidazo[4,5-c]pyridin-2-yl)-5-(6-cyclopropyl-4-methoxypyridin-3-yl)-1,6-naphthyridin-2(1H)-one,
3-(6-chloro-4-ethyl-1H-imidazo[4,5-c]pyridin-2-yl)-5-[4-methoxy-6-(trifluoromethyl)pyridin-3-yl]-1,6-naphthyridin-2(1H)-one,
3-(6-chloro-4-ethyl-1H-imidazo[4,5-c]pyridin-2-yl)-5-[6-(difluoromethoxy)-4-methoxypyridin-3-yl]-1,6-naphthyridin-2(1H)-one,
3-(6-chloro-4-methyl-1H-imidazo[4,5-c]pyridin-2-yl)-5-[6-(1,1-difluoroethyl)-4-methoxypyridin-3-yl]-1,6-naphthyridin-2(1H)-one,
3-(6-chloro-4-methyl-1H-imidazo[4,5-c]pyridin-2-yl)-5-(4-methoxy-6-methylpyridin-3-yl)-1,6-naphthyridin-2(1H)-one,
3-(6-chloro-4-ethyl-1H-imidazo[4,5-c]pyridin-2-yl)-5-(4-methoxy-6-methylpyridin-3-yl)-1,6-naphthyridin-2(1H)-one,
3-(6-chloro-1H-imidazo[4,5-c]pyridin-2-yl)-5-[4-methoxy-6-(propan-2-yl)pyridin-3-yl]-1,6-naphthyridin-2(1H)-one,
3-(6-chloro-1H-imidazo[4,5-c]pyridin-2-yl)-5-(6-cyclopropyl-4-methoxypyridin-3-yl)-1,6-naphthyridin-2(1H)-one,
3-(6-chloro-4-methyl-1H-imidazo[4,5-c]pyridin-2-yl)-5-[6-(difluoromethyl)-4-methoxypyridin-3-yl]-1,6-naphthyridin-2(1H)-one,
3-(6-chloro-4-methyl-1H-imidazo[4,5-c]pyridin-2-yl)-5-[4-methoxy-6-(trifluoromethyl)pyridin-3-yl]-1,6-naphthyridin-2(1H)-one,
3-(6-chloro-4-ethyl-1H-imidazo[4,5-c]pyridin-2-yl)-5-[6-(1,1-difluoroethyl)-4-methoxypyridin-3-yl]-1,6-naphthyridin-2(1H)-one,
3-(6-chloro-1H-imidazo[4,5-c]pyridin-2-yl)-5-(2-methoxy-6-methylpyridin-3-yl)-1,6-naphthyridin-2(1H)-one,
3-(6-chloro-4-methyl-1H-imidazo[4,5-c]pyridin-2-yl)-5-(2-methoxy-6-methylpyridin-3-yl)-1,6-naphthyridin-2(1H)-one,
3-(6-chloro-4-ethyl-1H-imidazo[4,5-c]pyridin-2-yl)-5-(2-methoxy-6-methylpyridin-3-yl)-1,6-naphthyridin-2(1H)-one,
3-{6-chloro-4-[(2,2-difluoroethyl)amino]-1H-imidazo[4,5-c]pyridin-2-yl}-5-(4-methoxy-2-methylpyrimidin-5-yl)-1,6-naphthyridin-2(1H)-one,
3-(6-chloro-4-ethyl-1H-imidazo[4,5-c]pyridin-2-yl)-5-(2,4-dimethoxypyrimidin-5-yl)-1,6-naphthyridin-2(1H)-one,
3-{6-chloro-4-[(2R,4R)-4-fluoro-2-methylpyrrolidin-1-yl]-1H-imidazo[4,5-c]pyridin-2-yl}-5-(2,4-dimethoxypyrimidin-5-yl)-1,6-naphthyridin-2(1H)-one,
3-{6-chloro-4-[(2,2-difluoroethyl)amino]-1H-imidazo[4,5-c]pyridin-2-yl}-5-(2,4-dimethoxypyrimidin-5-yl)-1,6-naphthyridin-2(1H)-one,
3-(6-chloro-4-ethyl-1H-imidazo[4,5-c]pyridin-2-yl)-5-(6-ethyl-4-methoxypyridin-3-yl)-1,6-naphthyridin-2(1H)-one,
3-(6-chloro-4-methyl-1H-imidazo[4,5-c]pyridin-2-yl)-5-(6-cyclopropyl-4-methoxypyridin-3-yl)-1,6-naphthyridin-2(1H)-one,
3-(6-chloro-4-ethyl-1H-imidazo[4,5-c]pyridin-2-yl)-5-[6-cyclopropyl-4-(difluoromethoxy)pyridin-3-yl]-1,6-naphthyridin-2(1H)-one,
3-(6-chloro-4-ethyl-1H-imidazo[4,5-c]pyridin-2-yl)-5-[6-(difluoromethyl)-4-methoxypyridin-3-yl]-1,6-naphthyridin-2(1H)-one,
3-{6-chloro-4-[(2,2,2-trifluoroethyl)amino]-1H-imidazo[4,5-c]pyridin-2-yl}-5-(2-methoxypyridin-3-yl)-1,6-naphthyridin-2(1H)-one,
3-{6-chloro-4-[(2,2-difluoroethyl)amino]-1H-imidazo[4,5-c]pyridin-2-yl}-5-(2-methoxypyridin-3-yl)-1,6-naphthyridin-2(1H)-one,
3-(6-chloro-4-methyl-1H-imidazo[4,5-c]pyridin-2-yl)-5-(4,6-dimethoxypyridin-3-yl)-1,6-naphthyridin-2(1H)-one,
3-(6-chloro-4-ethyl-1H-imidazo[4,5-c]pyridin-2-yl)-5-[1-(difluoromethyl)-3,5-dimethyl-1H-pyrazol-4-yl]-1,6-naphthyridin-2(1H)-one,
3-(6-chloro-4-methyl-1H-imidazo[4,5-c]pyridin-2-yl)-5-[1-(difluoromethyl)-3,5-dimethyl-1H-pyrazol-4-yl]-1,6-naphthyridin-2(1H)-one,
3-{6-chloro-4-[(2R,4S)-4-hydroxy-2-methylpyrrolidin-1-yl]-1H-imidazo[4,5-c]pyridin-2-yl}-5-[1-(difluoromethyl)-3,5-dimethyl-1H-pyrazol-4-yl]-1,6-naphthyridin-2(1H)-one,
3-(6-chloro-1H-imidazo[4,5-c]pyridin-2-yl)-5-(1-methyl-1H-pyrrol-3-yl)-1,6-naphthyridin-2(1H)-one,
3-(6-chloro-4-ethyl-1H-imidazo[4,5-c]pyridin-2-yl)-5-(1,4-dimethyl-1H-pyrazol-5-yl)-1,6-naphthyridin-2(1H)-one,
3-(6-chloro-1H-imidazo[4,5-c]pyridin-2-yl)-5-(1,3,5-trimethyl-1H-pyrazol-4-yl)-1,6-naphthyridin-2(1H)-one,
3-(6-chloro-4-ethyl-1H-imidazo[4,5-c]pyridin-2-yl)-5-(1,3,5-trimethyl-1H-pyrazol-4-yl)-1,6-naphthyridin-2(1H)-one,
3-(6-chloro-1H-imidazo[4,5-c]pyridin-2-yl)-5-[1-(cyclopropylmethyl)-3,5-dimethyl-1H-pyrazol-4-yl]-1,6-naphthyridin-2(1H)-one,
3-(6-chloro-4-methyl-1H-imidazo[4,5-c]pyridin-2-yl)-5-[3-cyclopropyl-1-(difluoromethyl)-5-methyl-1H-pyrazol-4-yl]-1,6-naphthyridin-2(1H)-one,
3-(6-chloro-4-ethyl-1H-imidazo[4,5-c]pyridin-2-yl)-5-[3-cyclopropyl-1-(difluoromethyl)-5-methyl-1H-pyrazol-4-yl]-1,6-naphthyridin-2(1H)-one,
3-(6-chloro-1H-imidazo[4,5-c]pyridin-2-yl)-5-[3-cyclopropyl-1-(2,2-difluoroethyl)-5-methyl-1H-pyrazol-4-yl]-1,6-naphthyridin-2(1H)-one,
3-(6-chloro-1H-imidazo[4,5-c]pyridin-2-yl)-5-[3-(difluoromethyl)-5-methyl-1-(propan-2-yl)-1H-pyrazol-4-yl]-1,6-naphthyridin-2(1H)-one,
3-(6-chloro-1H-imidazo[4,5-c]pyridin-2-yl)-5-(6,7-dihydro-5H-pyrazolo[5,1-b][1,3]oxazin-3-yl)-1,6-naphthyridin-2(1H)-one,
3-{6-chloro-4-[(2R)-2-methylazetidin-1-yl]-1H-imidazo[4,5-c]pyridin-2-yl}-5-[1-(difluoromethyl)-3,5-dimethyl-1H-pyrazol-4-yl]-1,6-naphthyridin-2(1H)-one,
3-(6-chloro-4-ethyl-1H-imidazo[4,5-c]pyridin-2-yl)-5-[1-(difluoromethyl)-1H-pyrazol-3-yl]-1,6-naphthyridin-2(1H)-one,
3-(6-chloro-4-ethyl-1H-imidazo[4,5-c]pyridin-2-yl)-5-(1,5-dimethyl-1H-pyrazol-3-yl)-1,6-naphthyridin-2(1H)-one,
3-(6-chloro-4-{[(2R)-1,1,1-trifluoropropan-2-yl]amino}-1H-imidazo[4,5-c]pyridin-2-yl)-5-(1,5-dimethyl-1H-pyrazol-4-yl)-1,6-naphthyridin-2(1H)-one,
3-[6-chloro-4-(tetrahydro-2H-pyran-4-yl)-1H-imidazo[4,5-c]pyridin-2-yl]-5-[3-cyclopropyl-1-(difluoromethyl)-5-methyl-1H-pyrazol-4-yl]-1,6-naphthyridin-2(1H)-one,
3-(6-chloro-4-ethyl-1H-imidazo[4,5-c]pyridin-2-yl)-5-(6-cyclopropyl-4-methoxypyridin-3-yl)-3,4-dihydropyrido[4,3-d]pyrimidin-2(1H)-one,
3-(6-chloro-1H-imidazo[4,5-c]pyridin-2-yl)-5-(2-chlorophenyl)-3,4-dihydropyrido[4,3-d]pyrimidin-2(1H)-one,
3-(6-chloro-1H-imidazo[4,5-c]pyridin-2-yl)-5-(2-methoxyphenyl)-3,4-dihydropyrido[4,3-d]pyrimidin-2(1H)-one,
3-(6-chloro-1H-imidazo[4,5-c]pyridin-2-yl)-5-[1-(cyclopropylsulfonyl)-1,2,5,6-tetrahydropyridin-3-yl]-1,6-naphthyridin-2(1H)-one,
3-(6-chloro-1H-imidazo[4,5-c]pyridin-2-yl)-5-[1-(cyclopropylcarbonyl)-1,2,5,6-tetrahydropyridin-3-yl]-1,6-naphthyridin-2(1H)-one,
3-(6-chloro-1H-imidazo[4,5-c]pyridin-2-yl)-5-[1-(phenylsulfonyl)azepan-3-yl]-1,6-naphthyridin-2(1H)-one,
7-chloro-18-fluoro-10,14-dimethyl-3,8,10,14,21,25,31-heptaazahexacyclo[18.6.2.2^{16,19}.1^{2, 5}.0^{4,9}.0^{24,28}]hentriacont-1(27),2,4,6,8,16,18,20(28),21,23,29-undecaen-15,26-dione,
7-chloro-10,14,30-trimethyl-3,8,10,14,22,26,31-heptaazahexacyclo[19.6.2.1^{2,5}.1^{16,20}.0^{4,9}.0^{2 5,29}]hentriacont-1(28),2,4,6,8,16(30),17,19,21(29),22,24-undecaen-15,27-dione,
7-chloro-10,14,18-trimethyl-3,8,10,14,21,25,31-heptaazahexacyclo[18.6.2.2^{16,19}.1^{2,5}.0^{4,9}.0^{2 4,28}]hentriacont-1(27),2,4,6,8,16,18,20(28),21,23,29-undecaen-13,26-dione,
7-chloro-32-methyl-17-oxa-3,8,10,14,24,28,35-heptaazaheptacyclo[21.6.2.2^{10,14}.1^{2,5}.1^{18,22}. 0^{4,9}.0^{27,31}]pentatriacont-1(30),2,4,6,8,18(32),19,21,23(31),24,26-undecaen-15,29-dione,
7-chloro-12,12,31-trimethyl-16-oxa-3,8,10,13,23,27,32-heptaazahexacyclo[20.6.2.1^{2,5}.1^{17,2 1}.0^{4,9}.0^{26,30}]dotriacont-1(29),2,4,6,8,17(31),18,20,22(30),23,25-undecaen-14,28-dione,
7-chloro-17-thia-3,8,10,14,24,28,35-heptaazaheptacyclo[21.6.2.2^{10,14}.1^{2,5}.1^{18,22}.0^{4,9}.0^{27,31}]p entatriacont-1(30),2,4,6,8,18(32),19,21,23(31),24,26-undecaen-15,29-dione 17,17-dioxid e,
7-chloro-10,15,20,31-tetramethyl-3,8,10,15,18,19,23,27,32-nonaazahexacyclo[20.6.2.1^{2,5}. 1^{18,21}0^{4,9}.0^{26,30}]dotriacont-1(29),2,4,6,8,19,21(31),22(30),23,25-decaen-16,28-dione,
7-chloro-10,14,16,19,30-pentamethyl-3,8,10,14,17,18,22,26,31-nonaazahexacyclo[19.6.2. 1^{2,5}.1^{17,20}.0^{4,9}.0^{25,29}]hentriacont-1(28),2,4,6,8,18,20(30),21(29),22,24-decaen-15,27-dione,
7-chloro-12,12,19,30-tetramethyl-3,8,10,14,17,18,22,26,31-nonaazahexacyclo[19.6.2.1^{2,5}. 1^{17,20}.0^{4,9}.0^{25,29}]hentriacont-1(28),2,4,6,8,18,20(30),21(29),22,24-decaen-15,27-dione, or
7-chloro-3,8,10,14,18,19,23,27,34-nonaazaheptacyclo[20.6.2.2^{10,14}.1^{2,5}1^{18,21}.0^{4,9}.0^{26,30}]tetr atriacont-1(29),2,4,6,8,19,21(31),22(30),23,25-decaen-15,28-dione.

Preferred aspects of the present invention are compounds selected from the following, or pharmaceutically acceptable salts thereof.
3-{6-Chloro-4-[(3R)-3-methylmorpholin-4-yl]-1H-imidazo[4,5-c]pyridin-2-yl}-1,6-naphthyridin-2(1H)-one,
3-(6-chloro-4-{[(2R)-1,1,1-trifluoropropan-2-yl]amino}-1H-imidazo[4,5-c]pyridin-2-yl)-5-[4-(dimethylamino)piperidin-1-yl]-1,6-naphthyridin-2(1H)-one,
3-{6-chloro-4-[(2R,4R)-4-fluoro-2-methylpyrrolidin-1-yl]-1H-imidazo[4,5-c]pyridin-2-yl}-1,6-naphthyridin-2(1H)-one,
3-{6-chloro-4-[(2R)-2-methylazetidin-1-yl]-1H-imidazo[4,5-c]pyridin-2-yl}-5-methyl-1,6-naphthyridin-2(1H)-one,
3-{6-chloro-4-[(3R)-3-methylmorpholin-4-yl]-1H-imidazo[4,5-c]pyridin-2-yl}-5-methyl-1,6-naphthyridin-2(1H)-one,
3-[6-chloro-4-(tetrahydro-2H-pyran-4-yl)-1H-imidazo[4,5-c]pyridin-2-yl]-5-methyl-1,6-naphthyridin-2(1H)-one,
3-[6-chloro-4-(4-hydroxy-2,2-dimethyltetrahydro-2H-pyran-4-yl)-1H-imidazo[4,5-c]pyridin-2-yl]-1,6-naphthyridin-2(1H)-one,
3-[6-chloro-4-(tetrahydro-2H-pyran-4-yl)-1H-imidazo[4,5-c]pyridin-2-yl]-1,6-naphthyridin-2(1H)-one,
3-[6-chloro-4-(tetrahydro-2H-pyran-4-yl)-1H-imidazo[4,5-c]pyridin-2-yl]-5-ethyl-1,6-naphthyridin-2(1H)-one,
3-[6-chloro-4-(tetrahydro-2H-pyran-4-yl)-1H-imidazo[4,5,c]pyridin-2-yl]-5-(1-methylcyclopropyl)-1,6-naphthyridin-2(1H)-one,
3-(6-chloro-4-methyl-1H-imidazo[4,5-c]pyridin-2-yl)-5-{2-[(²H₃)methyloxy]phenyl}-1,6-naphthyridin-2(1H)-one,
3-(6-chloro-1H-imidazo[4,5-c]pyridin-2-yl)-5-(2-chlorophenyl)-1,6-naphthyridin-2(1H)-one,
3-(6-chloro-4-methyl-1H-imidazo[4,5-c]pyridin-2-yl)-5-(2-chlorophenyl)-1,6-naphthyridin-2(1H)-one,
5-(2-chloro-6-fluorophenyl)-3-(6-chloro-4-methyl-1H-imidazo[4,5-c]pyridin-2-yl)-1,6-naphthyridin-2(1H)-one,
3-(6-chloro-1H-imidazo[4,5-c]pyridin-2-yl)-5-(2-methylphenyl)-1,6-naphthyridin-2(1H)-one,
3-(6-chloro-1H-imidazo[4,5-c]pyridin-2-yl)-5-(2-methoxyphenyl)-1,6-naphthyridin-2(1H)-one,
3-(6-chloro-1H-imidazo[4,5-c]pyridin-2-yl)-5-(2-fluoro-6-methoxyphenyl)-1,6-naphthyridin-2(1H)-one,
3-(6-chloro-4-ethyl-1H-imidazo[4,5-c]pyridin-2-yl)-5-(5-fluoro-2-methoxyphenyl)-1,6-naphthyridin-2(1H)-one,
3-(6-chloro-4-ethyl-1H-imidazo[4,5-c]pyridin-2-yl)-5-{2-[(²H₃)methyloxy]phenyl}-1,6-naphthyridin-2(1H)-one,
3-[6-chloro-4-(propan-2-yl)-1H-imidazo[4,5-c]pyridin-2-yl]-5-{2-[(²H₃)methyloxy]phenyl}-1,6-naphthyridin-2(1H)-one,
3-{6-chloro-4-[(2,2,2-trifluoroethyl)amino]-1H-imidazo[4,5-c]pyridin-2-yl}-5-(2-methylphenyl)-1,6-naphthyridin-2(1H)-one,
3-(6-chloro-4-methyl-1H-imidazo[4,5-c]pyridin-2-yl)-5-(2-fluoro-6-methoxyphenyl)-1,6-naphthyridin-2(1H)-one,
3-(6-chloro-4-methyl-1H-imidazo[4,5-c]pyridin-2-yl)-5-(5-fluoro-2-methoxyphenyl)-1,6-naphthyridin-2(1H)-one,
3-(6-chloro-4-methyl-1H-imidazo[4,5-c]pyridin-2-yl)-5-[2-(difluoromethoxy)phenyl]-1,6-naphthyridin-2(1H)-one,
3-(6-chloro-1H-imidazo[4,5-c]pyridin-2-yl)-5-{2-[(²H₃)methyloxy]phenyl}-1,6-naphthyridin-2(1H)-one,
3-(6-chloro-1H-imidazo[4,5-c]pyridin-2-yl)-5-{2-fluoro-6-[(²H₃)methyloxy]phenyl}-1,6-naphthyridin-2(1H)-one,
3-(6-chloro-4-ethyl-1H-imidazo[4,5-c]pyridin-2-yl)-5-{2-fluoro-6-[(²H₃)methyloxy]phenyl}-1,6-naphthyridin-2(1H)-one,
3-(6-chloro-4-ethyl-1H-imidazo[4,5-c]pyridin-2-yl)-5-(2-fluoro-6-hydroxyphenyl)-1,6-naphthyridin-2(1H)-one,
3-(6-chloro-4-ethyl-1H-imidazo[4,5-c]pyridin-2-yl)-5-(6-cyclopropyl-4-methoxypyridin-3-yl)-1,6-naphthyridin-2(1H)-one,
3-(6-chloro-4-ethyl-1H-imidazo[4,5-c]pyridin-2-yl)-5-[4-methoxy-6-(trifluoromethyl)pyridin-3-yl]-1,6-naphthyridin-2(1H)-one,
3-(6-chloro-4-ethyl-1H-imidazo[4,5-c]pyridin-2-yl)-5-[6-(difluoromethoxy)-4-methoxypyridin-3-yl]-1,6-naphthyridin-2(1H)-one,
3-(6-chloro-4-ethyl-1H-imidazo[4,5-c]pyridin-2-yl)-5-[6-(1,1-difluoroethyl)-4-methoxypyridin-3-yl]-1,6-naphthyridin-2(1H)-one,
3-(6-chloro-1H-imidazo[4,5-c]pyridin-2-yl)-5-[4-methoxy-6-(propan-2-yl)pyridin-3-yl]-1,6-naphthyridin-2(1H)-one,
3-(6-chloro-4-ethyl-1H-imidazo[4,5-c]pyridin-2-yl)-5-(2-methoxy-6-methylpyridin-3-yl)-1,6-naphthyridin-2(1H)-one,
3-(6-chloro-4-ethyl-1H-imidazo[4,5-c]pyridin-2-yl)-5-(2,4-dimethoxypyrimidin-5-yl)-1,6-naphthyridin-2(1H)-one,
3-{6-chloro-4-[(2,2-difluoroethyl)amino]-1H-imidazo[4,5-c]pyridin-2-yl}-5-(2,4-dimethoxypyrimidin-5-yl)-1,6-naphthyridin-2(1H)-one,
3-(6-chloro-4-ethyl-1H-imidazo[4,5-c]pyridin-2-yl)-5-(6-ethyl-4-methoxypyridin-3-yl)-1,6-naphthyridin-2(1H)-one,
3-(6-chloro-4-methyl-1H-imidazo[4,5-c]pyridin-2-yl)-5-(6-cyclopropyl-4-methoxypyridin-3-yl)-1,6-naphthyridin-2(1H)-one,
3-(6-chloro-4-ethyl-1H-imidazo[4,5-c]pyridin-2-yl)-5-[6-(difluoromethyl)-4-methoxypyridin-3-yl]-1,6-naphthyridin-2(1H)-one,
3-{6-chloro-4-[(2,2,2-trifluoroethyl)amino]-1H-imidazo[4,5-c]pyridin-2-yl}-5-(2-methoxypyridin-3-yl)-1,6-naphthyridin-2(1H)-one,
3-{6-chloro-4-[(2,2-difluoroethyl)amino]-1H-imidazo[4,5-c]pyridin-2-yl}-5-(2-methoxypyridin-3-yl)-1,6-naphthyridin-2(1H)-one,
3-(6-chloro-4-methyl-1H-imidazo[4,5-c]pyridin-2-yl)-5-(4,6-dimethoxypyridin-3-yl)-1,6-naphthyridin-2(1H)-one,
3-(6-chloro-4-ethyl-1H-imidazo[4,5-c]pyridin-2-yl)-5-[1-(difluoromethyl)-3,5-dimethyl-1H-pyrazol-4-yl]-1,6-naphthyridin-2(1H)-one,
3-(6-chloro-4-methyl-1H-imidazo[4,5-c]pyridin-2-yl)-5-[1-(difluoromethyl)-3,5-dimethyl-1H-pyrazol-4-yl]-1,6-naphthyridin-2(1H)-one,
3-{6-chloro-4-[(2R,4S)-4-hydroxy-2-methylpyrrolidin-1-yl]-1H-imidazo[4,5-c]pyridin-2-yl}-5-[1-(difluoromethyl)-3,5-dimethyl-1H-pyrazol-4-yl]-1,6-naphthyridin-2(1H)-one,
3-(6-chloro-4-methyl-1H-imidazo[4,5-c]pyridin-2-yl)-5-[3-cyclopropyl-1-(difluoromethyl)-5-methyl-1H-pyrazol-4-yl]-1,6-naphthyridin-2(1H)-one,
3-(6-chloro-4-ethyl-1H-imidazo[4,5-c]pyridin-2-yl)-5-[3-cyclopropyl-1-(difluoromethyl)-5-methyl-1H-pyrazol-4-yl]-1,6-naphthyridin-2(1H)-one,
3-{6-chloro-4-[(2R)-2-methylazetidin-1-yl]-1H-imidazo[4,5-c]pyridin-2-yl}-5-[1-(difluoromethyl)-3,5-dimethyl-1H-pyrazol-4-yl]-1,6-naphthyridin-2(1H)-one,
3-(6-chloro-4-{[(2R)-1,1,1-trifluoropropan-2-yl]amino}-1H-imidazo[4,5-c]pyridin-2-yl)-5-(1,5-dimethyl-1H-pyrazol-4-yl)-1,6-naphthyridin-2(1H)-one,
3-[6-chloro-4-(tetrahydro-2H-pyran-4-yl)-1H-imidazo[4,5-c]pyridin-2-yl]-5-[3-cyclopropyl-1-(difluoromethyl)-5-methyl-1H-pyrazol-4-yl]-1,6-naphthyridin-2(1H)-one, or
3-(6-chloro-4-ethyl-1H-imidazo[4,5-c]pyridin-2-yl)-5-(6-cyclopropyl-4-methoxypyridin-3-yl)-3,4-dihydropyrido[4,3-d]pyrimidin-2(1H)-one.

More preferred aspects of the present invention are compounds selected from the following, or pharmaceutically acceptable salts thereof.
3-(6-Chloro-4-ethyl-1H-imidazo[4,5-c]pyridin-2-yl)-5-(6-cyclopropyl-4-methoxypyridin-3-yl)-1,6-naphthyridin-2(1H)-one,
3-(6-chloro-4-ethyl-1H-imidazo[4,5-c]pyridin-2-yl)-5-[4-methoxy-6-(trifluoromethyl)pyridin-3-yl]-1,6-naphthyridin-2(1H)-one,
3-(6-chloro-4-{[(2R)-1,1,1-trifluoropropan-2-yl]amino}-1H-imidazo[4,5-c]pyridin-2-yl)-5-(1,5-dimethyl-1H-pyrazol-4-yl)-1,6-naphthyridin-2(1H)-one,
3-(6-chloro-4-ethyl-1H-imidazo[4,5-c]pyridin-2-yl)-5-[6-(1,1-difluoroethyl)-4-methoxypyridin-3-yl]-1,6-naphthyridin-2(1H)-one,
3-(6-chloro-4-ethyl-1H-imidazo[4,5-c]pyridin-2-yl)-5-[6-(difluoromethoxy)-4-methoxypyridin-3-yl]-1,6-naphthyridin-2(1H)-one,
3-{6-chloro-4-[(2,2-difluoroethyl)amino]-1H-imidazo[4,5-c]pyridin-2-yl}-5-(2-methoxypyridin-3-yl)-1,6-naphthyridin-2(1H)-one, or
3-(6-chloro-4-ethyl-1H-imidazo[4,5-c]pyridin-2-yl)-5-(6-cyclopropyl-4-methoxypyridin-3-yl)-3,4-dihydropyrido[4,3-d]pyrimidin-2(1H)-one.

Preferred aspects of the present invention in another aspect are salts of compounds selected from the following.
3-[4-(3-Aminophenyl)-6-chloro-1H-imidazo[4,5-c]pyridin-2-yl]-1H-1,6-naphthyridin-2-one trifluoroacetate,
3-[4-(3-aminopiperidin-1-yl)-6-chloro-1H-imidazo[4,5-c]pyridin-2-yl]-5-(2-methylphenyl)-1,6-naphthyridin-2(1H)-one hydrochloride,
3-[6-chloro-4-(tetrahydro-2H-pyran-4-yl)-1H-imidazo[4,5-c]pyridin-2-yl]-5-methyl-1,6-naphthyridin-2(1H)-one methanesulfonate,
3-[6-chloro-4-(tetrahydro-2H-pyran-4-yl)-1H-imidazo[4,5-c]pyridin-2-yl]-1,6-naphthyridin-2(1H)-one methanesulfonate,
3-[6-chloro-4-(tetrahydro-2H-pyran-4-yl)-1H-imidazo[4,5-c]pyridin-2-yl]-5-ethyl-1,6-naphthyridin-2(1H)-one methanesulfonate,
3-[6-chloro-4-(tetrahydro-2H-pyran-4-yl)-1H-imidazo[4,5,c]pyridin-2-yl]-5-(1-methylcyclopropyl)-1,6-naphthyridin-2(1H)-one methanesulfonate,
3-{6-chloro-4-[(2,2,2-trifluoroethyl)amino]-1H-imidazo[4,5-c]pyridin-2-yl}-5-(2-methylphenyl)-1,6-naphthyridin-2(1H)-one methanesulfonate,
3-(6-chloro-4-methyl-1H-imidazo[4,5-c]pyridin-2-yl)-5-(2-fluoro-6-methoxyphenyl)-1,6-naphthyridin-2(1H)-one methanesulfonate,
3-(6-chloro-4-methyl-1H-imidazo[4,5-c]pyridin-2-yl)-5-(5-fluoro-2-methoxyphenyl)-1,6-naphthyridin-2(1H)-one methanesulfonate,
3-(6-chloro-4-methyl-1H-imidazo[4,5-c]pyridin-2-yl)-5-[2-(difluoromethoxy)phenyl]-1,6-naphthyridin-2(1H)-one methanesulfonate,
3-(6-chloro-1H-imidazo[4,5-c]pyridin-2-yl)-5-{2-[(²H₃)methyloxy]phenyl}-1,6-naphthyridin-2(1H)-one methanesulfonate,
3-{6-chloro-4-[(3S)-tetrahydrofuran-3-ylamino]-1H-imidazo[4,5-c]pyridin-2-yl}-5-{2-[(²H₃)methyloxy]phenyl}-1,6-naphthyridin-2(1H)-one methanesulfonate,
3-(6-chloro-1H-imidazo[4,5-c]pyridin-2-yl)-5-{2-fluoro-6-[(²H₃)methyloxy]phenyl}-1,6-naphthyridin-2(1H)-one methanesulfonate,
3-(6-chloro-4-ethyl-1H-imidazo[4,5-c]pyridin-2-yl)-5-{2-fluoro-6-[(²H₃)methyloxy]phenyl}-1,6-naphthyridin-2(1H)-one methanesulfonate,
3-(6-chloro-4-ethyl-1H-imidazo[4,5-c]pyridin-2-yl)-5-(2-fluoro-6-hydroxyphenyl)-1,6-naphthyridin-2(1H)-one methanesulfonate,
3-(6-chloro-4-ethyl-1H-imidazo[4,5-c]pyridin-2-yl)-5-(6-cyclopropyl-4-methoxypyridin-3-yl)-1,6-naphthyridin-2(1H)-one methanesulfonate,
3-(6-chloro-4-ethyl-1H-imidazo[4,5-c]pyridin-2-yl)-5-(6-ethyl-4-methoxypyridin-3-yl)-1,6-naphthyridin-2(1H)-one methanesulfonate,
3-(6-chloro-4-methyl-1H-imidazo[4,5-c]pyridin-2-yl)-5-(6-cyclopropyl-4-methoxypyridin-3-yl)-1,6-naphthyridin-2(1H)-one methanesulfonate,
3-(6-chloro-4-ethyl-1H-imidazo[4,5-c]pyridin-2-yl)-5-[6-(difluoromethyl)-4-methoxypyridin-3-yl]-1,6-naphthyridin-2(1H)-one methanesulfonate,
3-{6-chloro-4-[(2,2,2-trifluoroethyl)amino]-1H-imidazo[4,5-c]pyridin-2-yl}-5-(2-methoxypyridin-3-yl)-1,6-naphthyridin-2(1H)-one methanesulfonate,
3-{6-chloro-4-[(2,2-difluoroethyl)amino]-1H-imidazo[4,5-c]pyridin-2-yl}-5-(2-methoxypyridin-3-yl)-1,6-naphthyridin-2(1H)-one methanesulfonate,
3-(6-chloro-4-methyl-1H-imidazo[4,5-c]pyridin-2-yl)-5-(4,6-dimethoxypyridin-3-yl)-1,6-naphthyridin-2(1H)-one methanesulfonate,
3-(6-chloro-4-ethyl-1H-imidazo[4,5-c]pyridin-2-yl)-5-[4-methoxy-6-(trifluoromethyl)pyridin-3-yl]-1,6-naphthyridin-2(1H)-one methanesulfonate,
3-(6-chloro-4-ethyl-1H-imidazo[4,5-c]pyridin-2-yl)-5-[6-(difluoromethoxy)-4-methoxypyridin-3-yl]-1,6-naphthyridin-2(1H)-one methanesulfonate,
3-(6-chloro-4-ethyl-1H-imidazo[4,5-c]pyridin-2-yl)-5-[6-(1,1-difluoroethyl)-4-methoxypyridin-3-yl]-1,6-naphthyridin-2(1H)-one methanesulfonate,
3-(6-chloro-4-ethyl-1H-imidazo[4,5-c]pyridin-2-yl)-5-[1-(difluoromethyl)-3,5-dimethyl-1H-pyrazol-4-yl]-1,6-naphthyridin-2(1H)-one methanesulfonate,
3-{6-chloro-4-[(2R)-2-methylazetidin-1-yl]-1H-imidazo[4,5-c]pyridin-2-yl}-5-[1-(difluoromethyl)-3,5-dimethyl-1H-pyrazol-4-yl]-1,6-naphthyridin-2(1H)-one methanesulfonate,
3-(6-chloro-4-ethyl-1H-imidazo[4,5-c]pyridin-2-yl)-5-[1-(difluoromethyl)-1H-pyrazol-3-yl]-1,6-naphthyridin-2(1H)-one methanesulfonate,
3-(6-chloro-4-ethyl-1H-imidazo[4,5-c]pyridin-2-yl)-5-(1,5-dimethyl-1H-pyrazol-3-yl)-1,6-naphthyridin-2(1H)-one methanesulfonate,
3-(6-chloro-4-{[(2R)-1,1,1-trifluoropropan-2-yl]amino}-1H-imidazo[4,5-c]pyridin-2-yl)-5-(1,5-dimethyl-1H-pyrazol-4-yl)-1,6-naphthyridin-2(1H)-one methanesulfonate,
3-[6-chloro-4-(tetrahydro-2H-pyran-4-yl)-1H-imidazo[4,5-c]pyridin-2-yl]-5-[3-cyclopropyl-1-(difluoromethyl)-5-methyl-1H-pyrazol-4-yl]-1,6-naphthyridin-2(1H)-one methanesulfonate,
3-(6-chloro-4-ethyl-1H-imidazo[4,5-c]pyridin-2-yl)-5-(6-cyclopropyl-4-methoxypyridin-3-yl)-3,4-dihydropyrido[4,3-d]pyrimidin-2(1H)-one methanesulfonate,
3-(6-chloro-4-ethyl-1H-imidazo[4,5-c]pyridin-2-yl)-5-(6-cyclopropyl-4-methoxypyridin-3-yl)-1,6-naphthyridin-2(1H)-one 1,2-ethanedisulfonate,
3-(6-chloro-4-ethyl-1H-imidazo[4,5-c]pyridin-2-yl)-5-(6-cyclopropyl-4-methoxypyridin-3-yl)-1,6-naphthyridin-2(1H)-one methanesulfonate,
3-(6-chloro-4-ethyl-1H-imidazo[4,5-c]pyridin-2-yl)-5-(6-cyclopropyl-4-methoxypyridin-3-yl)-1,6-naphthyridin-2(1H)-one benzenesulfonate, or
3-(6-chloro-4-ethyl-1H-imidazo[4,5-c]pyridin-2-yl)-5-(6-cyclopropyl-4-methoxypyridin-3-yl)-1,6-naphthyridin-2(1H)-one adipate.

More preferably, they are
3-(6-chloro-4-ethyl-1H-imidazo[4,5-c]pyridin-2-yl)-5-(6-cyclopropyl-4-methoxypyridin-3-yl)-1,6-naphthyridin-2(1H)-one 1,2-ethanedisulfonate,
3-(6-chloro-4-ethyl-1H-imidazo[4,5-c]pyridin-2-yl)-5-(6-cyclopropyl-4-methoxypyridin-3-yl)-1,6-naphthyridin-2(1H)-one methanesulfonate,
3-(6-chloro-4-ethyl-1H-imidazo[4,5-c]pyridin-2-yl)-5-(6-cyclopropyl-4-methoxypyridin-3-yl)-1,6-naphthyridin-2(1H)-one benzenesulfonate, or
3-(6-chloro-4-ethyl-1H-imidazo[4,5-c]pyridin-2-yl)-5-(6-cyclopropyl-4-methoxypyridin-3-yl)-1,6-naphthyridin-2(1H)-one adipate.

In another preferred aspect of the present invention, the compound represented by formula (1), or a pharmaceutically acceptable salt thereof, is a crystal.

In the present invention, a crystal refers to a solid in which atoms, atomic groups, molecules, or ions are spatially and regularly arranged in the internal structure, and is distinguished from an amorphous solid or an amorphous substance that does not have such a regular internal structure.

In the present invention, the fact that the compound represented by formula (1), or a pharmaceutically acceptable salt thereof, is in a crystal form can be confirmed by observation with a polarization microscope, powder X-ray crystallography, or single-crystal X-ray diffraction measurement. Furthermore, the type of crystal can be identified by comparing it with data based on indicators that have been measured in advance for the characteristics of the crystal. Therefore, according to a preferred embodiment of the present invention, the crystal according to the present invention can be confirmed to be a crystal by using such measuring means. In the present invention, the difference in crystal form is particularly distinguished by powder X-ray diffraction.

In the present invention, not only crystals with perfectly matching diffraction angles in powder X-ray diffraction but also crystals with matching diffraction angles within a range of ±0.2 are included in the present invention. This is because there are inherent variations in peak values from measurement to measurement (from sample preparation to measurement) even for the same crystal, generally due to the effects of particle size (particle diameter) non-uniformity and preferred orientation, which is a common practice (see, for example, the Japanese Pharmacopoeia 18th Edition, "2.58 X-ray Powder Diffraction Measurement Method" and "5. Qualitative Phase Analysis (Identification of Phases)"). This is because the diffraction angle (2θ) in powder X-ray diffraction may have an error within a range of ±0.2, so the above diffraction angle value should be understood as including a numerical value within a range of about ±0.2.

The crystal of the compound of the present invention or a pharmaceutically acceptable salt thereof is preferably:
a crystal of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one 1,2-ethanedisulfonate, having at least five peaks at diffraction angles (2θ) selected from 6.33 ± 0.2, 8.40 ± 0.2, 12.68 ± 0.2, 16.87 ± 0.2, 18.36 ± 0.2, 20.73 ± 0.2, 22.39 ± 0.2, 24.33 ± 0.2, and 25.55 ± 0.2 in powder X-ray diffraction using CuKα radiation;
a crystal of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one methanesulfonate, having at least five peaks at diffraction angles (2θ) selected from 4.65 ± 0.2, 9.33 ± 0.2, 10.13 ± 0.2, 12.73 ± 0.2, 18.76 ± 0.2, 19.51 ± 0.2, 21.73 ± 0.2, 23.92 ± 0.2, 26.02 ± 0.2, and 27.11 ± 0.2 in powder X-ray diffraction using CuKα radiation;
a crystal of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one benzenesulfonate, having at least five peaks at diffraction angles (2θ) selected from 5.75 ± 0.2, 7.08 ± 0.2, 11.55 ± 0.2, 12.76 ± 0.2, 13.34 ± 0.2, 16.04 ± 0.2, 17.18 ± 0.2, 22.91 ± 0.2, 25.41 ± 0.2, and 25.73 ± 0.2 in powder X-ray diffraction using CuKα radiation; or
a crystal of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one adipate, having at least five peaks at diffraction angles (2θ) selected from 6.76 ± 0.2, 8.11 ± 0.2, 12.23 ± 0.2, 13.54 ± 0.2, 15.88 ± 0.2, 16.75 ± 0.2, 18.88 ± 0.2, 19.71 ± 0.2, 21.35 ± 0.2, and 25.76 ± 0.2 in powder X-ray diffraction using CuKα radiation.

More preferably, it is
a crystal of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one 1,2-ethanedisulfonate, having peaks at diffraction angles (2θ) of 6.33 ± 0.2, 12.68 ± 0.2, 16.87 ± 0.2, 18.36 ± 0.2, and 20.73 ± 0.2 in powder X-ray diffraction using CuKα radiation;
a crystal of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one methanesulfonate, having peaks at diffraction angles (2θ) of 4.65 ± 0.2, 9.33 ± 0.2, 12.73 ± 0.2, 18.76 ± 0.2, and 27.11 ± 0.2 in powder X-ray diffraction using CuKα radiation;
a crystal of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one benzenesulfonate, having peaks at diffraction angles (2θ) of 5.75 ± 0.2, 7.08 ± 0.2, 11.55 ± 0.2, 13.34 ± 0.2, 25.41 ± 0.2, and 25.73 ± 0.2 in powder X-ray diffraction using CuKα radiation; or
a crystal of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one adipate, having peaks at diffraction angles (2θ) of 6.76 ± 0.2, 8.11 ± 0.2, 13.54 ± 0.2, 21.35 ± 0.2, and 25.76 ± 0.2 in powder X-ray diffraction using CuKα radiation.

The compound of the present invention or a pharmaceutically acceptable salt thereof exhibits SMG1 inhibitory activity, NMD inhibitory activity, an anticancer effect as a single agent, and an anticancer effect in combination with an immune checkpoint inhibitor, and is useful as a pharmaceutical.

One aspect of the present invention relates to a pharmaceutical composition for treating cancer, comprising the compound of the present invention or a pharmaceutically acceptable salt thereof as an active ingredient, and optionally containing a pharmaceutically acceptable carrier.

Another aspect of the present invention relates to a method for treating cancer, comprising administering an effective amount of the compound of the present invention or a pharmaceutically acceptable salt thereof.

In the present invention, the type of cancer to be treated is not particularly limited as long as it is a cancer for which sensitivity to the compound of the present invention is confirmed. In this specification, "cancer" is synonymous with malignant tumor and is broadly classified into solid cancer, which consists of epithelial cell cancer and sarcoma, and hematological cancer, and also includes rare cancers such as mesothelioma.

"Hematological cancer" is a cancer of blood cells; examples include leukemia, lymphoma, and multiple myeloma.

"Solid cancer" is a general term for epithelial cell cancer and sarcoma. Of these, "epithelial cell cancer" is a cancer that develops from epithelial cells; examples include gastric cancer, lung cancer, colorectal cancer, esophageal cancer, head and neck cancer, breast cancer, liver cancer, kidney cancer, uterine cancer, ovarian cancer, prostate cancer, cervical cancer, pancreatic cancer, skin cancer, adrenal cancer, biliary tract cancer, thymic cancer, bladder cancer, and brain tumor.

"Sarcoma" is a malignant tumor that develops in connective tissues such as bones and soft tissues (fat, muscle, nerves, etc.); examples include osteosarcoma, chordoma, chondrosarcoma, and Ewing sarcoma.

Preferably, the cancer is hematological cancer selected from the group consisting of leukemia, lymphoma, and multiple myeloma; sarcoma selected from the group consisting of osteosarcoma, chordoma, chondrosarcoma, and Ewing sarcoma; or solid cancer selected from the group consisting of gastric cancer, lung cancer, colorectal cancer, esophageal cancer, head and neck cancer, breast cancer, liver cancer, kidney cancer, tongue cancer, thyroid cancer, uterine cancer, ovarian cancer, prostate cancer, cervical cancer, pancreatic cancer, skin cancer, adrenal cancer, biliary tract cancer, thymic cancer, mesothelioma, bladder cancer, and brain tumor.

As used herein, "treat" and its derivatives mean the remission, alleviation, and/or delay of worsening of the clinical condition of cancer in a patient with cancer.

Another aspect of the present invention relates to a pharmaceutical composition for treating cancer, wherein the compound of the present invention or a pharmaceutically acceptable salt thereof is administered in combination with an immune checkpoint inhibitor.

Another aspect of the present invention relates to a method for treating cancer, wherein the compound of the present invention or a pharmaceutically acceptable salt thereof is administered in combination with an immune checkpoint inhibitor.

The compound of the present invention or a pharmaceutically acceptable salt thereof, and the immune checkpoint inhibitor may be each contained as an active ingredient in different pharmaceutical formulations, or may be contained in a single pharmaceutical formulation. When they are contained as active ingredients in different pharmaceutical formulations, they may be administered simultaneously or at different times.

The "immune checkpoint inhibitor" in the present specification is an agent that binds to an immune checkpoint molecule or its ligand and inhibits the transmission of immunosuppressive signals to release the suppression of T cell activation.

Examples of immune checkpoint inhibitors include anti-PD-1 antibodies, anti-PD-L1 antibodies, or anti-CTLA-4 antibodies. Examples of anti-PD-1 antibodies include, but are not limited to, nivolumab and pembrolizumab; examples of anti-PD-L1 antibodies include atezolizumab, avelumab, and durvalumab; and examples of anti-CTLA-4 antibodies include ipilimumab.

Another aspect of the present invention is an SMG1 inhibitor comprising the compound of the present invention or a pharmaceutically acceptable salt thereof as an active ingredient.

The "SMG1 inhibitor" in the present specification is an agent that has an inhibitory activity against SMG1 kinase activity and exerts a pharmacological effect. Since the kinase activity of SMG1 is essential for the nonsense-mediated mRNA decay (NMD) mechanism, an SMG1 inhibitor is an agent that has an NMD inhibitory activity.

Another aspect of the present invention is an NMD inhibitor comprising the compound of the present invention or a pharmaceutically acceptable salt thereof as an active ingredient.

The "NMD inhibitor" in the present specification is an agent that inhibits the nonsense-mediated mRNA decay (NMD) mechanism and exerts a pharmacological effect.

Nonsense-mediated mRNA decay (NMD) is one of the quality control mechanisms of mRNA. Specifically, it is a mechanism in which premature termination codons on mRNA generated by nonsense mutations or frameshift mutations in the genome, or by abnormalities in RNA splicing, are recognized by NMD and led to degradation. Some long non-coding RNAs are also subject to NMD.

A premature termination codon is a termination codon that appears upstream of the original termination codon.

### (Production Method)

A typical method for producing the compound represented by formula (1) or a pharmaceutically acceptable salt thereof will be described below. The compound of the present invention can be produced by various production methods, and the production methods shown below are merely examples, and the present invention should not be construed as being limited thereto.

The compound represented by formula (1) or a pharmaceutically acceptable salt thereof can be produced by applying various known production methods, utilizing the characteristics based on the basic skeleton or the type of substituent. Known methods include, for example, those described in "ORGANIC FUNCTIONAL GROUP PREPARATIONS", 2nd edition, ACADEMIC PRESS, INC., 1989, and "Comprehensive Organic Transformations", VCH Publishers Inc., 1989.

In this case, depending on the type of functional group present in the compound, it may be effective in production technology to protect the functional group with a suitable protecting group at the stage of raw material or intermediate, or to replace it with a group that can be easily converted to the functional group.

Examples of such functional groups include amino groups, hydroxyl groups, and carboxyl groups, and examples of protecting groups for them include those described in Greene's Protective Groups in Organic Synthesis (4th edition, John Wiley & Sons, Inc., 2006) by T. W. Greene and P. G. Wuts.

The protecting group or the group that can be easily converted to the functional group may be appropriately selected and used depending on the reaction conditions of each production method for producing the compound.

According to such methods, after introducing the group and carrying out the reaction, the desired compound can be obtained by removing the protecting group or converting it to a desired group as needed.

The compound represented by formula (1) can be produced, for example, by the following [Method A1], [Method A2], [Method B1], [Method B2], [Method B3], [Method B4], [Method C1], [Method C2], [Method C3], [Method D1], [Method D2], [Method D3], and [Method E]. This production method allows obtaining the compound from commercially available compounds or through the production methods shown in the Reference Examples described later.

### [Method A1]

Method A1 is a method for producing compound 1a, which is a compound represented by formula (1). 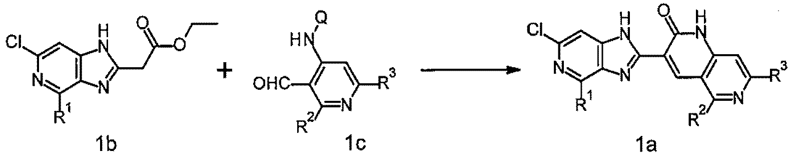 [wherein R¹, R², and R³ have the same meanings as defined above. Q represents a hydrogen atom or an alkoxycarbonyl group].

### (Step A1-1) Step of constructing a 1,6-naphthyridin-2(1H)-one structure

This step involves reacting compound 1b and 1c using a base in a solvent to obtain compound 1a.

Examples of the base include piperidine, pyrrolidine, triethylamine, N,N-diisopropylethylamine, and pyridine.

Examples of the solvent include ethanol, n-butanol, 2-propanol, methanol, dimethyl sulfoxide, N,N-dimethylformamide, acetonitrile, and tetrahydrofuran.

The reaction temperature is usually 50°C to 120°C, and the reaction time is usually about 1 hour to 24 hours.

### [Method A2]

When the structure of compound 1a is represented by 1a-1, Method A2 is a method for producing compound 1a-1. 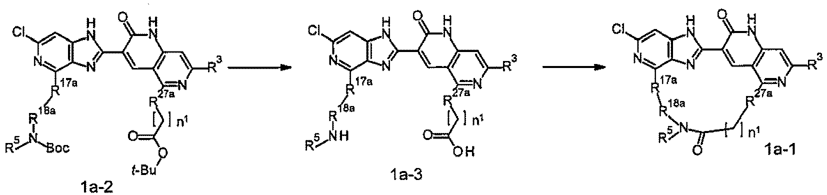 [wherein R^{17a} represents NR^{19a} or CHR^{19a}, R^{18a} represents a C1-C6 alkyl group optionally substituted with a C1-C6 alkyl group or a C3-C8 cycloalkyl group, R^{27a} represents a phenyl group, a heteroaryl group, or a 4- to 7-membered heterocyclic group, n¹ represents 0, 1, or 2, R⁵ represents a hydrogen atom, a C1-C6 alkyl group optionally substituted with a halogen atom, or a C3-C8 cycloalkyl group, R^{19a} represents a hydrogen atom or a C1-C6 alkyl group. R^{19a} and R⁵ may be bonded to each other to form a ring structure. Boc represents a tert-butoxycarbonyl group, and t-Bu represents a tert-butyl group].

### (Step A2-1) Step of performing deprotection

This step involves reacting compound 1a-2 using an acid in a solvent to obtain compound 1a-3.

Examples of the acid include trifluoroacetic acid, hydrochloric acid, sulfuric acid, and hydrogen chloride.

Examples of the solvent include dichloromethane, 1,4-dioxane, toluene, and ethyl acetate.

The reaction temperature is usually about room temperature to 100°C, and the reaction time is usually about 30 minutes to 24 hours.

### (Step A2-2) Step of constructing a macrocycle

This step involves reacting compound 1a-3 using a base and a condensing agent in a solvent to obtain compound 1a-1.

Examples of the base include N,N-diisopropylethylamine, triethylamine, N-methylmorpholine, and pyridine.

Examples of the condensing agent include O-(7-azabenzotriazol-1-yl)-N,N,N',N'-tetramethyluronium hexafluorophosphate, 1-(3-dimethylaminopropyl)-3-ethylcarbodiimide hydrochloride, and N,N'-dicyclohexylcarbodiimide.

Examples of the solvent include N,N-dimethylformamide, N-methylpyrrolidone, dichloromethane, tetrahydrofuran, and acetonitrile.

The reaction temperature is usually about room temperature to 80°C, and the reaction time is usually about 1 hour to 3 days.

### [Method B1]

Method B1 is a method for producing compound 1b. 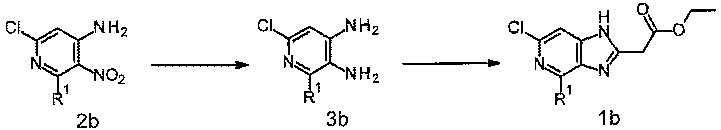 [wherein R¹ has the same meaning as defined above].

### (Step B 1-1) Step of reducing a nitro group

This step involves reacting compound 2b using an acid and a reducing metal in a solvent to obtain compound 3b.

Examples of the acid include ammonium chloride, hydrochloric acid, and acetic acid.

Examples of the metal include iron, tin, and zinc.

Examples of the solvent include ethanol, methanol, 2-propanol, tetrahydrofuran, 1,4-dioxane, water, or a mixture thereof.

The reaction temperature is usually about 50°C to 120°C, and the reaction time is usually about 1 hour to 2 days.

### (Step B1-2) Step of constructing an imidazo[4,5-c]pyridine structure

This step involves reacting compound 3b using ethyl 3-ethoxy-3-iminopropanoate hydrochloride in a solvent to obtain compound 1b.

Examples of the solvent include ethanol, acetic acid, dimethyl sulfoxide, N,N-dimethylformamide, acetonitrile, tetrahydrofuran, 1,4-dioxane, or a mixture thereof.

The reaction temperature is usually about room temperature to 120°C, and the reaction time is usually about 1 hour to 2 days.

### [Method B2]

When the structure of compound 2b is represented by 2b-1, Method B2 is a method for producing compound 2b-1. 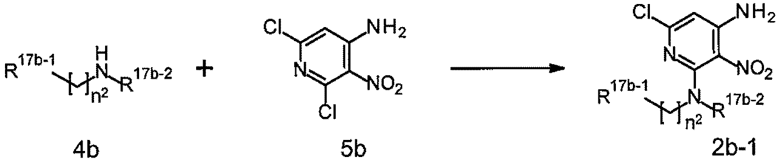 [wherein R^{17b-1} represents a C1-C6 alkyl group optionally substituted with a halogen atom, a hydroxyl group, an amino group, an alkoxycarbonyl group, an aminocarbonyl group, a C3-C8 cycloalkyl group, a phenyl group, a heteroaryl group, or a 4- to 7-membered heterocyclic group, n² represents 0, 1, or 2, and R^{17b-2} represents a hydrogen atom or a C1-C6 alkyl group. R^{17b-1} and R^{17b-2} may be bonded to each other to form a ring structure].

### (Step B2-1) Step of performing aromatic nucleophilic substitution

This step involves reacting compound 4b using compound 5b and a base in a solvent to obtain compound 2b-1.

Examples of the base include calcium carbonate, cesium carbonate, sodium carbonate, N,N-diisopropylethylamine, triethylamine, N-methylmorpholine, sodium hydride, and 1,8-diazabicyclo[5,4,0]-7-undecene.

Examples of the solvent include N,N-dimethylformamide, dimethyl sulfoxide, acetonitrile, 2-propanol, ethanol, and methanol.

The reaction temperature is usually about room temperature to 120°C, and the reaction time is usually about 1 hour to 6 days.

### [Method B3]

When the structure of compound 2b is represented by 2b-2, Method B3 is a method for producing compound 2b-2. 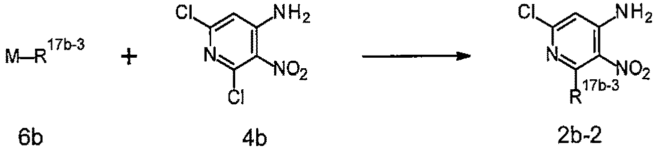 [wherein R^{17b-3} represents a C1-C6 alkyl group optionally halogenated, a C3-C8 cycloalkyl group, a phenyl group, or a heteroaryl group, and M represents an organoboron such as dialkoxyboron, an organozinc such as monoalkyl zinc, a zinc halide, an organotin such as trialkyltin, a magnesium halide, an organoaluminum such as dialkylaluminum, or an organozirconium such as monoalkyl zirconium].

### (Step B3-1) Step of performing cross-coupling

This step involves reacting compound 6b using compound 4b and a palladium catalyst, and optionally a base, in a solvent to obtain compound 2b-2.

Examples of the palladium catalyst include [1,1'-bis(diphenylphosphino)ferrocene]dichloropalladium(II) dichloromethane adduct, tetrakis(triphenylphosphine)palladium(0), and bis(triphenylphosphine)palladium(II) dichloride.

Examples of the base include sodium carbonate, potassium carbonate, cesium carbonate, tripotassium phosphate, and triethylamine.

Examples of the solvent include 1,4-dioxane, 2,2-dimethoxyethane, tetrahydrofuran, toluene, N,N-dimethylformamide, water, or a mixture thereof.

The reaction temperature is usually about room temperature to 120°C, and the reaction time is usually about 1 hour to 2 days.

### [Method B4]

When the structure of compound 3b is represented by 3b-1, Method B4 is a method for producing compound 3b-1. 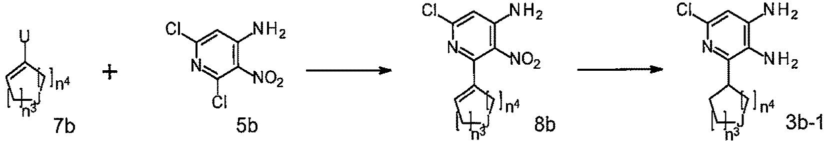 [wherein U represents a dihydroxyboryl group or a (pinacolato)boryl group, J represents an oxygen atom or NR^{17b-4}, n³ and n⁴ each independently represent the same or different 1, 2, or 3, and R^{17b-4} represents a hydrogen atom, a C1-C6 alkyl group, a C3-C8 cycloalkyl group, or a tert-butoxycarbonyl group].

### (Step B4-1) Step of performing cross-coupling

This step involves reacting compound 7b using compound 5b, a palladium catalyst, and a base in a solvent to obtain compound 8b.

The same palladium catalyst and base as in Step B3-1 can be used, and the reaction can be carried out at the same reaction temperature and reaction time.

### (Step B4-2) Step of reducing a double bond and a nitro group

This step involves reacting compound 8b using hydrogen and a platinum catalyst to obtain compound 3b-1.

Examples of the platinum catalyst include platinum(IV) oxide and platinum sulfided on carbon.

Examples of the solvent include ethanol, tetrahydrofuran, ethyl acetate, methanol, or a mixture thereof.

The reaction temperature is usually about room temperature to 80°C, and the reaction time is usually about 1 hour to 2 days.

Compounds 6b and 7b are known or are produced according to known methods or similar methods using known compounds as starting materials. Known compounds can be purchased from commercial suppliers, or can be readily synthesized by methods described in the literature or similar methods. Known literature includes, for example, J. Am. Chem. Soc., 132, 50, 17701-17703 (2010), Angew. Chem. Int. Ed., 50, 8230-8232 (2011), WO2010018113, US20100298575, Chem. Commun., (18), 2143-2145 (2008), Adv. Synth. & Catal., 359, 2741-2746 (2017), Heterocycles, 91, 1654-1659 (2015), WO2008156726 A1, WO2009048527 A1, US20090118284 A1, WO2009058801 A1, US20090197864 A1, Eur. J. Org. Chem., 2021, 6551-6560 (2021), Angew. Chem. Int. Ed., 47, 4851-4854 (2008), J. Am. Chem. Soc., 139, 7741-7744 (2017), Eur. J. Org. Chem., 2016, 83-86 (2016), and J. Org. Chem., 65, 5653-5658 (2000).

### [Method C1]

When the structure of compound 1c is represented by 1c-1, Method C1 is a method for producing compound 1c-1. 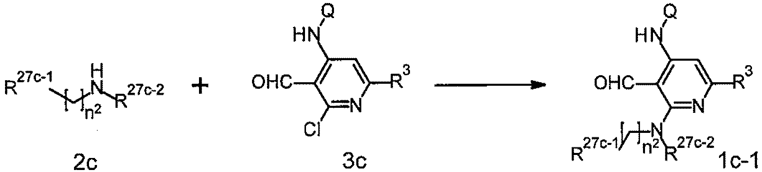 [wherein R³, Q, and n² have the same meanings as defined above. R^{27c-1} represents a C1-C6 alkyl group optionally substituted with a halogen atom, a hydroxyl group, an amino group, a halogeno group, an alkoxycarbonyl group, an aminocarbonyl group, a C3-8 cycloalkyl group, a phenyl group, a heteroaryl group, or a 4- to 7-membered heterocyclic group, and R^{27c-2} represents a hydrogen atom or a C1-C6 alkyl group. R^{27c-1} and R^{27c-2} may be bonded to each other to form a ring structure].

### (Step C1-1) Step of performing aromatic nucleophilic substitution

This step involves reacting compound 2c using compound 3c and a base in a solvent to obtain compound 1c-1.

Examples of the base include calcium carbonate, cesium carbonate, sodium carbonate, N,N-diisopropylethylamine, triethylamine, N-methylmorpholine, sodium hydride, and 1,8-diazabicyclo[5,4,0]-7-undecene.

Examples of the solvent include N,N-dimethylformamide, dimethyl sulfoxide, acetonitrile, 2-propanol, ethanol, and methanol.

The reaction temperature is usually about room temperature to 120°C, and the reaction time is usually about 1 hour to 6 days.

### [Method C2]

When the structure of compound 1c is represented by 1c-2, Method C2 is a method for producing compound 1c-2. 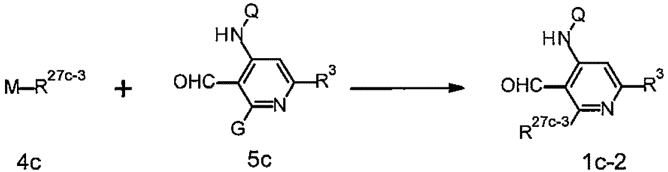 [wherein R³, M, and Q have the same meanings as defined above. R^{27c-3} represents a phenyl group, a heteroaryl group, a C1-C6 alkyl group, or a C3-C8 cycloalkyl group, and G represents a chloro group, a bromo group, or an iodo group].

### (Step C2-1) Step of performing cross-coupling

This step involves reacting compound 4c using compound 5c and a palladium catalyst, and optionally a base, in a solvent to obtain compound 1c-2.

Examples of the palladium catalyst include [1,1'-bis(diphenylphosphino)ferrocene]dichloropalladium(II) dichloromethane adduct, tetrakis(triphenylphosphine)palladium(0), and bis(triphenylphosphine)palladium(II) dichloride.

Examples of the base include sodium carbonate, potassium carbonate, cesium carbonate, tripotassium phosphate, and triethylamine.

Examples of the solvent include 1,4-dioxane, 2,2-dimethoxyethane, tetrahydrofuran, toluene, N,N-dimethylformamide, water, or a mixture thereof.

The reaction temperature is usually about room temperature to 120°C, and the reaction time is usually about 1 hour to 2 days.

### [Method C3]

When the structure of compound 1c is represented by 1c-3, Method C3 is a method for producing compound 1c-3. 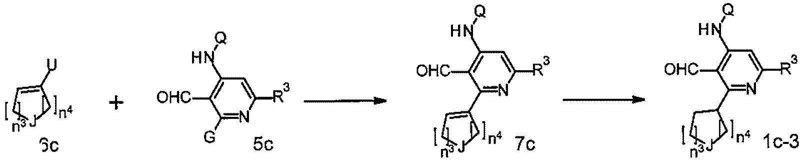 [wherein R³, G, U, J, Q, n³, and n⁴ have the same meanings as defined above].

### (Step C3-1) Step of performing cross-coupling

This step involves reacting compound 6c using compound 5c, a palladium catalyst, and a base in a solvent to obtain compound 7c.

The same palladium catalyst and base as in Step C2-1 can be used, and the reaction can be carried out at the same reaction temperature and reaction time.

### (Step C3-2) Step of reducing a double bond

This step involves reacting compound 7c using hydrogen and a transition metal catalyst in a solvent to obtain compound 1c-3.

Examples of the transition metal catalyst include palladium carbon, platinum(IV) oxide, platinum sulfided on carbon, and tris(triphenylphosphine)rhodium(I) chloride.

Examples of the solvent include ethyl acetate, ethanol, tetrahydrofuran, methanol, toluene, or a mixture thereof.

The reaction temperature is usually about room temperature to 80°C, and the reaction time is usually about 1 hour to 2 days.

Compound 4c and compound 6c are known or are produced according to known methods or similar methods using known compounds as starting materials. Known compounds can be purchased from commercial suppliers, or can be readily synthesized by methods described in the literature or similar methods. Known literature includes the same literature as that given in the production methods of compound 6b and compound 7b.

### [Method D1]

Method D1 is a method for producing compound 1e, which is a compound represented by formula (1). 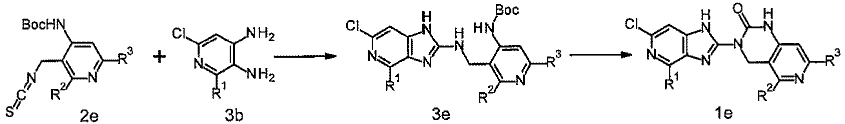 [wherein R¹, R², R³, and Boc have the same meanings as defined above].

### (Step D1-1) Step of constructing a 2-aminoimidazo[4,5-c]pyridine structure

This step involves reacting compound 2e using compound 3b, a base, and a diimide compound in a solvent to obtain compound 3e.

Examples of the base include imidazole, pyridine, and 4-dimethylaminopyridine.

Examples of the diimide compound include N,N'-diisopropylcarbodiimide, N,N'-dicyclohexylcarbodiimide, and 1-(3-dimethylaminopropyl)-3-ethylcarbodiimide hydrochloride.

Examples of the solvent include N,N-dimethylformamide, dimethyl sulfoxide, and acetonitrile.

The reaction temperature is usually about room temperature to 120°C, and the reaction time is usually about 1 hour to 24 hours.

### (Step D1-2) Step of constructing a 3,4-dihydropyrido[4,3-d]pyrimidin structure

This step involves reacting compound 3e by removing the Boc group using an acid and then reacting using a base and 1,1'-carbonyldiimidazole in a solvent, or by reacting using a base in a solvent to obtain compound 1e.

Examples of the acid include hydrogen chloride and trifluoroacetic acid.

Examples of the base include triethylamine, sodium hydride, N,N-diisopropylethylamine, and N-methylmorpholine.

Examples of the solvent include dichloromethane, 1,4-dioxane, tetrahydrofuran, and ethyl acetate.

The reaction temperature is usually about room temperature to 110°C, and the reaction time is usually about 1 hour to 24 hours.

### [Method D2]

Method D2 is a method for producing compound 2e. 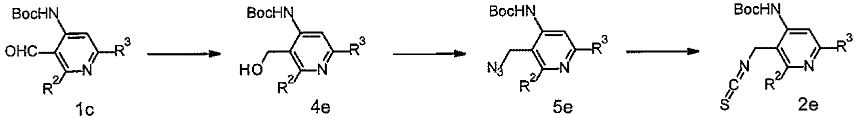 [wherein R², R³, and Boc have the same meanings as defined above].

### (Step D2-1) Step of reducing a formyl group

This step involves reacting compound 1c using a reducing agent in a solvent to obtain compound 4e.

Examples of the reducing agent include sodium borohydride.

Examples of the solvent include methanol, ethanol, tetrahydrofuran, water, or a mixture thereof.

The reaction temperature is usually about room temperature to 80°C, and the reaction time is usually about 1 hour to 10 hours.

### (Step D2-2) Step of azidation

This step involves reacting compound 4e using an azidating reagent, and optionally a base, in a solvent to obtain compound 5e.

Examples of the azidating reagent include diphenylphosphoryl azide alone, a combination of carbon tetrachloride and sodium azide, a combination of diethyl azodicarboxylate, triphenylphosphine, and hydrazoic acid, and a combination of triphenylphosphine, iodine, and sodium azide.

Examples of the base include 1,8-diazabicyclo[5,4,0]-7-undecene, imidazole, and triethylamine.

Examples of the solvent include tetrahydrofuran, N,N-dimethylformamide, and dimethyl sulfoxide.

The reaction temperature is usually about room temperature to 120°C, and the reaction time is usually about 1 hour to 24 hours.

### (Step D2-3) Step of isothiocyanation

This step involves reacting compound 5e using triphenylphosphine and carbon disulfide in a solvent to obtain compound 2e.

Examples of the solvent include acetonitrile, tetrahydrofuran, dichloromethane, chloroform, and toluene.

The reaction temperature is usually about room temperature to 110°C, and the reaction time is usually about 1 hour to 2 days.

### [Method D3]

Method D3 is a method for producing compound 3e, instead of Method E1. 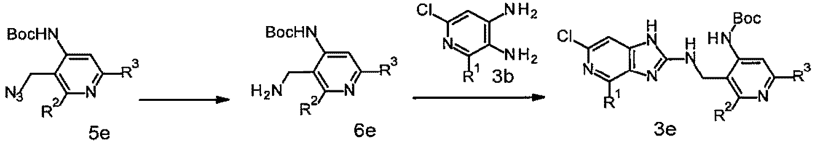 [wherein R¹, R², R³, and Boc have the same meanings as defined above].

### (Step D3-1) Step of reducing an azide

This step involves reacting compound 5e using triphenylphosphine in a solvent to obtain compound 6e.

Examples of the solvent include a mixture of tetrahydrofuran and water, and methanol.

The reaction temperature is usually about room temperature to 60°C, and the reaction time is usually about 1 hour to 2 days.

### (Step D3-2) Step of constructing a 2-aminoimidazo[4,5-c]pyridine structure

This step involves reacting compound 6e using compound 3b, 1,1'-thiocarbonyldiimidazole, and a diimide compound in a solvent to obtain compound 3e.

Examples of the diimide compound include N,N'-diisopropylcarbodiimide, N,N'-dicyclohexylcarbodiimide, and 1-(3-dimethylaminopropyl)-3-ethylcarbodiimide hydrochloride.

Examples of the solvent include acetonitrile, N,N-dimethylformamide, dimethyl sulfoxide, and tetrahydrofuran.

The reaction temperature is usually about room temperature to 120°C, and the reaction time is usually about 1 hour to 24 hours.

### [Method E]

Method E is a method for producing a methanesulfonic acid salt of a compound represented by formula (1). 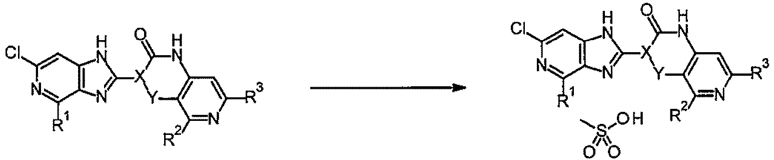 [wherein R¹, R², R³, X, and Y have the same meanings as defined above].

### (Step E-1) Step of forming methanesulfonate

This step involves reacting a compound represented by formula (1) using methanesulfonic acid to obtain a salt thereof.

Examples of the solvent include ethanol, acetonitrile, dichloromethane, and ethyl acetate.

The reaction temperature is usually about room temperature to 90°C, and the reaction time is usually about 1 hour to 24 hours.

The compounds produced by the above methods can be isolated and purified by known methods, for example, extraction, precipitation, distillation, chromatography, fractional recrystallization, and recrystallization.

When a compound or an intermediate in production has an asymmetric carbon, optical isomers exist. Each of these optical isomers can be isolated and purified by conventional methods such as fractional recrystallization (salt resolution) by recrystallization with an appropriate salt, and column chromatography. Reference literature for methods of resolving optical isomers from racemates includes *"*Enantiomers, Racemates and Resolution, John Wiley And Sons, Inc." by J. Jacques et al.

The SMG1 kinase inhibitory activity of a compound of the present invention or a pharmaceutically acceptable salt thereof can be measured, for example, by the method described below. Using purified human SMG1 protein and a substrate peptide as materials, the kinase activity can be measured by the ADP-Glo Kinase Assay; the SMG1 kinase inhibitory activity of a test substance can be evaluated by comparing the kinase activity in the presence and absence of the test substance.

The NMD inhibitory activity of a compound of the present invention or a pharmaceutically acceptable salt thereof in cells can be measured, for example, by the method described below. EMT6 murine breast cancer cell line is treated with a test substance *in vitro* for 6 hours, and the increase in the transcript of *Snhg1,* a known NMD target gene, is quantified as an indicator of NMD inhibition. Quantification is performed by real-time PCR.

The NMD inhibitory activity of a compound of the present invention or a pharmaceutically acceptable salt thereof *in vivo* can be measured, for example, by the method described below. Using mice subcutaneously implanted with EMT6 murine breast cancer cell line, tumor tissues 6 hours after oral administration of a test substance are excised, and the increase in *Snhg1* transcript is quantified as an indicator of NMD inhibition in the tumor, in the same manner as in the *in vitro* method.

The antitumor effect of a compound of the present invention or a pharmaceutically acceptable salt thereof in an syngeneic model can be evaluated, for example, by the method described below. MC38 mouse colorectal cancer cell line is subcutaneously implanted in C57BL6/J mice, and the tumor volume is measured over time. The suppression of tumor growth in the groups administered with the test substance (single agent), the immune checkpoint inhibitor (single agent), and the test substance and the immune checkpoint inhibitor (combination) is quantified as an antitumor effect to evaluate the effect of the test substance alone and the effect in combination with the immune checkpoint inhibitor.

The compound of the present invention or a pharmaceutically acceptable salt thereof can be used in combination with various therapeutic or prophylactic agents for diseases for which it is considered to be effective, not limited to immune checkpoint inhibitors.

The compound of the present invention or a pharmaceutically acceptable salt thereof may be used in combination with other antitumor agents. Examples of other antitumor agents include alkylating agents, antimetabolites, antitumor antibiotics, antitumor plant components, BRMs (biological response modifiers), hormones, vitamins, antitumor antibodies, molecularly targeted drugs, and other antitumor agents.

More specifically, examples of the alkylating agent include alkylating agents such as nitrogen mustard, nitrogen mustard-N-oxide, and chlorambucil, aziridine alkylating agents such as carboquone and thiotepa, epoxide alkylating agents such as dibromomannitol and dibromodulcitol, nitrosourea alkylating agents such as carmustine, lomustine, semustine, nimustine hydrochloride, streptozocin, chlorozotocin, and ranimustine, and busulfan, improsulfan tosylate, or dacarbazine.

Examples of various antimetabolites include purine antimetabolites such as 6-mercaptopurine, 6-thioguanine, and thioinosine, pyrimidine antimetabolites such as fluorouracil, tegafur, tegafur-uracil, carmofur, doxifluridine, broxuridine, cytarabine, and enocitabine, and folic acid antimetabolites such as pemetrexed, methotrexate, and trimetrexate.

Examples of antitumor antibiotics include anthracycline antibiotic antitumor agents such as mitomycin C, bleomycin, peplomycin, daunorubicin, aclarubicin, doxorubicin, pirarubicin, THP-adriamycin, 4'-epidoxorubicin, and epirubicin, and chromomycin A3 and actinomycin D.

Examples of antitumor plant components include vinca alkaloids such as vindesine, vincristine, and vinblastine, taxanes such as paclitaxel and docetaxel, or epipodophyllotoxins such as etoposide and teniposide.

Examples of the BRM include tumor necrosis factor and indomethacin.

Examples of the hormone include hydrocortisone, dexamethasone, methylprednisolone, prednisolone, prasterone, betamethasone, triamcinolone, oxymetholone, nandrolone, metenolone, fosfestrol, ethinylestradiol, chlormadinone, and medroxyprogesterone.

Examples of the vitamin include vitamin C and vitamin A.

Examples of antitumor antibodies and molecularly targeted drugs include trastuzumab, rituximab, cetuximab, nimotuzumab, denosumab, bevacizumab, infliximab, imatinib, gefitinib, erlotinib, sunitinib, lapatinib, sorafenib, dasatinib, nilotinib, vemurafenib, and osimertinib.

Examples of other antitumor agents include cisplatin, carboplatin, oxaliplatin, tamoxifen, camptothecin, ifosfamide, cyclophosphamide, melphalan, L-asparaginase, acecraton, schizophyllan, Picibanil, procarbazine, pipobroman, neocarzinostatin, hydroxyurea, ubenimex, and Krestin.

The combination may be administered simultaneously, or separately and sequentially or at desired time intervals. Formulations for simultaneous administration may be a combined preparation or separately formulated.

The pharmaceutical composition of the present invention comprises the compound of the present invention or a pharmaceutically acceptable salt thereof, and may contain a pharmaceutically acceptable carrier, and can be administered as various injections such as intravenous injection, intramuscular injection, and subcutaneous injection, or by various methods such as oral administration and transdermal administration. A pharmaceutically acceptable carrier refers to a pharmaceutically acceptable material (e.g., excipients, diluents, additives, solvents, etc.) that is involved in transporting the compound of the present invention or the composition comprising the compound of the present invention from one organ or tissue to another organ or tissue.

A pharmaceutical formulation containing the compound of the present invention or a pharmaceutically acceptable salt thereof as an active ingredient is prepared using additives such as carriers and excipients used in ordinary formulations. The compound of the present invention may be administered in any form, such as orally in the form of tablets, pills, capsules, granules, powders, or solutions, or parenterally in the form of injections for intra-articular, intravenous, or intramuscular administration, suppositories, eye drops, ophthalmic ointments, transdermal solutions, ointments, transdermal patches, transmucosal solutions, transmucosal patches, or inhalants.

For oral administration, solid compositions such as tablets, powders, and granules are used. Such solid compositions comprise one or more active ingredients and at least one inactive excipient such as lactose, mannitol, glucose, hydroxypropyl cellulose, microcrystalline cellulose, starch, polyvinylpyrrolidone, and/or magnesium aluminometasilicate. The solid composition may also contain inactive additives such as lubricants such as magnesium stearate, disintegrants such as carboxymethyl starch sodium, stabilizers, and solubilizers according to conventional methods. Tablets or pills may be optionally coated with sugar coating or a film of gastric-soluble or enteric-soluble substances.

When used as tablets, the carriers include, for example, excipients such as lactose, sucrose, sodium chloride, glucose, urea, starch, calcium carbonate, kaolin, crystalline cellulose, and silicic acid; binders such as water, ethanol, propanol, simple syrup, glucose solution, starch solution, gelatin solution, carboxymethylcellulose, shellac, methylcellulose, potassium phosphate, and polyvinylpyrrolidone; disintegrants such as dried starch, sodium alginate, agar powder, laminarin powder, sodium bicarbonate, calcium carbonate, polyoxyethylene sorbitan fatty acid ester, sodium lauryl sulfate, monoglyceride stearate, starch, and lactose; disintegration inhibitors such as sucrose, stearin, cacao butter, and hydrogenated oil; absorption enhancers such as quaternary ammonium salts and sodium lauryl sulfate; humectants such as glycerin and starch; adsorbents such as starch, lactose, kaolin, bentonite, and colloidal silicic acid; and lubricants such as purified talc, stearates, boric acid powder, and polyethylene glycol. Tablets with ordinary coatings, such as sugar-coated tablets, gelatin-coated tablets, enteric-coated tablets, film-coated tablets, double tablets, or multilayer tablets, can also be used as needed.

When used as pills, the carriers include, for example, excipients such as glucose, lactose, cacao butter, starch, hydrogenated vegetable oil, kaolin, and talc; binders such as gum arabic powder, tragacanth powder, gelatin, and ethanol; and disintegrants such as laminarin and agar.

Liquid compositions for oral administration include pharmaceutically acceptable emulsions, solutions, suspensions, syrups, or elixirs. Inert diluents commonly used in such liquid compositions, such as purified water or ethanol, can be added to the composition. In addition to the inert diluent, the liquid composition may also contain adjuvants such as solubilizers and wetting agents, sweeteners, flavors, fragrances, and preservatives.

Injections for parenteral administration include sterile aqueous or non-aqueous solutions, suspensions, or emulsions. Aqueous solvents include, for example, distilled water for injection and physiological saline. Non-aqueous solvents include, for example, propylene glycol, polyethylene glycol, vegetable oils such as olive oil, alcohols such as ethanol, and Polysorbate 80. Such injectable compositions may further contain tonicity adjusting agents, preservatives, wetting agents, emulsifiers, dispersants, stabilizers, or solubilizers. These injectable compositions can be sterilized, for example, by filtration through a bacteria-retaining filter, by incorporating a bactericide, or by irradiation. These injectable compositions can also be used by producing a sterile solid composition and dissolving or suspending it in sterile water or a sterile solvent for injection before use.

When used as an injection, it can be used as a solution, emulsion, or suspension. These solutions, emulsions, or suspensions are preferably sterilized and isotonic with blood. The solvent used for producing these solutions, emulsions, or suspensions is not particularly limited as long as it can be used as a diluent for medical use; examples include water, ethanol, propylene glycol, ethoxylated isostearyl alcohol, polyoxylated isostearyl alcohol, and polyoxyethylene sorbitan fatty acid esters. In this case, the preparation may contain a sufficient amount of sodium chloride, glucose, or glycerin in the preparation to prepare an isotonic solution, and may also contain ordinary solubilizers, buffers, analgesics, and the like.

Furthermore, the above formulations may also contain colorants, preservatives, fragrances, flavors, sweeteners, and the like, as needed, and may further contain other pharmaceuticals.

The amount of the compound contained in the above formulations is not particularly limited and is appropriately selected within a wide range, but usually 0.5 to 70% by weight, preferably 1 to 30% by weight, of the total composition.

The dose and frequency of administration of the compound of the present invention are appropriately determined in consideration of the symptoms, age, etc. of the patient (warm-blooded animal, particularly human). Usually, in the case of oral administration, a suitable daily dose is about 0.001 to 100 mg/kg, preferably 0.1 to 30 mg/kg, more preferably 0.1 to 10 mg/kg body weight, which is administered once a day or in two or more divided doses. In the case of intravenous administration, a suitable daily dose is about 0.0001 to 10 mg/kg body weight, which is administered once a day or in multiple divided doses.

### Examples

The present invention will be specifically described below with reference to Reference Examples and Examples, but the present invention is not limited thereto, and these are not to be construed as limiting in any sense. In this specification, reagents, solvents, and starting materials not specifically described are readily available from commercial sources.

### Reference Example 1

### Production of ethyl {6-chloro-4-[(3R)-3-methylmorpholin-4-yl]-1H-imidazo[4,5-c]pyridin-2-yl}acetate

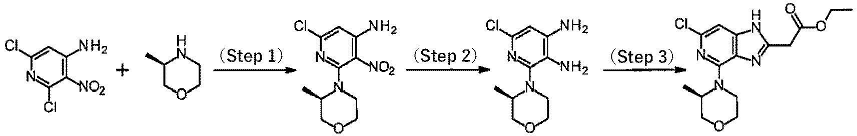

### (Step 1) 6-Chloro-2-[(3R)-3-methylmorpholin-4-yl]-3-nitropyridin-4-amine

To a solution of (R)-3-methylmorpholine (4.00 mL) and 2,6-dichloro-3-nitropyridin-4-amine (6.00 g) in N,N-dimethylformamide (60 mL) was added potassium carbonate (8.00 g) at room temperature, and the mixture was stirred at the same temperature for 23 hours. Water was added to the reaction mixture, and the mixture was extracted three times with ethyl acetate. The obtained organic layer was washed twice with water and once with saturated sodium chloride solution, and then dried over anhydrous sodium sulfate. The obtained organic layer was filtered, and the solvent was evaporated under reduced pressure. The residue was purified by silica gel column chromatography (n-hexane/ethyl acetate) to obtain the title compound (6.93 g).
¹H-NMR (CDCl₃) δ: 6.04 (1H, s), 6.01 (2H, br s), 4.59-4.51 (1H, m), 3.88-3.83 (1H, m), 3.79 (1H, dd, J = 11.6, 3.1 Hz), 3.72 (1H, d, J = 11.6 Hz), 3.61-3.51 (2H, m), 2.84-2.74 (1H, m), 1.37 (3H, d, J = 6.7 Hz).

### (Step 2) 6-Chloro-2-[(3R)-3-methylmorpholin-4-yl]pyridin-3,4-diamine

To a mixed solution of the compound (6.93 g) obtained in Step 1 above in ethanol (100 mL)/water (50 mL) were added iron powder (4.30 g) and ammonium chloride (1.36 g) at room temperature, and the mixture was stirred at 80°C for 2.5 hours. After cooling the reaction mixture to room temperature, insoluble materials were removed by filtration through Celite, and the residue was washed with ethyl acetate and water. The combined filtrate was concentrated under reduced pressure, ethanol was distilled off, and the residue was extracted three times with ethyl acetate. The obtained organic layer was washed with saturated sodium chloride solution and then dried over anhydrous sodium sulfate. The obtained organic layer was filtered, and the solvent was evaporated under reduced pressure. A mixture of n-hexane/ethyl acetate (1:1) was added to the residue to solidify the residue. The precipitated solid was collected by filtration to obtain the title compound (4.47 g). ¹H-NMR (CDCl₃) δ: 6.49 (1H, s), 3.96 (2H, br s), 3.95-3.85 (2H, m), 3.76 (1H, td, J = 11.0, 2.6 Hz), 3.60 (2H, br s), 3.52-3.44 (1H, m), 3.37 (1H, dd, J = 11.0, 9.2 Hz), 3.02 (1H, ddd, J = 12.2, 9.2, 2.6 Hz), 2.83 (1H, dt, J = 12.2, 2.6 Hz), 0.83 (3H, d, J = 6.1 Hz).

### (Step 3) Ethyl {6-chloro-4-[(3R)-3-methylmorpholin-4-yl]-1H-imidazo[4,5-c]pyridin-2-yl}acetate

A mixture of the compound (5.04 g) obtained in Step 2 above, ethyl 3-ethoxy-3-iminopropanoate hydrochloride (8.11 g), and ethanol (70 mL) was stirred under reflux for 9 hours. After cooling the reaction mixture to room temperature, the solvent was evaporated under reduced pressure. The residue was purified by silica gel column chromatography (n-hexane/ethyl acetate), and the obtained oily substance was solidified with a mixture of n-hexane/ethyl acetate (10:1). The precipitated solid was collected by filtration, washed with n-hexane, and then dried under reduced pressure to obtain the title compound (6.33 g). ¹H-NMR (CDCl₃) δ: 10.07 (1H, br s), 6.76 (1H, s), 5.48-5.37 (1H, m), 4.77 (1H, d, J = 13.4 Hz), 4.30 (2H, q, J = 7.3 Hz), 4.06-4.01 (1H, m), 4.00 (2H, s), 3.87 (1H, dd, J = 11.0, 3.1 Hz), 3.80 (1H, d, J = 11.0 Hz), 3.69 (1H, td, J = 11.0, 3.1 Hz), 3.47 (1H, td, J = 12.8, 3.1 Hz), 1.38-1.33 (6H, m).

### Reference Example 2

### Production of ethyl {6-chloro-4-[(3S)-3-(difluoromethyl)morpholin-4-yl]-1H-imidazo[4,5-c]pyridin-2-yl}acetate

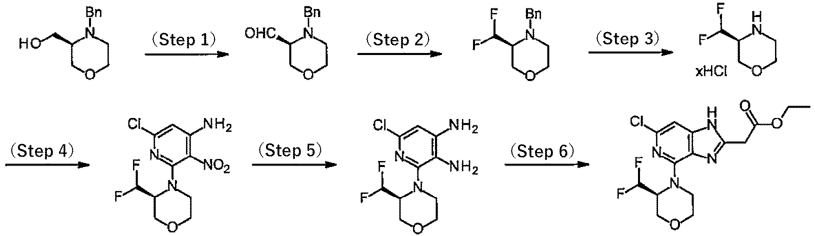

### (Step 1) (3S)-4-Benzylmorpholin-3-carbaldehyde

To a solution of [(3R)-4-benzylmorpholin-3-yl]methanol (300 mg, manufactured by BLD PHARM) in acetonitrile (15 mL) were added 2-hydroxy-2-azaadamantane (11.1 mg), copper(I) chloride (7.2 mg), 2,2'-bipyridyl (11.3 mg), and 4-dimethylaminopyridine (17.7 mg) at room temperature, and the mixture was stirred at the same temperature for 17 hours. Saturated sodium thiosulfate solution and saturated sodium bicarbonate solution were added to the reaction mixture, and the mixture was extracted three times with dichloromethane. The obtained organic layer was washed with saturated sodium chloride solution, and then dried over anhydrous sodium sulfate. The obtained organic layer was filtered, and the solvent was evaporated under reduced pressure to obtain the crude title compound (297 mg).

### (Step 2) (3S)-4-Benzyl-3-(difluoromethyl)morpholine

To a solution of the compound (297 mg) obtained in Step 1 above in dichloromethane (7 mL) was added bis(2-methoxyethyl)aminosulfur trifluoride (0.78 mL) at 0°C, and the mixture was stirred at room temperature for 4 hours. Saturated sodium bicarbonate solution was added to the reaction mixture, and the mixture was extracted three times with dichloromethane. The obtained organic layer was washed with saturated sodium chloride solution, and then dried over anhydrous sodium sulfate. The obtained organic layer was filtered, and the solvent was evaporated under reduced pressure. The residue was purified by silica gel column chromatography (n-hexane/ethyl acetate) to obtain the title compound (71.4 mg).
¹H-NMR (CDCl₃) δ: 7.39-7.22 (5H, m), 6.21 (1H, td, J = 55.5, 5.5 Hz), 3.93 (1H, dd, J = 14.1, 3.1 Hz), 3.90-3.79 (2H, m), 3.78-3.64 (3H, m), 2.90-2.80 (2H, m), 2.45-2.37 (1H, m).

### (Step 3) (3S)-3-(Difluoromethyl)morpholine hydrochloride

To a solution of the compound (70 mg) obtained in Step 2 above in 1,2-dichloroethane (1 mL) was added 1-chloroethyl chloroformate (0.1 mL) at room temperature, and the mixture was stirred under reflux for 8 hours. Subsequently, after cooling the reaction mixture to room temperature, methanol (1 mL) was added, and the mixture was stirred under reflux for 1 hour. After cooling the reaction mixture to room temperature, the solvent was evaporated under reduced pressure to obtain the crude title compound (54 mg).

### (Step 4) 6-Chloro-2-[(3S)-3-(difluoromethyl)morpholin-4-yl]-3-nitropyridin-4-amine

To a solution of the compound (50.1 mg) obtained in Step 3 above in 1,4-dioxane (1.5 mL) were added N,N-diisopropylethylamine (155 µL) and 2,6-dichloro-3-nitropyridin-4-amine (60 mg) at room temperature, and the mixture was stirred at 90°C for 7.5 hours. After cooling the reaction mixture to room temperature, the solvent was evaporated under reduced pressure. The residue was subjected to silica gel column chromatography (n-hexane/ethyl acetate) to obtain the crude title compound (64.5 mg).

### (Step 5) 6-Chloro-2-[(3S)-3-(difluoromethyl)morpholin-4-yl]pyridin-3,4-diamine

To a mixed solution of the compound (64.5 mg) obtained in Step 4 above in ethanol (2 mL)/water (1 mL) were added iron powder (76 mg) and ammonium chloride (36 mg) at room temperature, and the mixture was stirred at 80°C for 1 hour. After cooling the reaction mixture to room temperature, insoluble materials were removed by filtration through Celite, and the residue was washed with ethyl acetate and water. The combined filtrate was extracted twice with dichloromethane, and the obtained organic layer was washed with saturated sodium chloride solution, and then dried over anhydrous sodium sulfate. The obtained organic layer was filtered, and the solvent was evaporated under reduced pressure to obtain the crude title compound (37.9 mg).

### (Step 6) Ethyl {6-chloro-4-[(3S)-3-(difluoromethyl)morpholin-4-yl]-1H-imidazo[4,5-c]pyridin-2-yl}acetate

A mixture of the compound (37.9 mg) obtained in Step 5 above, ethyl 3-ethoxy-3-iminopropanoate hydrochloride (68 mg), and ethanol (1 mL) was stirred under reflux for 1.5 hours. After cooling the reaction mixture to room temperature, the solvent was evaporated under reduced pressure, and the residue was purified by silica gel column chromatography (n-hexane/ethyl acetate) to obtain the title compound (22.3 mg).
¹H-NMR (CDCl₃) δ: 10.14 (1H, br s), 6.81 (1H, s), 6.26 (1H, td, J = 56.3, 5.9 Hz), 5.80-5.78 (1H, m), 4.81 (1H, d, J = 14.1 Hz), 4.32-4.22 (3H, m), 4.05 (1H, dd, J = 11.7, 3.1 Hz), 3.99 (2H, d, J = 1.8 Hz), 3.90-3.80 (1H, m), 3.69 (1H, td, J = 11.7, 3.1 Hz), 3.49 (1H, td, J = 13.0, 3.3 Hz), 1.34 (3H, t, J = 7.4 Hz).

### Reference Example 3

### Production of ethyl {6-chloro-4-[(trans-4-hydroxycyclohexyl)(methyl)amino]-1H-imidazo[4, 5-c]pyridin-2-yl } acetate

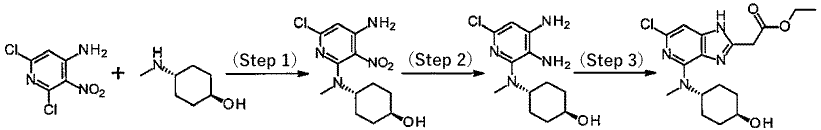

### (Step 1) trans-4-[(4-Amino-6-chloro-3-nitropyridin-2-yl)(methyl)amino]cyclohexanol

To a solution of trans-4-(methylamino)cyclohexanol (155 mg) and 2,6-dichloro-3-nitropyridin-4-amine (208 mg) in N,N-dimethylformamide (3.3 mL) was added potassium carbonate (276 mg) at room temperature, and the mixture was stirred at 40°C for 2 hours. The reaction mixture was cooled to room temperature, water was added, and the mixture was extracted twice with ethyl acetate. The obtained organic layer was washed with saturated sodium chloride solution, and then dried over anhydrous sodium sulfate. The obtained organic layer was filtered, and the solvent was evaporated under reduced pressure to obtain the crude title compound.

### (Steps 2 and 3) Ethyl {6-chloro-4-[(trans-4-hydroxycyclohexyl)(methyl)amino]-1H-imidazo[4,5-c]pyridin-2-yl}acetate

The title compound was obtained by using the compound obtained in Step 1 above as a production material and performing the same operations as in Steps 5 and 6 of Reference Example 2.
¹H-NMR (DMSO-D₆) δ: 12.57 (1H, s), 6.70 (1H, s), 5.24-5.06 (1H, m), 4.58 (1H, d, J = 4.9 Hz), 4.14 (2H, q, J = 7.0 Hz), 3.93 (2H, s), 3.45-3.37 (1H, m), 3.14 (3H, s), 1.93-1.85 (2H, m), 1.70-1.56 (4H, m), 1.37-1.25 (2H, m), 1.21 (3H, t, J = 7.0 Hz).

### Reference Example 4

### Production of ethyl (6-chloro-4-{[(2R)-1, 1, 1-trifluoropropan-2-yl]amino}-1H-imidazo[4,5-c]pyridin-2-yl)acetate

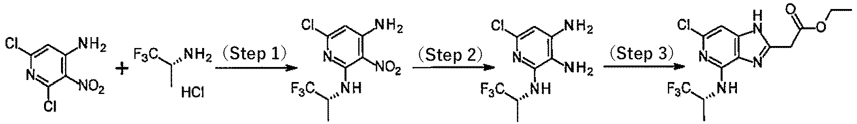

### (Step 1) 6-Chloro-3-nitro-N-2-[(2R)-1,1,1-trifluoropropan-2-yl]pyridin-2,4-diamine

A mixture of (R)-1,1,1-trifluoropropan-2-amine hydrochloride (863 mg), 2,6-dichloro-3-nitropyridin-4-amine (600 mg), N,N-diisopropylethylamine (2.00 mL), and dimethyl sulfoxide (6.0 mL) was stirred at 120°C for 7 hours.

The reaction mixture was cooled to room temperature, water was added, and the mixture was extracted twice with ethyl acetate. The obtained organic layer was washed twice with water and once with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (723 mg). ¹H-NMR (CDCl₃) δ: 9.00 (1H, br d, J = 9.4 Hz), 6.07 (1H, s), 5.31-5.17 (1H, m), 2.62 (2H, s), 1.44 (3H, d, J = 7.0 Hz).

### (Steps 2 and 3) Ethyl (6-chloro-4-{[(2R)-1,1,1-trifluoropropan-2-yl]amino}-1H-imidazo[4,5-c]pyridin-2-yl)acetate

The title compound was obtained by using the compound obtained in Step 1 above as a production material and performing the same operations as in Steps 5 and 6 of Reference Example 2.
¹H-NMR (DMSO-D₆) δ: 12.69 (1H, s), 7.56 (1H, d, J = 9.4 Hz), 6.84 (1H, s), 5.31-5.10 (1H, m), 4.13 (2H, q, J = 7.0 Hz), 3.96 (2H, s), 1.38 (3H, d, J = 7.0 Hz), 1.20 (3H, t, J = 7.0 Hz)

### Reference Example 5

### Production of ethyl (6-chloro-4-{[dimethyl(oxido)-λ⁶-sulfanylidene]amino}-1H-imidazo[4,5-c]pyridin-2-yl)acetate

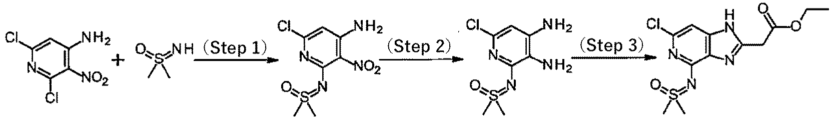

### (Step 1) 6-Chloro-2-{[dimethyl(oxido)-1⁶-sulfanylidene]amino}-3-nitropyridin-4-amine

A mixture of S,S-dimethylsulfoximine (559 mg), 2,6-dichloro-3-nitropyridin-4-amine (416 mg), N,N-diisopropylethylamine (1.05 mL), and 1,4-dioxane (5.0 mL) was stirred under reflux for 128 hours. The reaction mixture was cooled to room temperature, saturated sodium bicarbonate solution and water were added, and the mixture was extracted twice with ethyl acetate. The obtained organic layer was washed with saturated sodium chloride solution, and then dried over anhydrous sodium sulfate. The obtained organic layer was filtered, and the solvent was evaporated under reduced pressure. The obtained residue was solidified with a mixed solution of ethyl acetate (3 mL)/n-hexane (9 mL). The precipitated solid was collected by filtration, washed with a mixture of n-hexane/ethyl acetate, and then dried under reduced pressure to obtain the title compound (346 mg).
¹H-NMR (DMSO-D₆) δ: 6.96 (2H, s), 6.30 (1H, s), 3.39 (6H, s).

### (Steps 2 and 3) Ethyl (6-chloro-4-{[dimethyl(oxido)-λ⁶-sulfanylidene]amino}-1H-imidazo[4,5-c]pyridin-2-yl)acetate

The title compound was obtained by using the compound obtained in Step 1 above as a production material and performing the same operations as in Steps 5 and 6 of Reference Example 2.
¹H-NMR (CDCl₃) δ: 10.51-10.22 (1H, m), 7.18-6.91 (1H, m), 4.32-4.23 (2H, m), 4.08-4.03 (2H, m), 3.51-3.43 (6H, m), 1.36-1.30 (3H, m).

The following compounds were synthesized by performing the same operations as in any of the steps of Reference Examples 1 to 5 (Table 1-1 and Table 1-2).

**Table 1-1**

| Reference Example No. | Production Material | Synthesis Method | Structure of Compound Synthesized |
|---|---|---|---|
| 6 | 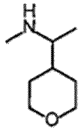 | Step 1 of Reference Example 3, Steps 5 and 6 of Reference Example 2 | 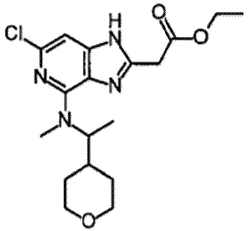 |
| 7 | 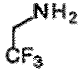 | Step 1 of Reference Example 4, Steps 5 and 6 of Reference Example 2 | 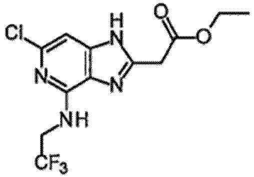 |
| 8 | 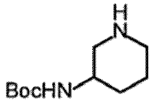 | Step 1 of Reference Example 1, Steps 5 and 6 of Reference Example 2 | 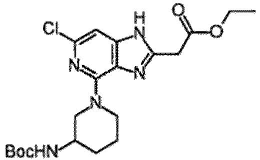 |
| 9 | 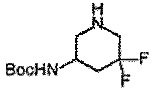 | Step 1 of Reference Example 3, Steps 5 and 6 of Reference Example 2 | 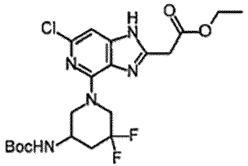 |
| 10 | 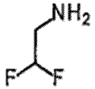 | Step 1 of Reference Example 3, Steps 5 and 6 of Reference Example 2 | 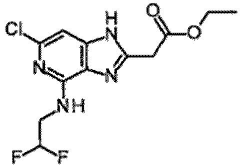 |
| 11 | 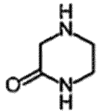 | Step 1 of Reference Example 1, Steps 5 and 6 of Reference Example 2 | 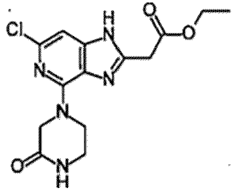 |

**Table 1-2**

| Reference Example No. | Name of Compound Synthesized | Spectral Data |
|---|---|---|
| 6 | ethyl (6-chloro-4-{methyl[1-(tetrahydro-2H-pyran-4-yl)ethyl]amino}-1H-imidazo[4,5-c]pyridin-2-yl)acetate | ¹H-NMR (DMSO-D₆) δ: 12.57 (1H, s), 6.69 (1H, s), 5.65-5.34 (1H, m), 4.14 (2H, q, J = 7.1 Hz), 3.93 (2H, s), 3.91-3.84 (1H, m), 3.77-3.69 (1H, m), 3.31-3.15 (2H, m), 3.06 (3H, s), 1.83-1.59 (2H, m), 1.36-1.04 (9H, m). |
| 7 | ethyl (6-chloro-4-[(2,2,2-trifluoroethyl)amino]-1H-imidazo[4,5-c]pyridin-2-yl)acetate | ¹H-NMR (DMSO-D₆) δ: 12.70 (1H, s), 7.67 (1H, t, J = 6.7 Hz), 6.86 (1H, s), 4.28-4.16 (2H, m), 4.13 (2H, q, J = 7.0 Hz), 3.96 (2H, s), 1.20 (3H, t, J = 7.0 Hz). |
| 8 | ethyl (4-{3-[(tert-butoxycarbonyl)amino]piperidin -1-yl}-6-chloro-1H-imidazo[4,5-c]pyridin-2-yl)acetate | ¹H-NMR (DMSO-D₆) δ: 12.64 (1H, s), 6.94-6.87 (1H, m), 6.79 (1H, s), 5.07-4.97 (1H, m), 4.82-4.73 (1H, m), 4.13 (2H, q, J = 7.0 Hz), 3.95 (2H, s), 3.45-3.23 (1H, m), 3.07-2.96 (1H, m), 2.92-2.82 (1H, m), 1.91-1.84 (1H, m), 1.79-1.71 (1H, m), 1.52-1.39 (11H, m), 1.20 (3H, t, J = 7.0 Hz). |
| 9 | ethyl (4-{5-[(tert-butoxycarbonyl)amino]-3,3-difluoropiperidin-1-yl}-6-chloro-1H-imidazo[4,5-c]pyridin-2-yl)acetate | ¹H-NMR (CDCl₃) δ: 10.16 (1H, br s), 6.83 (1H, s), 5.43 (1H, s), 5.22-5.05 (1H, m), 4.84-4.72 (1H, m), 4.28 (2H, q, J = 7.1 Hz), 4.16-4.10 (1H, m), 4.01-3.98 (2H, m), 3.89-3.68 (1H, m), 3.68-3.52 (1H, m), 2.49-2.13 (2H, m), 1.42 (9H, s), 1.33 (3H, t, J = 7.1 Hz). |
| 10 | ethyl (6-chloro-4-[(2,2-difluoroethyl)amino]-1H-imidazo[4,5-c]pyridin-2-yl)acetate | ¹H-NMR (DMSO-D₆) δ: 12.65 (1H, s), 7.41 (1H, t, J = 6.3 Hz), 6.81 (1H, s), 6.15 (1H, tt, J = 56.7, 4.3 Hz), 4.13 (2H, q, J = 7.0 Hz), 3.95 (2H, s), 3.86-3.71 (2H, m), 1.20 (3H, t, J = 7.0 Hz) |
| 11 | ethyl [6-chloro-4-(3-oxopiperazin-1-yl)-1H-imidazo[4,5-c]pyridin-2-yl]acetate | ¹H-NMR (CDCl₃) δ: 10.16 (1H, br s), 6.81 (1H, s), 6.02 (1H, br s), 4.89 (2H, s), 4.36 (2H, t, J = 5.5 Hz), 4.28 (2H, q, J = 7.1 Hz), 3.99 (2H, s), 3.54 (2H, td, J = 5.2, 2.2 Hz), 1.34 (3H, t, J = 7.1 Hz). |

### Reference Example 12

### Production of tert-butyl (2R,4R)-4-hydroxy-2-methylpyrrolidin-1-carboxylate

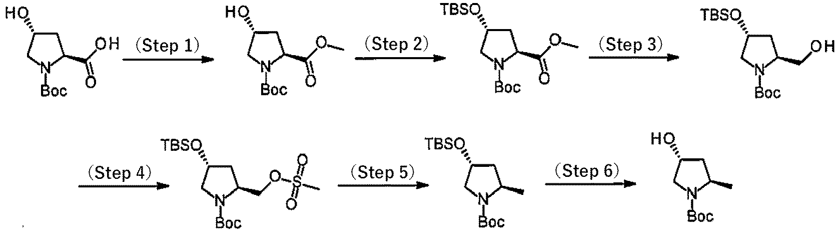

### (Step 1) 1-tert-Butyl 2-methyl (2S,4R)-4-hydroxypyrrolidin-1,2-dicarboxylate

To a solution of (4R)-1-(tert-butoxycarbonyl)-4-hydroxy-L-proline (2.0 g) in N,N-dimethylformamide (20 mL) were added potassium carbonate (1.8 g) and iodomethane (1.1 mL) at 0°C, and the mixture was stirred at room temperature for 2 hours. Water was added to the reaction mixture, and the mixture was extracted three times with ethyl acetate. The obtained organic layer was washed once with 5% sodium thiosulfate solution, twice with water, and once with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (2.0 g).
¹H-NMR (CDCl₃) δ: 4.55-4.49 (1H, m), 4.48-4.36 (1H, m), 3.77-3.71 (3H, m), 3.68-3.62 (1H, m), 3.59-3.54 (0.67H, m), 3.49-3.43 (0.33H, m), 2.36-2.20 (1H, m), 2.14-2.02 (1H, m), 1.76-1.68 (1H, m), 1.49-1.38 (9H, m).

### (Step 2) 1-tert-Butyl 2-methyl (2S,4R)-4-{[tert-butyl(dimethyl)silyl]oxy}pyrrolidin-1,2-dicarboxylate

To a solution of the compound (2.0 g) obtained in Step 1 above in N,N-dimethylformamide (20 mL) were added imidazole (1.1 g) and tert-butyldimethylchlorosilane (1.4 g) at 0°C, and the mixture was stirred at room temperature for 7.5 hours. Water was added to the reaction mixture, and the mixture was extracted three times with ethyl acetate. The obtained organic layer was washed three times with water and once with saturated sodium chloride solution, and then dried over anhydrous sodium sulfate. The obtained organic layer was filtered, and the solvent was evaporated under reduced pressure. The obtained residue was subjected to silica gel column chromatography (n-hexane/ethyl acetate) to obtain the crude title compound (3.0 g).

### (Step 3) tert-Butyl (2S,4R)-4-{[tert-butyl(dimethyl)silyl]oxy}-2-(hydroxymethyl)pyrrolidin-1-carboxylate

To a solution of the compound (3.0 g) obtained in Step 2 above in ethanol (30 mL) was added sodium borohydride (1.2 g) at 0°C, and the mixture was stirred at room temperature for 5 hours. Saturated sodium bicarbonate solution was added to the reaction mixture at 0°C, and the mixture was extracted three times with ethyl acetate. The obtained organic layer was washed with water and saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (2.5 g).
¹H-NMR (CDCl₃) δ: 4.92 (1H, d, J = 8.0 Hz), 4.28 (1H, br s), 3.73-3.66 (1H, m), 3.58-3.51 (1H, m), 3.43 (1H, d, J = 11.7 Hz), 3.34 (1H, dd, J = 11.7, 3.7 Hz), 1.98-1.93 (1H, m), 1.61-1.54 (1H, m), 1.47 (9H, s), 0.87 (9H, s), 0.06 (6H, s).

### (Step 4) tert-Butyl (2S,4R)-4-{[tert-butyl(dimethyl)silyl]oxy}-2-{[(methylsulfonyl)oxymethyl}pyrrolidin-1-carboxylate

To a solution of the compound (2.5 g) obtained in Step 3 above in dichloromethane (30 mL) were added triethylamine (3.1 mL) and methanesulfonic anhydride (2.9 g) at 0°C, and the mixture was stirred at room temperature for 1.5 hours. Water was added to the reaction mixture, and the layers were separated. The aqueous layer was extracted twice with chloroform. The combined organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (2.9 g).

### (Step 5) tert-Butyl (2R,4R)-4-{[tert-butyl(dimethyl)silyl]oxy}-2-methylpyrrolidin-1-carboxylate

To a solution of the compound (2.9 g) obtained in Step 4 above in tetrahydrofuran (30 mL) was added a 1.01 M solution of lithium triethylborohydride in tetrahydrofuran (35 mL) at 0°C, and the mixture was stirred at room temperature for 3.5 hours. Saturated ammonium chloride solution was added to the reaction mixture at 0°C, and the mixture was extracted three times with ethyl acetate. The obtained organic layer was washed with water and saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (2.5 g).
¹H-NMR (CDCl₃) δ: 4.36-4.31 (1H, m), 4.03-3.70 (1H, m), 3.49-3.23 (2H, m), 2.04-1.93 (1H, m), 1.46 (9H, s), 1.32-1.18 (4H, m), 0.87 (9H, s), 0.05 (6H, s).

### (Step 6) tert-Butyl (2R,4R)-4-hydroxy-2-methylpyrrolidin-1-carboxylate

To a solution of the compound (2.5 g) obtained in Step 5 above in tetrahydrofuran (20 mL) was added a 1.0 M solution of tetra-n-butylammonium fluoride in tetrahydrofuran (10 mL) at 0°C, and the mixture was stirred at room temperature for 5 hours. The reaction mixture was concentrated under reduced pressure. The obtained residue was purified by silica gel column chromatography (n-hexane/ethyl acetate) to obtain the title compound (1.3 g).
¹H-NMR (CDCl₃) δ: 4.42-4.37 (1H, m), 4.29-3.94 (1H, m), 3.51-3.43 (2H, m), 2.16-2.04 (1H, m), 1.76-1.68 (1H, m), 1.47 (9H, s), 1.24 (3H, d, J = 5.5 Hz)

The following compounds were synthesized by performing the same operation as in any of Steps 1 to 6 of Reference Example 12 (Table 2-1 and Table 2-2).

**Table 2-1**

| Reference Example No. | Production Material | Synthesis Method | Structure of Compound Synthesized |
|---|---|---|---|
| 13 | 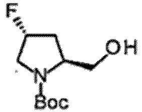 Can be produced using the method described in US20110082165A1 etc. | Reference Example 12 Steps 4 and 5 | 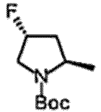 |
| 14 | 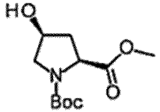 | Reference Example 12 Steps 2, 3, 4, 5, and 6 | 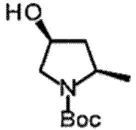 |
| 15 | 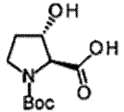 Can be produced using the method described in J. Med. Chem. 2006, 49(21), 6254-6263 etc. | Reference Example 12 Steps 1 and 3 | 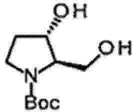 |
| 16 | 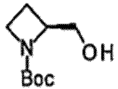 Can be produced using the method described in J. Med. Chem. 2005, 48(24), 7637-7647 etc. | Reference Example 12 Steps 4 and 5 | 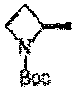 |

**Table 2-2**

| Reference Example No. | Name of Compound Synthesized | Spectral Data |
|---|---|---|
| 13 | tert-butyl (2R,4R)-4-fluoro-2-methylpyrrolidin-1-carboxylate | ¹H-NMR (CDCl₃) δ: 5.10 (1H, d, J = 53.1 Hz), 4.19-3.70 (2H, m), 3.43 (1H, ddd, J = 37.2, 13.4, 3.1 Hz), 2.49-2.35 (1H, m), 1.80-1.57 (1H, m), 1.47 (9H, s), 1.33-1.21 (4H, m). |
| 14 | tert-butyl (2R,4S)-4-hydroxy-2-methylpyrrolidin-1-carboxylate | ¹H-NMR (CDCl₃) δ: 4.32-4.27 (1H, m), 3.99-3.48 (2H, m), 3.28-3.15 (1H, m), 2.22-2.09 (1H, m), 1.47 (9H, s), 1.36-1.22 (4H, m), 0.88 (9H, s), 0.06 (6H, s). |
| 15 | tert-butyl (2R,3S)-3-hydroxy-2-(hydroxymethyl)pyrrolidin-1-carboxylate | ¹H-NMR (CDCl₃) δ: 4.18-3.96 (1H, m), 3.87-3.52 (3H, m), 3.49-3.38 (1H, m), 2.05 (1H, br s), 1.92-1.83 (1H, m), 1.74 (1H, d, J = 3.7 Hz), 1.48 (9H, s). |
| 16 | tert-butyl (2R)-2-methylazetidin-1-carboxylate | ¹H-NMR (CDCl₃) δ: 4.33-4.24 (1H, m), 3.81 (2H, t, J = 7.6 Hz), 2.33-2.23 (1H, m), 1.81-1.72 (1H, m), 1.44 (9H, s), 1.37 (3H, d, J = 6.1 Hz). |

### Reference Example 17

### Production of tert-butyl (2R,3S)-3-hydroxy-2-methylpyrrolidin-1-carboxylate

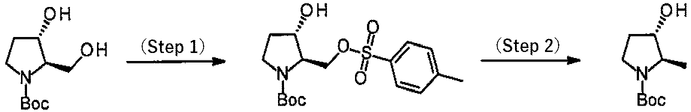

### (Step 1) tert-Butyl (2R,3S)-3-hydroxy-2-({[(4-methylphenyl)sulfonyl]oxy}methyl)pyrrolidin-1-carboxylate

To a solution of the compound (0.50 g) obtained in Reference Example 15 in pyridine (2.0 mL) was added 4-methylbenzenesulfonyl chloride (0.66 g) at room temperature, and the mixture was stirred at the same temperature for 6 hours. Water was added to the reaction mixture, and the mixture was extracted three times with ethyl acetate. The obtained organic layer was washed three times with water and once with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (0.55 g).
¹H-NMR (CDCl₃) δ: 7.77 (2H, d, J = 6.7 Hz), 7.39-7.31 (2H, m), 4.39 (1H, br s), 4.21-3.73 (3H, m), 3.59-3.32 (2H, m), 2.45 (3H, s), 2.14-1.73 (3H, m), 1.33-1.44 (9H, m).

### (Step 2) tert-Butyl (2R,3S)-3-hydroxy-2-methylpyrrolidin-1-carboxylate

The crude title compound was obtained by using the compound obtained in Step 1 above as a production material and performing the same operation as in Step 5 of Reference Example 12.

### Reference Example 18

### Production of ethyl {6-chloro-4-[(2R,4R)-4-hydroxy-2-methylpyrrolidin-1-yl]-1H-imidazo [4, 5-c]pyridin-2-yl } acetate

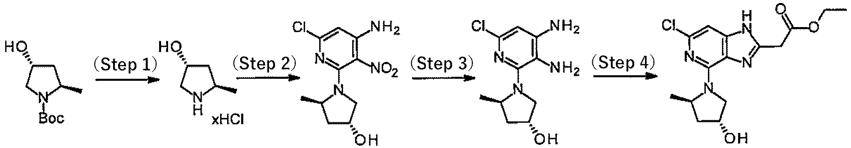

### (Step 1) (3R,5R)-5-Methylpyrrolidin-3-ol hydrochloride

To the compound (1.3 g) obtained in Step 6 of Reference Example 12 was added a 4 M solution of hydrogen chloride in 1,4-dioxane (5 mL) at room temperature, and the mixture was stirred at the same temperature for 5 hours. The reaction mixture was concentrated under reduced pressure, toluene was added to the obtained residue, and the mixture was concentrated again under reduced pressure to obtain the crude title compound (0.90 g).

### (Step 2) (3R,5R)-1-(4-Amino-6-chloro-3-nitropyridin-2-yl)-5-methylpyrrolidin-3-ol

To a solution of the compound (0.90 g) obtained in Step 1 above in N,N-dimethylformamide (20 mL) were added 2,6-dichloro-3-nitropyridin-4-amine (1.0 g) and potassium carbonate (2.0 g) at room temperature, and the mixture was stirred at the same temperature for 27 hours. Water was added to the reaction mixture, and the mixture was extracted three times with ethyl acetate. The obtained organic layer was washed three times with water and once with saturated sodium chloride solution, and then dried over anhydrous sodium sulfate. The obtained organic layer was filtered, and the solvent was evaporated under reduced pressure to obtain the crude title compound (1.6 g).

### (Step 3) (3R,5R)-1-(3,4-Diamino-6-chloropyridin-2-yl)-5-methylpyrrolidin-3-ol

A mixture of the compound (1.6 g) obtained in Step 2 above, iron powder (1.3 g), ammonium chloride (0.13 g), ethanol (20 mL), and water (5 mL) was stirred at 90°C for 1.5 hours. After cooling the reaction mixture to room temperature, insoluble materials were removed by filtration through Celite, and the residue was washed with ethanol. Saturated sodium bicarbonate solution was added to the combined filtrate, and the mixture was extracted three times with ethyl acetate. The obtained organic layer was washed with water and saturated sodium chloride solution, and then dried over anhydrous sodium sulfate. The obtained organic layer was filtered, and the solvent was evaporated under reduced pressure to obtain the crude title compound (1.4 g).

### (Step 4) Ethyl {6-chloro-4-[(2R,4R)-4-hydroxy-2-methylpyrrolidin-1-yl]-1H-imidazo[4,5-c]pyridin-2-yl}acetate

A mixture of the compound (1.4 g) obtained in Step 3 above, ethyl 3-ethoxy-3-iminopropanoate hydrochloride (1.9 g), ethanol (20 mL), and acetic acid (2.7 mL) was stirred at 90°C for 4 hours. The reaction mixture was cooled to room temperature, saturated sodium bicarbonate solution was added, and the mixture was extracted three times with ethyl acetate. The obtained organic layer was washed with water and saturated sodium chloride solution, and then dried over anhydrous sodium sulfate. The obtained organic layer was filtered, and the solvent was evaporated under reduced pressure. The residue was purified by silica gel column chromatography (n-hexane/ethyl acetate) to obtain the title compound (1.15 g).
¹H-NMR (CDCl₃) δ: 10.00 (1H, br s), 6.65 (1H, s), 4.90-4.83 (1H, m), 4.61-4.55 (1H, m), 4.27 (2H, q, J = 7.1 Hz), 4.21-4.15 (1H, m), 4.06 (1H, dd, J = 12.2, 4.3 Hz), 3.97 (2H, s), 2.26-2.19 (1H, m), 1.95-1.88 (1H, m), 1.67 (1H, d, J = 4.9 Hz), 1.58 (9H, s), 1.35-1.31 (6H, m).

The following compounds were synthesized by performing the same operations as in Steps 1 to 4 of Reference Example 18 (Table 3-1).

**Table 3-1**

| Reference Example No. | Production Material | Structure of Compound Synthesized | Name of Compound Synthesized | Spectral Data |
|---|---|---|---|---|
| 19 | 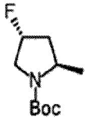 Reference Example 13 | 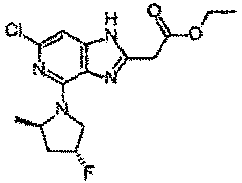 | ethyl {6-chloro-4-[(2R,4R)-4-fluoro-2-methylpyrrolidin-1-yl]-1H-imidazo[4,5-c]pyridin-2-yl}acetate | ¹H-NMR (CDCl₃) δ: 9.97 (1H, br s), 6.69 (1H, s), 5.30 (1H, d, J = 53.4 Hz), 4.86 (1H, q, J = 6.7 Hz), 4.63-4.52 (1H, m), 4.28 (2H, q, J = 6.7 Hz), 4.13-3.95 (1H, m), 3.98 (2H, s), 2.59-2.47 (1H, m), 1.97-1.81 (1H, m), 1.37 (3H, d, J = 6.1 Hz), 1.33 (3H, t, J = 6.7 Hz). |
| 20 | 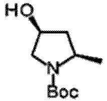 Reference Example 14 | 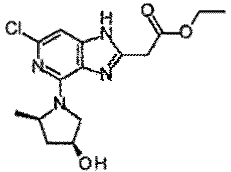 | ethyl {6-chloro-4-[(2R,4S)-4-hydroxy-2-methylpyrrolidin-1-yl]-1H-imidazo[4,5-c]pyridin-2-yl}acetate | ¹H-NMR (CDCl₃) δ: 9.96 (1H, br s), 6.64 (1H, s), 4.88-4.81 (1H, m), 4.60-4.55 (1H, m), 4.27 (2H, q, J = 6.7 Hz), 4.20 (1H, dd, J = 12.5, 5.2 Hz), 4.07 (1H, dd, J = 12.5, 2.7 Hz), 3.98 (2H, s), 2.40-2.33 (1H, m), 1.86-1.79 (1H, m), 1.69 (1H, d, J = 3.7 Hz), 1.48 (3H, d, J = 6.7 Hz), 1.33 (3H, t, J = 6.7 Hz). |
| 21 | 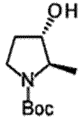 Reference Example 17 | 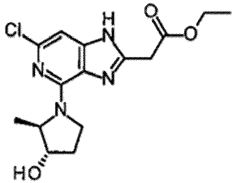 | ethyl {6-chloro-4-[(2R,3S)-3-hydroxy-2-methylpyrrolidin-1-yl]-1H-imidazo[4,5-c]pyridin-2-yl}acetate | ¹H-NMR (CDCl₃) δ: 9.94 (1H, br s), 6.63 (1H, s), 4.85-4.79 (1H, m), 4.27 (2H, q, J = 7.1 Hz), 4.22 (1H, t, J = 4.0 Hz), 4.14-4.05 (1H, m), 3.97 (2H, s), 4.02-3.95 (1H, m), 2.30-2.22 (1H, m), 2.05-1.98 (1H, m), 1.60 (1H, d, J = 4.3 Hz), 1.33 (3H, t, J = 7.1 Hz), 1.25 (3H, d, J = 6.1 Hz). |
| 22 | 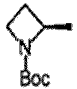 Reference Example 16 | 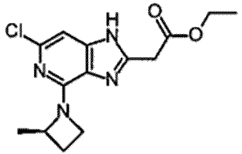 | ethyl {6-chloro-4-[(2R)-2-methylazetidin-1-yl]-1H-imidazo[4,5-c]pyridin-2-yl}acetate | ¹H-NMR (CDCl₃) δ: 9.98 (1H, br s), 6.65 (1H, s), 4.82-4.75 (1H, m), 4.41 (1H, td, J = 8.9, 5.5 Hz), 4.27 (2H, q, J = 7.3 Hz), 4.23-4.17 (1H, m), 3.98 (2H, s), 2.57-2.49 (1H, m), 2.07-1.99 (1H, m), 1.61 (3H, d, J = 6.1 Hz), 1.33 (3H, t, J = 7.3 Hz). |

### Reference Example 23

### Production of ethyl [6-chloro-4-(tetrahydro-2H-pyran-4-yl)-1H-imidazo[4,5-c]pyridin-2-yl]acetate

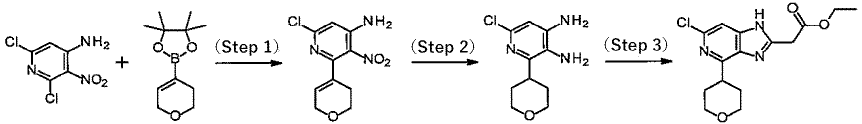

### (Step 1) 6-Chloro-2-(3,6-dihydro-2H-pyran-4-yl)-3-nitropyridin-4-amine

A mixture of 4-(4,4,5,5-tetramethyl-1,3,2-dioxaborolan-2-yl)-3,6-dihydro-2H-pyran (8.48 g), 2,6-dichloro-3-nitropyridin-4-amine (8.00 g), potassium carbonate (10.6 g), [1,1'-bis(diphenylphosphino)ferrocene]dichloropalladium(II) dichloromethane adduct (785 mg), 1,4-dioxane (80 mL), and water (20 mL) was stirred at 90°C for 7 hours under a nitrogen atmosphere. After cooling the reaction mixture to room temperature, water and ethyl acetate were added, and insoluble materials were removed by filtration through Celite. The filtrate was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (6.08 g).
¹H-NMR (CDCl₃) δ: 6.66 (1H, s), 5.95-5.88 (1H, m), 5.63 (2H, br s), 4.25 (2H, q, J = 2.7 Hz), 3.91 (2H, t, J = 5.2 Hz), 2.53-2.44 (2H, m).

### (Step 2) 6-Chloro-2-(tetrahydro-2H-pyran-4-yl)pyridin-3,4-diamine

To a solution of the compound (4.00 g) obtained in Step 1 above in ethanol (100 mL) was added 5%-platinum (sulfided) on carbon (916 mg) at room temperature, and the mixture was stirred at 50°C for 7 hours under a hydrogen atmosphere. After cooling the reaction mixture to room temperature, insoluble materials were removed by filtration through Celite, and the filtrate was concentrated under reduced pressure. Ethyl acetate (300 mL) was added to the obtained residue, insoluble materials were removed by filtration, and the solvent was evaporated under reduced pressure to obtain the crude title compound (2.95 g).

### (Step 3) Ethyl [6-chloro-4-(tetrahydro-2H-pyran-4-yl)-1H-imidazo[4,5-c]pyridin-2-yl]acetate

A mixture of the compound (3.10 g) obtained in Step 2 above, ethyl 3-ethoxy-3-iminopropanoate hydrochloride (5.33 g), ethanol (50 mL), and acetic acid (3.9 mL) was stirred under reflux for 4 hours. After cooling the reaction mixture to room temperature, water was added, and the mixture was extracted three times with ethyl acetate. The obtained organic layer was washed with saturated sodium chloride solution, and then dried over anhydrous sodium sulfate. The obtained organic layer was filtered and concentrated under reduced pressure. Toluene was added to the residue, and the mixture was concentrated again under reduced pressure. The obtained residue was solidified with diethyl ether. The precipitated solid was collected by filtration, washed with a mixture of n-hexane/ethyl acetate (1:3), and then dried under reduced pressure to obtain the title compound (2.10 g).
¹H-NMR (CD₃OD) δ: 7.36 (1H, s), 4.21 (2H, q, J = 7.2 Hz), 4.13-3.99 (4H, m), 3.72-3.56 (3H, m), 2.21-2.03 (2H, m), 1.83-1.71 (2H, m), 1.26 (3H, t, J = 7.2 Hz).

### Reference Example 24

### Production of ethyl [6-chloro-4-(4-hydroxy-2,2-dimethyltetrahydro-2H-pyran-4-yl)-1H-imidazo[4,5-c]pyridin-2-yl]acetate

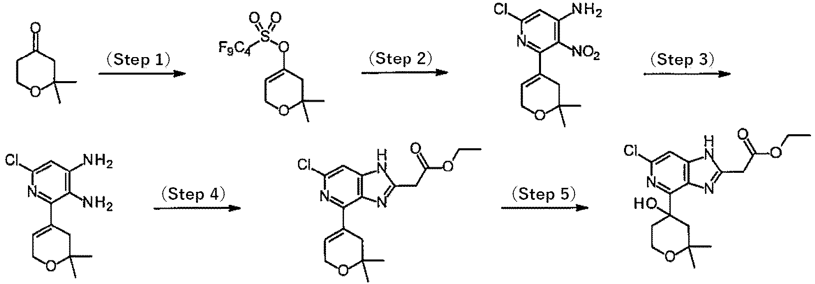

### (Step 1) 2,2-Dimethyl-3,6-dihydro-2H-pyran-4-yl nonafluorobutan-1-sulfonate

To a solution of 2,2-dimethyltetrahydro-4H-pyran-4-one (708 mg) in tetrahydrofuran (15 mL) was added a 1.08 M solution of lithium diisopropylamide in n-hexan-tetrahydrofuran (6.2 mL) at -70°C, and the mixture was stirred at the same temperature for 1 hour. Then, a solution of nonafluorobutan-1-sulfonyl fluoride (1.16 mL) in tetrahydrofuran (2.0 mL) was added, and the mixture was warmed to 0°C and stirred for another 2.5 hours. Saturated ammonium chloride solution was added to the reaction mixture, and the mixture was extracted twice with hexane. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain a mixture (1.60 g) of the title compound and a double bond positional isomer (6,6-dimethyl-3,6-dihydro-2H-pyran-4-yl nonafluorobutan-1-sulfonate).
¹H-NMR (CDCl₃) δ: 5.85-5.81 (0.75H, m), 5.74-5.71 (0.25H, m), 4.30-4.25 (1.5H, m), 3.93-3.88 (0.5H, m), 2.42-2.38 (0.5H, m), 2.32-2.28 (1.5H, m), 1.33 (1.5H, s), 1.31-1.27 (4.5H, m).

### (Step 2) 6-Chloro-2-(2,2-dimethyl-3,6-dihydro-2H-pyran-4-yl)-3-nitropyridin-4-amine

A mixture of the compound (1.00 g) obtained in Step 1 above, bis(pinacolato)diboron (743 mg), potassium acetate (718 mg), [1,1'-bis(diphenylphosphino)ferrocene]dichloropalladium(II) dichloromethane adduct (200 mg), and 1,4-dioxane (12 mL) was stirred at 80°C for 7.5 hours under a nitrogen atmosphere. After cooling the reaction mixture to room temperature, 2,6-dichloro-3-nitropyridin-4-amine (360 mg), cesium carbonate (1.67 g), [1,1'-bis(diphenylphosphino)ferrocene]dichloropalladium(II) dichloromethane adduct (100 mg), and water (3.0 mL) were added, and the mixture was stirred at 100°C for 2 hours under a nitrogen atmosphere. After cooling the reaction mixture to room temperature, insoluble materials were removed by filtration through Celite, and the filtrate was concentrated. The obtained residue was purified by silica gel column chromatography (n-hexane/ethyl acetate) to obtain a mixture (299 mg) of the title compound and a double bond positional isomer (6-chloro-2-(6,6-dimethyl-3,6-dihydro-2H-pyran-4-yl)-3-nitropyridin-4-amine).
¹H-NMR (CDCl₃) δ: 6.66-6.63 (1H, m), 5.93-5.90 (0.72H, m), 5.71-5.69 (0.28H, m), 5.57 (2H, br s), 4.28-4.23 (1.44H, m), 3.95-3.89 (0.56H, m), 2.47-2.42 (0.56H, m), 2.37-2.33 (1.44H, m), 1.31 (6.48H, s), 1.27 (2.52H, d, J = 4.9 Hz).

### (Step 3) 6-Chloro-2-(2,2-dimethyl-3,6-dihydro-2H-pyran-4-yl)pyridin-3,4-diamine

A mixture of the compound (299 mg) obtained in Step 2 above, iron powder (590 mg), ammonium chloride (290 mg), ethanol (8.0 mL), and water (4.0 mL) was stirred at 80°C for 2.5 hours. After cooling the reaction mixture to room temperature, insoluble materials were removed by filtration through Celite. The filtrate was extracted with dichloromethane. The obtained organic layer was washed with saturated sodium chloride solution, and then dried over anhydrous sodium sulfate. The obtained organic layer was filtered, and the solvent was evaporated under reduced pressure to obtain a mixture (262 mg) of the crude title compound and a double bond positional isomer (6-chloro-2-(6,6-dimethyl-3,6-dihydro-2H-pyran-4-yl)pyridin-3,4-diamine).

### (Step 4) Ethyl [6-chloro-4-(2,2-dimethyl-3,6-dihydro-2H-pyran-4-yl)-1H-imidazo[4,5-c]pyridin-2-yl]acetate

A mixture of the compound (260 mg) obtained in Step 3 above, ethyl 3-ethoxy-3-iminopropanoate hydrochloride (400 mg), ethanol (14 mL), and acetic acid (0.50 mL) was stirred under reflux for 3 hours. After cooling the reaction mixture to room temperature, the solvent was evaporated under reduced pressure, and the residue was purified by silica gel column chromatography (n-hexane/ethyl acetate) to obtain a mixture (223 mg) of the title compound and a double bond positional isomer (ethyl [6-chloro-4-(6,6-dimethyl-3,6-dihydro-2H-pyran-4-yl)-1H-imidazo[4,5-c]pyridin-2-yl]acetate).
¹H-NMR (CDCl₃) δ: 10.90-10.66 (0.5H, m), 10.47-10.29 (0.5H, m), 7.77 (0.5H, s), 7.53 (0.5H, d, J = 13.4 Hz), 7.33-7.19 (0.5H, m), 6.55-6.38 (0.5H, m), 4.52-4.44 (1.5H, m), 4.36-4.24 (2H, m), 4.16-4.05 (2H, m), 4.02-3.94 (0.5H, m), 2.85-2.65 (2H, m), 1.70-1.19 (9H, m).

### (Step 5) Ethyl [6-chloro-4-(4-hydroxy-2,2-dimethyltetrahydro-2H-pyran-4-yl)-1H-imidazo[4, 5-c]pyridin-2-yl] acetate

To a mixed solution of the compound (88.0 mg) obtained in Step 4 above in 2-propanol (2.8 mL)/dichloromethane (0.40 mL) was added tris(2,2,6,6-tetramethyl-3,5-heptanedionato)manganese(III) (15 mg) at 0°C, and the mixture was stirred at room temperature for 3 hours under an oxygen atmosphere. Ethyl acetate and saturated sodium thiosulfate solution were added to the reaction mixture, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (31.7 mg).
¹H-NMR (CDCl₃) δ: 11.12-10.39 (1H, m), 7.55-7.32 (1H, m), 4.38-4.24 (3H, m), 4.11-4.07 (2H, m), 3.94-3.79 (1H, m), 2.82-2.67 (1H, m), 2.56-2.48 (1H, m), 1.80-1.57 (2H, m), 1.52 (3H, s), 1.39-1.32 (3H, m), 1.31-1.25 (3H, m).

### Reference Example 25

### Production of ethyl 2-{4-[3-(tert-butoxycarbonylamino)phenyl]-6-chloro-1H-imidazo[4,5-c]pyridin-2-yl}acetate

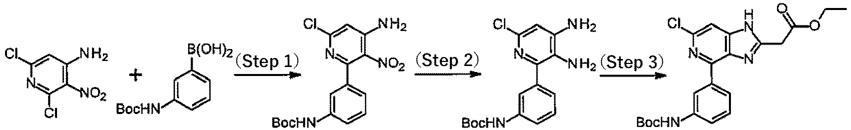

### (Step 1) tert-Butyl N-[3-(4-amino-6-chloro-3-nitro-2-pyridyl)phenyl]carbamate

A mixture of 3-(N-tert-butoxycarbonylamino)phenylboronic acid (228 mg), 2,6-dichloro-3-nitropyridin-4-amine (200 mg), cesium carbonate (284 mg), [1,1'-bis(diphenylphosphino)ferrocene]dichloropalladium(II) dichloromethane adduct (42.7 mg), 1,4-dioxane (4.0 mL), and water (1.0 mL) was stirred at 100°C for 5 hours under a nitrogen atmosphere. The reaction mixture was cooled to room temperature, water was added, and the mixture was extracted twice with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (138 mg).
¹H-NMR (CDCl₃) δ: 7.59 (1H, br s), 7.41-7.36 (1H, m), 7.31 (1H, t, J = 7.8 Hz), 7.12-7.08 (1H, m), 6.71 (1H, s), 6.61 (1H, br s), 5.81 (2H, s), 1.52 (9H, s).

### (Step 2) tert-Butyl N-[3-(3,4-diamino-6-chloro-2-pyridyl)phenyl]carbamate

To a mixture of the compound (138 mg) obtained in Step 1 above, ammonium chloride (20 mg), ethanol (4 mL), and water (2 mL) was added iron powder (211 mg) at 80°C, and the mixture was stirred at the same temperature for 2 hours. After cooling the reaction mixture to room temperature, insoluble materials were removed by filtration through Celite, and the filtrate was concentrated under reduced pressure. Water was added to the residue, and the mixture was extracted twice with ethyl acetate. The obtained organic layer was washed with saturated sodium chloride solution, and then dried over anhydrous sodium sulfate. The obtained organic layer was filtered, and the solvent was evaporated under reduced pressure to obtain the crude title compound (120 mg).

### (Step 3) Ethyl 2-{4-[3-(tert-butoxycarbonylamino)phenyl]-6-chloro-1H-imidazo[4,5-c]pyridin-2-yl}acetate

A mixture of the compound (120 mg) obtained in Step 2 above, ethyl 3-ethoxy-3-iminopropanoate hydrochloride (140 mg), acetic acid (0.4 mL), and ethanol (4 mL) was stirred at 90°C for 8 hours. The reaction mixture was cooled to room temperature, insoluble materials were removed by filtration, and the solvent was evaporated under reduced pressure. The obtained residue was purified by silica gel column chromatography (n-hexane/ethyl acetate) to obtain the title compound (114 mg).
¹H-NMR (DMSO-D₆) δ: 10.93 (0.3H, br s), 10.60 (0.7H, br s), 8.34-8.22 (1H, m), 8.05-7.97 (0.3H, m), 7.75-7.50 (1H, m), 7.48-7.27 (1.7H, m), 6.74-6.54 (1H, m), 4.29 (2H, q, J = 7.0 Hz), 4.12 (2H, s), 1.54 (9H, s), 1.34 (3H, t, J = 7.0 Hz).

### Reference Example 26

### Production of ethyl [6-chloro-4-(2-fluorophenyl)-1H-imidazo[4,5-c]pyridin-2-yl]acetate

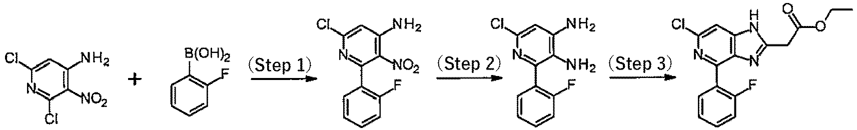

### (Steps 1 to 3) Ethyl [6-chloro-4-(2-fluorophenyl)-1H-imidazo[4,5-c]pyridin-2-yl]acetate

The title compound was obtained by using 2-fluorophenylboronic acid as a production material and performing the same operations as in Steps 1 to 3 of Reference Example 25.
¹H-NMR (CDCl₃) δ: 11.01-10.87 (1H, m), 8.04-8.02 (1H, m), 7.67 (1H, s), 7.47-7.37 (2H, m), 7.25-7.16 (1H, m), 4.31 (2H, q, J = 6.1 Hz), 4.14 (2H, s), 1.36-1.34 (3H, m).

### Reference Example 27

### Production of ethyl (6-chloro-4-{[(3S)-tetrahydrofuran-3-yl]amino}-1H-imidazo[4,5-c]pyridin-2-yl)acetate

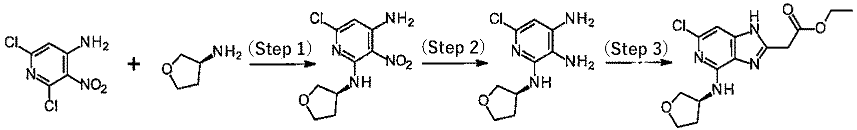

### (Steps 1 to 3) Ethyl (6-chloro-4-{[(3S)-tetrahydrofuran-3-yl]amino}-1H-imidazo[4,5-c]pyridin-2-yl)acetate

The title compound was obtained by using (S)-3-aminotetrahydrofuran as a production material and performing the same operations as in Step 1 of Reference Example 1 and Steps 2 and 3 of Reference Example 25.
¹H-NMR (CDCl₃) δ: 10.19 (1H, br s), 6.72 (1H, s), 5.52 (1H, d, J = 8.0 Hz), 4.92-4.85 (1H, m), 4.28 (2H, q, J = 7.2 Hz), 3.99 (2H, s), 4.07-3.96 (2H, m), 3.90-3.82 (1H, m), 3.77 (1H, dd, J = 9.2, 3.7 Hz), 2.42-2.33 (1H, m), 1.98-1.88 (1H, m), 1.33 (3H, t, J = 7.2 Hz).

### Reference Example 28

### Production of ethyl (6-chloro-1H-imidazo[4,5-c]pyridin-2-yl)acetate

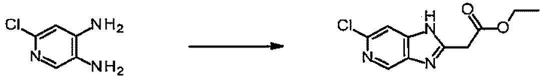

### Ethyl (6-chloro-1H-imidazo[4,5-c]pyridin-2-yl)acetate

A mixture of 6-chloro-3,4-pyridinediamine (16.7 g), ethyl 3-ethoxy-3-iminopropanoate hydrochloride (25.0 g), and acetic acid (50 mL) was stirred at 100°C for 4 hours. After cooling the reaction mixture to room temperature, water (150 mL) was added, and the mixture was cooled to 0°C. The precipitated solid was collected by filtration, washed twice with cold water, and then dried to obtain a crude product. Ethanol (50 mL) and water (250 mL) were added to the obtained crude product, and the mixture was stirred at 60°C for 20 minutes. Insoluble materials were removed by filtration while the mixture was still warm. The filtrate was cooled to 0°C. The precipitated solid was collected by filtration, washed with water, and then dried under reduced pressure to obtain the title compound (14.9 g).
¹H-NMR (DMSO-D₆) δ: 13.25-12.83 (1H, m), 8.67 (1H, s), 7.64 (1H, br s), 4.15 (2H, q, J = 7.0 Hz), 4.07 (2H, s), 1.21 (3H, t, J = 7.0 Hz).

### Reference Example 29

### Production of ethyl (6-chloro-4-methyl-1H-imidazo[4,5-c]pyridin-2-yl)acetate

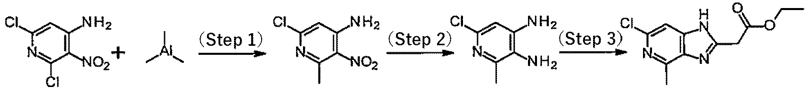

### (Step 1) 6-Chloro-2-methyl-3-nitropyridin-4-amine

To a solution of 2,6-dichloro-3-nitropyridin-4-amine (2.08 g) and tetrakis(triphenylphosphine)palladium(O) (1.16 g) in N,N-dimethylformamide (24 mL) was added dropwise a 2.0 M solution of trimethylaluminum in toluene (6.0 mL) at room temperature, and the mixture was stirred at 70°C for 3 hours. The reaction mixture was cooled to room temperature, ice water and ethyl acetate were added, and then insoluble materials were removed by filtration. The obtained filtrate was extracted twice with ethyl acetate. Saturated sodium bicarbonate solution was then added to the aqueous layer, and the mixture was extracted twice with ethyl acetate. The combined organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (855 mg).
¹H-NMR (CDCl₃) δ: 6.64 (1H, s), 5.94 (2H, br s), 2.71 (3H, s).

### (Step 2) 6-Chloro-2-methylpyridin-3,4-diamine

A mixture of the compound (853 mg) obtained in Step 1 above, iron powder (635 mg), ammonium chloride (608 mg), ethanol (16 mL), and water (8.0 mL) was stirred at 80°C for 2.5 hours. After cooling the reaction mixture to room temperature, insoluble materials were removed by filtration through Celite. Saturated sodium bicarbonate solution was added to the filtrate, and the mixture was extracted four times with dichloromethane, twice with ethyl acetate, and twice with a mixture of ethyl acetate/tetrahydrofuran (4: 1). The combined organic layer was dried over anhydrous sodium sulfate. The obtained organic layer was filtered, and the solvent was evaporated under reduced pressure to obtain the crude title compound (629 mg).

### (Step 3) Ethyl (6-chloro-4-methyl-1H-imidazo[4,5-c]pyridin-2-yl)acetate

A mixture of the compound (629 mg) obtained in Step 2 above, ethyl 3-ethoxy-3-iminopropanoate hydrochloride (1.56 g), and ethanol (20 mL) was stirred at 80°C for 4.5 hours. After cooling the reaction mixture to room temperature, the solvent was evaporated under reduced pressure, and the obtained residue was purified by silica gel column chromatography (n-hexane/ethyl acetate). The obtained product was solidified with a mixture of ethyl acetate (2 mL)/n-hexane (8 mL), collected by filtration, washed with a mixture of n-hexane/ethyl acetate (4: 1), and dried under reduced pressure to obtain the title compound (660 mg).
¹H-NMR (DMSO-D₆) δ: 12.89 (1H, br s), 7.45 (1H, s), 4.14 (2H, q, J = 7.0 Hz), 4.04 (2H, s), 2.64 (3H, s), 1.21 (3H, t, J = 7.0 Hz).

### Reference Example 30

### Production of ethyl (6-chloro-4-ethyl-1H-imidazo[4,5-c]pyridin-2-yl)acetate

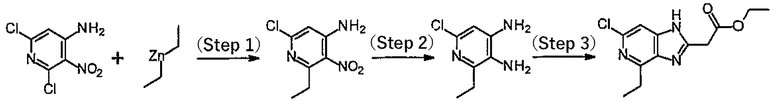

### (Step 1) 6-Chloro-2-ethyl-3-nitropyridin-4-amine

To a mixture of 2,6-dichloro-3-nitropyridin-4-amine (4.00 g), [1,1'-bis(diphenylphosphino)ferrocene]dichloropalladium(II) dichloromethane adduct (785 mg), and 1,4-dioxane (40 mL) was added a 1.12 M solution of diethylzinc in n-hexane (21.0 mL) at room temperature, and the mixture was stirred at 70°C for 3 hours. After cooling the reaction mixture to room temperature, saturated sodium bicarbonate solution was added, and the mixture was stirred at room temperature for 10 minutes. Insoluble materials were removed by filtration, and the filtrate was extracted with ethyl acetate. The obtained organic layer was washed with water and saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (1.87 g).
¹H-NMR (CDCl₃) δ: 6.61 (1H, s), 5.75 (2H, br s), 2.93 (2H, q, J = 7.4 Hz), 1.33 (3H, t, J = 7.4 Hz).

### (Step 2) 6-Chloro-2-ethylpyridin-3,4-diamine

To a mixed solution of the compound (1.87 g) obtained in Step 1 above in tetrahydrofuran (20 mL)/ethanol (10 mL)/water (5.0 mL) was added iron powder (1.55 g) at room temperature, and the mixture was heated to 70°C. Then, ammonium chloride (51.2 mg) was added, and the mixture was stirred at the same temperature for 3 hours. After cooling the reaction mixture to room temperature, insoluble materials were removed by filtration through Celite, and the filtrate was concentrated under reduced pressure and dried to obtain the crude title compound (1.61 g).

### (Step 3) Ethyl (6-chloro-4-ethyl-1H-imidazo[4,5-c]pyridin-2-yl)acetate

A mixture of the compound (1.61 g) obtained in Step 2 above, ethyl 3-ethoxy-3-iminopropanoate hydrochloride (3.63 g), and ethanol (25 mL) was stirred at 80°C for 9 hours. After cooling the reaction mixture to room temperature, 10% citric acid solution was added, and the mixture was stirred at room temperature for several minutes. Subsequently, saturated sodium bicarbonate solution was added to the mixture, and the mixture was extracted twice with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate). The obtained oily substance was solidified with a mixture of ethyl acetate (2.0 mL)/n-hexane (20 mL), collected by filtration, washed with a mixture of n-hexane/ethyl acetate (10:1), and dried under reduced pressure to obtain the title compound (1.91 g).
¹H-NMR (DMSO-D₆) δ: 13.21-12.74 (1H, m), 7.44 (1H, br s), 4.15 (2H, q, J = 7.0 Hz), 4.05 (2H, s), 3.09-2.92 (2H, m), 1.28 (3H, t, J = 7.4 Hz), 1.21 (3H, t, J = 7.0 Hz).

### Reference Example 31

### Production of ethyl [6-chloro-4-(propan-2-yl)-1H-imidazo[4,5-c]pyridin-2-yl]acetate

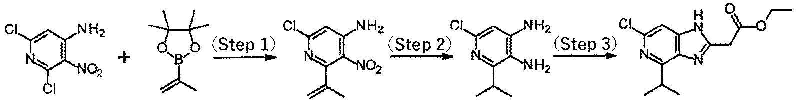

### (Step 1) 6-Chloro-3-nitro-2-(prop-1-en-2-yl)pyridin-4-amine

A mixture of isopropenylboronic acid pinacol ester (677 µL), 2,6-dichloro-3-nitropyridin-4-amine (624 mg), [1,1'-bis(diphenylphosphino)ferrocene]dichloropalladium(II) dichloromethane adduct (86 mg), potassium carbonate (829 mg), 1,4-dioxane (12 mL), and water (4.0 mL) was stirred at 90°C for 6 hours under a nitrogen atmosphere. After cooling the reaction mixture to room temperature, insoluble materials were removed by filtration through Celite. The filtrate was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (356 mg).
¹H-NMR (CDCl₃) δ: 6.65 (1H, s), 5.64 (2H, s), 5.21 (1H, s), 5.06 (1H, s), 2.16 (3H, s).

### (Step 2) 6-Chloro-2-(propan-2-yl)pyridin-3,4-diamine

A mixture of the compound (354 mg) obtained in Step 1 above, platinum(IV) oxide (56 mg), and ethanol (17 mL) was stirred at 50°C for 6 hours under a hydrogen atmosphere. After cooling the reaction mixture to room temperature, insoluble materials were removed by filtration through Celite, and the solvent was evaporated under reduced pressure to obtain the crude title compound.

### (Step 3) Ethyl [6-chloro-4-(propan-2-yl)-1H-imidazo[4,5-c]pyridin-2-yl]acetate

The title compound (338 mg) was obtained by using the compound obtained in Step 2 above as a production material and performing the same operation as in Step 3 of Reference Example 25.
¹H-NMR (DMSO-D₆) δ: 12.87 (1H, s), 7.43 (1H, s), 4.15 (2H, q, J = 7.3 Hz), 4.05 (2H, s), 3.71-3.57 (1H, m), 1.29 (6H, d, J = 7.3 Hz), 1.21 (3H, t, J = 7.3 Hz).

### Reference Example 32

### Production of 6-chloro-3-nitro-2-propylpyridin-4-amine

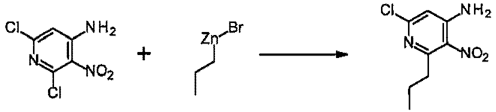

### 6-Chloro-3-nitro-2-propylpyridin-4-amine

To a mixture of 2,6-dichloro-3-nitropyridin-4-amine (624 mg), bis(triphenylphosphine)palladium(II) dichloride (211 mg), and tetrahydrofuran (7.5 mL) was added a 0.5 M solution of propylzinc bromide in tetrahydrofuran (12 mL) at room temperature, and the mixture was stirred at 70°C for 5.5 hours under a nitrogen atmosphere. After cooling the reaction mixture to room temperature, the mixture was concentrated under reduced pressure, and the obtained residue was purified by silica gel column chromatography (n-hexane/ethyl acetate) and (n-hexane/dichloromethane) to obtain the title compound (395 mg).
¹H-NMR (CDCl₃) δ: 6.60 (1H, s), 5.69 (2H, s), 2.90-2.83 (2H, m), 1.82-1.71 (2H, m), 1.00 (3H, t, J = 7.3 Hz).

### Reference Example 33

### Production of 6-chloro-2-cyclopropyl-3-nitropyridin-4-amine

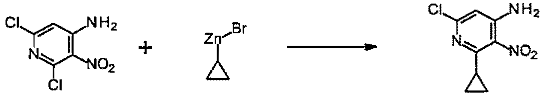

### 6-Chloro-2-cyclopropyl-3 -nitropyridin-4-amine

The title compound was obtained by using a 0.5 M solution of cyclopropylzinc bromide in tetrahydrofuran as a production material and performing the same operation as in Reference Example 32.
¹H-NMR (CDCl₃) δ: 6.47 (1H, s), 5.60 (2H, s), 2.48-2.39 (1H, m), 1.24-1.19 (2H, m), 1.09-1.03 (2H, m).

### Reference Example 34

### Production of 2-(4-amino-6-chloro-3-nitropyridin-2-yl)-N-tert-butylacetamide

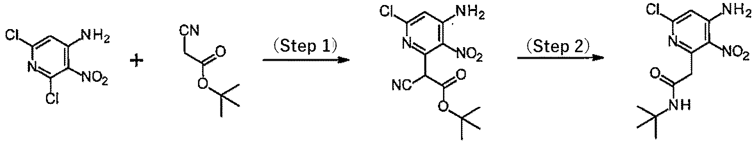

### (Step 1) tert-Butyl (4-amino-6-chloro-3-nitropyridin-2-yl)(cyano)acetate

To a solution of 2,6-dichloro-3-nitropyridin-4-amine (500 mg) in N,N-dimethylformamide (8.0 mL) were added tert-butyl cyanoacetate (343 µL) and potassium carbonate (664 mg) at room temperature, and the mixture was stirred at 80°C for 1 hour. The reaction mixture was cooled to room temperature, 1 M hydrochloric acid was added, and the mixture was extracted twice with ethyl acetate. The obtained organic layer was washed with water and saturated sodium chloride solution, and then dried over anhydrous sodium sulfate. The obtained organic layer was filtered, and the solvent was evaporated under reduced pressure to obtain the crude title compound (752 mg).

### (Step 2) 2-(4-Amino-6-chloro-3-nitropyridin-2-yl)-N-tert-butylacetamide

To a solution of the compound (752 mg) obtained in Step 1 above in dichloromethane (5.0 mL) was added trifluoroacetic acid (2.50 mL) at room temperature, and the mixture was stirred at the same temperature for 20 hours. The reaction mixture was concentrated under reduced pressure, saturated sodium bicarbonate solution was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (132 mg).
¹H-NMR (CDCl₃) δ: 6.67 (1H, s), 6.29 (2H, br s), 5.64 (1H, br s), 3.97 (2H, s), 1.39 (9H, s).

The following compounds were synthesized by performing the same operations as in Steps 2 and 3 of Reference Example 25 (Table 4-1).

**Table 4-1**

| Reference Example No. | Production Material | Structure of Compound Synthesized | Name of Compound Synthesized | Spectral Data |
|---|---|---|---|---|
| 35 | 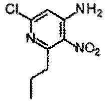 Reference Example 32 | 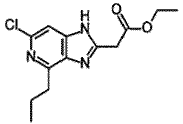 | ethyl (6-chloro-4-propyl-1H-imidazo[4,5-c]pyridin-2-yl)acetate | ¹H-NMR (DMSO-D₆) δ: 12.87 (1H, s), 7.44 (1H, s), 4.14 (2H, q, J = 7.0 Hz), 4.04 (2H, s), 3.02-2.88 (2H, m), 1.83-1.69 (2H, m), 1.21 (3H, t, J = 7.0 Hz), 0.93 (3H, t, J = 7.3 Hz). |
| 36 | 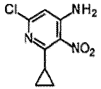 Reference Example 33 | 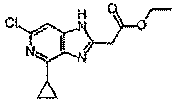 | ethyl (6-chloro-4-cyclopropyl-1H-imidazo[4,5-c]pyridin-2-yl)acetate | ¹H-NMR (DMSO-D₆) δ: 12.85 (1H, s), 7.33 (1H, s), 4.15 (2H, q, J = 7.0 Hz), 4.05 (2H, s), 2.73-2.57 (1H, m), 1.21 (3H, t, J = 7.0 Hz), 1.07 (4H, d, J = 5.5 Hz). |
| 37 | 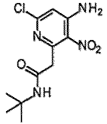 Reference Example 34 | 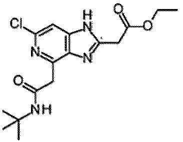 | ethyl {4-[2-(tert-butylamino)-2-oxoethyl]-6-chloro-1H-imidazo[4,5-c]pyridin-2-yl}acetate | ¹H-NMR (CDCl₃) δ: 11.70-10.49 (1H, m), 7.62-7.31 (1H, m), 6.13 (1H, br s), 4.38-4.29 (2H, m), 4.12-4.08 (2H, m), 4.08-3.86 (2H, m), 1.38-1.33 (9H, m). |

### Reference Example 38

### Production of ethyl [6-chloro-4-(1-hydroxyethyl)-1H-benzimidazol-2-yl]acetate

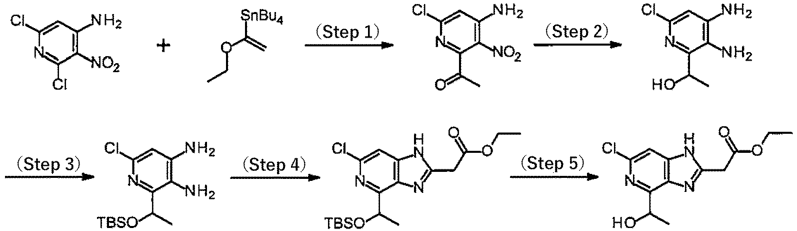

### (Step 1) 1-(4-Amino-6-chloro-3-nitropyridin-2-yl)ethanone

To a solution of 2,6-dichloro-3-nitropyridin-4-amine (2.75 g) in N,N-dimethylformamide (25 mL) were added tributyl(1-ethoxyvinyl)tin (4.60 mL) and bis(triphenylphosphine)palladium(II) chloride (232 mg) at room temperature, and the mixture was stirred at 90°C for 3.5 hours. The reaction mixture was cooled to room temperature, water was added, and the mixture was extracted three times with ethyl acetate. The obtained organic layer was washed twice with water and once with saturated sodium chloride solution, and then dried over anhydrous sodium sulfate. The obtained organic layer was filtered, and the solvent was evaporated under reduced pressure. 1,4-Dioxane (25 mL) and 2 M hydrochloric acid (30 mL) were added to the obtained residue, and the mixture was stirred at room temperature for 1 hour. A 1 M aqueous solution of sodium hydroxide (60 mL) was added to the reaction mixture at 0°C, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was solidified with a mixture of n-hexane/ethyl acetate/dichloromethane (10:1:1). The precipitated solid was collected by filtration, washed with a mixture of n-hexane/ethyl acetate/dichloromethane (10:1:1), and dried under reduced pressure to obtain the crude title compound (2.24 g).

### (Step 2) 1-(3,4-Diamino-6-chloropyridin-2-yl)ethanol

To a solution of the compound (2.24 g) obtained in Step 1 above in ethanol (40 mL) was added sodium borohydride (1.18 g) at 0°C, and the mixture was stirred at room temperature for 1 hour. Then, sodium borohydride (590 mg) was added again, and the mixture was stirred at the same temperature for another 1 hour. Water was added to the reaction mixture, and insoluble materials were removed by filtration through Celite. The filtrate was extracted three times with ethyl acetate. The obtained organic layer was washed with water and saturated sodium chloride solution, and then dried over anhydrous sodium sulfate. The obtained organic layer was filtered, and the solvent was evaporated under reduced pressure to obtain the crude title compound (1.55 g).

### (Step 3) 2-(1-{[tert-Butyl(dimethyl)silyl]oxy}ethyl)-6-chloropyridin-3,4-diamine

To a solution of the compound (1.55 g) obtained in Step 2 above in N,N-dimethylformamide (21 mL) were added imidazole (1.55 g) and tert-butyldimethylchlorosilane (3.50 g) at room temperature, and the mixture was stirred at the same temperature for 16 hours. Ice was added to the reaction mixture, and the mixture was stirred at room temperature for 30 minutes. The precipitated solid was collected by filtration, washed twice with water and twice with hexane, and then dried under reduced pressure to obtain the crude title compound (1.64 g).

### (Step 4) Ethyl [4-(1-{[tert-butyl(dimethyl)silyl]oxy}ethyl)-6-chloro-1H-imidazo[4,5-c]pyridin-2-yl]acetate

A mixture of the compound (1.64 g) obtained in Step 3 above, ethyl 3-ethoxy-3-iminopropanoate hydrochloride (2.13 g), and ethanol (14 mL) was stirred under reflux for 2.5 hours. After cooling the reaction mixture to room temperature, the solvent was evaporated under reduced pressure, and the obtained residue was purified by silica gel column chromatography (n-hexane/ethyl acetate) to obtain the title compound (1.47 g).
¹H-NMR (CDCl₃) δ: 10.86 (1H, br s), 7.50 (1H, s), 5.23 (1H, q, J = 6.1 Hz), 4.30 (2H, q, J = 7.3 Hz), 4.09 (2H, s), 1.57-1.51 (3H, m), 1.34 (3H, t, J = 7.3 Hz), 0.92 (9H, s), 0.18 (3H, s), 0.10 (3H, s).

### (Step 5) Ethyl [6-chloro-4-(1-hydroxyethyl)-1H-benzimidazol-2-yl]acetate

To a solution of the compound (1.47 g) obtained in Step 4 above in 1,4-dioxane (12 mL) was added 6 M hydrochloric acid (3.0 mL) at room temperature, and the mixture was stirred at the same temperature for 2.5 hours. A 1 M aqueous solution of sodium hydroxide (19 mL) was added to the reaction mixture at 0°C, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The residue was solidified with a mixture of n-hexane/ethyl acetate (2:1), and then the precipitated solid was collected by filtration. The obtained solid was suspended in ethyl acetate, insoluble materials were removed by filtration, and the filtrate was concentrated under reduced pressure to obtain the title compound (836 mg).
¹H-NMR (CDCl₃) δ: 11.05-10.45 (1H, m), 7.57-7.32 (1H, m), 5.47-5.24 (1H, m), 4.39-4.24 (2H, m), 4.10 (2H, s), 1.73-1.60 (3H, m), 1.42-1.26 (3H, m).

### Reference Example 39

### Production of tert-butyl 6-chloro-2-(2-ethoxy-2-oxoethyl)-4-(1-fluoroethyl)-1H-imidazo[4,5-c]pyridin-1-carboxylate

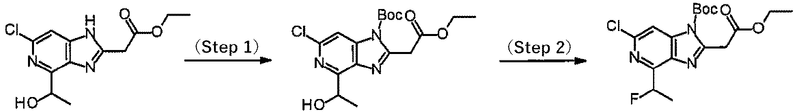

### (Step 1) tert-Butyl 6-chloro-2-(2-ethoxy-2-oxoethyl)-4-(1-hydroxyethyl)-1H-imidazo[4,5-c]pyridin-1-carboxylate

To a solution of the compound (60.0 mg) obtained in Step 5 of Reference Example 38 in tetrahydrofuran (1.0 mL) were added di-tert-butyl dicarbonate (51.0 mg) and 4-dimethylaminopyridine (5.0 mg) at room temperature, and the mixture was stirred at the same temperature for 1 hour. The reaction mixture was concentrated under reduced pressure, and the obtained residue was purified by silica gel column chromatography (n-hexane/ethyl acetate) to obtain the title compound (71.9 mg).
¹H-NMR (CDCl₃) δ: 7.76 (1H, s), 5.43-5.31 (1H, m), 4.27 (2H, s), 4.21 (2H, q, J = 7.1 Hz), 1.69 (9H, s), 1.64 (3H, d, J = 6.7 Hz), 1.27 (3H, t, J = 7.1 Hz).

### (Step 2) tert-Butyl 6-chloro-2-(2-ethoxy-2-oxoethyl)-4-(1-fluoroethyl)-1H-imidazo[4,5-c]pyridin-1-carboxylate

To a solution of the compound (71 mg) obtained in Step 1 above in dichloromethane (1 mL) was added bis(2-methoxyethyl)aminosulfur trifluoride (80 µL) at -78°C, and the mixture was warmed to 0°C and stirred for 3 hours. The reaction mixture was purified by silica gel column chromatography (n-hexane/ethyl acetate) to obtain the title compound (59 mg).
¹H-NMR (CDCl₃) δ: 7.83 (1H, d, J = 1.2 Hz), 6.20 (1H, dq, J = 47.3, 6.7 Hz), 4.29 (2H, s), 4.21 (2H, q, J = 7.1 Hz), 1.84 (3H, dd, J = 23.8, 6.7 Hz), 1.69 (9H, s), 1.27 (3H, t, J = 7.1 Hz).

### Reference Example 40

### Production of ethyl {4-[{4-[(tert-butoxycarbonyl)(methyl)amino]butyl}(methyl)amino]-6-chloro-1H-imidazo[4,5-c]pyridin-2-yl}acetate

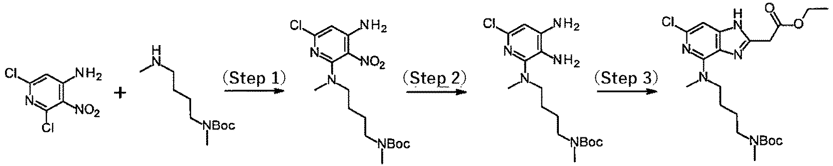

### (Step 1) tert-Butyl {4-[(4-amino-6-chloro-3-nitropyridin-2-yl)(methyl)amino]butyl}methylcarbamate

To a solution of tert-butyl N-methyl-N-[4-(methylamino)butyl]carbamate (1.70 g) and 2,6-dichloro-3-nitropyridin-4-amine (1.60 g) in N,N-dimethylformamide (38 mL) was added potassium carbonate (3.20 g) at room temperature, and the mixture was stirred at the same temperature for 6 hours. Water was added to the reaction mixture, and the mixture was extracted twice with ethyl acetate. The obtained organic layer was washed three times with water and once with saturated sodium chloride solution, and then dried over anhydrous sodium sulfate. The obtained organic layer was filtered, and the solvent was evaporated under reduced pressure to obtain the crude title compound (3.00 g).

### (Step 2) tert-Butyl {4-[(3,4-diamino-6-chloropyridin-2-yl)(methyl)amino]butyl}methylcarbamate

A mixture of the compound (3.00 g) obtained in Step 1 above, iron powder (2.23 g), ammonium chloride (215 mg), ethanol (39 mL), and water (19 mL) was stirred at 90°C for 1.5 hours. After cooling the reaction mixture to room temperature, insoluble materials were removed by filtration through Celite. Saturated sodium bicarbonate solution was added to the filtrate, and the mixture was extracted three times with ethyl acetate. The obtained organic layer was washed with water and saturated sodium chloride solution, and then dried over anhydrous sodium sulfate. The obtained organic layer was filtered, and the solvent was evaporated under reduced pressure to obtain the crude title compound (2.85 g).

### (Step 3) Ethyl {4-[{4-[(tert-butoxycarbonyl)(methyl)amino]butyl}(methyl)amino]-6-chloro-1H-imidazo[4,5-c]pyridin-2-yl}acetate

A mixture of the compound (2.80 g) obtained in Step 2 above, ethyl 3-ethoxy-3-iminopropanoate hydrochloride (3.10 g), ethanol (20 mL), and acetic acid (4.5 mL) was stirred at 100°C for 3.5 hours. The reaction mixture was cooled to room temperature, saturated sodium bicarbonate solution was added, and the mixture was extracted three times with ethyl acetate. The obtained organic layer was washed with water and saturated sodium chloride solution, and then dried over anhydrous sodium sulfate. The obtained organic layer was filtered, and the solvent was evaporated under reduced pressure. The residue was purified by silica gel column chromatography (n-hexane/ethyl acetate) to obtain the title compound (2.30 g).
¹H-NMR (CDCl₃) δ: 10.20 (1H, br s), 6.66 (1H, s), 4.28 (2H, q, J = 7.2 Hz), 4.06-3.98 (4H, m), 3.37 (3H, br s), 3.27 (2H, br s), 2.83 (3H, s), 1.70-1.62 (2H, m), 1.62-1.54 (2H, m), 1.46 (9H, s), 1.34 (3H, t, J = 7.2 Hz).

The following compounds were synthesized by performing the same operations as in Steps 1 to 3 of Reference Example 40 (Table 5-1).

**Table 5-1**

| Reference Example No. | Production Material | Structure of Compound Synthesized | Name of Compound Synthesized | Spectral Data |
|---|---|---|---|---|
| 41 | 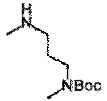 | 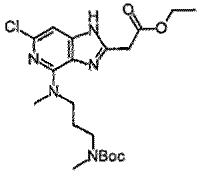 | ethyl {4-[{3-[(tert-butoxycarbonyl)(methyl) amino]propyl)(methyl) amino]-6-chloro-1H-imidazo[4,5-c]pyridin-2-yl} acetate | ¹H-NMR (CDCl₃) δ: 9.96 (1H, br s), 6.64 (1H, s), 4.27 (2H, q, J = 7.1 Hz), 3.98 (2H, s), 4.09-3.98 (2H, m), 3.36 (3H, s), 3.29 (2H, br s), 2.87 (3H, s), 1.92-1.85 (2H, m), 1.44 (9H, br s), 1.33 (3H, t, J = 7.1 Hz). |
| 42 | 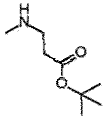 | 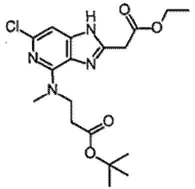 | tert-butyl N-[6-chloro-2-(2-ethoxy-2-oxoethyl)-1H-imidazo[4,5-c]pyridin-4-yl]-N-methyl-beta-alaninate | ¹H-NMR (CDCl₃) δ: 9.95 (1H, br s), 6.65 (1H, s), 4.30-4.23 (4H, m), 3.96 (2H, s), 3.41 (3H, s), 2.62 (2H, t, J = 7.0 Hz), 1.42 (9H, s), 1.33 (3H, t, J = 7.3 Hz). |
| 43 | 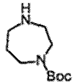 | 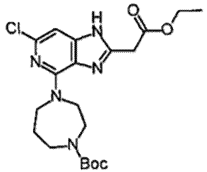 | tert-butyl 4-[6-chloro-2-(2-ethoxy-2-oxoethyl)-1H-imidazo[4,5-c]pyridin-4-yl]-1,4-diazepan-1-carboxylate | ¹H-NMR (CDCl₃) δ: 9.97 (1H, br s), 6.65 (1H, s), 4.27 (2H, q, J = 7.2 Hz), 4.29-4.23 (1H, m), 4.18-4.08 (2H, m), 4.01 (1H, t, J = 5.8 Hz), 3.96 (2H, s), 3.66-3.58 (2H, m), 3.36 (1H, t, J = 5.8 Hz), 3.27 (1H, t, J = 6.1 Hz), 2.07-2.01 (2H, m), 1.47-1.35 (9H, m), 1.33 (3H, t, J = 7.2 Hz). |
| 44 | 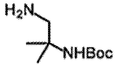 | 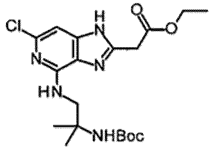 | ethyl [4-({2-[(tert-butoxycarbonyl)amino]-2-methylpropyl}amino)-6-chloro-1H-imidazo[4,5-c]pyridin-2-yl]acetate | ¹H-NMR (CDCl₃) δ: 10.17 (1H, s), 6.70 (1H, s), 5.99 (1H, br s), 5.76 (1H, br s), 4.28 (2H, q, J = 7.1 Hz), 4.00 (2H, s), 3.67 (2H, d, J = 6.7 Hz), 1.43 (9H, s), 1.39 (6H, s), 1.34 (3H, t, J = 7.1 Hz). |
| 45 | 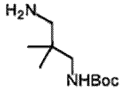 | 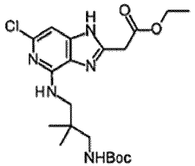 | ethyl [4-({3-[(tert-butoxycarbonyl)amino]-2,2-dimethylpropyl}amino)-6-chloro-1H-imidazo[4,5-c]pyridin-2-yl]acetate | ¹H-NMR (CDCl₃) δ: 10.15 (1H, br s), 6.70 (1H, s), 6.69 (1H, br s), 5.60 (1H, br s), 4.28 (2H, q, J = 7.2 Hz), 4.01 (2H, s), 3.39 (2H, d, J = 7.4 Hz), 2.90 (2H, d, J = 6.7 Hz), 1.47 (9H, s), 1.34 (3H, t, J = 7.2 Hz), 0.95 (6H, s). |

### Reference Example 46

### Production of tert-butyl (3-formyl-2-methylpyridin-4-yl)carbamate

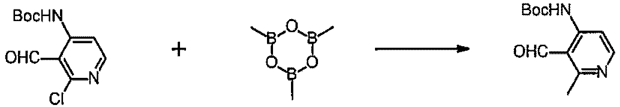

### tert-Butyl (3-formyl-2-methylpyridin-4-yl)carbamate

To a solution of tert-butyl 2-chloro-3-formylpyridin-4-ylcarbamate (4.00 g) in 1,2-dimethoxyethane (30 mL) were added a 50% solution of trimethylboroxine in tetrahydrofuran (13.4 mL), potassium carbonate (6.46 g), and [1,1'-bis(diphenylphosphino)ferrocene]dichloropalladium(II) dichloromethane adduct (318 mg) at room temperature, and the mixture was stirred under reflux for 3 hours under a nitrogen atmosphere. The reaction mixture was cooled to room temperature, insoluble materials were removed by filtration through Celite, and then the filtrate was concentrated under reduced pressure. The obtained residue was purified by silica gel column chromatography (n-hexane/ethyl acetate) to obtain the title compound (3.26 g).
¹H-NMR (CDCl₃) δ: 10.98 (1H, br s), 10.46 (1H, s), 8.43 (1H, d, J = 6.1 Hz), 8.25 (1H, d, J = 6.1 Hz), 2.85 (3H, s), 1.54 (9H, s).

### Reference Example 47

### Production of 4-amino-2,6-dimethylpyridin-3-carbaldehyde

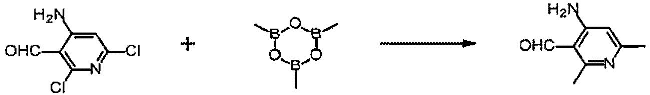

### 4-Amino-2,6-dimethylpyridin-3-carbaldehyde

The title compound was obtained by using 4-amino-2,6-dichloropyridin-3-carbaldehyde and a 50% solution of trimethylboroxine in tetrahydrofuran as production materials and performing the same operation as in Reference Example 46.
¹H-NMR (CDCl₃) δ: 10.36 (1H, s), 6.26 (1H, s), 2.72 (3H, s), 2.39 (3H, s).

### Reference Example 48

### Production of tert-butyl (2-ethyl-3-formylpyridin-4-yl)carbamate

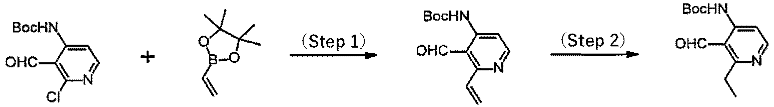

### (Step 1) tert-Butyl (2-ethenyl-3-formylpyridin-4-yl)carbamate

A mixture of tert-butyl 2-chloro-3-formylpyridin-4-ylcarbamate (2.00 g), 4,4,5,5-tetramethyl-2-vinyl-1,3,2-dioxaborolane (1.47 mL), cesium carbonate (7.68 g), [1,1'-bis(diphenylphosphino)ferrocene]dichloropalladium(II) dichloromethane adduct (318 mg), 1,4-dioxane (24 mL), and water (6.0 mL) was stirred under reflux for 2.5 hours under a nitrogen atmosphere. The reaction mixture was cooled to room temperature, water was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (1.60 g).
¹H-NMR (CDCl₃) δ: 10.97 (1H, br s), 10.50 (1H, s), 8.56 (1H, d, J = 6.1 Hz), 8.32 (1H, d, J = 6.1 Hz), 7.33 (2H, dd, J = 17.1, 10.7 Hz), 6.27 (1H, dd, J = 17.1, 1.8 Hz), 5.83 (1H, dd, J = 10.7, 1.8 Hz), 1.57 (9H, s).

### (Step 2) tert-Butyl (2-ethyl-3-formylpyridin-4-yl)carbamate

To a solution of the compound (2.55 g) obtained in Step 1 above in ethyl acetate (35 mL) was added 10%-palladium on carbon (50% water content, 273 mg) at room temperature, and the mixture was stirred at the same temperature for 2 hours under a hydrogen atmosphere. Insoluble materials were removed by filtration through Celite, and the solvent was evaporated under reduced pressure. The obtained residue was purified by silica gel column chromatography (n-hexane/ethyl acetate) to obtain the title compound (2.30 g).
¹H-NMR (CDCl₃) δ: 10.98 (1H, br s), 10.43 (1H, s), 8.46 (1H, d, J = 6.1 Hz), 8.23 (1H, d, J = 6.1 Hz), 3.15 (2H, q, J = 7.6 Hz), 1.53 (9H, s), 1.35 (3H, t, J = 7.6 Hz).

### Reference Example 49

### Production of tert-butyl [3-formyl-2-(1-methylcyclopropyl)pyridin-4-yl]carbamate

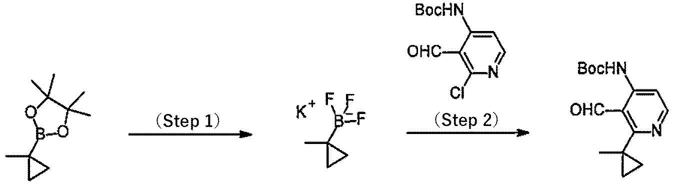

### (Step 1) Potassium trifluoro(1-methylcyclopropyl)borate(1-)

To a mixed solution of 4,4,5,5-tetramethyl-2-(1-methylcyclopropyl)-1,3,2-dioxaborolane (383 mg) in acetonitrile (6.0 mL)/water (0.9 mL) was added potassium hydrogen difluoride (510 mg) at room temperature, and the mixture was stirred at the same temperature for 4.5 hours. The reaction mixture was concentrated under reduced pressure. Diethyl ether was added to the residue, and the precipitated solid was collected by filtration. The obtained solid was washed with diethyl ether and methanol to obtain the crude title compound (341 mg).

### (Step 2) tert-Butyl [3-formyl-2-(1-methylcyclopropyl)pyridin-4-yl]carbamate

To a mixed suspension of the compound (287 mg) obtained in Step 1 above and tert-butyl 2-chloro-3-formylpyridin-4-ylcarbamate (350 mg) in toluene (8.0 mL)/water (2.0 mL) were added cesium carbonate (1.33 g) and [1,1'-bis(diphenylphosphino)ferrocene]dichloropalladium(II) dichloromethane adduct (111 mg) at room temperature, and the mixture was stirred at 100°C for 4 hours under a nitrogen atmosphere. After cooling the reaction mixture to room temperature, the mixture was concentrated under reduced pressure. The obtained residue was purified by silica gel column chromatography (n-hexane/ethyl acetate) to obtain the title compound (119 mg).
¹H-NMR (CDCl₃) δ: 10.97 (1H, br s), 10.90 (1H, s), 8.47 (1H, d, J = 6.1 Hz), 8.25 (1H, d, J = 6.1 Hz), 1.55 (9H, s), 1.53 (3H, s), 1.11 (2H, dd, J = 6.1, 4.3 Hz), 0.94 (2H, dd, J = 6.1, 4.3 Hz).

### Reference Example 50

### Production of tert-butyl [3-formyl-2-(tetrahydro-2H-pyran-4-yl)pyridin-4-yl]carbamate

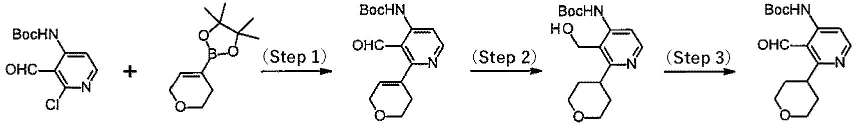

### (Step 1) tert-Butyl [2-(3,6-dihydro-2H-pyran-4-yl)-3-formylpyridin-4-yl]carbamate

The title compound was obtained by using 4-(4,4,5,5-tetramethyl-1,3,2-dioxaborolan-2-yl)-3,6-dihydro-2H-pyran as a production material and performing the same operation as in Step 1 of Reference Example 48.
¹H-NMR (CDCl₃) δ: 10.85 (1H, br s), 10.15 (1H, s), 8.53 (1H, d, J = 6.1 Hz), 8.28 (1H, d, J = 6.1 Hz), 5.79 (1H, br s), 4.40-4.32 (2H, m), 3.98 (2H, t, J = 5.5 Hz), 2.74-2.65 (2H, m), 1.55 (9H, s).

### (Step 2) tert-Butyl [3-(hydroxymethyl)-2-(tetrahydro-2H-pyran-4-yl)pyridin-4-yl]carbamate

To a solution of the compound (660 mg) obtained in Step 1 above in ethanol (20 mL) was added 10%-palladium on carbon (50% water content, 300 mg) at room temperature, and the mixture was stirred at 50°C for 4.5 hours under a hydrogen atmosphere. The reaction mixture was cooled to room temperature, insoluble materials were removed by filtration through Celite, and then the solvent was evaporated under reduced pressure. The obtained residue was purified by silica gel column chromatography (n-hexane/ethyl acetate) to obtain the title compound (250 mg).
¹H-NMR (CDCl₃) δ: 8.42 (1H, d, J = 5.5 Hz), 7.93 (1H, br s), 7.83 (1H, d, J = 5.5 Hz), 4.85 (2H, d, J = 5.5 Hz), 4.12-4.06 (2H, m), 3.55 (2H, t, J = 11.0 Hz), 3.22-3.14 (1H, m), 2.18-2.07 (2H, m), 1.97 (1H, t, J = 5.5 Hz), 1.63-1.57 (2H, m), 1.53 (9H, s).

### (Step 3) tert-Butyl [3-formyl-2-(tetrahydro-2H-pyran-4-yl)pyridin-4-yl]carbamate

To a solution of the compound (250 mg) obtained in Step 2 above in dichloromethane (10 mL) was added manganese dioxide (250 mg) at room temperature, and the mixture was stirred at the same temperature for 9.5 hours. Then, manganese dioxide (500 mg) was added, and the mixture was stirred at the same temperature for 9.5 hours. Subsequently, manganese dioxide (500 mg) was added again to the reaction mixture at room temperature, and the mixture was stirred at the same temperature for another 7 hours. Insoluble materials were removed by filtration through Celite, and the residue was washed with dichloromethane. The combined filtrate was concentrated under reduced pressure to obtain the title compound (220 mg).
¹H-NMR (CDCl₃) δ: 11.02 (1H, br s), 10.55 (1H, s), 8.52 (1H, d, J = 6.1 Hz), 8.25 (1H, d, J = 6.1 Hz), 4.11 (2H, dd, J = 11.3, 4.0 Hz), 3.63-3.53 (3H, m), 2.29-2.18 (2H, m), 1.74-1.67 (2H, m), 1.53 (9H, s).

### Reference Example 51

### Production of tert-butyl [3-formyl-2-(4-methylpiperazin-1-yl)pyridin-4-yl]carbamate

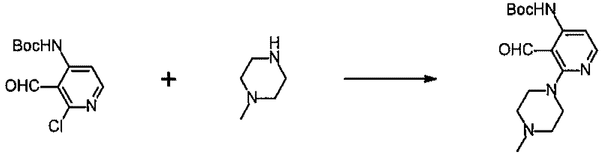

### tert-Butyl [3-formyl-2-(4-methylpiperazin-1-yl)pyridin-4-yl]carbamate

A mixture of tert-butyl 2-chloro-3-formylpyridin-4-ylcarbamate (200 mg), 1-methylpiperazine (172 µL), potassium carbonate (215 mg), and N,N-dimethylformamide (1.5 mL) was stirred at 110°C for 7.5 hours. The reaction mixture was purified by silica gel column chromatography (dichloromethane/methanol) to obtain the title compound (118 mg).
¹H-NMR (CDCl₃) δ: 10.76 (1H, br s), 9.96 (1H, s), 8.20 (1H, d, J = 6.1 Hz), 7.87 (1H, d, J = 6.1 Hz), 3.45 (4H, t, J = 4.9 Hz), 2.63-2.52 (4H, m), 2.36 (3H, s), 1.53 (9H, s).

### Reference Example 52

Production of tert-butyl {2-[4-(dimethylamino)piperidin-1-yl]-3-formylpyridin-4-yl}carbamate

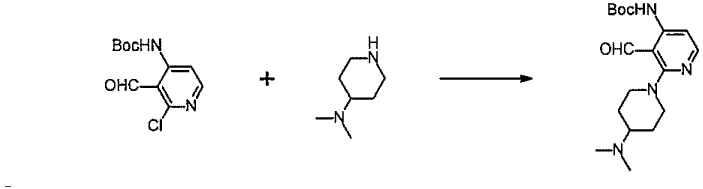

### tert-Butyl {2-[4-(dimethylamino)piperidin-1-yl]-3-formylpyridin-4-yl}carbamate

The title compound was obtained by using 4-(dimethylamino)piperidine as a production material and performing the same operation as in Reference Example 51. ¹H-NMR (CDCl₃) δ: 10.78 (1H, br s), 9.92 (1H, s), 8.18 (1H, d, J = 6.1 Hz), 7.85 (1H, d, J = 6.1 Hz), 3.82-3.72 (2H, m), 3.14-3.04 (2H, m), 2.38-2.29 (7H, m), 2.00-1.88 (2H, m), 1.71-1.61 (2H, m), 1.53 (9H, s).

### Reference Example 53

### Production of {2-[(²H₃)methyloxy]phenyl}boronic acid

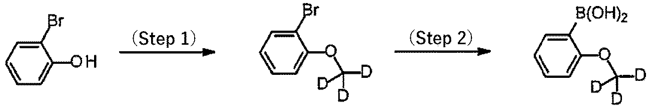

### (Step 1) 1-Bromo-2-[(²H₃)methyloxy]benzene

To a solution of 2-bromophenol (3.80 g) and iodomethane-d3 (4.78 g) in N,N-dimethylformamide (40 mL) was added sodium carbonate (4.66 g) at 0°C, and the mixture was stirred at room temperature for 18 hours. Water was added to the reaction mixture, and the mixture was extracted three times with diethyl ether. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/diethyl ether) to obtain the title compound (3.33 g).
¹H-NMR (CDCl₃) δ: 7.54 (1H, dd, J = 7.9, 1.8 Hz), 7.31-7.23 (1H, m), 6.91 (1H, dd, J = 8.5, 1.2 Hz), 6.87-6.81 (1H, m).

### (Step 2) {2-[(²H₃)Methyloxy]phenyl}boronic acid

To a solution of the compound (3.33 g) obtained in Step 1 above in tetrahydrofuran (35 mL) was added dropwise a 1.58 M solution of n-butyllithium in n-hexane (13.3 mL) at -78°C, and the mixture was stirred at 0°C for 15 minutes. Then, triisopropyl borate (5.22 mL) was added dropwise at -78°C, and the mixture was stirred at 0°C for another 1 hour. Saturated ammonium chloride solution was added to the reaction mixture, and the mixture was extracted three times with ethyl acetate. The obtained organic layer was dried over anhydrous sodium sulfate. The obtained organic layer was filtered, and the solvent was evaporated under reduced pressure to obtain the crude title compound (2.44 g).

### Reference Example 54

### Production of {2-fluoro-6-[(²H₃)methyloxy]phenyl}boronic acid

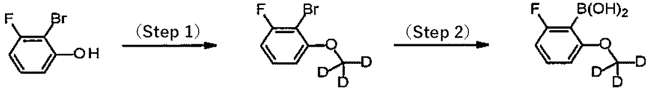

### (Steps 1 and 2) {2-Fluoro-6-[(²H₃)methyloxy]phenyl}boronic acid

The crude title compound was obtained by using 2-bromo-3-fluorophenol as a production material and performing the same operations as in Steps 1 and 2 of Reference Example 53.

### Reference Example 55

### Production of [2-fluoro-6-(methoxymethoxy)phenyl]boronic acid

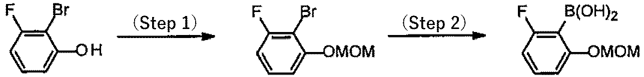

### (Step 1) 2-Bromo-1-fluoro-3-(methoxymethoxy)benzene

To a solution of 2-bromo-3-fluorophenol (1.00 g) in acetone (18 mL) were added chloro(methoxy)methane (510 µL) and potassium carbonate (1.10 g) at room temperature, and the mixture was stirred at the same temperature for 15 hours. Insoluble materials were removed by filtration through Celite, and the filtrate was concentrated under reduced pressure. The obtained residue was purified by silica gel column chromatography (n-hexane/ethyl acetate) to obtain the title compound (1.18 g).
¹H-NMR (CDCl₃) δ: 7.22 (1H, td, J = 8.4, 6.5 Hz), 6.97-6.93 (1H, m), 6.85-6.79 (1H, m), 5.27 (2H, s), 3.52 (3H, s).

### (Step 2) [2-Fluoro-6-(methoxymethoxy)phenyl]boronic acid

The title compound was obtained by using the compound obtained in Step 1 above as a production material, performing the same operation as in Step 2 of Reference Example 53, washing the obtained solid with n-hexane, and then drying under reduced pressure. ¹H-NMR (CDCl₃) δ: 7.40 (1H, ddd, J = 8.5, 7.9, 7.3 Hz), 7.00 (1H, d, J = 7.9 Hz), 6.81 (1H, dd, J = 10.4, 8.5 Hz), 6.32-6.26 (2H, m), 5.31 (2H, s), 3.52 (3H, s).

### Reference Example 56

### Production of 8-[3-(4,4,5,5-tetramethyl-1,3,2-dioxaborolan-2-yl)benzyl]-3-oxa-8-azabicyclo[3.2.1]octane

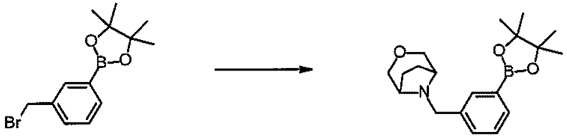

### 8-[3-(4,4,5,5-Tetramethyl-1,3,2-dioxaborolan-2-yl)benzyl]-3-oxa-8-azabicyclo[3.2.1]octane

To a solution of 2-[3-(bromomethyl)phenyl]-4,4,5,5-tetramethyl-1,3,2-dioxaborolane (151 mg) and 3-oxa-8-azabicyclo[3.2.1]octane hydrochloride (94 mg) in N,N-dimethylformamide (1.5 mL) was added potassium carbonate (212 mg) at room temperature, and the mixture was stirred at the same temperature for 30 minutes. Water was added to the reaction mixture, and the mixture was extracted twice with ethyl acetate. The obtained organic layer was dried over anhydrous sodium sulfate. The obtained organic layer was filtered, and the solvent was evaporated under reduced pressure to obtain the crude title compound (167 mg).

### Reference Example 57

### Production of tert-butyl (3-formyl-2-{2-[(²H₃)methyloxy]phenyl}pyridin-4-yl)carbamate

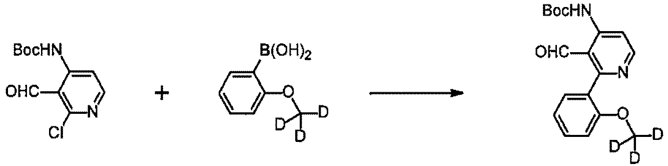

tert-Butyl (3-formyl-2-{2-[(²H₃)methyloxy]phenyl}pyridin-4-yl)carbamate

A mixture of tert-butyl 2-chloro-3-formylpyridin-4-ylcarbamate (3.85 g), the compound (2.44 g) obtained in Step 2 of Reference Example 53, sodium carbonate (3.18 g), tetrakis(triphenylphosphine)palladium(0) (867 mg), 1,4-dioxane (38 mL), and water (19 mL) was stirred at 100°C for 1 hour under a nitrogen atmosphere. After cooling the reaction mixture to room temperature, the mixture was concentrated under reduced pressure. Water was added to the residue, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (3.76 g).
¹H-NMR (DMSO-D₆) δ: 10.91 (1H, br s), 9.72 (1H, s), 8.63 (1H, d, J = 6.1 Hz), 8.36 (1H, d, J = 6.1 Hz), 7.50-7.43 (2H, m), 7.14-7.10 (1H, m), 6.96 (1H, d, J = 8.5 Hz), 1.55 (9H, s).

The following compounds were synthesized by performing the same operation as in Reference Example 57 (Table 6-1).

**Table 6-1**

| Reference Example No. | Production Material | Structure of Compound Synthesized | Name of Compound Synthesized | Spectral Data |
|---|---|---|---|---|
| 58 | 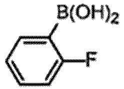 | 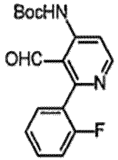 | tert-butyl [2-(2-fluorophenyl)-3-formylpyridin-4-yl] carbamate | ¹H-NMR (CDCl₃) δ: 10.94 (1H, s), 9.86 (1H, d, J = 4.3 Hz), 8.65 (1H, d, J = 6.1 Hz), 8.43 (1H, d, J = 6.1 Hz), 7.60-7.53 (1H, m), 7.53-7.46 (1H, m), 7.35-7.30 (1H, m), 7.21-7.14 (1H, m), 1.56 (9H, s). |
| 59 | 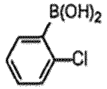 | 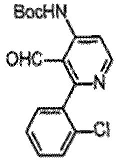 | tert-butyl [2-(2-chlorophenyl)-3-formylpyridin-4-yl] carbamate | ¹H-NMR (CDCl₃) δ: 10.94 (1H, br s), 9.73 (1H, d, J = 0.8 Hz), 8.63 (1H, dd, J = 5.9, 0.8 Hz), 8.45 (1H, dd, J = 5.9, 0.8 Hz), 7.51-7.40 (4H, m), 1.56 (9H, s). |
| 60 | 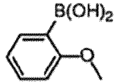 | 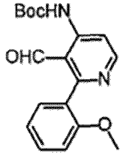 | tert-butyl [3-formyl-2-(2-methoxyphenyl)pyridin-4-yl]carbamate | ¹H-NMR (CDCl₃) δ: 10.93 (1H, s), 9.73 (1H, s), 8.64 (1H, d, J = 6.1 Hz), 8.38 (1H, d, J = 6.1 Hz), 7.48 (2H, dd, J = 15.3, 7.3 Hz), 7.13 (1H, t, J = 7.3 Hz), 6.98 (1H, d, J = 8.5 Hz), 3.75 (3H, s), 1.56 (9H, s). |
| 61 | 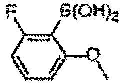 | 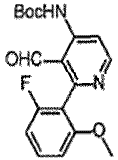 | tert-butyl [2-(2-fluoro-6-methoxyphenyl)-3-formylpyridin-4-yl] carbamate | ¹H-NMR (CDCl₃) δ: 10.92 (1H, s), 9.90 (0.1H, s), 9.78 (0.9H, s), 8.68 (0.9H, d, J = 6.1 Hz), 8.44 (0.9H, d, J = 6.1 Hz), 8.22 (0.1H, d, J = 6.1 Hz), 8.12(0.1H, d, J = 6.1 Hz), 7.47-7.38 (0.9H, m), 7.28-7.23 (0.1H, m), 6.90-6.78 (1.8H, m), 6.73-6.64 (0.2H, m), 3.79 (0.3H, s), 3.77 (2.7H, s), 1.57 (8.1H, s), 1.53 (0.9H, s). |
| 62 | 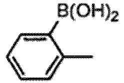 | 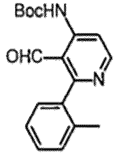 | tert-butyl [3-formyl-2-(2-methylphenyl)pyridin-4-yl]carbamate | ¹H-NMR (CDCl₃) δ: 10.97 (1H, s), 9.74 (1H, s), 8.64 (1H, d, J = 5.5 Hz), 8.42 (1H, d, J = 5.5 Hz), 7.42-7.36 (1H, m), 7.33-7.24 (3H, m), 2.18 (3H, s), 1.58 (9H, s). |
| 63 | 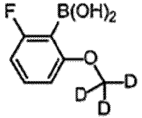 Reference Example 54 | 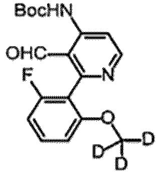 | tert-butyl (2-{2-fluoro-6-[(²H₃)methyloxy]phenyl}-3-formylpyridin-4-yl) carbamate | ¹H-NMR (CDCl₃) δ: 10.91 (1H, s), 9.77 (1H, s), 8.67 (1H, d, J = 5.5 Hz), 8.43 (1H, d, J = 5.5 Hz), 7.41 (1H, td, J = 8.5, 6.7 Hz), 6.85 (1H, t, J = 8.5 Hz), 6.79 (1H, d, J = 8.5 Hz), 1.56 (9H, s). |
| 64 | 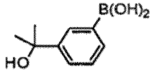 | 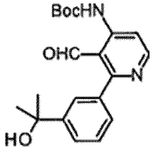 | tert-butyl {3-formyl-2-[3-(2-hydroxypropan-2-yl)phenyl]pyridin-4-yl} carbamate | ¹H-NMR (CDCl₃) δ: 10.98 (1H, s), 9.92 (1H, s), 8.63 (1H, d, J = 6.1 Hz), 8.40 (1H, d, J = 6.1 Hz), 7.71 (1H, t, J = 1.5 Hz), 7.64 (1H, dt, J = 7.9, 1.5 Hz), 7.48 (1H, t, J = 7.9 Hz), 7.38 (1H, dt, J = 7.9, 1.5 Hz), 1.72 (1H, s), 1.64 (6H, s), 1.58 (9H, s). |
| 65 | 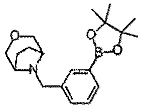 Reference Example 56 | 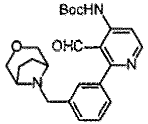 | tert-butyl {3-formyl-2-[3-(3-oxa-8-azabicyclo[3.2.1]octan-8-ylmethyl)phenyl] pyridin-4-yl}carbamate | ¹H-NMR (CDCl₃) δ: 10.97 (1H, s), 9.94 (1H, s), 8.64 (1H, d, J = 6.1 Hz), 8.40 (1H, d, J = 6.1 Hz), 7.70-7.68 (1H, m), 7.60-7.58 (1H, m), 7.57-7.56 (2H, m), 7.51-7.47 (1H, m), 3.74 (2H, d, J = 10.4 Hz), 3.56 (2H, s), 3.52 (2H, dd, J = 10.4, 1.8 Hz), 3.04 (2H, br s), 2.05-1.98 (2H, m), 1.96-1.89 (2H, m), 1.58 (9H, s). |
| 66 | 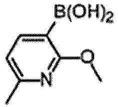 | 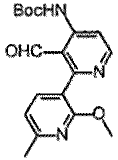 | tert-butyl (3-formyl-2'-methoxy-6'-methyl[2,3'-bipyridine]-4-yl) carbamate | ¹H-NMR (CDCl₃) δ: 10.91 (1H, s), 9.77 (1H, s), 8.63 (1H, d, J = 6.1 Hz), 8.39 (1H, d, J = 6.1 Hz), 7.76 (1H, d, J = 7.4 Hz), 6.95 (1H, d, J = 7.4 Hz), 3.90 (3H, s), 2.55 (3H, s), 1.58 (9H, s). |
| 67 | 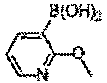 | 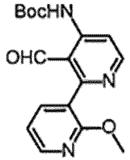 | tert-butyl (3-formyl-2'-methoxy[2,3'-pyridine]-4-yl)carbamate | ¹H-NMR (CDCl₃) δ: 10.89 (1H, s), 9.74 (1H, s), 8.62 (1H, d, J = 6.1 Hz), 8.40 (1H, d, J = 6.1 Hz), 8.31 (1H, dd, J = 5.5, 1.8 Hz), 7.85 (1H, dd, J = 7.3, 1.8 Hz), 7.09 (1H, dd, J = 7.3, 5.5 Hz), 3.90 (3H, s), 1.56 (9H, s). |
| 68 | 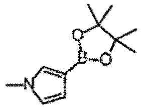 | 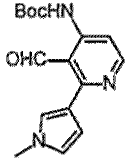 | tert-butyl [3-formyl-2-(1-methyl-1H-pyrrol-3-yl)pyridin-4-yl] carbamate | ¹H-NMR (CDCl₃) δ: 10.91 (1H, br s), 10.22 (1H, s), 8.52 (1H, d, J = 5.9 Hz), 8.17 (1H, d, J = 5.9 Hz), 6.70-6.97 (1H, m), 6.71-6.67 (1H, m), 6.44-6.40 (1H, m), 3.74 (3H, s), 1.55 (9H, s). |

### Reference Example 69

### Production of 4-amino-1'-methyl-1',2',5',6'-tetrahydro[2,3'-bipyridin]-5-carbaldehyde

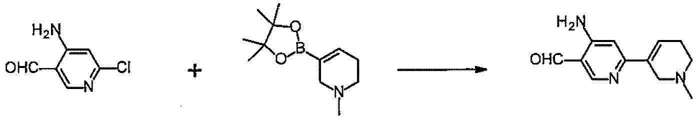

### 4-Amino-1'-methyl-1',2',5',6'-tetrahydro[2,3'-bipyridin]-5-carbaldehyde

The title compound was obtained by using 4-amino-6-chloronicotinaldehyde and 1-methyl-5-(4,4,5,5-tetramethyl-1,3,2-dioxaborolan-2-yl)-1,2,3,6-tetrahydropyridine as production materials and performing the same operation as in Reference Example 57. ¹H-NMR (CD₃OD) δ: 9.88 (1H, s), 8.49 (1H, s), 8.28 (2H, br s), 6.84 (1H, s), 6.83-6.79 (1H, m), 4.20-4.15 (2H, m), 3.44-3.35 (2H, m), 3.01 (3H, s), 2.74-2.67 (2H, m).

### Reference Example 70

### Production of methyl 3-{4-[(tert-butoxycarbonyl)amino]-3-formylpyridin-2-yl}-2-methylbenzoate

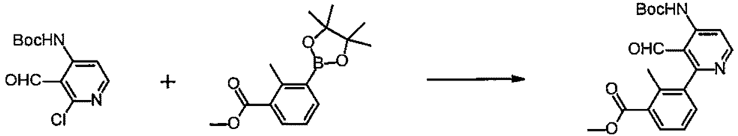

### Methyl 3-{4-[(tert-butoxycarbonyl)amino]-3-formylpyridin-2-yl}-2-methylbenzoate

A mixture of tert-butyl 2-chloro-3-formylpyridin-4-ylcarbamate (200 mg), methyl 2-methyl-3-(4,4,5,5-tetramethyl-1,3,2-dioxaborolan-2-yl)benzoate (240 mg), cesium carbonate (0.76 g), [1,1'-bis(diphenylphosphino)ferrocene]dichloropalladium(II) dichloromethane adduct (95 mg), 1,4-dioxane (10 mL), and water (5 mL) was stirred at 100°C for 1.5 hours under a nitrogen atmosphere. After cooling the reaction mixture to room temperature, water was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (240 mg).
¹H-NMR (CDCl₃) δ: 10.93 (1H, s), 9.68 (1H, s), 8.62 (1H, d, J = 6.1 Hz), 8.43 (1H, d, J = 6.1 Hz), 7.96 (1H, dd, J = 7.4, 1.8 Hz), 7.40-7.35 (2H, m), 3.92 (3H, s), 2.33 (3H, s), 1.56 (9H, s).

The following compounds were synthesized by performing the same operation as in Reference Example 70 (Table 7-1).

**Table 7-1**

| Reference Example No. | Production Material | Structure of Compound Synthesized | Name of Compound Synthesized | Spectral Data |
|---|---|---|---|---|
| 71 | 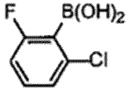 | 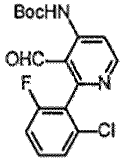 | tert-butyl [2-(2-chloro-6-fluorophenyl)-3-formylpyridin-4-yl] carbamate | ¹H-NMR (CDCl₃) δ: 10.90 (1H, s), 9.75 (1H, s), 8.68 (1H, d, J = 6.1 Hz), 8.49 (1H, d, J = 6.1 Hz), 7.46-7.32 (2H, m), 7.19-7.12 (1H, m), 1.56 (9H, s). |
| 72 | 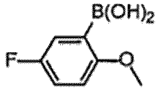 | 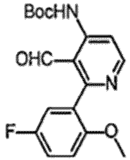 | tert-butyl [2-(5-fluoro-2-methoxyphenyl)-3-formylpyridin-4-yl] carbamate | ¹H-NMR (CDCl₃) δ: 10.91 (1H, br s), 9.74 (1H, s), 8.65 (1H, d, J = 6.1 Hz), 8.42 (1H, d, J = 6.1 Hz), 7.30-7.26 (1H, m), 7.21-7.14 (1H, m), 6.93 (1H, dd, J = 9.2, 4.3 Hz), 3.74 (3H, s), 1.58 (9H, s). |
| 73 | 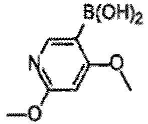 | 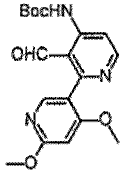 | tert-butyl (3-formyl-4',6'-dimethoxy[2,3'-bipyridine]-4-yl) carbamate | ¹H-NMR (CDCl₃) δ: 10.87 (1H, s), 9.73 (1H, s), 8.62 (1H, d, J = 5.5 Hz), 8.38 (1H, d, J = 5.5 Hz), 8.18 (1H, s), 6.28 (1H, s), 3.99 (3H, s), 3.77 (3H, s), 1.55 (9H, s). |
| 74 | 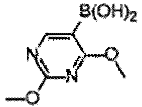 | 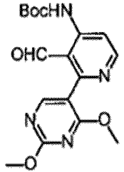 | tert-butyl [2-(2,4-dimethoxypyrimidin-5-yl)-3-formylpyridin-4-yl] carbamate | ¹H-NMR (CDCl₃) δ: 10.85 (1H, br s), 9.76 (1H, s), 8.61 (1H, d, J = 6.1 Hz), 8.50 (1H, s), 8.41 (1H, d, J = 6.1 Hz), 4.08 (3H, s), 3.96 (3H, s), 1.56 (9H, s). |
| 75 | 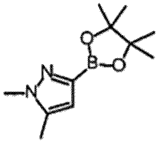 | 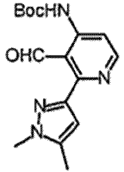 | tert-butyl [2-(1,5-dimethyl-1H-pyrazol-3-yl)-3-formylpyridin-4-yl] carbamate | ¹H-NMR (CDCl₃) δ: 10.93 (1H, s), 10.02 (1H, s), 8.58 (1H, d, J = 6.1 Hz), 8.28 (1H, d, J = 6.1 Hz), 7.52 (1H, s), 3.87 (3H, s), 2.39 (3H, s), 1.55 (9H, s). |
| 76 | 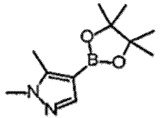 | 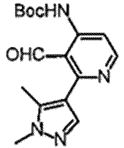 | tert-butyl [2-(1,5-dimethyl-1H-pyrazol-4-yl)-3-formylpyridin-4-yl] carbamate | ¹H-NMR (CDCl₃) δ: 10.93 (1H, br s), 10.02 (1H, s), 8.58 (1H, d, J = 6.1 Hz), 8.28 (1H, d, J = 6.1 Hz), 7.52 (1H, s), 3.87 (3H, s), 2.39 (3H, s), 1.55 (9H, s). |
| 77 | 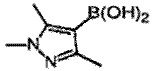 | 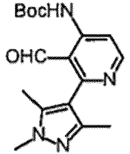 | tert-butyl [3-formyl-2-(1,3,5-trimethyl-1H-pyrazol-4-yl)pyridin-4-yl]carbamate | ¹H-NMR (CDCl₃) δ: 10.94 (1H, s), 9.85 (1H, s), 8.63 (1H, d, J = 6.1 Hz), 8.32 (1H, d, J = 6.1 Hz), 3.81 (3H, s), 2.25 (3H, s), 2.17 (3H, s), 1.57 (9H, s). |
| 78 | 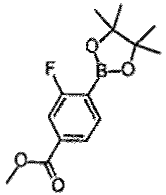 | 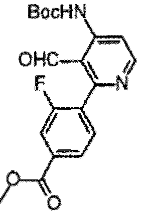 | methyl 4-{4-[(tert-butoxycarbonyl)amino]-3-formylpyridin-2-yl}-3-fluorobenzoate | ¹H-NMR (CDCl₃) δ: 10.91 (1H, s), 9.83 (1H, d, J = 4.9 Hz), 8.66 (1H, d, J = 6.1 Hz), 8.47 (1H, d, J = 6.1 Hz), 8.00 (1H, dd, J = 8.0, 1.8 Hz), 7.84 (1H, dd, J = 8.0, 1.8 Hz), 7.66 (1H, t, J = 8.0 Hz), 3.98 (3H, s), 1.56 (9H, s). |

### Reference Example 79

### Production of tert-butyl [2-(2-cyano-6-fluorophenyl)-3-formylpyridin-4-yl]carbamate

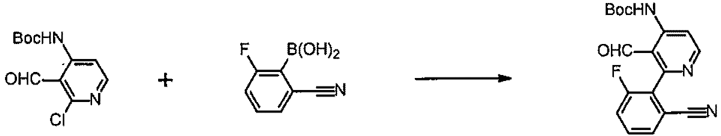

### tert-Butyl [2-(2-cyano-6-fluorophenyl)-3-formylpyridin-4-yl]carbamate

A mixture of tert-butyl 2-chloro-3-formylpyridin-4-ylcarbamate (437 mg), 2-cyano-6-fluorophenylboronic acid (312 mg), cesium fluoride (515 mg), copper(I) iodide (67.1 mg), tetrakis(triphenylphosphine)palladium(0) (401 mg), and N,N-dimethylformamide (5.6 mL) was stirred at room temperature for 1 hour under a nitrogen atmosphere. Water was added to the reaction mixture, and the mixture was extracted three times with ethyl acetate. The obtained organic layer was dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (200 mg).
¹H-NMR (CDCl₃) δ: 10.88 (1H, br s), 9.78 (1H, d, J = 3.1 Hz), 8.71 (1H, d, J = 6.1 Hz), 8.55 (1H, d, J = 6.1 Hz), 7.68 (1H, dd, J = 7.9, 1.2 Hz), 7.61 (1H, ddd, J = 8.5, 7.9, 4.9 Hz), 7.45 (1H, td, J = 8.5, 1.2 Hz), 1.56 (9H, s).

### Reference Example 80

### Production of tert-butyl {2-[2-fluoro-6-(methoxymethoxy)phenyl]-3-formylpyridin-4-yl}carbamate

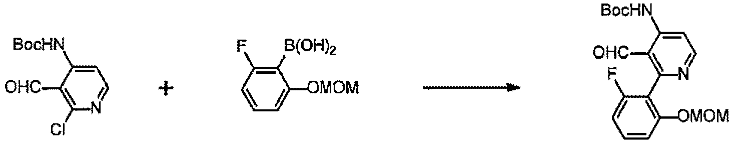

### tert-Butyl {2-[2-fluoro-6-(methoxymethoxy)phenyl]-3-formylpyridin-4-yl}carbamate

A mixture of tert-butyl 2-chloro-3-formylpyridin-4-ylcarbamate (215 mg), the compound (170 mg) obtained in Step 2 of Reference Example 55, cesium carbonate (550 mg), chloro(2-dicyclohexylphosphino-2',4',6'-triisopropyl-1,1'-biphenyl)[2-(2'-amino-1,1'-biphenyl)]palladium(II) (66.0 mg), 1,4-dioxane (8.0 mL), and water (2.0 mL) was stirred at 100°C for 1.5 hours under a nitrogen atmosphere. After cooling the reaction mixture to room temperature, the mixture was concentrated under reduced pressure. The obtained residue was purified by silica gel column chromatography (n-hexane/ethyl acetate) to obtain the title compound (225 mg).
¹H-NMR (CDCl₃) δ: 10.90 (1H, br s), 9.81 (1H, s), 8.66 (1H, d, J = 6.1 Hz), 8.43 (1H, d, J = 6.1 Hz), 7.39 (1H, td, J = 8.5, 6.7 Hz), 7.03 (1H, d, J = 8.5 Hz), 6.89 (1H, t, J = 8.5 Hz), 5.13 (1H, d, J = 7.3 Hz), 5.04 (1H, d, J = 7.3 Hz), 3.32 (3H, s), 1.55 (9H, s).

### Reference Example 81

### Production of tert-butyl {2-[3-(difluoromethoxy)-2-methylphenyl]-3-formylpyridin-4-yl}carbamate

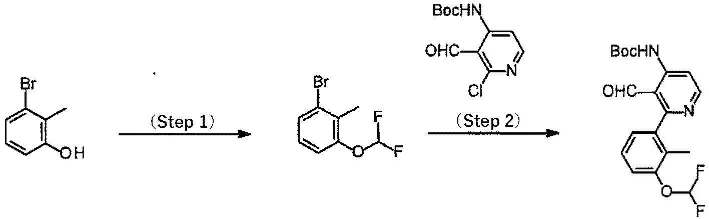

### (Step 1) 1-Bromo-3-(difluoromethoxy)-2-methylbenzene

To a solution of 3-bromo-2-methylphenol (500 mg) in N,N-dimethylformamide (10 mL) were added sodium 2-chloro-2,2-difluoroacetate (820 mg) and cesium carbonate (1.75 g) at room temperature, and the mixture was stirred at 90°C for 9.5 hours. After cooling the reaction mixture to room temperature, water was added, and the mixture was extracted three times with ethyl acetate. The obtained organic layer was washed three times with water and once with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (400 mg).
¹H-NMR (CDCl₃) δ: 7.44-7.41 (1H, m), 7.07-7.05 (2H, m), 6.49 (1H, t, J = 73.9 Hz), 2.38 (3H, s).

### (Step 2) tert-Butyl {2-[3-(difluoromethoxy)-2-methylphenyl]-3-formylpyridin-4-yl}carbamate

A mixture of the compound (400 mg) obtained in Step 1 above, bis(pinacolato)diboron (640 mg), potassium acetate (500 mg), [1,1'-bis(diphenylphosphino)ferrocene]dichloropalladium(II) dichloromethane adduct (140 mg), and 1,4-dioxane (20 mL) was stirred at 90°C for 6 hours under a nitrogen atmosphere. The reaction mixture was cooled to room temperature, and water (5.0 mL), tert-butyl 2-chloro-3-formylpyridin-4-ylcarbamate (300 mg), and cesium carbonate (1.10 g) were added. The mixture was stirred at 90°C for 2 hours under a nitrogen atmosphere. The reaction mixture was cooled to room temperature, water was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (220 mg).
¹H-NMR (CDCl₃) δ: 10.93 (1H, br s), 9.71 (1H, s), 8.62 (1H, d, J = 6.1 Hz), 8.43 (1H, d, J = 6.1 Hz), 7.31 (1H, t, J = 8.5 Hz), 7.21 (1H, d, J = 8.5 Hz), 7.13 (1H, d, J = 8.5 Hz), 6.55 (1H, t, J = 73.9 Hz), 2.08 (3H, s), 1.56 (9H, s).

### Reference Example 82

### Production of tert-butyl {2-[2-(difluoromethoxy)phenyl]-3-formylpyridin-4-yl}carbamate

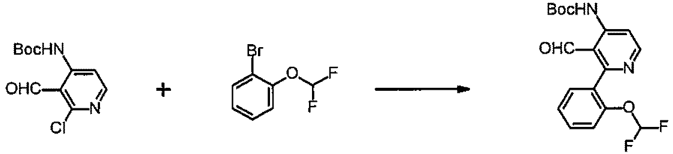

### tert-Butyl {2-[2-(difluoromethoxy)phenyl]-3-formylpyridin-4-yl}carbamate

The title compound was obtained by using 1-bromo-2-(difluoromethoxy)benzene as a production material and performing the same operation as in Step 2 of Reference Example 81.
¹H-NMR (CDCl₃) δ: 10.93 (1H, br s), 9.77 (1H, s), 8.62 (1H, d, J = 6.1 Hz), 8.43 (1H, d, J = 6.1 Hz), 7.50-7.54 (2H, m), 7.39 (1H, t, J = 7.9 Hz), 7.27 (1H, d, J = 7.9 Hz), 6.40 (1H, t, J = 76.0 Hz), 1.56 (9H, s).

### Reference Example 83

### Production of tert-butyl (6'-chloro-3-formyl-4'-methoxy[2,3'-bipyridin]-4-yl)carbamate

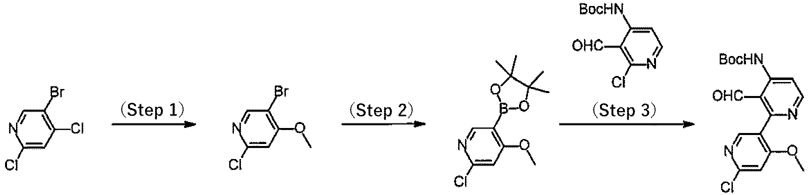

### (Step 1) 5-Bromo-2-chloro-4-methoxypyridine

To a solution of 5-bromo-2,4-dichloropyridine (45.0 g) in methanol (250 mL) was added a 1 M solution of sodium methoxide in methanol (200 mL) at 0°C, and the mixture was stirred at room temperature for 24 hours. Ice water (300 mL) was added to the reaction mixture, and the mixture was stirred for 30 minutes. The precipitated solid was collected by filtration, washed once with water and once with cold methanol, and then subjected to azeotropic removal of water with toluene three times to obtain the title compound (38.0 g). ¹H-NMR (CDCl₃) δ: 8.35 (1H, s), 6.84 (1H, s), 3.97 (3H, s).

### (Step 2) 2-Chloro-4-methoxy-5-(4,4,5,5-tetramethyl-1,3,2-dioxaborolan-2-yl)pyridine

To a solution of the compound (13.0 g) obtained in Step 1 above and 2-isopropoxy-4,4,5,5-tetramethyl-1,3,2-dioxaborolane (13.1 g) in tetrahydrofuran (240 mL) was added dropwise a 1.57 M solution of n-butyllithium in hexane (52.0 mL) at -78°C, and the mixture was stirred at the same temperature for 2 hours. Saturated ammonium chloride solution was added to the reaction mixture at -78°C. The mixture was warmed to room temperature, and then extracted three times with ethyl acetate. The obtained organic layer was washed with saturated sodium chloride solution and dried over anhydrous sodium sulfate. The obtained organic layer was filtered, and the solvent was evaporated under reduced pressure to obtain the crude title compound (15.8 g).

### (Step 3) tert-Butyl (6'-chloro-3-formyl-4'-methoxy[2,3'-bipyridin]-4-yl)carbamate

A mixture of the compound (15.8 g) obtained in Step 2 above and tert-butyl 2-chloro-3-formylpyridin-4-ylcarbamate (13.0 g), potassium carbonate (14.0 g), [1,1'-bis(diphenylphosphino)ferrocene]dichloropalladium(II) dichloromethane adduct (825 mg), 1,4-dioxane (120 mL), and water (60 mL) was stirred at 100°C for 6 hours under a nitrogen atmosphere. The reaction mixture was cooled to room temperature, water was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate). The obtained solid was washed with a mixture of n-hexane/ethyl acetate (6:1), and then dried under reduced pressure to obtain the title compound (11.0 g). ¹H-NMR (CDCl₃) δ: 10.84 (1H, s), 9.69 (1H, s), 8.63 (1H, d, J = 6.1 Hz), 8.44 (1H, d, J = 6.1 Hz), 8.39 (1H, s), 6.94 (1H, s), 3.84 (3H, s), 1.55 (9H, s).

### Reference Example 84

### Production of tert-butyl (3-formyl-4'-methoxy-6'-methyl[2,3'-bipyridin]-4-yl)carbamate

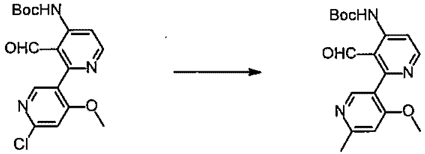

### tert-Butyl (3-formyl-4'-methoxy-6'-methyl[2,3'-bipyridin]-4-yl)carbamate

To a solution of the compound (800 mg) obtained in Step 3 of Reference Example 83 in 1,2-dimethoxyethane (12 mL) were added a 50% solution of trimethylboroxine in tetrahydrofuran (750 µL), potassium carbonate (610 mg), and [1,1'-bis(diphenylphosphino)ferrocene]dichloropalladium(II) dichloromethane adduct (90 mg) at room temperature, and the mixture was stirred under reflux for 5 hours. After cooling the reaction mixture to room temperature, insoluble materials were removed by filtration through Celite, and the filtrate was concentrated under reduced pressure. The obtained residue was purified by silica gel column chromatography (dichloromethane/methanol). The obtained solid was washed with a mixture of n-hexane/ethyl acetate (5:1), and then dried under reduced pressure to obtain the title compound (623 mg).
¹H-NMR (CDCl₃) δ: 10.90 (1H, br s), 9.73 (1H, s), 8.66 (1H, d, J = 5.5 Hz), 8.50 (1H, s), 8.42 (1H, d, J = 5.5 Hz), 6.79 (1H, s), 3.83 (3H, s), 2.65 (3H, s), 1.58 (9H, s).

### Reference Example 85

### Production of tert-butyl (6'-ethyl-3-formyl-4'-methoxy[2,3'-bipyridin]-4-yl)carbamate

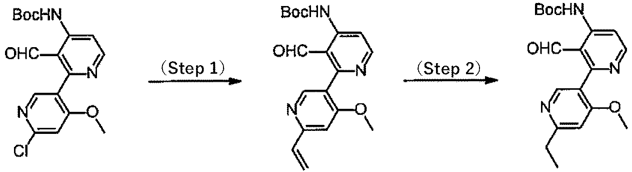

### (Steps 1 and 2) tert-Butyl (6'-ethyl-3-formyl-4'-methoxy[2,3'-bipyridin]-4-yl)carbamate

The title compound was obtained by using the compound obtained in Step 3 of Reference Example 83 as a production material and performing the same operations as in Steps 1 and 2 of Reference Example 48.
¹H-NMR (CDCl₃) δ: 10.88 (1H, br s), 9.72 (1H, s), 8.64 (1H, d, J = 6.1 Hz), 8.50 (1H, s), 8.40 (1H, d, J = 6.1 Hz), 6.76 (1H, s), 3.82 (3H, s), 2.88 (2H, q, J = 7.6 Hz), 1.55 (9H, s), 1.35 (3H, t, J = 7.6 Hz).

### Reference Example 86

### Production of tert-butyl (6'-cyclopropyl-3-formyl-4'-methoxy[2,3'-bipyridin]-4-yl)carbamate

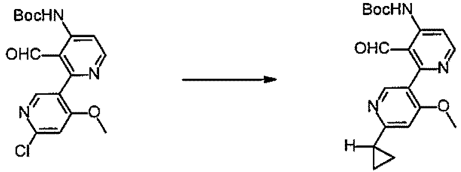

### tert-Butyl (6'-cyclopropyl-3-formyl-4'-methoxy[2,3'-bipyridin]-4-yl)carbamate

A mixture of the compound (12.2 g) obtained in Step 3 of Reference Example 83, potassium cyclopropyl trifluoroborate (6.45 g), cesium carbonate (32.8 g), palladium(II) acetate (565 mg), butyl[di(tricyclo[3.3.1.13,7]decan-1-yl)]phosphine (1.80 g), toluene (170 mL), and water (17 mL) was stirred at 100°C for 11 hours under a nitrogen atmosphere. The reaction mixture was cooled to room temperature, water was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate). A mixture of ethanol (30 mL)/water (30 mL) was added to the obtained oily substance, and the mixture was stirred at 70°C for 15 minutes. Then, the mixture was cooled to room temperature. The precipitated solid was collected by filtration, washed sequentially with water and a mixture of ethanol/water (1:2), and then dried under reduced pressure to obtain the title compound (10.6 g).
¹H-NMR (CDCl₃) δ: 10.87 (1H, br s), 9.71 (1H, s), 8.62 (1H, d, J = 6.1 Hz), 8.40 (1H, s), 8.38 (1H, d, J = 6.1 Hz), 6.76 (1H, s), 3.81 (3H, s), 2.12-2.01 (1H, m), 1.55 (9H, s), 1.21-0.99 (4H, m).

### Reference Example 87

### Production of tert-butyl [6'-cyclopropyl-4'-(difluoromethoxy)-3-formyl[2,3'-bipyridin]-4-yl]carbamate

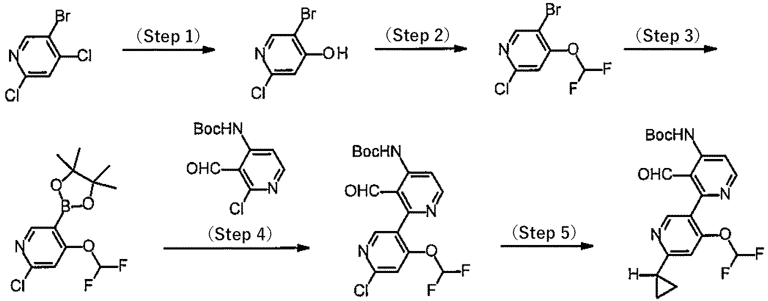

### (Step 1) 5-Bromo-2-chloropyridin-4-ol

To a suspension of 5-bromo-2,4-dichloropyridine (300 mg) in 1,4-dioxane (1 mL) was added 12 M hydrochloric acid (1 mL) at room temperature, and the mixture was stirred at 100°C for 5 hours. The reaction mixture was cooled to room temperature, a 1 M aqueous solution of sodium hydroxide (12 mL) was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (188 mg).
¹H-NMR (DMSO-D₆) δ: 12.27 (1H, br s), 8.33 (1H, s), 6.90 (1H, s).

### (Step 2) 5-Bromo-2-chloro-4-(difluoromethoxy)pyridine

To a solution of the compound (185 mg) obtained in Step 1 above in N,N-dimethylformamide (4.4 mL) were added cesium carbonate (578 mg) and sodium bromodifluoroacetate (262 mg) at room temperature, and the mixture was stirred at 80°C for 2 hours. The reaction mixture was cooled to room temperature, and then purified by silica gel column chromatography (n-hexane/ethyl acetate) to obtain the title compound (156 mg).
¹H-NMR (CDCl₃) δ: 8.53 (1H, s), 7.19-7.15 (1H, m), 6.70 (1H, t, J = 71.1 Hz).

### (Steps 3 and 4) tert-Butyl [6'-chloro-4'-(difluoromethoxy)-3-formyl[2,3'-bipyridin]-4-yl]carbamate

The title compound was obtained by using the compound obtained in Step 2 above as a production material and performing the same operations as in Steps 2 and 3 of Reference Example 83.
¹H-NMR (CDCl₃) δ: 10.86 (1H, br s), 9.76 (1H, s), 8.64 (1H, d, J = 6.1 Hz), 8.52 (1H, s), 8.50 (1H, d, J = 6.1 Hz), 6.57 (1H, t, J = 71.1 Hz), 1.56 (9H, s)., MS (m/z): 400 (M+H)⁺.

### (Step 5) tert-Butyl [6'-cyclopropyl-4'-(difluoromethoxy)-3-formyl[2,3'-bipyridin]-4-yl]carbamate

A mixture of the compound (78.0 mg) obtained in Step 4 above, potassium cyclopropyl trifluoroborate (86.0 mg), cesium carbonate (127 mg), [1,1'-bis(diphenylphosphino)ferrocene]dichloropalladium(II) dichloromethane adduct (16.0 mg), toluene (2.0 mL), and water (0.5 mL) was stirred at 100°C for 6 hours under a nitrogen atmosphere. After cooling the reaction mixture to room temperature, the solvent was evaporated under reduced pressure. The obtained residue was purified by silica gel column chromatography (n-hexane/ethyl acetate) to obtain the title compound (18.0 mg).
¹H-NMR (CDCl₃) δ: 10.89 (1H, br s), 9.77 (1H, s), 8.62 (1H, d, J = 6.1 Hz), 8.51 (1H, s), 8.44 (1H, d, J = 6.1 Hz), 7.01 (1H, s), 6.56 (1H, t, J = 72.1 Hz), 2.14-2.03 (1H, m), 1.55 (9H, s), 1.22-0.99 (4H, m).

### Reference Example 88

### Production of tert-butyl [3-formyl-2-(4-methoxy-2-methylpyrimidin-5-yl)pyridin-4-yl]carbamate

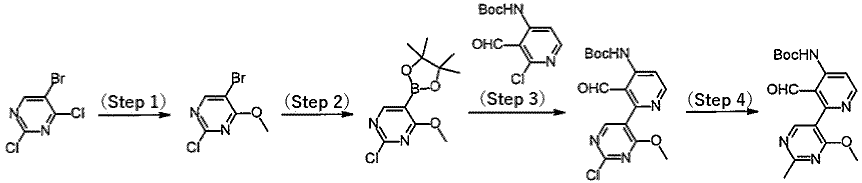

### (Steps 1 to 4) tert-Butyl [3-formyl-2-(4-methoxy-2-methylpyrimidin-5-yl)pyridin-4-yl]carbamate

The title compound was obtained by using 5-bromo-2,4-dichloropyrimidine as a production material and performing the same operations as in Steps 1 and 2 of Reference Example 83, Reference Example 70, and Reference Example 84.
¹H-NMR (CDCl₃) δ: 10.88 (1H, s), 9.76 (1H, s), 8.64 (1H, d, J = 6.1 Hz), 8.60 (1H, s), 8.45 (1H, d, J = 6.1 Hz), 3.97 (3H, s), 2.73 (3H, s), 1.57 (9H, s).

### Reference Example 89

### Production of tert-butyl [3-formyl-4'-methoxy-6'-(trifluoromethyl)[2,3'-bipyridin]-4-yl]carbamate

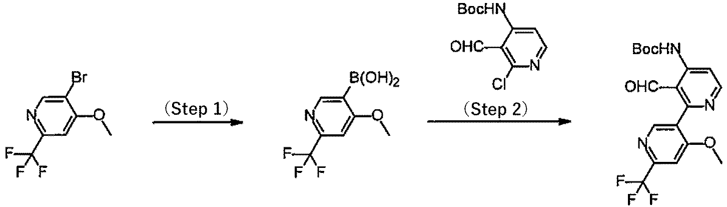

### (Step 1) [4-Methoxy-6-(trifluoromethyl)pyridin-3-yl]boronic acid

To a solution of 5-bromo-4-methoxy-2-(trifluoromethyl)pyridine (200 mg) in tetrahydrofuran (4.0 mL) was added a 1.56 M solution of n-butyllithium in hexane (500 µL) at -78°C, and the mixture was stirred at the same temperature for 30 minutes. Then, triisopropyl borate (270 µL) was added, and the mixture was warmed to 0°C and stirred for another 20 minutes. Saturated ammonium chloride solution was added to the reaction mixture, and the mixture was extracted three times with ethyl acetate. The obtained organic layer was dried over anhydrous sodium sulfate. The obtained organic layer was filtered, and the solvent was evaporated under reduced pressure to obtain the crude title compound (172 mg).

### (Step 2) tert-Butyl [3-formyl-4'-methoxy-6'-(trifluoromethyl)[2,3'-bipyridin]-4-yl]carbamate

A mixture of the compound (171 mg) obtained in Step 1 above, tert-butyl 2-chloro-3-formylpyridin-4-ylcarbamate (299 mg), cesium carbonate (758 mg), [1,1'-bis(diphenylphosphino)ferrocene]dichloropalladium(II) dichloromethane adduct (129 mg), 1,4-dioxane (4.0 mL), and water (1.5 mL) was stirred at 90°C for 3.5 hours under a nitrogen atmosphere. The reaction mixture was cooled to room temperature, water was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (127 mg).
¹H-NMR (CDCl₃) δ: 10.85 (1H, s), 9.70 (1H, s), 8.71 (1H, s), 8.66 (1H, d, J = 6.1 Hz), 8.48 (1H, d, J = 6.1 Hz), 7.29 (1H, s), 3.92 (3H, s), 1.56 (9H, s).

### Reference Example 90

### Production of tert-butyl [6'-(1,1-difluoroethyl)-3-formyl-4'-methoxy[2,3'-bipyridin]-4-yl]carbamate

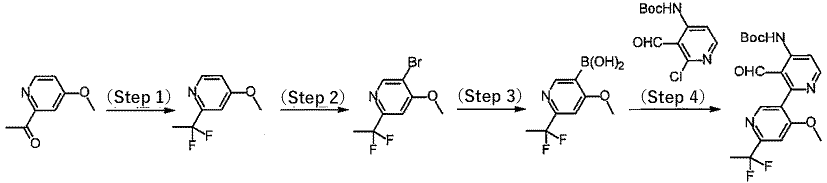

### (Step 1) 2-(1,1-Difluoroethyl)-4-methoxypyridine

To a solution of 1-(4-methoxypyridin-2-yl)ethanone (1.00 g) in dichloromethane (7.0 mL) was added (diethylamino)sulfur trifluoride (8.60 mL) at 0°C, and the mixture was stirred at room temperature for 9 hours. The reaction mixture was cooled to 0°C, and saturated sodium bicarbonate solution was carefully added. Then, the mixture was extracted twice with dichloromethane, and the obtained organic layer was dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (937 mg).
¹H-NMR (CDCl₃) δ: 8.46 (1H, d, J = 5.5 Hz), 7.17 (1H, d, J = 2.4 Hz), 6.86 (1H, dd, J = 5.5, 2.4 Hz), 3.89 (3H, s), 2.00 (3H, t, J = 17.7 Hz).

### (Step 2) 5-Bromo-2-(1,1-difluoroethyl)-4-methoxypyridine

Sulfuric acid (1.7 mL) and N-bromosuccinimide (312 mg) were carefully added to the compound (300 mg) obtained in Step 1 above at 0°C, and the mixture was stirred at 60°C for 1.5 hours. The reaction mixture was cooled to 0°C, and ethyl acetate and a 10 M aqueous solution of sodium hydroxide were added little by little. Then, the mixture was extracted three times with ethyl acetate, and the obtained organic layer was dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (297 mg).
¹H-NMR (CDCl₃) δ: 8.60 (1H, s), 7.19 (1H, s), 4.03 (3H, s), 2.03 (3H, t, J = 17.4 Hz).

### (Step 3) [6-(1,1-Difluoroethyl)-4-methoxypyridin-3-yl]boronic acid

To a solution of the compound (297 mg) obtained in Step 2 above in tetrahydrofuran (6.0 mL) was added dropwise a 1.56 M solution of n-butyllithium in n-hexane (760 µL) at -78°C. Then, triisopropyl borate (540 µL) was added, and the mixture was stirred at the same temperature for 30 minutes. Saturated ammonium chloride solution and water were added to the reaction mixture, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure to obtain the crude title compound (255 mg).

### (Step 4) tert-Butyl [6'-(1,1-difluoroethyl)-3-formyl-4'-methoxy[2,3'-bipyridin]-4-yl]carbamate

A mixture of the compound (256 mg) obtained in Step 3 above, tert-butyl 2-chloro-3-formylpyridin-4-ylcarbamate (303 mg), cesium carbonate (1.14 g), [1,1'-bis(diphenylphosphino)ferrocene]dichloropalladium(II) dichloromethane adduct (193 mg), 1,4-dioxane (6.0 mL), and water (2.0 mL) was stirred at 90°C for 2 hours under a nitrogen atmosphere. The reaction mixture was cooled to room temperature, water was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (181 mg).
¹H-NMR (CDCl₃) δ: 10.89 (1H, s), 9.73 (1H, s), 8.67 (1H, d, J = 6.1 Hz), 8.64 (1H, s), 8.47 (1H, d, J = 6.1 Hz), 3.91 (3H, s), 2.08 (3H, t, J = 18.6 Hz), 1.58 (9H, s).

### Reference Example 91

### Production of tert-butyl [6'-(difluoromethyl)-3-formyl-4'-methoxy[2,3'-bipyridin]-4-yl]carbamate

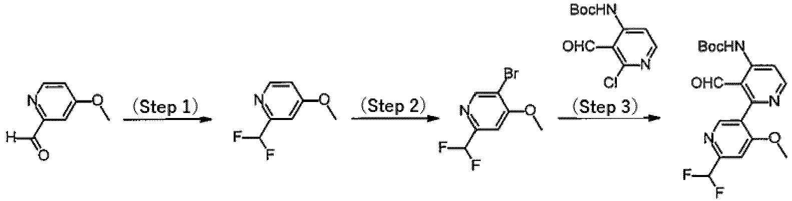

### (Steps 1 to 3) tert-Butyl [6'-(difluoromethyl)-3-formyl-4'-methoxy[2,3'-bipyridin]-4-yl]carbamate

The title compound was obtained by using 4-methoxypyridin-2-carbaldehyde as a production material and performing the same operations as in Steps 1 and 2 of Reference Example 90 and Step 2 of Reference Example 81.
¹H-NMR (CDCl₃) δ: 10.88 (1H, s), 9.72 (1H, s), 8.68 (1H, d, J = 6.1 Hz), 8.65 (1H, s), 8.48 (1H, d, J = 6.1 Hz), 8.41 (1H, s), 6.84-6.50 (1H, m), 3.92 (3H, s), 1.58 (9H, s).

### Reference Example 92

### Production of tert-butyl [3-formyl-4'-methoxy-6'-(propan-2-yl)[2,3'-bipyridin]-4-yl]carbamate

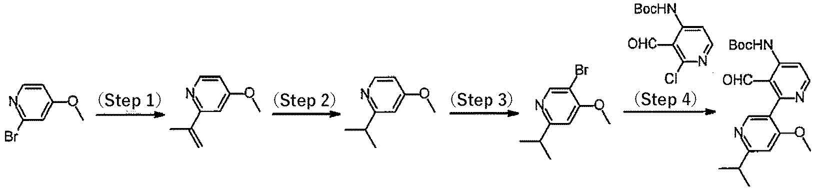

### (Steps 1 to 4) tert-Butyl [3-formyl-4'-methoxy-6'-(propan-2-yl)[2,3'-bipyridin]-4-yl]carbamate

The title compound was obtained by using 2-bromo-4-methoxypyridine as a production material and performing the same operations as in Steps 1 and 2 of Reference Example 48, Step 2 of Reference Example 90, and Step 2 of Reference Example 81. ¹H-NMR (CDCl₃) δ: 10.89 (1H, s), 9.74 (1H, s), 8.65 (1H, d, J = 6.1 Hz), 8.52 (1H, s), 8.41 (1H, d, J = 6.1 Hz), 6.77 (1H, s), 3.84 (3H, s), 1.56 (9H, s), 1.36 (6H, d, J = 6.7 Hz).

### Reference Example 93

### Production of tert-butyl [6'-(difluoromethoxy)-3-formyl-4'-methoxy[2,3'-bipyridin]-4-yl]carbamate

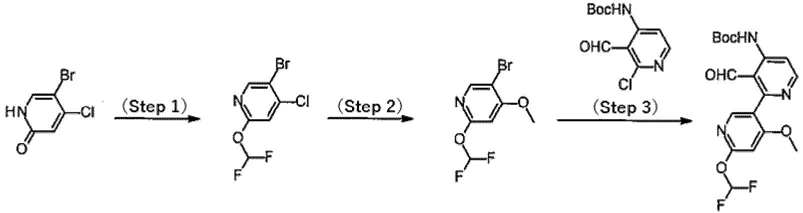

### (Step 1) 5-Bromo-4-chloro-2-(difluoromethoxy)pyridine

To a mixture of 5-bromo-4-chloro-1H-pyridin-2-one (938 mg), cesium fluoride (752 mg), trimethylsilyl difluoro(fluorosulfonyl)acetate (3.60 mL), and acetonitrile (15 mL) was added 55% sodium hydride in oil (241 mg) at 0°C, and the mixture was stirred at the same temperature for 18 hours. Water and ethyl acetate were added to the reaction mixture, and insoluble materials were removed by filtration. The filtrate was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (578 mg).
¹H-NMR (CDCl₃) δ: 8.34 (1H, s), 7.39 (1H, t, J = 72.3 Hz), 7.06 (1H, s).

### (Step 2) 5-Bromo-2-(difluoromethoxy)-4-methoxypyridine

A mixture of the compound (533 mg) obtained in Step 1 above, sodium hydroxide (423 mg), and methanol (4.2 mL) was stirred at 70°C for 1 hour. The reaction mixture was cooled to room temperature, water was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (162 mg).
¹H-NMR (CD₃OD) δ: 8.18 (1H, s), 7.52 (1H, t, J = 72.6 Hz), 6.68 (1H, s), 3.99 (3H, s).

### (Step 3) tert-Butyl [6'-(difluoromethoxy)-3-formyl-4'-methoxy[2,3'-bipyridin]-4-yl]carbamate

A mixture of the compound (162 mg) obtained in Step 2 above, bis(pinacolato)diboron (199 mg), potassium acetate (189 mg), [1,1'-bis(diphenylphosphino)ferrocene]dichloropalladium(II) dichloromethane adduct (115 mg), and N,N-dimethylformamide (1.6 mL) was stirred at 90°C for 30 minutes under a nitrogen atmosphere. Subsequently, tert-butyl 2-chloro-3-formylpyridin-4-ylcarbamate (216 mg), sodium carbonate (221 mg), N,N-dimethylformamide (1.6 mL), and water (1.6 mL) were added to the reaction mixture at 90°C, and the mixture was stirred at the same temperature for another 1 hour. The reaction mixture was cooled to room temperature, water was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (49.1 mg).
¹H-NMR (CDCl₃) δ: 10.85 (1H, s), 9.71 (1H, s), 8.63 (1H, d, J = 6.1 Hz), 8.43 (1H, d, J = 6.1 Hz), 8.20 (1H, s), 7.54 (1H, t, J = 72.9 Hz), 6.46 (1H, s), 3.83 (3H, s), 1.56 (9H, s).

### Reference Example 94

### Production of tert-butyl {2-[1-(difluoromethyl)-3,5-dimethyl-1H-pyrazol-4-yl]-3-formylpyridin-4-yl}carbamate

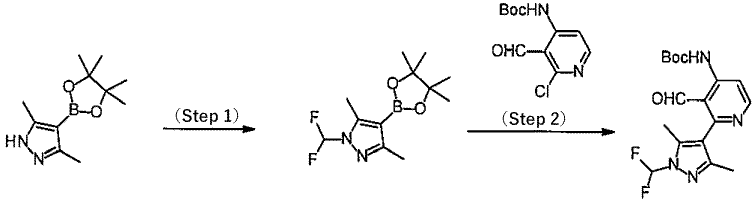

### (Step 1) 1-(Difluoromethyl)-3,5-dimethyl-4-(4,4,5,5-tetramethyl-1,3,2-dioxaborolan-2-yl)-1H-pyrazole

To a solution of 3,5-dimethyl-4-(4,4,5,5-tetramethyl-1,3,2-dioxaborolan-2-yl)-1H-pyrazole (200 mg) in acetonitrile (10 mL) were added sodium 2-chloro-2,2-difluoroacetate (165 mg) and 18-crown-6 (50.0 mg) at room temperature, and the mixture was stirred at 90°C for 3 hours. The reaction mixture was cooled to room temperature, saturated ammonium chloride solution was added, and the mixture was extracted three times with ethyl acetate. The obtained organic layer was washed with water and saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (220 mg).
¹H-NMR (CDCl₃) δ: 7.13 (1H, t, J = 59.8 Hz), 2.57 (3H, s), 2.33 (3H, s), 1.31 (12H, s).

### (Step 2) tert-Butyl {2-[1-(difluoromethyl)-3,5-dimethyl-1H-pyrazol-4-yl]-3-formylpyridin-4-yl}carbamate

A mixture of the compound (180 mg) obtained in Step 1 above, tert-butyl 2-chloro-3-formylpyridin-4-ylcarbamate (125 mg), cesium carbonate (0.48 g), [1,1'-bis(diphenylphosphino)ferrocene]dichloropalladium(II) dichloromethane adduct (60 mg), 1,4-dioxane (4 mL), and water (2 mL) was stirred at 100°C for 1.5 hours under a nitrogen atmosphere. After cooling the reaction mixture to room temperature, water was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (150 mg).
¹H-NMR (CDCl₃) δ: 10.90 (1H, s), 9.83 (1H, s), 8.64 (1H, d, J = 6.1 Hz), 8.40 (1H, d, J = 6.1 Hz), 7.20 (1H, t, J = 59.2 Hz), 2.37 (3H, s), 2.18 (3H, s), 1.56 (9H, s).

### Reference Example 95

### Production of tert-butyl {2-[3-cyclopropyl-1-(difluoromethyl)-5-methyl-1H-pyrazol-4-yl]-3-formylpyridin-4-yl}carbamate

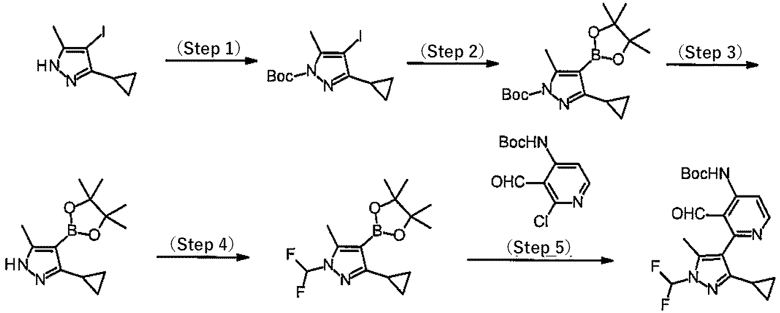

### (Step 1) tert-Butyl 3-cyclopropyl-4-iodo-5-methyl-1H-pyrazole-1-carboxylate

To a solution of 3-cyclopropyl-4-iodo-5-methyl-1H-pyrazole (1.00 g, which can be produced by the methods described in US20140256706A1 and the like) in tetrahydrofuran (20 mL) were added di-tert-butyl dicarbonate (1.10 g), 4-dimethylaminopyridine (100 mg), and N,N-diisopropylethylamine (1.10 mL) at room temperature, and the mixture was stirred at the same temperature for 2 hours. The reaction mixture was concentrated under reduced pressure, and the obtained residue was purified by silica gel column chromatography (n-hexane/ethyl acetate) to obtain the title compound (1.30 g).
¹H-NMR (CDCl₃) δ: 2.53 (3H, s), 1.86-1.77 (1H, m), 1.62 (9H, s), 1.02-0.89 (4H, m).

### (Step 2) tert-Butyl 3-cyclopropyl-5-methyl-4-(4,4,5,5-tetramethyl-1,3,2-dioxaborolan-2-yl)-1H-pyrazole-1-carboxylate

The title compound was obtained by using the compound obtained in Step 1 above as a production material and performing the same operation as in Step 2 of Reference Example 83.
¹H-NMR (CDCl₃) δ: 2.64 (3H, s), 2.30-2.22 (1H, m), 1.60 (9H, s), 1.31 (12H, s), 1.02-0.97 (2H, m), 0.89-0.83 (2H, m).

### (Step 3) 3-Cyclopropyl-5-methyl-4-(4,4,5,5-tetramethyl-1,3,2-dioxaborolan-2-yl)-1H-pyrazole

The compound (370 mg) obtained in Step 2 above was heated at 180°C for 20 minutes. After cooling the reaction mixture to room temperature, ethyl acetate was added, and the solvent was evaporated under reduced pressure to obtain the title compound (260 mg).
¹H-NMR (CDCl₃) δ: 2.38 (3H, s), 2.35-2.28 (1H, m), 1.31 (12H, s), 0.96-0.89 (2H, m), 0.86-0.80 (2H, m).

### (Steps 4 and 5) tert-Butyl {2-[3-cyclopropyl-1-(difluoromethyl)-5-methyl-1H-pyrazol-4-yl]-3-formylpyridin-4-yl}carbamate

The title compound was obtained by using the compound obtained in Step 3 above as a production material and performing the same operations as in Steps 1 and 2 of Reference Example 94.
¹H-NMR (CDCl₃) δ: 10.95 (1H, s), 9.93 (1H, s), 8.65 (1H, d, J = 6.1 Hz), 8.39 (1H, d, J = 6.1 Hz), 7.14 (1H, t, J = 59.2 Hz), 2.40 (3H, s), 1.56 (9H, s), 1.04-0.98 (1H, m), 0.88-0.77 (4H, m).

### Reference Example 96

### Production of 1-(cyclopropylmethyl)-3,5-dimethyl-4-(4,4,5,5-tetramethyl-1,3,2-dioxaborolan-2-yl)-1H-pyrazole

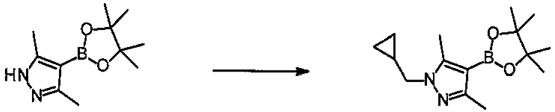

### 1-(Cyclopropylmethyl)-3,5-dimethyl-4-(4,4,5,5-tetramethyl-1,3,2-dioxaborolan-2-yl)-1H-pyrazole

To a solution of 3,5-dimethyl-4-(4,4,5,5-tetramethyl-1,3,2-dioxaborolan-2-yl)-1H-pyrazole (200 mg) in N,N-dimethylformamide (10 mL) were added (bromomethyl)cyclopropane (360 mg) and cesium carbonate (450 mg) at room temperature, and the mixture was stirred at 90°C for 10 hours. The reaction mixture was cooled to room temperature, saturated sodium bicarbonate solution was added, and the mixture was extracted three times with ethyl acetate. The obtained organic layer was washed three times with water and once with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (205 mg).
¹H-NMR (CDCl₃) δ: 3.85 (2H, d, J = 7.3 Hz), 2.40 (3H, s), 2.35 (3H, s), 1.29 (12H, s), 1.19-1.27 (1H, m), 0.51-0.57 (2H, m), 0.31-0.36 (2H, m).

### Reference Example 97

### Production of 3-cyclopropyl-1-(2,2-difluoroethyl)-5-methyl-4-(4,4,5,5-tetramethyl-1,3,2-dioxaborolan-2-yl)-1H-pyrazole

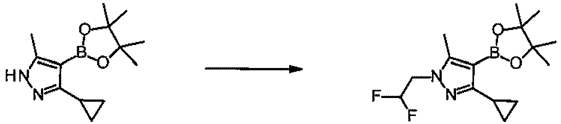

### 3-Cyclopropyl-1-(2,2-difluoroethyl)-5-methyl-4-(4,4,5,5-tetramethyl-1,3,2-dioxaborolan-2-yl)-1H-pyrazole

To a solution of the compound (250 mg) obtained in Step 3 of Reference Example 95 in N,N-dimethylformamide (10 mL) was added 55% sodium hydride in oil (70.0 mg) at 0°C, and the mixture was stirred at room temperature for 30 minutes. Then, a solution of 1,1-difluoro-2-iodoethane (0.40 g) in N,N-dimethylformamide (2.0 mL) was added at 0°C, and the mixture was stirred at room temperature for another 2 hours. Subsequently, 55% sodium hydride in oil (70.0 mg) and a solution of 1,1-difluoro-2-iodoethane (0.40 g) in N,N-dimethylformamide (2.0 mL) were added again to the reaction mixture at 0°C, and the mixture was stirred at room temperature for 5 hours. Water was added to the reaction mixture, and the mixture was extracted three times with ethyl acetate. The obtained organic layer was washed three times with water and once with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (160 mg).
¹H-NMR (CDCl₃) δ: 6.04 (1H, tt, J = 55.8, 4.6 Hz), 4.27 (2H, td, J = 13.3, 4.6 Hz), 2.40 (3H, s), 2.24-2.33 (1H, m), 1.30 (12H, s), 0.82-0.90 (4H, m).

### Reference Example 98

### Production of 3-(4,4,5,5-tetramethyl-1,3,2-dioxaborolan-2-yl)-6,7-dihydro-5H-pyrazolo[5,1-b][1,3]oxazine

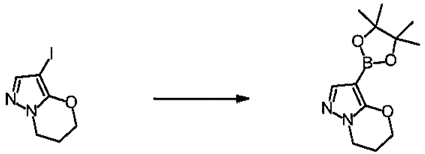

### 3-(4,4,5,5-Tetramethyl-1,3,2-dioxaborolan-2-yl)-6,7-dihydro-5H-pyrazolo[5,1-b][1,3]oxazine

To a solution of 3-iodo-6,7-dihydro-5H-pyrazolo[5,1-b][1,3]oxazine (620 mg) and 2-isopropoxy-4,4,5,5-tetramethyl-1,3,2-dioxaborolane (677 µL) in tetrahydrofuran (15 mL) was added a 1.3 M solution of isopropylmagnesium chloride lithium chloride complex in tetrahydrofuran (2.5 mL) at 0°C, and the mixture was stirred at the same temperature for 2.5 hours. Methanol (3.0 mL) was added to the reaction mixture, and the mixture was concentrated under reduced pressure. The obtained residue was purified by silica gel column chromatography (n-hexane/ethyl acetate) to obtain the title compound (327 mg). ¹H-NMR (CDCl₃) δ: 7.59 (1H, s), 4.40-4.34 (2H, m), 4.21-4.12 (2H, m), 2.31-2.18 (2H, m), 1.31 (12H, s).

The following compounds were synthesized by performing the same operation as in Step 2 of Reference Example 94 (Table 8-1).

**Table 8-1**

| Reference Example No. | Production Material | Structure of Compound Synthesized | Name of Compound Synthesized | Spectral Data |
|---|---|---|---|---|
| 99 | 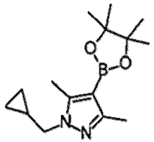 Reference Example 96 | 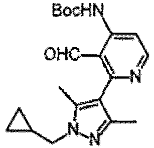 | tert-butyl {2-[1-(cyclopropylmethyl)-3,5-dimethyl-1H-pyrazol-4-yl]-3-formylpyridin-4-yl} carbamate | ¹H-NMR (CDCl₃) δ: 10.93 (1H, s), 9.84 (1H, s), 8.62 (1H, d, J = 6.1 Hz), 8.31 (1H, d, J = 6.1 Hz), 3.93 (2H, d, J = 6.7 Hz), 2.24 (3H, s), 2.17 (3H, s), 1.55 (9H, s), 1.22-1.29 (1H, m), 0.58-0.63 (2H, m), 0.36-0.42 (2H, m). |
| 100 | 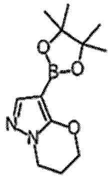 Reference Example 98 | 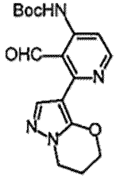 | tert-butyl [2-(6,7-dihydro-5H-pyrazolo[5,1-b][1,3]oxazin-3-yl)-3-formylpyridin-4-yl] carbamate | ¹H-NMR (CDCl₃) δ: 10.90 (1H, br s), 9.96 (1H, s), 8.55 (1H, d, J = 6.1 Hz), 8.20 (1H, d, J = 6.1 Hz), 7.72 (1H, s), 4.37 (2H, t, J = 5.2 Hz), 4.25 (2H, t, J = 6.1 Hz), 2.38-2.29 (2H, m), 1.54 (9H, s). |
| 101 | 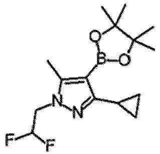 Reference Example 97 | 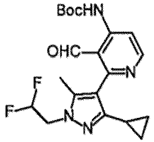 | tert-butyl {2-[3-cyclopropyl-1-(2,2-difluoroethyl)-5-methyl-1H-pyrazol-4-yl]-3-formylpyridin-4-yl}carbamate | ¹H-NMR (CDCl₃) δ: 10.96 (1H, s), 9.93 (1H, s), 8.63 (1H, d, J = 6.1 Hz), 8.34 (1H, d, J = 6.1 Hz), 6.12 (1H, tt, J = 55.5, 4.5 Hz), 4.40-4.31 (2H, m), 2.28 (3H, s), 2.17 (3H, s), 1.55 (9H, s), 1.29-1.23 (1H, m), 0.95-0.74 (4H, m). |

### Reference Example 102

### Production of tert-butyl {2-[1-(difluoromethyl)-1H-pyrazol-3-yl]-3-formylpyridin-4-yl}carbamate

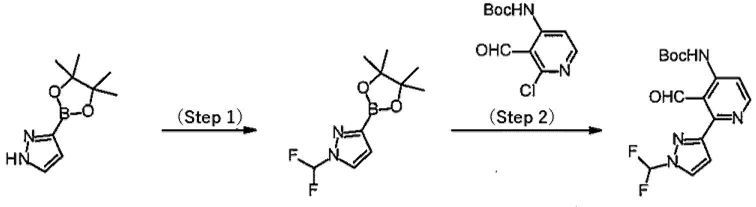

### (Steps 1 and 2) tert-Butyl {2-[1-(difluoromethyl)-1H-pyrazol-3-yl]-3-formylpyridin-4-yl}carbamate

The title compound was obtained by using 3-(4,4,5,5-tetramethyl-1,3,2-dioxaborolan-2-yl)-1H-pyrazole as a production material and performing the same operations as in Steps 1 and 2 of Reference Example 94.
¹H-NMR (CDCl₃) δ: 10.99 (1H, s), 10.51 (1H, s), 8.61 (1H, d, J = 6.1 Hz), 8.41 (1H, d, J = 6.1 Hz), 7.95 (1H, d, J = 2.4 Hz), 7.27 (1H, t, J = 60.4 Hz), 7.09 (1H, d, J = 2.4 Hz), 1.56 (9H, s).

### Reference Example 103

### Production of tert-butyl {2-[3-(difluoromethyl)-5-methyl-1-(propan-2-yl)-1H-pyrazol-4-yl]-3-formylpyridin-4-yl}carbamate

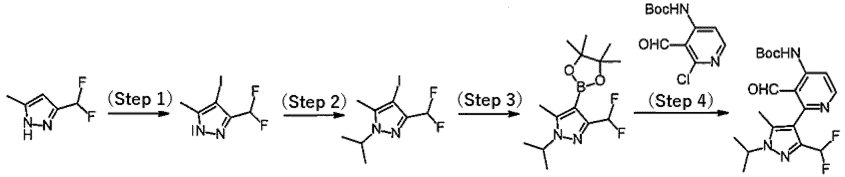

### (Step 1) 3-(Difluoromethyl)-4-iodo-5-methyl-1H-pyrazole

To a solution of 3-(difluoromethyl)-5-methyl-1H-pyrazole (1.00 g) in acetonitrile (15 mL) was added N-iodosuccinimide (1.70 g) at room temperature, and the mixture was stirred at room temperature for 1 hour. 5% Sodium thiosulfate solution was added to the reaction mixture, and the mixture was extracted three times with ethyl acetate. The obtained organic layer was washed with saturated sodium bicarbonate solution and saturated sodium chloride solution, and then dried over anhydrous sodium sulfate. The obtained organic layer was filtered, and the solvent was evaporated under reduced pressure to obtain the crude title compound (2.00 g).

### (Steps 2 to 4) tert-Butyl {2-[3-(difluoromethyl)-5-methyl-1-(propan-2-yl)-1H-pyrazol-4-yl]-3-formylpyridin-4-yl}carbamate

The title compound was obtained by using the compound obtained in Step 2 above and 2-iodopropane as production materials and performing the same operations as in Reference Example 97, Step 2 of Reference Example 83, and Step 2 of Reference Example 94.
¹H-NMR (CDCl₃) δ: 10.95 (1H, br s), 9.87 (1H, s), 8.61 (1H, d, J = 6.1 Hz), 8.39 (1H, d, J = 6.1 Hz), 6.70 (1H, t, J = 54.3 Hz), 4.53-4.46 (1H, m), 2.18 (3H, s), 1.55 (9H, s), 1.55 (3H, d, J = 6.7 Hz), 1.51 (3H, d, J = 6.7 Hz).

### Reference Example 104

### Production of tert-butyl [2-(1,4-dimethyl-1H-pyrazol-5-yl)-3-formylpyridin-4-yl]carbamate

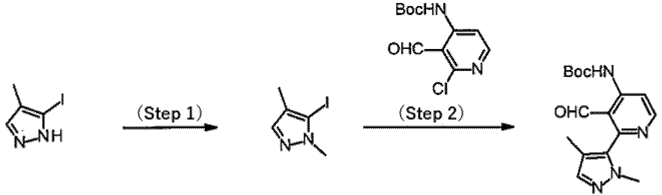

### (Step 1) 5-Iodo-1,4-dimethyl-1H-pyrazole

To a solution of 3-iodo-4-methyl-1H-pyrazole (1.10 g) in tetrahydrofuran (20 mL) was added 55% sodium hydride in oil (0.46 g) at 0°C, and the mixture was stirred at room temperature for 30 minutes. The reaction mixture was cooled again to 0°C, iodomethane (1.30 mL) was added, and the mixture was stirred at room temperature for 2 hours. Saturated sodium bicarbonate solution was added to the reaction mixture, and the mixture was extracted three times with ethyl acetate. The obtained organic layer was washed with 5% sodium thiosulfate solution and saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (350 mg).
¹H-NMR (CDCl₃) δ: 7.05 (1H, s), 3.87 (3H, s), 1.98 (3H, s).

### (Step 2) tert-Butyl [2-(1,4-dimethyl-1H-pyrazol-5-yl)-3-formylpyridin-4-yl]carbamate

The title compound was obtained by using the compound obtained in Step 1 above as a production material and performing the same operation as in Step 2 of Reference Example 81.
¹H-NMR (CDCl₃) δ: 10.89 (1H, s), 9.75 (1H, s), 8.68 (1H, d, J = 5.5 Hz), 8.45 (1H, d, J = 5.5 Hz), 7.40 (1H, s), 3.84 (3H, s), 1.93 (3H, s), 1.56 (9H, s).

### Reference Example 105

### Production of tert-butyl [6'-cyclopropyl-3-(isothiocyanatomethyl)-4'-methoxy[2,3'-bipyridin]-4-yl]carbamate

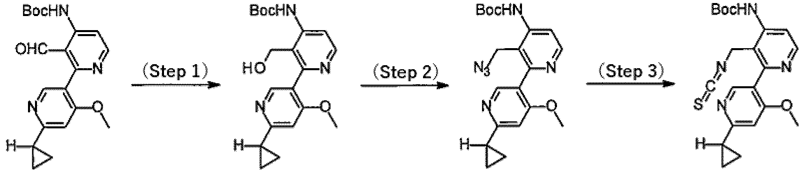

### (Step 1) tert-Butyl [6'-cyclopropyl-3-(hydroxymethyl)-4'-methoxy[2,3'-bipyridin]-4-yl]carbamate

To a solution of the compound (800 mg) obtained in Reference Example 86 in ethanol (10 mL) was added sodium borohydride (161 mg) at 0°C, and the mixture was stirred at the same temperature for 1.5 hours. Saturated ammonium chloride solution was added to the reaction mixture, and the mixture was extracted three times with dichloromethane. The obtained organic layer was washed with saturated sodium chloride solution, and then dried over anhydrous sodium sulfate. The obtained organic layer was filtered, and the solvent was evaporated under reduced pressure to obtain the crude title compound (759 mg).

### (Step 2) tert-Butyl [3-(azidomethyl)-6'-cyclopropyl-4'-methoxy[2,3'-bipyridin]-4-yl]carbamate

To a solution of the compound (390 mg) obtained in Step 1 above in tetrahydrofuran (6.0 mL) were added diphenylphosphoryl azide (455 µL) and 1,8-diazabicyclo[5,4,0]-7-undecene (315 µL) at room temperature, and the mixture was stirred at the same temperature for 15.5 hours. Water was added to the reaction mixture, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (276 mg).
¹H-NMR (CDCl₃) δ: 8.55 (1H, d, J = 5.5 Hz), 8.21 (1H, s), 8.12 (1H, d, J = 5.5 Hz), 7.43 (1H, br s), 6.78 (1H, s), 4.27 (1H, d, J = 14.7 Hz), 4.16 (1H, d, J = 14.1 Hz), 3.84 (3H, s), 2.13-2.01 (1H, m), 1.56 (9H, s), 1.18-0.99 (4H, m).

### (Step 3) tert-Butyl [6'-cyclopropyl-3-(isothiocyanatomethyl)-4'-methoxy[2,3'-bipyridin]-4-yl]carbamate

To a solution of the compound (236 mg) obtained in Step 2 above in tetrahydrofuran (3.0 mL) was added triphenylphosphine (161 mg) at room temperature, and the mixture was stirred at 50°C for 3 hours. The reaction mixture was cooled to room temperature, and the solvent was evaporated under reduced pressure. Acetonitrile (3.0 mL) and carbon disulfide (360 µL) were added to the obtained residue at room temperature, and the mixture was stirred at 80°C for 1 hour. The reaction mixture was cooled to room temperature, and then the precipitated solid was collected by filtration. The obtained solid was washed once with acetonitrile (3.0 mL) and dried under reduced pressure to obtain the title compound (200 mg).
¹H-NMR (CDCl₃) δ: 8.58 (1H, d, J = 6.1 Hz), 8.26 (1H, s), 7.00 (1H, d, J = 6.1 Hz), 6.80 (1H, s), 6.67 (1H, br s), 4.52-4.08 (2H, m), 3.88 (3H, s), 2.14-2.01 (1H, m), 1.71 (9H, s), 1.18-1.03 (4H, m).

### Reference Example 106

### Production of tert-butyl [3-(aminomethyl)-2-(2-chlorophenyl)pyridin-4-yl]carbamate

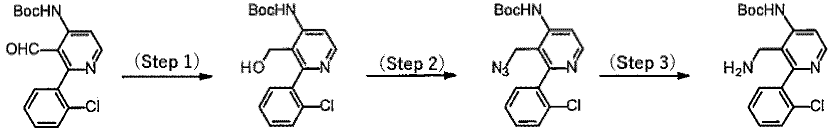

### (Steps 1 and 2) tert-Butyl [3-(azidomethyl)-2-(2-chlorophenyl)pyridin-4-yl]carbamate

The title compound was obtained by using the compound obtained in Reference Example 59 as a production material and performing the same operations as in Steps 1 and 2 of Reference Example 105.
¹H-NMR (CDCl₃) δ: 8.54 (1H, d, J = 6.1 Hz), 8.17 (1H, d, J = 6.1 Hz), 7.53-7.46 (1H, m), 7.44-7.30 (4H, m), 4.29 (1H, d, J = 14.0 Hz), 4.17 (1H, d, J = 14.0 Hz), 1.57 (9H, s).

### (Step 3) tert-Butyl [3-(aminomethyl)-2-(2-chlorophenyl)pyridin-4-yl]carbamate

To a solution of the compound (170 mg) obtained in Step 2 above in tetrahydrofuran (3.0 mL)/water (3.0 mL) was added triphenylphosphine (161 mg) at room temperature, and the mixture was stirred at the same temperature for 22 hours. Water was added to the reaction mixture, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The residue was purified by silica gel column chromatography (n-hexane/ethyl acetate) to obtain the title compound (152 mg).
¹H-NMR (CDCl₃) δ: 10.37 (1H, br s), 8.48 (1H, d, J = 6.1 Hz), 8.12 (1H, d, J = 6.1 Hz), 7.51-7.43 (1H, m), 7.40-7.31 (3H, m), 3.86 (1H, d, J = 13.4 Hz), 3.74 (1H, d, J = 13.4 Hz), 1.57 (9H, s).

### Reference Example 107

### Production of tert-butyl [3-(aminomethyl)-2-(2-methoxyphenyl)pyridin-4-yl]carbamate

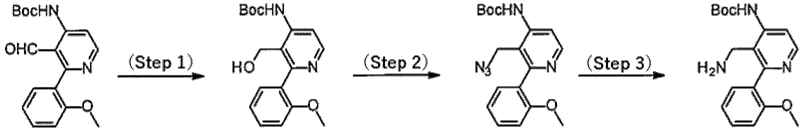

### (Steps 1 to 3) tert-Butyl [3-(aminomethyl)-2-(2-methoxyphenyl)pyridin-4-yl]carbamate

The title compound was obtained by using the compound obtained in Reference Example 60 as a production material and performing the same operations as in Steps 1 and 2 of Reference Example 105 and Step 3 of Reference Example 106.
¹H-NMR (CDCl₃) δ: 8.48 (1H, d, J = 6.1 Hz), 8.06 (1H, d, J = 6.1 Hz), 7.43-7.36 (1H, m), 7.35-7.31 (1H, m), 7.08 (1H, t, J = 7.9 Hz), 6.98 (1H, d, J = 7.9 Hz), 3.86 (1H, d, J = 13.4 Hz), 3.78 (3H, s), 3.73 (1H, d, J = 13.4 Hz), 1.57 (9H, s).

### Reference Example 108

### Production of tert-butyl [1'-(cyclopropanecarbonyl)-3-formyl-1',2',5',6'-tetrahydro[2,3'-bipyridin]-4-yl]carbamate

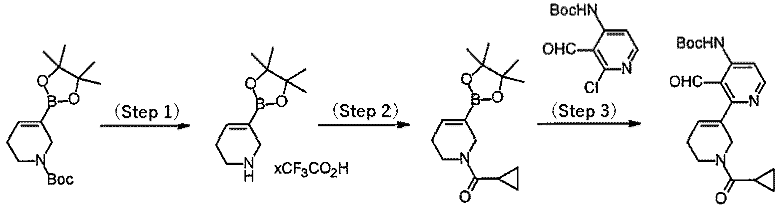

### (Step 1) 5-(4,4,5,5-Tetramethyl-1,3,2-dioxaborolan-2-yl)-1,2,3,6-tetrahydropyridine trifluoroacetate

To a solution of tert-butyl 5-(4,4,5,5-tetramethyl-1,3,2-dioxaborolan-2-yl)-3,6-dihydropyridin-1(2H)-carboxylate (1.00 g) in dichloromethane (7.0 mL) was added trifluoroacetic acid (2.50 mL) at 0°C, and the mixture was stirred at room temperature for 4 hours. The reaction mixture was concentrated under reduced pressure, and excess trifluoroacetic acid was removed by azeotropic distillation with toluene to obtain the crude title compound (1.04 g).

### (Step 2) Cyclopropyl[5-(4,4,5,5-tetramethyl-1,3,2-dioxaborolan-2-yl)-3,6-dihydropyridin-1(2H)-yl]methanone

To a solution of the compound (207 mg) obtained in Step 1 above in dichloromethane (2.0 mL) were added triethylamine (266 µL) and cyclopropanecarbonyl chloride (75.0 µL) at 0°C, and the mixture was stirred at room temperature for 3 hours. The reaction mixture was concentrated under reduced pressure, and the residue was purified by silica gel column chromatography (n-hexane/ethyl acetate) to obtain the title compound (110 mg).
¹H-NMR (CDCl₃) δ: 6.83-6.57 (1H, m), 4.33-4.14 (2H, m), 3.80-3.60 (2H, m), 2.38-2.17 (2H, m), 1.88-1.71 (1H, m), 1.32-1.22 (12H, m), 1.03-0.95 (2H, m), 0.81-0.71 (2H, m).

### (Step 3) tert-Butyl [1'-(cyclopropanecarbonyl)-3-formyl-1',2',5',6'-tetrahydro[2,3'-bipyridin]-4-yl]carbamate

The title compound was obtained by using the compound obtained in Step 2 above as a production material and performing the same operation as in Reference Example 70.
¹H-NMR (CDCl₃) δ: 10.84 (1H, br s), 10.18-10.06 (1H, m), 8.58-8.45 (1H, m), 8.40-8.21 (1H, m), 6.05-5.79 (1H, m), 4.73 (1H, s), 4.58 (1H, s), 3.96-3.76 (2H, m), 2.55-2.35 (2H, m), 1.91-1.77 (1H, m), 1.55 (9H, s), 1.32-1.18 (1H, m), 1.12-0.98 (2H, m), 0.86-0.74 (2H, m).

### Reference Example 109

### Production of tert-butyl [1'-(cyclopropylsulfonyl)-3-formyl-1',2',5',6'-tetrahydro[2,3'-bipyridin]-4-yl]carbamate

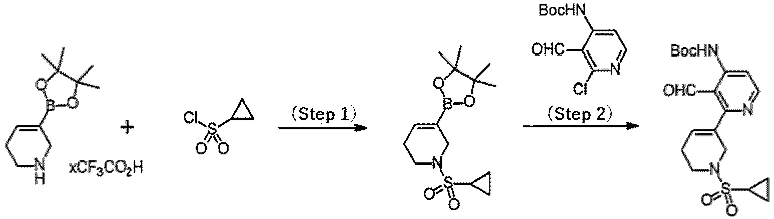

### (Steps 1 and 2) tert-Butyl [1'-(cyclopropylsulfonyl)-3-formyl-1',2',5',6'-tetrahydro[2,3'-bipyridin]-4-yl]carbamate

The title compound was obtained by using the compound obtained in Step 1 of Reference Example 108 and cyclopropanesulfonyl chloride as production materials and performing the same operations as in Step 2 of Reference Example 108 and Reference Example 57.
¹H-NMR (CDCl₃) δ: 10.84 (1H, br s), 10.12 (1H, s), 8.50 (1H, d, J = 5.9 Hz), 8.31 (1H, d, J = 5.9 Hz), 5.92-5.88 (1H, m), 4.38-4.33 (2H, m), 3.59-3.53 (2H, m), 2.57-2.51 (2H, m), 2.42-2.35 (1H, m), 1.28-1.19 (11H, m), 1.05-0.97 (2H, m).

### Reference Example 110

### Production of 4-amino-2-[1-(phenylsulfonyl)azepan-3-yl]pyridin-3-carbaldehyde

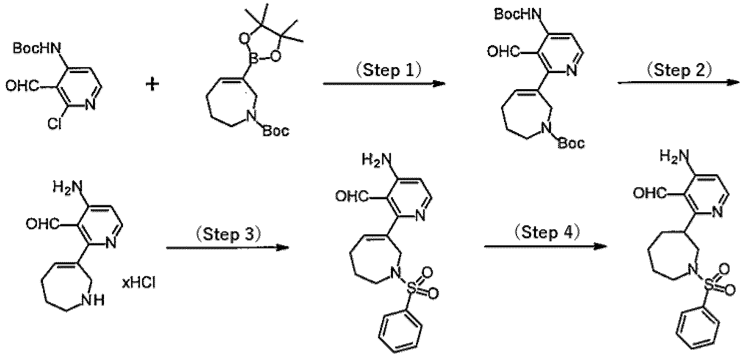

### (Step 1) tert-Butyl 6-{4-[(tert-butoxycarbonyl)amino]-3-formylpyridin-2-yl}-2,3,4,7-tetrahydro-1H-azepin-1-carboxylate

A mixture (242 mg) of the title compound and a double bond positional isomer (tert-butyl 6-{4-[(tert-butoxycarbonyl)amino]-3-formylpyridin-2-yl}-2,3,4,5-tetrahydro-1H-azepin-1-carboxylate) were obtained by using tert-butyl 6-(4,4,5,5-tetramethyl-1,3,2-dioxaborolan-2-yl)-2,3,4,7-tetrahydro-1H-azepin-1-carboxylate [251 mg, containing a double bond positional isomer (tert-butyl 6-(4,4,5,5-tetramethyl-1,3,2-dioxaborolan-2-yl)-2,3,4,5-tetrahydro-1H-azepin-1-carboxylate)] as a production material and performing the same operation as in Reference Example 57.
¹H-NMR (CDCl₃) δ: 10.88 (0.6H, br s), 10.84 (0.4H, br s), 10.10 (0.6H, s), 10.06 (0.4H, s), 8.49 (0.6H, d, J = 5.9 Hz), 8.47 (0.4H, d, J = 5.9 Hz), 8.26 (0.6H, d, J = 5.9 Hz), 8.22 (0.4H, d, J = 5.9 Hz), 5.84-5.78 (0.6H, m), 5.76-5.71 (0.4H, m), 4.43-4.30 (2H, m), 3.82-3.56 (2H, m), 2.55-2.43 (2H, m), 1.96-1.85 (2H, m), 1.55 (5.4H, s), 1.54 (3.6H, s), 1.45 (3.6H, s), 1.23 (5.4H, s).

### (Step 2) 4-Amino-2-(2,5,6,7-tetrahydro-1H-azepin-3-yl)pyridin-3-carbaldehyde hydrochloride

A mixture of the compound (53 mg) obtained in Step 1 above, ethyl acetate (1 mL), and a 4 M solution of hydrogen chloride in 1,4-dioxane (0.3 mL) was stirred at room temperature for 18.5 hours. The reaction mixture was concentrated under reduced pressure to obtain a mixture (41 mg) of the crude title compound and a double bond positional isomer (4-amino-2-(4,5,6,7-tetrahydro-1H-azepin-3-yl)pyridin-3-carbaldehyde hydrochloride).

### (Step 3) 4-Amino-2-[1-(benzenesulfonyl)-2,5,6,7-tetrahydro-1H-azepin-3-yl]pyridin-3-carbaldehyde

A mixture of the title compound and a double bond positional isomer (4-amino-2-[1-(benzenesulfonyl)-4,5,6,7-tetrahydro-1H-azepin-3-yl]pyridin-3-carbaldehyde) was obtained by using the compound obtained in Step 2 above and benzenesulfonyl chloride as production materials and performing the same operation as in Step 2 of Reference Example 108.
¹H-NMR (CDCl₃) δ: 10.04 (1H, s), 8.13 (1H, d, J = 5.9 Hz), 7.78 (2H, dd, J = 8.2, 1.2 Hz), 7.59-7.42 (3H, m), 6.42 (1H, d, J = 5.9 Hz), 5.89-5.82 (1H, m), 4.39-4.26 (2H, m), 3.59-3.50 (2H, m), 2.53-2.40 (2H, m), 2.03-1.94 (2H, m).

### (Step 4) 4-Amino-2-[1-(phenylsulfonyl)azepan-3-yl]pyridin-3-carbaldehyde

A mixture of the compound (30 mg) obtained in Step 3 above, 10%-palladium on carbon (30 mg), and ethyl acetate (2 mL) was stirred at room temperature for 2.5 hours under a hydrogen atmosphere. Insoluble materials were removed by filtration through Celite, and the solvent was evaporated under reduced pressure to obtain the crude title compound (25 mg).

### Reference Example 111

### Production of tert-butyl methyl[3-methyl-4-(4,4,5,5-tetramethyl-1,3,2-dioxaborolan-2-yl)benzyl]carbamate

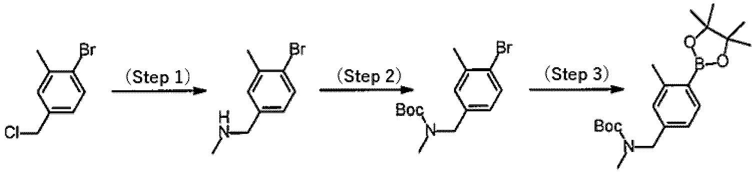

### (Step 1) 1-(4-Bromo-3-methylphenyl)-N-methylmethanamine

To a 2 M solution of methylamine in tetrahydrofuran (6.80 mL) was added a solution of 1-bromo-4-(chloromethyl)-2-methylbenzene (1.00 g) in N,N-dimethylformamide (20 mL) at 0°C, and the mixture was stirred at room temperature for 4.5 hours. Water was added to the reaction mixture, and the mixture was extracted three times with ethyl acetate. The obtained organic layer was washed three times with water and once with saturated sodium chloride solution, and then dried over anhydrous sodium sulfate. The obtained organic layer was filtered, and the solvent was removed under reduced pressure to obtain the title compound (1.00 g).
¹H-NMR (CDCl₃) δ: 7.47 (1H, d, J = 7.9 Hz), 7.20 (1H, d, J = 1.8 Hz), 6.99 (1H, dd, J = 7.9, 1.8 Hz), 3.67 (2H, s), 2.39 (3H, s).

### (Step 2) tert-Butyl (4-bromo-3-methylbenzyl)methylcarbamate

To a solution of the compound (1.00 g) obtained in Step 1 above in ethanol (20 mL) was added di-tert-butyl dicarbonate (1.20 g) at room temperature, and the mixture was stirred at the same temperature for 1 hour. The reaction mixture was concentrated under reduced pressure, and the obtained residue was purified by silica gel column chromatography (n-hexane/ethyl acetate) to obtain the title compound (1.00 g).
¹H-NMR (CDCl₃) δ: 7.49 (1H, d, J = 7.9 Hz), 7.08 (1H, br s), 6.91 (1H, br s), 4.34 (2H, s), 2.85-2.75 (3H, m), 2.38 (3H, s), 1.47 (9H, s).

### (Step 3) tert-Butyl methyl[3-methyl-4-(4,4,5,5-tetramethyl-1,3,2-dioxaborolan-2-yl)benzyl]carbamate

A mixture of the compound (1.00 g) obtained in Step 2 above, bis(pinacolato)diboron (1.20 g), potassium acetate (0.95 g), [1,1'-bis(diphenylphosphino)ferrocene]dichloropalladium(II) dichloromethane adduct (0.26 g), and 1,4-dioxane (20 mL) was stirred at 90°C for 10 hours under a nitrogen atmosphere. After cooling the reaction mixture to room temperature, the mixture was diluted with ethyl acetate, and insoluble materials were removed by filtration through Celite. The obtained filtrate was washed with water and saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (1.00 g).
¹H-NMR (CDCl₃) δ: 7.72 (1H, d, J = 7.9 Hz), 7.06-6.98 (2H, m), 4.38 (2H, br s), 2.86-2.72 (3H, m), 2.52 (3H, s), 1.51-1.43 (9H, m), 1.34 (12H, s).

### Reference Example 112

### Production of tert-butyl [2-methyl-3-(4,4,5,5-tetramethyl-1,3,2-dioxaborolan-2-yl)phenoxy]acetate

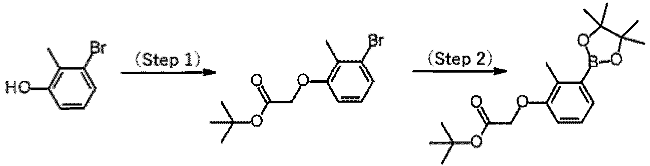

### (Step 1) tert-Butyl (3-bromo-2-methylphenoxy)acetate

To a solution of 3-bromo-2-methylphenol (500 mg) in N,N-dimethylformamide (20 mL) were added tert-butyl bromoacetate (0.59 mL) and potassium carbonate (550 mg) at room temperature, and the mixture was stirred at 90°C for 7.5 hours. After cooling the reaction mixture to room temperature, water was added, and the mixture was extracted three times with ethyl acetate. The obtained organic layer was washed three times with water and once with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (0.80 g).
¹H-NMR (CDCl₃) δ: 7.19 (1H, d, J = 7.9 Hz), 6.98 (1H, t, J = 7.9 Hz), 6.64 (1H, d, J = 7.9 Hz), 4.52 (2H, s), 2.38 (3H, s), 1.48 (9H, s).

### (Step 2) tert-Butyl [2-methyl-3-(4,4,5,5-tetramethyl-1,3,2-dioxaborolan-2-yl)phenoxy]acetate

The title compound was obtained by using the compound obtained in Step 1 above as a production material and performing the same operation as in Step 3 of Reference Example 111.
¹H-NMR (CDCl₃) δ: 7.37 (1H, d, J = 8.0 Hz), 7.11 (1H, t, J = 8.0 Hz), 6.79 (1H, d, J = 8.0 Hz), 4.51 (2H, s), 2.49 (3H, s), 1.48 (9H, s), 1.34 (12H, s).

### Reference Example 113

### Production of tert-butyl {[3-(4,4,5,5-tetramethyl-1,3,2-dioxaborolan-2-yl)phenyl]sulfonyl}acetate

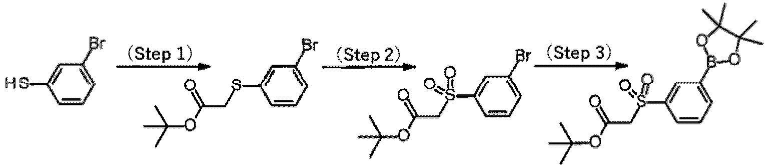

### (Step 1) tert-Butyl [(3-bromophenyl)sulfanyl]acetate

To a solution of 3-bromobenzenethiol (1.00 g) in N,N-dimethylformamide (20 mL) were added tert-butyl bromoacetate (1.20 mL) and potassium carbonate (1.10 g) at room temperature, and the mixture was stirred at the same temperature for 8 hours. Water was added to the reaction mixture, and the mixture was extracted three times with ethyl acetate. The obtained organic layer was washed three times with water and once with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (1.60 g).
¹H-NMR (CDCl₃) δ: 7.53 (1H, t, J = 1.8 Hz), 7.35-7.29 (2H, m), 7.15 (1H, t, J = 7.9 Hz), 3.56 (2H, s), 1.42 (9H, s).

### (Step 2) tert-Butyl [(3-bromophenyl)sulfonyl]acetate

To a solution of the compound (1.60 g) obtained in Step 1 above in 1,4-dioxane (20 mL) were added water (20 mL) and potassium peroxymonosulfate (9.70 g) at 0°C, and the mixture was stirred at room temperature for 1.5 hours. Water was added to the reaction mixture, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (1.10 g).
¹H-NMR (CDCl₃) δ: 8.09 (1H, t, J = 1.8 Hz), 7.89 (1H, dt, J = 7.9, 1.8 Hz), 7.82 (1H, dt, J = 7.9, 1.8 Hz), 7.47 (1H, t, J = 7.9 Hz), 4.05 (2H, s), 1.39 (9H, s).

### (Step 3) tert-Butyl {[3-(4,4,5,5-tetramethyl-1,3,2-dioxaborolan-2-yl)phenyl]sulfonyl}acetate

The title compound was obtained by using the compound obtained in Step 2 above as a production material and performing the same operation as in Step 3 of Reference Example 111.
¹H-NMR (CDCl₃) δ: 8.37 (1H, br s), 8.08 (1H, t, J = 7.7 Hz), 8.02 (1H, t, J = 7.7 Hz), 7.58 (1H, t, J = 7.7 Hz), 4.05 (2H, s), 1.36 (9H, s), 1.35 (12H, s).

### Reference Example 114

### Production of tert-butyl [3,5-dimethyl-4-(4,4,5,5-tetramethyl-1,3,2-dioxaborolan-2-yl)-1H-pyrazol-1-yl]acetate

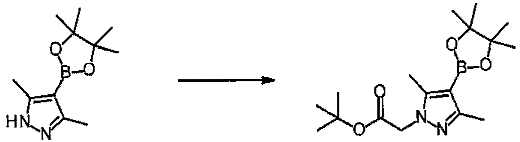

### tert-Butyl [3,5-dimethyl-4-(4,4,5,5-tetramethyl-1,3,2-dioxaborolan-2-yl)-1H-pyrazol-1-yl]acetate

To a solution of 3,5-dimethylpyrazole-4-boronic acid pinacol ester (500 mg) in N,N-dimethylformamide (10 mL) were added tert-butyl bromoacetate (0.40 mL) and cesium carbonate (1.10 g) at room temperature, and the mixture was stirred at the same temperature for 8 hours. Water was added to the reaction mixture, and the mixture was extracted three times with ethyl acetate. The obtained organic layer was washed three times with water and once with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (0.76 g).
¹H-NMR (CDCl₃) δ: 4.67 (2H, s), 2.35 (3H, s), 2.34 (3H, s), 1.57 (9H, s), 1.28 (12H, s).

### Reference Example 115

### Production of tert-butyl 2-[3,5-dimethyl-4-(4,4,5,5-tetramethyl-1,3,2-dioxaborolan-2-yl)-1H-pyrazol-1-yl]propanoate

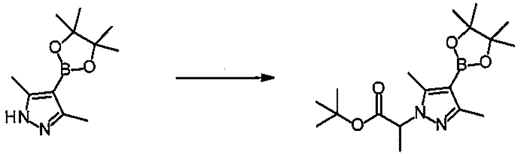

### tert-Butyl 2-[3,5-dimethyl-4-(4,4,5,5-tetramethyl-1,3,2-dioxaborolan-2-yl)-1H-pyrazol-1-yl]propanoate

To a solution of 3,5-dimethylpyrazole-4-boronic acid pinacol ester (400 mg) and tert-butyl 2-bromopropionate (0.9 mL) in N,N-dimethylformamide (9 mL) was added 55% sodium hydride in oil (91 mg) at room temperature, and the mixture was stirred at the same temperature for 1 hour. Saturated ammonium chloride solution was added to the reaction mixture, and the mixture was extracted with ethyl acetate. The obtained organic layer was washed three times with water and once with saturated sodium chloride solution, and then dried over anhydrous sodium sulfate. The obtained organic layer was filtered, and the solvent was evaporated under reduced pressure. The obtained residue was purified by silica gel column chromatography (n-hexane/ethyl acetate) to obtain the title compound (596 mg). ¹H-NMR (CDCl₃) δ: 4.82-4.71 (1H, m), 2.37 (2.1H, s), 2.35 (2.1H, s), 2.23 (0.9H, s), 2.21 (0.9H, s), 1.77 (2.1H, d, J = 7.4 Hz), 1.75 (0.9H, d, J = 7.4 Hz), 1.42 (9H, s), 1.31-1.24 (12H, m).

### Reference Example 116

### Production of tert-butyl 3-[4-(4,4,5,5-tetramethyl-1,3,2-dioxaborolan-2-yl)-1H-pyrazol-1-yl]propanoate

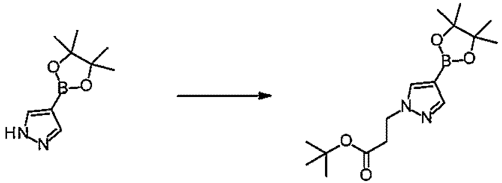

### tert-Butyl 3-[4-(4,4,5,5-tetramethyl-1,3,2-dioxaborolan-2-yl)-1H-pyrazol-1-yl]propanoate

To a solution of 4-(4,4,5,5-tetramethyl-1,3,2-dioxaborolan-2-yl)-1H-pyrazole (1.00 g) in acetonitrile (20 mL) were added tert-butyl acrylate (1.50 mL) and 1,8-diazabicyclo[5.4.0]-7-undecene (0.38 mL) at room temperature, and the mixture was stirred at the same temperature for 19.5 hours. The reaction mixture was concentrated under reduced pressure, and the obtained residue was purified by silica gel column chromatography (n-hexane/ethyl acetate) to obtain the title compound (1.40 g).
¹H-NMR (CDCl₃) δ: 7.78 (1H, s), 7.72 (1H, s), 4.38 (2H, t, J = 6.7 Hz), 2.81 (2H, t, J = 6.7 Hz), 1.41 (9H, s), 1.31 (12H, s).

The following compounds were synthesized by performing the same operation as in Reference Example 70 (Table 9-1).

**Table 9-1**

| Reference Example No. | Production Material | Structure of Compound Synthesized | Name of Compound Synthesized | Spectral Data |
|---|---|---|---|---|
| 117 | 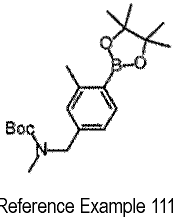 | 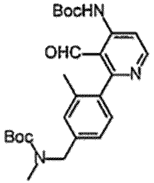 | tert-butyl (4-{4-[(tert-butoxycarbonyl) amino]-3-formylpyridin-2-yl}-3-methylbenzyl) methylcarbamate | ¹H-NMR (CDCl₃) δ: 10.94 (1H, s), 9.72 (1H, s), 8.61 (1H, d, J = 6.1 Hz), 8.39 (1H, d, J = 6.1 Hz), 7.22-7.10 (3H, m), 4.45 (2H, br s), 2.90-2.77 (3H, m), 2.15 (3H, s), 1.56 (9H, s), 1.50 (9H, br s). |
| 118 | 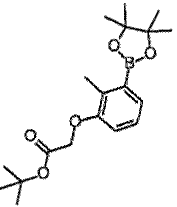 Reference Example 112 | 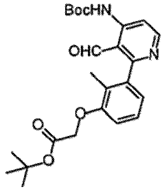 | tert-butyl (3-{4-[(tert-butoxycarbonyl) amino]-3-formylpyridin-2-yl}-2-methylphenoxy)acetate | ¹H-NMR (CDCl₃) δ: 10.94 (1H, br s), 9.71 (1H, s), 8.61 (1H, d, J = 6.1 Hz), 8.39 (1H, d, J = 6.1 Hz), 7.23 (1H, t, J = 7.9 Hz), 6.90 (1H, d, J = 7.9 Hz), 6.81 (1H, d, J = 7.9 Hz), 4.57 (2H, s), 2.07 (3H, s), 1.55 (9H, s), 1.50 (9H, s). |
| 119 | 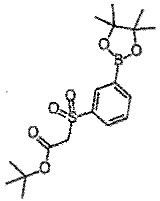 Reference Example 113 | 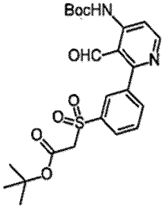 | tert-butyl [(3-{4-[(tert-butoxycarbonyl) amino]-3-formylpyridin-2-yl} phenyl)sulfonyl] acetate | ¹H-NMR (CDCl₃) δ: 10.95 (1H, br s), 9.92 (1H, s), 8.63 (1H, d, J = 6.1 Hz), 8.46 (1H, d, J = 6.1 Hz), 8.14 (1H, s), 8.10 (1H, d, J = 7.9 Hz), 7.85 (1H, d, J = 7.9 Hz), 7.74 (1H, t, J = 7.9 Hz), 4.10 (2H, s), 1.56 (9H, s), 1.41 (9H, s). |
| 120 | 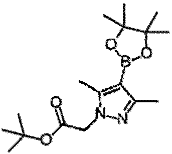 Reference Example 114 | 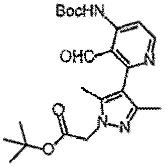 | tert-butyl (4-{4-[(tert-butoxycarbonyl) amino]-3-formylpyridin-2-yl}-3,5-dimethyl-1 H-pyrazol-1-yl)acetate | ¹H-NMR (CDCl₃) δ: 10.94 (1H, s), 9.86 (1H, s), 8.62 (1H, d, J = 6.1 Hz), 8.32 (1H, d, J = 6.1 Hz), 4.82 (1H, d, J = 17.2 Hz), 4.69 (1H, d, J = 17.2 Hz), 2.18 (3H, s), 2.17 (3H, s), 1.55 (9H, s), 1.49 (9H, s). |
| 121 | 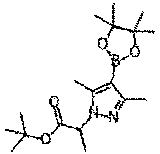 Reference Example 115 | 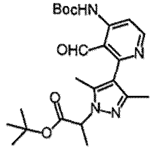 | tert-butyl 2-(4-{4-[(tert-butoxycarbonyl)amino]-3-formylpyridin-2-yl}-3,5-dimethyl-1 H-pyrazol-1-yl)propanoate | ¹H-NMR (CDCl₃) δ: 10.97-10.92 (1H, m), 9.87 (0.65H, s), 9.81 (0.35H, s), 8.66-8.62 (1H, m), 8.35-8.32 (1H, m), 4.93 (0.35H, q, J = 7.3 Hz), 4.82 (0.65H, q, J = 7.3 Hz), 2.21 (3H, s), 2.20-2.19 (3H, m), 1.85 (1.95H, d, J = 7.3 Hz), 1.80 (1.05H, d, J = 7.3 Hz), 1.57 (9H, s), 1.46 (9H, s). |
| 122 | 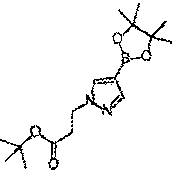 Reference Example 116 | 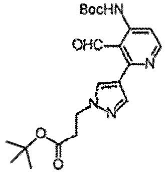 | tert-butyl 3-(4-{4-[(tert-butoxycarbonyl)amino]-3-formylpyridin-2-yl}-1 H-pyrazol-1-yl)propanoate | ¹H-NMR (CDCl₃) δ: 10.90 (1H, s), 10.19 (1H, s), 8.54 (1H, d, J = 6.1 Hz), 8.26 (1H, d, J = 6.1 Hz), 7.83 (1H, s), 7.75 (1H, s), 4.46 (2H, t, J = 6.7 Hz), 2.87 (2H, t, J = 6.7 Hz), 1.55 (9H, s), 1.43 (9H, s). |

### Example 1

### Production of 3-{6-chloro-4-[(3R)-3-methylmorpholin-4-yl]-1H-imidazo[4,5-c]pyridin-2-yl}-1,6-naphthyridin-2(1H)-one

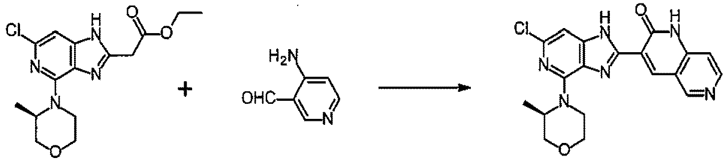

### 3-{6-Chloro-4-[(3R)-3-methylmorpholin-4-yl]-1H-imidazo[4,5-c]pyridin-2-yl}-1,6-naphthyridin-2(1H)-one

A mixture of the compound (3.60 g) obtained in Step 3 of Reference Example 1, 4-aminopyridin-3-carbaldehyde (1.30 g), piperidine (2.94 mL), and ethanol (55 mL) was stirred under reflux for 9 hours. The reaction mixture was cooled to room temperature, and the precipitated solid was collected by filtration. The obtained solid was washed twice with ethanol and dried under reduced pressure to obtain the title compound (3.76 g).
¹H-NMR (DMSO-D₆) δ: 12.84 (1H, br s), 12.73 (1H, br s), 9.13 (1H, s), 9.02 (1H, s), 8.55 (1H, d, J = 5.5 Hz), 7.31 (1H, d, J = 5.5 Hz), 6.99 (1H, s), 5.47-5.34 (1H, m), 4.96-4.75 (1H, m), 4.04-3.92 (1H, m), 3.84-3.69 (2H, m), 3.64-3.52 (1H, m), 3.43-3.35 (1H, m), 1.27 (3H, d, J = 6.7 Hz)., MS (m/z): 397 (M+H)⁺.

The following compounds were synthesized by performing the same operation as in Example 1 (Table 10-1 and Table 10-2).

**Table 10-1**

| Example No. | Production Material 1 | Production Material 2 | Structure of Compound Synthesized |
|---|---|---|---|
| 2 | 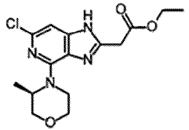 Reference Example 1, Step 3 | 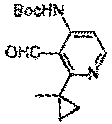 Reference Example 49, Step 2 | 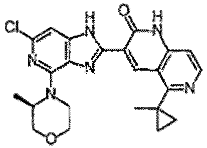 |
| 3 | 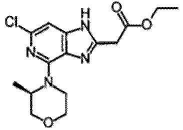 Reference Example 1, Step 3 | 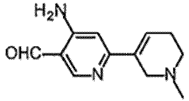 Reference Example 69 | 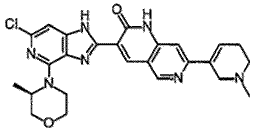 |
| 4 | 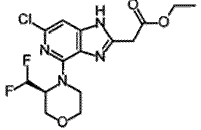 Reference Example 2, Step 6 | 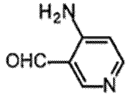 | 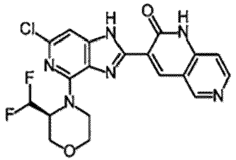 |
| 5 | 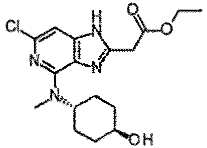 Reference Example 3, Step 3 | 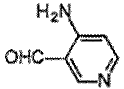 | 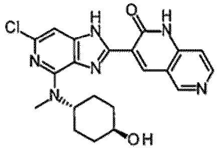 |
| 6 | 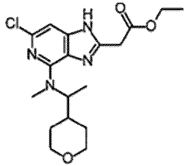 Reference Example 6, Step 3 | 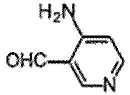 | 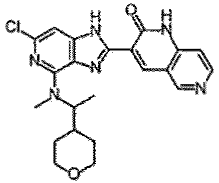 |
| 7 | 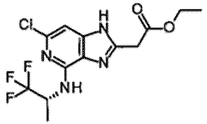 Reference Example 4, Step 3 | 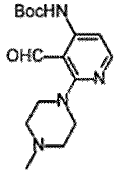 Reference Example 51 | 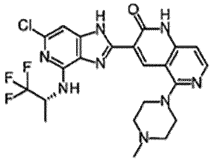 |
| 8 | 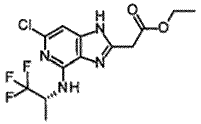 Reference Example 4, Step 3 | 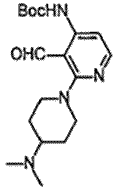 Reference Example 52 | 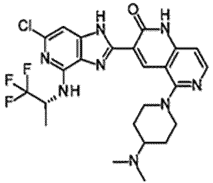 |

**Table 10-2**

| Example No. | Name of Compound Synthesized | Spectral Data |
|---|---|---|
| 2 | 3-{6-chloro-4-[(3R)-3-methylmorpholin-4-yl]-1H-imidazo[4,5-c]pyridin-2-yl}-5-(1-methylcyclopropyl)-1,6-naphthyridin-2(1H)-one | ¹H-NMR (DMSO-D₆) δ: 12.83-12.59 (2H, m), 9.43 (1H, s), 8.41 (1H, d, J = 5.5 Hz), 7.19 (1H, d, J = 5.5 Hz), 6.94 (1H, s), 5.60-5.42 (1H, m), 4.88-4.71 (1H, m), 4.04-3.93 (1H, m), 3.81-3.69 (2H, m), 3.62-3.52 (1H, m), 3.39-3.34 (1H, m), 1.45 (3H, s), 1.32 (3H, d, J = 6.7 Hz), 1.05-0.98 (2H, m), 0.92-0.85 (2H, m)., MS (m/z): 451 (M+H)⁺. |
| 3 | 3-{6-chloro-4-[(3R)-3-methylmorpholin-4-yl]-1H-imidazo[4,5-c]pyridin-2-yl}-7-(1-methyl-1,2,5,6-tetrahydropyridin-3-yl)-1,6-naphthyridin-2(1H)-one | ¹H-NMR (DMSO-D₆) δ: 12.79 (1H, br s), 12.58 (1H, br s), 9.05 (1H, s), 8.96 (1H, s), 7.28 (1H, s), 6.99 (1H, s), 6.84 (1H, br s), 5.50-5.34 (1H, m), 4.93-4.75 (1H, m), 4.04-3.92 (1H, m), 3.83-3.71 (2H, m), 3.64-3.52 (1H, m), 3.43-3.20 (3H, m), 2.54-2.44 (2H, m), 2.42-2.29 (5H, m), 1.27 (3H, d, J = 6.7 Hz)., MS (m/z): 492 (M+H)⁺. |
| 4 | 3-{6-chloro-4-[(3S)-3-(difluoromethyl)morpholin-4-yl]-1H-imidazo[4,5-c]pyridin-2-yl}-1,6-naphthyridin-2(1H)-one | ¹H-NMR (DMSO-D₆) δ: 12.89 (1H, br s), 12.72 (1H, br s), 9.10 (1H, s), 8.99 (1H, s), 8.55-8.50 (1H, m), 7.29 (1H, d, J = 4.9 Hz), 7.05 (1H, s), 6.53 (1H, td, J = 55.5, 6.1 Hz), 5.95-5.73 (1H, m), 5.01-4.70 (1H, m), 4.13 (1H, d, J = 12.3 Hz), 4.04-3.95 (1H, m), 3.89-3.79 (1H, m), 3.66-3.56 (1H, m), 3.56-3.43 (1H, m)., MS (m/z): 433 (M+H)⁺. |
| 5 | 3-{6-chloro-4-[(trans-4-hydroxycyclohexyl)(methyl)amino]-1H-imidazo[4,5-c]pyridin-2-yl}-1,6-naphthyridin-2(1H)-one | ¹H-NMR (DMSO-D₆) δ: 12.73 (2H, s), 9.08 (1H, s), 8.93 (1H, s), 8.55 (1H, d, J = 5.5 Hz), 7.32 (1H, d, J = 5.5 Hz), 6.87 (1H, s), 5.33-5.13 (1H, m), 4.59 (1H, d, J = 4.9 Hz), 3.51-3.41 (1H, m), 3.29 (3H, s), 2.02-1.91 (2H, m), 1.78-1.65 (4H, m), 1.50-1.36 (2H, m)., MS (m/z): 425 (M+H)⁺. |
| 6 | 3-(6-chloro-4-{methyl[1-(tetrahydro-2H-pyran-4-yl)ethyl]amino}-1H-imidazo[4,5-c]pyridin-2-yl)-1,6-naphthyridin-2(1H)-one | ¹H-NMR (DMSO-D₆) δ: 12.73 (1H, s), 12.72 (1H, s), 9.13 (1H, s), 8.94 (1H, s), 8.55 (1H, d, J = 6.1 Hz), 7.31 (1H, d, J = 6.1 Hz), 6.87 (1H, s), 5.84-5.26 (1H, m), 3.98-3.89 (1H, m), 3.80-3.71 (1H, m), 3.32-3.13 (5H, m), 1.92-1.66 (2H, m), 1.47-1.11 (6H, m)., MS (m/z): 439 (M+H)⁺. |
| 7 | 3-(6-chloro-4-{[(2R)-1,1,1-trifluoropropan-2-yl]amino}-1H-imidazo[4,5-c]pyridin-2-yl)-5-(4-methylpiperazin-1-yl)-1,6-naphthyridin-2(1H)-one | ¹H-NMR (DMSO-D₆) δ: 12.98-12.54 (2H, m), 9.09-8.90 (1H, m), 8.31-7.55 (2H, m), 7.04-6.92 (1H, m), 6.93-6.83 (1H, m), 5.86-4.90 (1H, m), 3.43-3.33 (4H, m), 2.59-2.51 (4H, m), 2.29-2.18 (3H, m), 1.49-1.33 (3H, m)., MS (m/z): 507 (M+H)⁺. |
| 8 | 3-(6-chloro-4-{[(2R)-1,1,1-trifluoropropan-2-yl]amino}-1H-imidazo[4,5-c]pyridin-2-yl)-5-[4-(dimethylamino)piperidin-1-yl]-1,6-naphthyridin-2(1H)-one | ¹H-NMR (DMSO-D₆) δ: 12.97-12.50 (2H, m), 9.03-8.92 (1H, m), 8.24-7.54 (2H, m), 7.05-6.92 (1H, m), 6.92-6.78 (1H, m), 5.69-4.99 (1H, m), 3.89-3.74 (2H, m), 3.08-2.91 (2H, m), 2.21 (6H, s), 1.96-1.80 (2H, m), 1.71-1.53 (2H, m), 1.46-1.38 (3H, m)., MS (m/z): 535 (M+H)⁺. |

### Example 9

### Production of 3-[4-(5-amino-3,3-difluoropiperidin-1-yl)-6-chloro-1H-imidazo[4,5-c]pyridin-2-yl]-1,6-naphthyridin-2(1H)-one trifluoroacetate

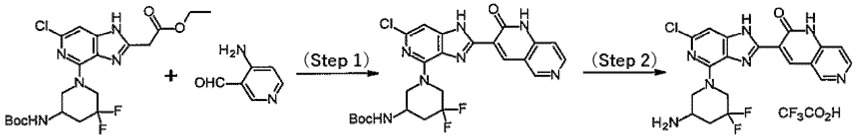

### (Step 1) tert-Butyl {1-[6-chloro-2-(2-oxo-1,2-dihydro-1,6-naphthyridin-3-yl)-1H-imidazo[4,5-c]pyridin-4-yl]-5,5-difluoropiperidin-3-yl}carbamate

The title compound was obtained by using the compound obtained in Step 3 of Reference Example 9 and 4-aminopyridin-3-carbaldehyde as production materials and performing the same operation as in Step 1 of Example 1.
¹H-NMR (DMSO-D₆) δ: 9.03 (1H, s), 9.00 (1H, s), 8.54 (1H, d, J = 6.1 Hz), 7.33 (1H, d, J = 6.1 Hz), 7.06 (1H, s), 6.84 (1H, br s), 5.59-5.41 (1H, m), 5.31-5.18 (1H, m), 3.88-3.73 (1H, m), 3.69-3.52 (1H, m), 2.53-2.34 (2H, m), 2.31-1.96 (1H, m), 1.42 (9H, s).

### (Step 2) 3-[4-(5-Amino-3,3-difluoropiperidin-1-yl)-6-chloro-1H-imidazo[4,5-c]pyridin-2-yl]-1,6-naphthyridin-2(1H)-one trifluoroacetate

To the compound (33.8 mg) obtained in Step 1 above was added trifluoroacetic acid (650 µL) at room temperature, and the mixture was stirred at the same temperature for 2 hours. The reaction mixture was concentrated under reduced pressure, and excess trifluoroacetic acid remaining was removed by azeotropic distillation with toluene. Ethyl acetate was added to the residue to solidify the residue. The solid was collected by filtration, washed with hexane, and then dried under reduced pressure to obtain the title compound (31.5 mg).
¹H-NMR (DMSO-D₆) δ: 13.02-12.87 (2H, m), 9.21 (1H, s), 9.10 (1H, s), 8.61 (1H, d, J = 5.5 Hz), 8.33-8.16 (3H, br s), 7.40 (1H, d, J = 5.5 Hz), 7.13 (1H, s), 5.69-5.49 (1H, m), 5.04-4.89 (1H, m), 4.19-4.03 (1H, m), 3.66-3.43 (2H, m), 2.45-2.13 (2H, m)., MS (m/z): 432 (M+H)⁺.

### Example 10

### Production of 3-{6-chloro-4-[(2R,4R)-4-fluoro-2-methylpyrrolidin-1-yl]-1H-imidazo[4,5-c]pyridin-2-yl}-1,6-naphthyridin-2(1H)-one

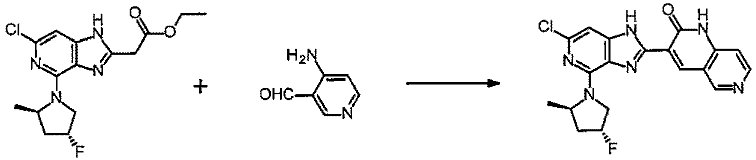

### 3-{6-Chloro-4-[(2R,4R)-4-fluoro-2-methylpyrrolidin-1-yl]-1H-imidazo[4,5-c]pyridin-2-yl}-1,6-naphthyridin-2(1H)-one

A mixture of the compound (60 mg) obtained in Reference Example 19, 4-aminopyridin-3-carbaldehyde (24 mg), piperidine (50 µL), and ethanol (5.0 mL) was stirred at 90°C for 6 hours. After cooling the reaction mixture to room temperature, the solvent was evaporated under reduced pressure. A mixture of n-hexane/ethyl acetate was added to the residue, and the solid was collected by filtration to obtain the title compound (48 mg). ¹H-NMR (DMSO-D₆) δ: 12.80 (2H, br s), 9.12 (1H, s), 9.03 (1H, s), 8.54 (1H, d, J = 6.1 Hz), 7.31 (1H, d, J = 6.1 Hz), 6.92 (1H, s), 5.45 (1H, br d, J = 54.3 Hz), 4.84-4.59 (2H, m), 4.14-3.96 (1H, m), 2.53-2.42 (1H, m), 2.11-1.78 (1H, m), 1.35 (3H, d, J = 6.1 Hz).

The following compounds were synthesized by performing the same operation as in Example 10 (Table 11-1 and Table 11-2).

**Table 11-1**

| Example No. | Production Material 1 | Production Material 2 | Structure of Compound Synthesized |
|---|---|---|---|
| 11 | 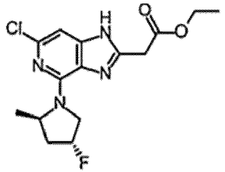 Reference Example 19 | 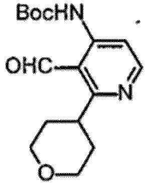 Reference Example 50, Step 3 | 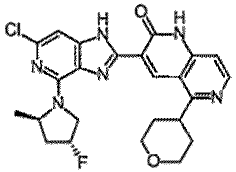 |
| 12 | 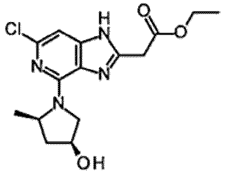 Reference Example 20 | 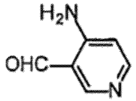 | 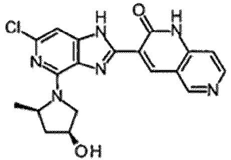 |
| 13 | 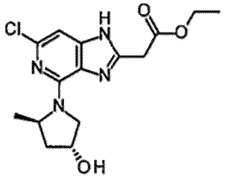 Reference Example 18, Step 4 | 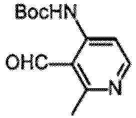 Reference Example 46 | 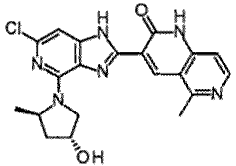 |
| 14 | 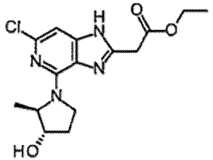 Reference Example 21 | 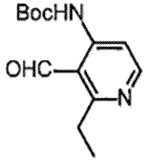 Reference Example 48, Step 2 | 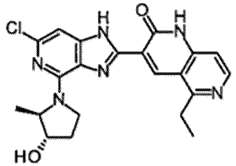 |
| 15 | 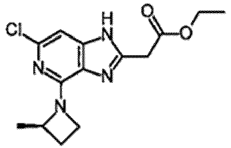 Reference Example 22 | 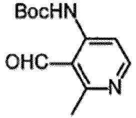 Reference Example 46 | 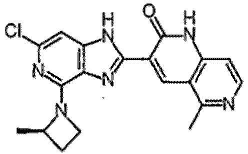 |
| 16 | 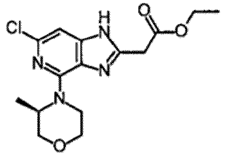 Reference Example 1, Step 3 | 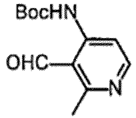 Reference Example 46 | 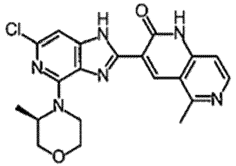 |

**Table 11-2**

| Example No. | Name of Compound Synthesized | Spectral Data |
|---|---|---|
| 11 | 3-{6-chloro-4-[(2R,4R)-4-fluoro-2-methylpyrrolidin-1-yl]-1H-imidazo[4,5-c]pyridin-2-yl}-5-(tetrahydro-2H-pyran-4-yl)-1,6-naphthyridin-2(1H)-one | ¹H-NMR (DMSO-D₆) δ: 9.18 (1H, s), 8.50 (1H, d, J = 6.1 Hz), 7.21 (1H, d, J = 6.1 Hz), 6.91 (1H, s), 5.45 (1H, d, J = 54.3 Hz), 4.83 (1H, br s), 4.58 (1H, br s), 4.09-3.94 (3H, m), 3.76-3.55 (3H, m), 2.49-2.44 (1H, m), 2.08-1.89 (3H, m), 1.79-1.71 (2H, m), 1.39 (3H, d, J = 6.1 Hz). |
| 12 | 3-{6-chloro-4-[(2R,4S)-4-hydroxy-2-methylpyrrolidin-1-yl]-1H-imidazo[4,5-c]pyridin-2-yl}-1,6-naphthyridin-2(1H)-one | ¹H-NMR (DMSO-D₆) δ: 12.72 (2H, br s), 9.12 (1H, s), 8.97 (1H, s), 8.53 (1H, d, J = 5.5 Hz), 7.30 (1H, d, J = 5.5 Hz), 6.84 (1H, s), 5.07 (1H, br s), 4.79 (1H, br s), 4.45-4.35 (1H, m), 4.19-3.90 (2H, m), 2.27 (1H, br s), 1.73 (1H, d, J = 12.2 Hz), 1.46 (3H, d, J = 5.5 Hz). |
| 13 | 3-{6-chloro-4-[(2R,4R)-4-hydroxy-2-methylpyrrolidin-1-yl]-1H-imidazo[4,5-c]pyridin-2-yl}-5-methyl-1,6-naphthyridin-2(1H)-one | ¹H-NMR (DMSO-D₆) δ: 12.71 (2H, br s), 9.01 (1H, s), 8.39 (1H, d, J = 5.5 Hz), 7.19 (1H, d, J = 5.5 Hz), 6.85 (1H, s), 4.96-4.91 (1H, m), 4.74 (1H, br s), 4.45 (1H, br s), 4.07-4.01 (1H, m), 2.81 (3H, s), 2.11-2.05 (1H, m), 1.88-1.79 (1H, m), 1.33 (3H, d, J = 6.1 Hz). |
| 14 | 3-{6-chloro-4-[(2R,3S)-3-hydroxy-2-methylpyrrolidin-1-yl]-1H-imidazo[4,5-c]pyridin-2-yl}-5-ethyl-1,6-naphthyridin-2(1H)-one | ¹H-NMR (DMSO-D₆) δ: 12.69 (2H, br s), 9.06 (1H, s), 8.45 (1H, d, J = 5.5 Hz), 7.20 (1H, d, J = 5.5 Hz), 6.83 (1H, s), 4.95 (1H, s), 4.72 (1H, br s), 4.09 (1H, s), 3.83 (1H, br s), 3.44 (1H, br s), 3.20-3.13 (1H, m), 2.25-2.13 (2H, m), 1.94-1.84 (1H, m), 1.34 (3H, t, J = 7.9 Hz), 1.29 (3H, d, J = 6.7 Hz). |
| 15 | 3-{6-chloro-4-[(2R)-2-methylazetidin-1-yl]-1H-imidazo[4,5-c]pyridin-2-yl}-5-methyl-1,6-naphthyridin-2(1H)-one | ¹H-NMR (DMSO-D₆) δ: 12.71 (1H, br s), 12.69 (1H, br s), 9.02 (1H, s), 8.40 (1H, d, J = 5.5 Hz), 7.19 (1H, d, J = 5.5 Hz), 6.89 (1H, s), 4.80-4.69 (1H, m), 4.33 (1H, br s), 4.19 (1H, d, J = 7.9 Hz), 2.79 (3H, s), 2.57-2.50 (1H, m), 2.12-1.99 (1H, m), 1.66 (3H, d, J = 5.5 Hz). |
| 16 | 3-{6-chloro-4-[(3R)-3-methylmorpholin-4-yl]-1H-imidazo[4,5-c]pyridin-2-yl}-5-methyl-1,6-naphthyridin-2(1H)-one | ¹H-NMR (DMSO-D₆) δ: 12.83 (1H, s), 12.70 (1H, s), 8.99 (1H, s), 8.40 (1H, d, J = 5.5 Hz), 7.19 (1H, d, J = 5.5 Hz), 6.98 (1H, s), 5.45-5.31 (1H, m), 4.93-4.79 (1H, m), 4.03-3.95 (1H, m), 3.81-3.72 (2H, m), 3.62-3.36 (2H, m), 2.81 (3H, s), 1.29 (3H, d, J = 6.7 Hz)., MS (m/z): 411 (M+H)⁺. |

### Example 17

### Production of 3-[6-chloro-4-(tetrahydro-2H-pyran-4-yl)-1H-imidazo[4,5-c]pyridin-2-yl]-5-methyl-1,6-naphthyridin-2(1H)-one

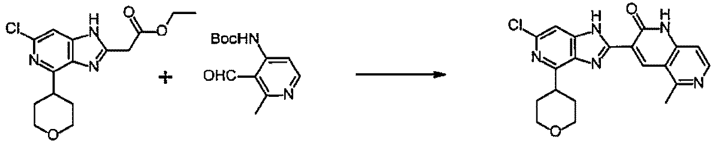

### 3-[6-Chloro-4-(tetrahydro-2H-pyran-4-yl)-1H-imidazo[4,5-c]pyridin-2-yl]-5-methyl-1,6-naphthyridin-2(1H)-one

A mixture of the compound (2.00 g) obtained in Step 3 of Reference Example 23, the compound (1.46 g) obtained in Reference Example 46, piperidine (1.70 mL), and ethanol (35 mL) was stirred under reflux for 4.5 hours. The reaction mixture was cooled to room temperature, and then the precipitated solid was collected by filtration. The obtained solid was washed twice with ethanol and then dried under reduced pressure to obtain the title compound (2.27 g).
¹H-NMR (DMSO-D₆) δ: 13.03 (1H, br s), 12.78 (1H, br s), 9.14 (1H, s), 8.43 (1H, d, J = 6.1 Hz), 7.54 (1H, s), 7.21 (1H, d, J = 6.1 Hz), 4.05-4.95 (2H, m), 3.82-3.70 (1H, m), 3.64-3.51 (2H, m), 2.86 (3H, s), 2.08-1.93 (2H, m), 1.86-1.75 (2H, m)., MS (m/z): 396 (M+H)⁺.

The following compounds were synthesized by performing the same operation as in Example 17 (Table 12-1 and Table 12-2).

**Table 12-1**

| Example No. | Production Material 1 | Production Material 2 | Structure of Compound Synthesized |
|---|---|---|---|
| 18 | 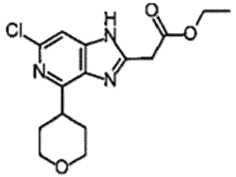 Reference Example 23, Step 3 | 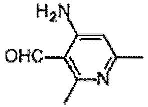 Reference Example 47 | 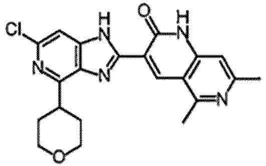 |
| 19 | 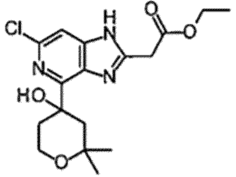 Reference Example 24, Step 5 | 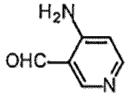 | 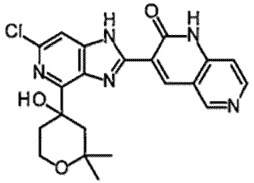 |
| 20 | 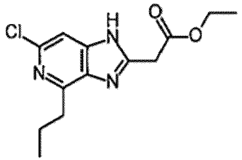 Reference Example 35 | 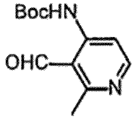 Reference Example 46 | 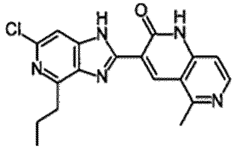 |
| 21 | 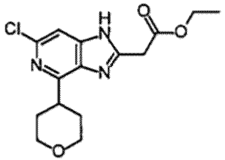 Reference Example 23, Step 3 | 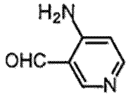 | 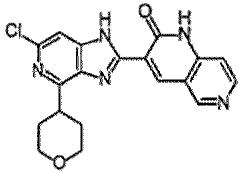 |
| 22 | 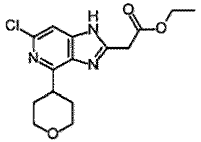 Reference Example 23, Step 3 | 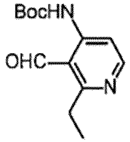 Reference Example 48, Step 2 | 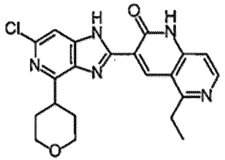 |
| 23 | 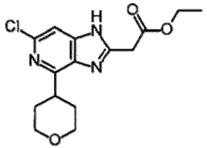 Reference Example 23, Step 3 | 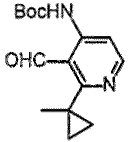 Reference Example 49, Step 2 | 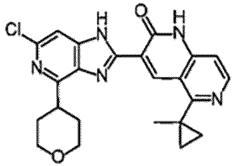 |

**Table 12-2**

| Example No. | Name of Compound Synthesized | Spectral Data |
|---|---|---|
| 18 | 3-[6-chloro-4-(tetrahydro-2H-pyran-4-yl)-1H-imidazo[4,5-c]pyridin-2-yl]-5,7-dimethyl-1,6-naphthyridin-2(1H)-one | ¹H-NMR (DMSO-D₆) δ: 9.11 (1H, s), 7.54 (1H, s), 7.03 (1H, s), 4.06-3.96 (2H, m), 3.86-3.70 (1H, m), 3.64-3.51 (2H, m), 2.82 (3H, s), 2.51 (3H, s), 2.07-1.93 (2H, m), 1.88-1.75 (2H, m)., MS (m/z): 410 (M+H)⁺. |
| 19 | 3-[6-chloro-4-(4-hydroxy-2,2-dimethyltetrahydro-2H-pyran-4-yl)-1H-imidazo[4,5-c]pyridin-2-yl]-1,6-naphthyridin-2(1H)-one | ¹H-NMR (DMSO-D₆) δ: 12.99-12.59 (2H, m), 9.35 (1H, s), 9.22 (1H, s), 8.61 (1H, d, J = 5.5 Hz), 7.66 (1H, s), 7.41-7.31 (1H, m), 6.21 (1H, br s), 4.12-3.99 (1H, m), 3.77-3.61 (1H, m), 2.24-2.01 (2H, m), 1.84-1.61 (2H, m), 1.47 (3H, s), 1.15 (3H, s)., MS (m/z): 426 (M+H)⁺. |
| 20 | 3-(6-chloro-4-propyl-1H-imidazo[4,5-c]pyridin-2-yl)-5-methyl-1,6-naphthyridin-2(1H)-one | ¹H-NMR (DMSO-D₆) δ: 12.99 (1H, s), 12.77 (1H, s), 9.14 (1H, s), 8.43 (1H, d, J = 6.1 Hz), 7.52 (1H, s), 7.20 (1H, d, J = 6.1 Hz), 3.12 (2H, t, J = 7.3 Hz), 2.85 (3H, s), 1.92-1.77 (2H, m), 0.98 (3H, t, J = 7.3 Hz)., MS (m/z): 354 (M+H)⁺. |
| 21 | 3-[6-chloro-4-(tetrahydro-2H-pyran-4-yl)-1H-imidazo[4,5-c]pyridin-2-yl]-1,6-naphthyridin-2(1H)-one | ¹H-NMR (DMSO-D₆) δ: 13.02 (1H, br s), 12.79 (1H, br s), 9.22 (1H, s), 9.20 (1H, s), 8.58 (1H, d, J = 5.9 Hz), 7.55 (1H, s), 7.33 (1H, d, J = 5.9 Hz), 4.09-3.93 (2H, m), 3.82-3.67 (1H, m), 3.62-3.49 (2H, m), 2.14-1.95 (2H, m), 1.88-1.74 (2H, m)., MS (m/z): 382 (M+H)⁺. |
| 22 | 3-[6-chloro-4-(tetrahydro-2H-pyran-4-yl)-1H-imidazo[4,5-c]pyridin-2-yl]-5-ethyl-1,6-naphthyridin-2(1H)-one | ¹H-NMR (DMSO-D₆) δ: 13.01 (1H, br s), 12.76 (1H, br s), 9.15 (1H, s), 8.47 (1H, d, J = 5.5 Hz), 7.52 (1H, s), 7.19 (1H, d, J = 5.5 Hz), 4.03-3.93 (2H, m), 3.81-3.69 (1H, m), 3.55 (2H, t, J = 11.0 Hz), 3.20 (2H, q, J = 7.6 Hz), 2.07-1.90 (2H, m), 1.84-1.74 (2H, m), 1.33 (3H, t, J = 7.6 Hz)., MS (m/z): 410 (M+H)⁺. |
| 23 | 3-[6-chloro-4-(tetrahydro-2H-pyran-4-yl)-1H-imidazo[4,5,c]pyridin-2-yl]-5-(1-methylcyclopropyl)-1,6-naphthyridin-2(1H)-one | ¹H-NMR (DMSO-D₆) δ: 12.89 (1H, br s), 12.60 (1H, br s), 9.54 (1H, s), 8.43 (1H, d, J = 5.5 Hz), 7.53 (1H, s), 7.22 (1H, d, J = 5.5 Hz), 4.05-3.95 (2H, m), 3.75-3.61 (1H, m), 3.55 (2H, t, J = 10.7 Hz), 2.14-1.95 (2H, m), 1.95-1.80 (2H, m), 1.50 (3H, s), 1.08-1.02 (2H, m), 0.96-0.89 (2H, m)., MS (m/z): 436 (M+H)⁺. |

### Example 24

### Production of 3-[4-(3-aminophenyl)-6-chloro-1H-imidazo[4,5-c]pyridin-2-yl]-1H-1,6-naphthyridin-2-one trifluoroacetate

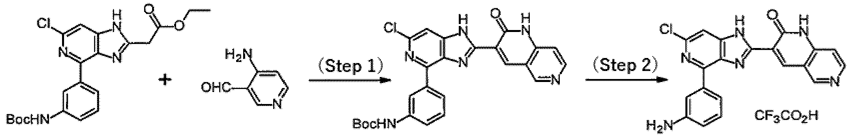

### (Steps 1 and 2) 3-[4-(3-Aminophenyl)-6-chloro-1H-imidazo[4,5-c]pyridin-2-yl]-1H-1,6-naphthyridin-2-one trifluoroacetate

The title compound was obtained by using the compound obtained in Step 3 of Reference Example 25 as a production material and performing the same operations as in Example 1 and Step 2 of Example 9.
¹H-NMR (DMSO-D₆) δ: 13.24 (2H, br s), 9.38 (1H, s), 9.32 (1H, s), 8.80-8.70 (1H, m), 8.67 (1H, d, J = 5.9 Hz), 8.51-8.41 (1H, m), 7.70 (1H, s), 7.56 (1H, t, J = 7.8 Hz), 7.49 (1H, d, J = 5.9 Hz), 7.22 (1H, dd, J = 7.8, 1.2 Hz)., MS (m/z): 389 (M+H)⁺.

### Example 25

### Production of 3-[6-chloro-4-(2-fluorophenyl)-1H-imidazo[4,5-c]pyridin-2-yl]-1,6-naphthyridin-2(1H)-one

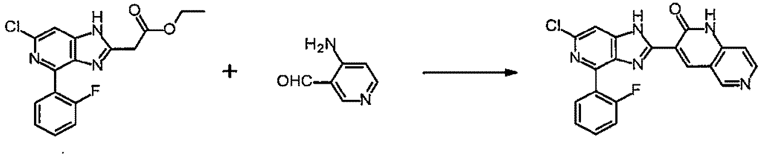

### 3-[6-Chloro-4-(2-fluorophenyl)-1H-imidazo[4,5-c]pyridin-2-yl]-1,6-naphthyridin-2(1H)-one

The title compound was obtained by using the compound obtained in Step 3 of Reference Example 26 as a production material and performing the same operation as in Example 1.
¹H-NMR (DMSO-D₆) δ: 13.16 (1H, s), 12.79 (1H, s), 9.15 (1H, s), 9.08 (1H, s), 8.54 (1H, d, J = 6.1 Hz), 7.79 (1H, td, J = 7.3, 1.8 Hz), 7.73 (1H, s), 7.60-7.56 (1H, m), 7.41-7.36 (2H, m), 7.30 (1H, d, J = 6.1 Hz)., MS (m/z): 392 (M+H)⁺.

### Example 26

### Production of 3-(6-chloro-4-methyl-1H-imidazo[4,5-c]pyridin-2-yl)-5-{2-[(²H₃)methyloxy]phenyl}-1,6-naphthyridin-2(1H)-one

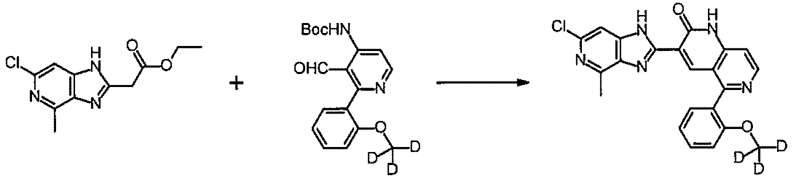

### 3-(6-Chloro-4-methyl-1H-imidazo[4,5-c]pyridin-2-yl)-5-{2-[(²H₃)methyloxy]phenyl}-1,6-naphthyridin-2(1H)-one

A mixture of the compound (34 mg) obtained in Step 3 of Reference Example 29, the compound (40 mg) obtained in Reference Example 57, piperidine (24 µL), and ethanol (1.5 mL) was stirred at 80°C for 5 hours. After cooling the reaction mixture to room temperature, the precipitated solid was collected by filtration. The obtained solid was washed with ethanol, and then dried under reduced pressure to obtain the title compound (48 mg).
¹H-NMR (DMSO-D₆) δ: 12.97 (1H, s), 12.87 (1H, s), 8.68 (1H, s), 8.64 (1H, d, J = 5.5 Hz), 7.64-7.56 (1H, m), 7.49 (1H, s), 7.46-7.40 (1H, m), 7.36 (1H, d, J = 5.5 Hz), 7.31 (1H, d, J = 7.9 Hz), 7.22-7.15 (1H, m), 2.63 (3H, s)., MS (m/z): 421 (M+H)⁺.

The following compounds were synthesized by performing the same operation as in Example 26 (Table 13-1 and Table 13-2).

**Table 13-1**

| Exampl e No. | Production Material 1 | Production Material 2 | Structure of Compound Synthesized |
|---|---|---|---|
| 27 | 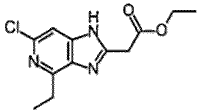 | 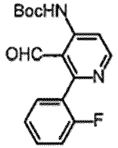 | 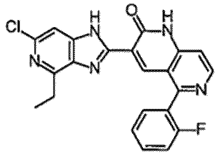 |
| | Reference Example 30, Step 3 | Reference Example 58 | |
| 28 | 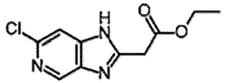 | 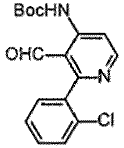 | 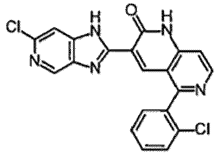 |
| | Reference Example 28 | Reference Example 59 | |
| 29 | 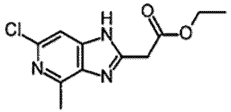 | 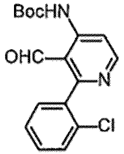 | 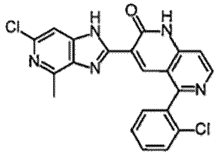 |
| | Reference Example 29, Step 3 | Reference Example 59 | |
| 30 | 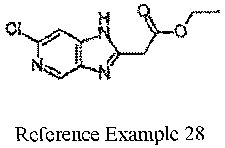 | 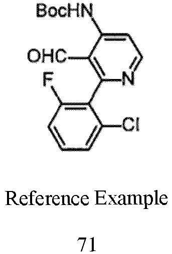 | 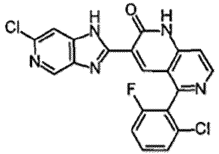 |
| 31 | 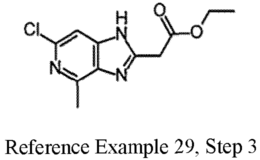 | 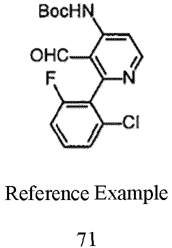 | 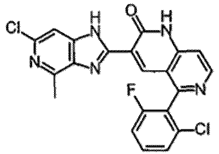 |
| 32 | 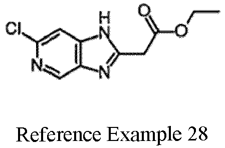 | 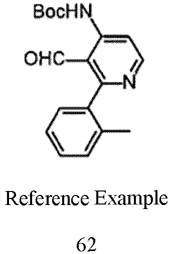 | 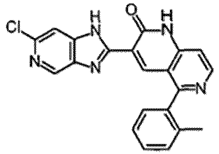 |
| 33 | 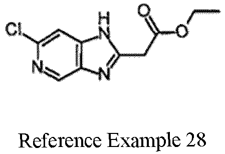 | 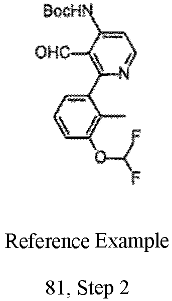 | 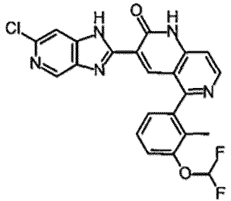 |
| 34 | 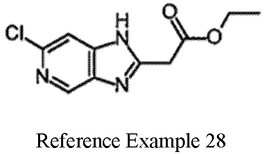 | 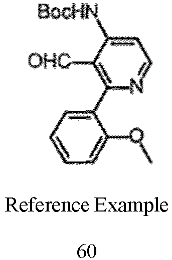 | 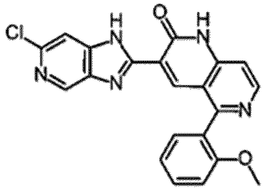 |
| 35 | 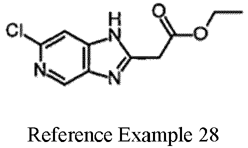 | 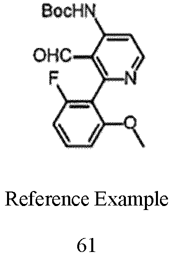 | 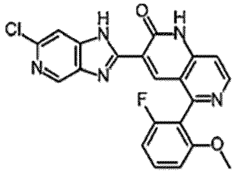 |
| 36 | 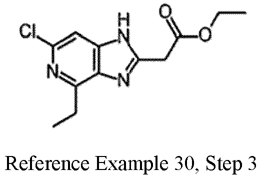 | 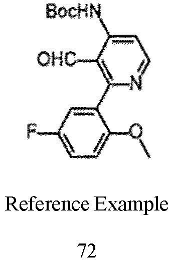 | 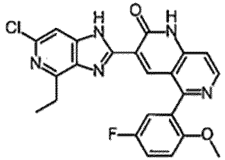 |
| 37 | 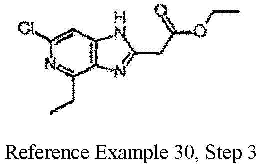 | 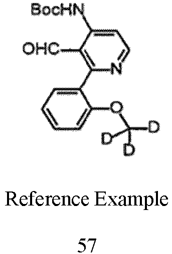 | 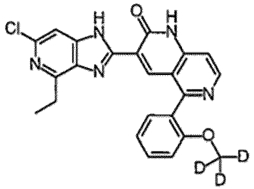 |
| 38 | 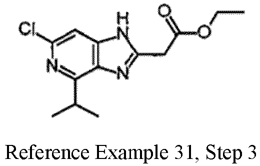 | 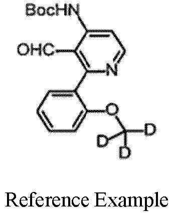 | 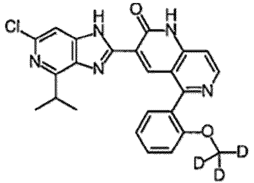 |
| | | 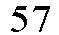 | |
| 39 | 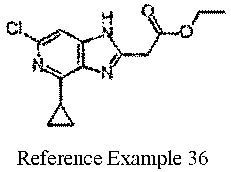 | 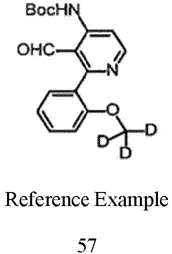 | 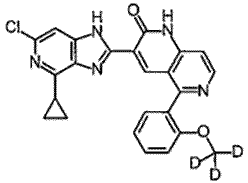 |
| 40 | 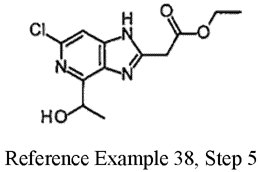 | 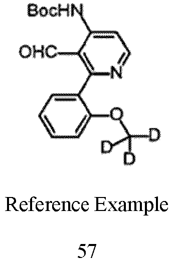 | 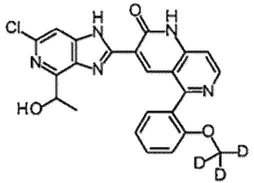 |
| 41 | 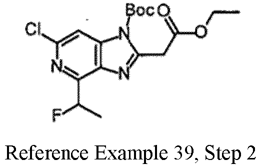 | 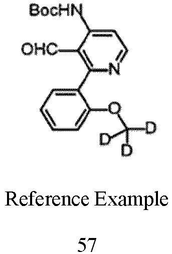 | 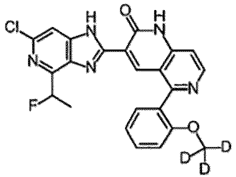 |
| 42 | 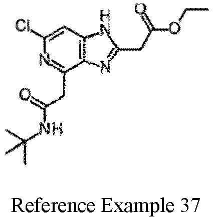 | 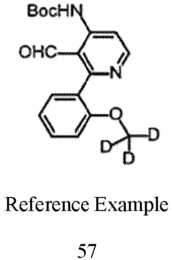 | 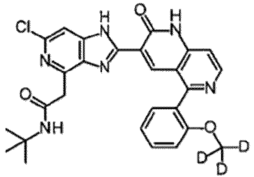 |
| 43 | 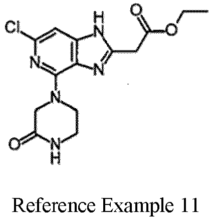 | 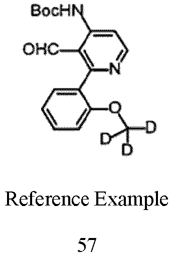 | 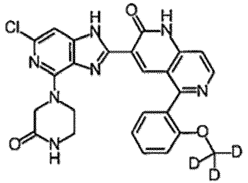 |
| 44 | 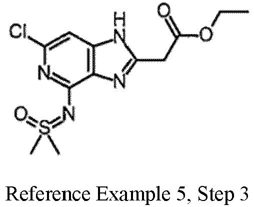 | 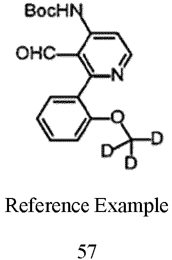 | 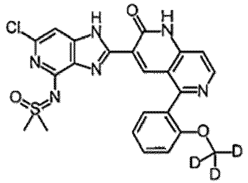 |
| 45 | 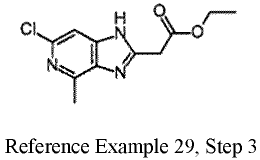 | 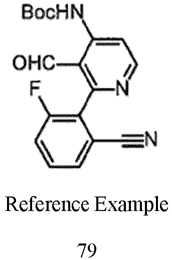 | 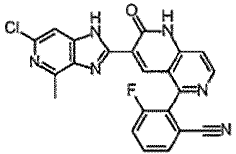 |
| 46 | 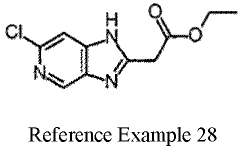 | 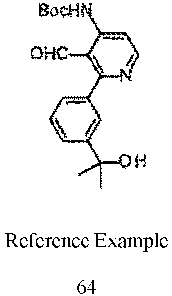 | 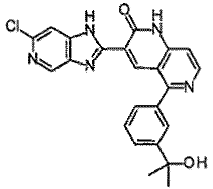 |
| 47 | 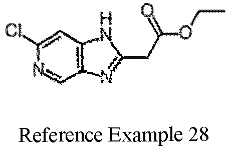 | 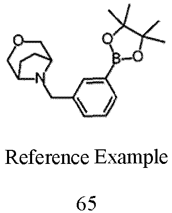 | 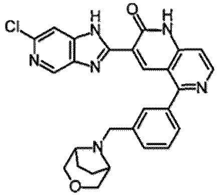 |
| 48 | 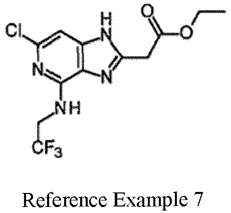 | 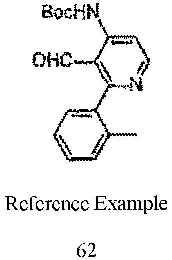 | 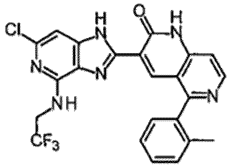 |
| 49 | 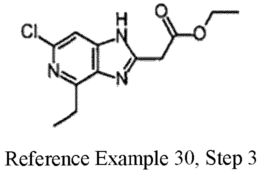 | 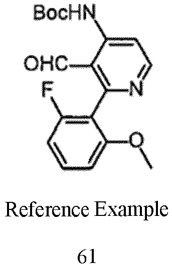 | 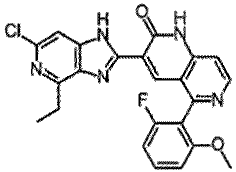 |
| 50 | 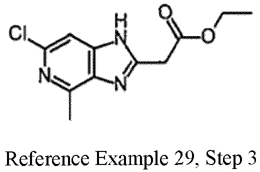 | 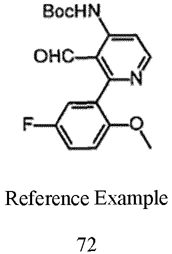 | 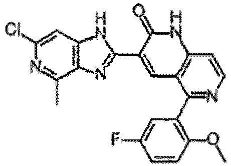 |
| 51 | 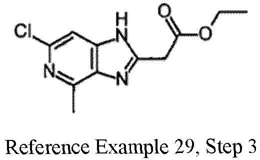 | 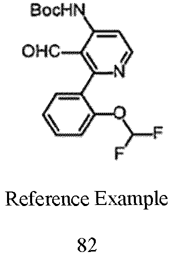 | 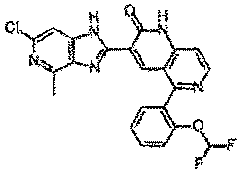 |
| 52 | 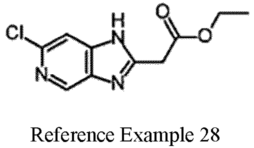 | 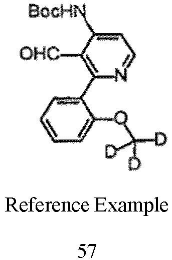 | 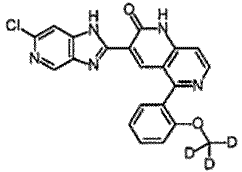 |
| 53 | 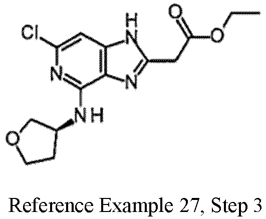 | 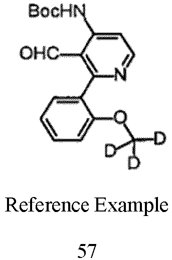 | 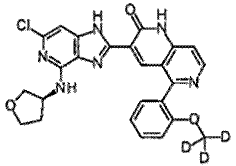 |
| 54 | 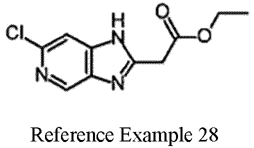 | 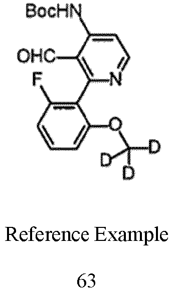 | 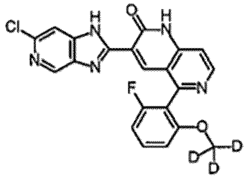 |
| 55 | 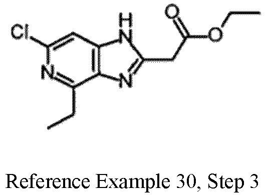 | 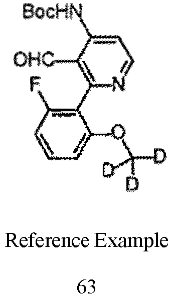 | 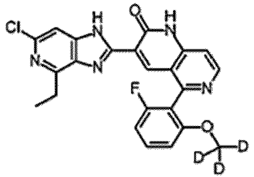 |

**Table 13-2**

| Example No. | Name of Compound Synthesized | Spectral Data |
|---|---|---|
| 27 | 3-(6-chloro-4-ethyl-1H-imidazo[4,5-c]pyridin-2-yl)-5-(2-fluorophenyl)-1,6-naphthyridin-2(1H)-one | ¹H-NMR (DMSO-D₆) δ: 13.00 (2H, s), 8.75-8.67 (2H, m), 7.74-7.39 (6H, m), 3.02 (2H, q, J = 7.3 Hz), 1.28 (3H, t, J = 7.3 Hz)., MS (m/z): 420 (M+H)⁺. |
| 28 | 3-(6-chloro-1H-imidazo [4,5-c]pyridin-2-yl)-5-(2-chlorophenyl)-1,6-naphthyridin-2(1H)-one | ¹H-NMR (DMSO-D₆) δ: 13.12-12.96 (1H, m), 8.81-8.72 (1H, m), 8.72-8.63 (1H, m), 8.63-8.51 (1H, m), 7.79-7.70 (1H, m), 7.70-7.52 (3H, m), 7.50-7.37 (1H, m)., MS (m/z): 408 (M+H)⁺. |
| 29 | 3-(6-chloro-4-methyl-1H-imidazo [4,5-c]pyridin-2-yl)-5-(2-chlorophenyl)-1,6-naphthyridin-2(1H)-one | ¹H-NMR (DMSO-D₆) δ: 13.00 (2H, s), 8.67 (1H, d, J = 5.5 Hz), 8.54 (1H, s), 7.76-7.41 (6H, m), 2.61 (3H, s)., MS (m/z): 422 (M+H)⁺. |
| 30 | 5-(2-chloro-6-fluorophenyl)-3-(6-chloro-1H-imidazo[4,5-c]pyridin-2-yl)-1,6-naphthyridin-2(1H)-one | ¹H-NMR (DMSO-D₆) δ: 13.08 (2H, s), 8.77 (1H, s), 8.73 (1H, d, J = 5.5 Hz), 8.49 (1H, s), 7.78-7.52 (4H, m), 7.48 (1H, d, J = 5.5 Hz)., MS (m/z): 426 (M+H)⁺. |
| 31 | 5-(2-chloro-6-fluorophenyl)-3-(6-chloro-4-methyl-1H-imidazo[4,5-c]pyridin-2-yl)-1,6-naphthyridin-2(1H)-one | ¹H-NMR (DMSO-D₆) δ: 13.07 (1H, s), 13.02 (1H, s), 8.72 (1H, d, J = 5.5 Hz), 8.46 (1H, s), 7.78-7.62 (2H, m), 7.59-7.45 (3H, m), 2.62 (3H, s)., MS (m/z): 440 (M+H)⁺. |
| 32 | 3-(6-chloro-1H-imidazo[4,5-c]pyridin-2-yl)-5-(2-methylphenyl)-1,6-naphthyridin-2(1H)-one | ¹H-NMR (DMSO-D₆) δ: 13.11-13.02 (2H, m), 8.74 (1H, s), 8.66 (1H, d, J = 6.1 Hz), 8.59 (1H, s), 7.72-7.65 (1H, m), 7.53-7.38 (5H, m), 7.36-7.31 (1H, m), 2.09 (3H, s)., MS (m/z): 388 (M+H)⁺. |
| 33 | 3-(6-chloro-1H-imidazo [4,5-c]pyridin-2-yl)-5-[3-(difluoromethoxy)-2-methylphenyl]-1,6-naphthyridin-2(1H)-one | ¹H-NMR (DMSO-D₆) δ: 8.75 (1H, s), 8.66 (1H, d, J = 5.5 Hz), 8.59 (1H, s), 7.68 (1H, br s), 7.49 (1H, t, J = 7.3 Hz), 7.43-7.38 (2H, m), 7.37 (1H, t, J = 74.5 Hz), 7.26 (1H, d, J = 7.3 Hz), 2.00 (3H, s). |
| 34 | 3-(6-chloro-1H-imidazo [4,5-c]pyridin-2-yl)-5-(2-methoxyphenyl)-1,6-naphthyridin-2(1H)-one | ¹H-NMR (DMSO-D₆) δ: 13.19-12.86 (2H, m), 8.75 (1H, s), 8.71-8.61 (2H, m), 7.72-7.64 (1H, m), 7.63-7.57 (1H, m), 7.41 (1H, dd, J = 8.5, 1.6 Hz, ), 7.37 (1H, d, J = 6.1 Hz), 7.30 (1H, br d, J = 7.8 Hz), 7.21-7.15 (1H, m), 3.71 (3H, s)., MS (m/z): 404 (M+H)⁺. |
| 35 | 3-(6-chloro-1H-imidazo [4,5-c]pyridin-2-yl)-5-(2-fluoro-6-methoxyphenyl)-1,6-naphthyridin-2(1H)-one | ¹H-NMR (DMSO-D₆) δ: 13.01 (2H, br s), 8.76 (1H, s), 8.67 (1H, d, J = 6.1 Hz), 8.55 (1H, s), 7.69 (1H, br s), 7.68-7.60 (1H, m), 7.41 (1H, d, J = 6.1 Hz), 7.16 (1H, d, J = 8.5 Hz), 7.08 (1H, t, J= 8.5 Hz), 3.72 (3H, s)., MS (m/z): 422 (M+H)⁺. |
| 36 | 3-(6-chloro-4-ethyl-1H-imidazo[4,5-c]pyridin-2-yl)-5-(5-fluoro-2-methoxyphenyl)-1,6-naphthyridin-2(1H)-one | ¹H-NMR (DMSO-D₆) δ: 12.95 (1H, br s), 12.87 (1H, br s), 8.64 (1H, s), 8.62 (1H, d, J = 5.5 Hz), 7.47 (1H, s), 7.43 (1H, td, J = 8.5, 3.3 Hz), 7.35 (1H, d, J = 5.5 Hz), 7.34-7.25 (2H, m), 3.71 (3H, s), 3.01 (2H, q, J = 7.6 Hz), 1.27 (3H, t, J = 7.6 Hz)., MS (m/z): 450 (M+H)⁺. |
| 37 | 3-(6-chloro-4-ethyl-1 H-imidazo[4,5-c]pyridin-2-yl)-5-{2-[(²H₃)methyloxy]phenyl}-1,6-naphthyridin-2(1H)-one | ¹H-NMR (DMSO-D₆) δ: 12.97 (1H, s), 12.87 (1H, s), 8.69 (1H, s), 8.64 (1H, d, J = 5.5 Hz), 7.64-7.57 (1H, m), 7.50 (1H, s), 7.46-7.42 (1H, m), 7.36 (1H, d, J = 5.5 Hz), 7.31 (1H, d, J = 7.9 Hz), 7.22-7.15 (1H, m), 3.10-2.94 (2H, m), 1.29 (3H, t, J = 7.6 Hz)., MS (m/z): 435 (M+H)⁺. |
| 38 | 3-[6-chloro-4-(propan-2-yl)-1H-imidazo[4,5-c]pyridin-2-yl]-5-{2-[(²H₃)methyloxy]phenyl}-1,6-naphthyridin-2(1H)-one | ¹H-NMR (DMSO-D₆) δ: 12.96 (1H, s), 12.86 (1H, s), 8.70 (1H, s), 8.64 (1H, d, J = 6.1 Hz), 7.64-7.57 (1H, m), 7.49 (1H, s), 7.47-7.42 (1H, m), 7.36 (1H, d, J = 6.1 Hz), 7.31 (1H, d, J = 7.9 Hz), 7.22-7.15 (1H, m), 3.64-3.57 (1H, m), 1.30 (6H, d, J = 6.7 Hz)., MS (m/z): 449 (M+H)⁺. |
| 39 | 3-(6-chloro-4-cyclopropyl-1H-imidazo[4,5-c]pyridin-2-yl)-5-{2-[(²H₃)methyloxy]phenyl}-1,6-naphthyridin-2(1H)-one | ¹H-NMR (DMSO-D₆) δ: 12.96 (1H, s), 12.87 (1H, s), 8.68 (1H, s), 8.64 (1H, d, J = 6.1 Hz), 7.62-7.56 (1H, m), 7.46-7.42 (1H, m), 7.39 (1H, s), 7.36 (1H, d, J = 6.1 Hz), 7.30 (1H, d, J = 8.0 Hz), 7.21-7.15 (1H, m), 2.64-2.56 (1H, m), 1.16-1.02 (4H, m)., MS (m/z): 447 (M+H)⁺. |
| 40 | 3-[6-chloro-4-(1-hydroxyethyl)-1H-imidazo[4,5-c]pyridin-2-yl]-5-{2-[(²H₃)methyloxy]phenyl}-1,6-naphthyridin-2(1H)-one | ¹H-NMR (DMSO-D₆) δ: 13.16-12.59 (2H, m), 8.74-8.60 (2H, m), 7.65-7.53 (2H, m), 7.50-7.34 (2H, m), 7.34-7.14 (2H, m), 6.31-5.02 (2H, m), 1.61-1.40 (3H, m)., MS (m/z): 451 (M+H)⁺. |
| 41 | 3-[6-chloro-4-(1-fluoroethyl)-1H-imidazo[4,5-c]pyridin-2-yl]-5-{2-[(²H₃)methyloxy]phenyl}-1,6-naphthyridin-2(1H)-one | ¹H-NMR (DMSO-D₆) δ: 13.27-12.70 (2H, m), 8.72 (1H, s), 8.65 (1H, d, J = 6.1 Hz), 7.69 (1H, s), 7.63-7.55 (1H, m), 7.48-7.41 (1H, m), 7.40-7.35 (1H, m), 7.31 (1H, d, J = 8.5 Hz), 7.23-7.16 (1H, m), 6.30-5.99 (1H, m), 1.75 (3H, dd, J = 24.4, 6.1 Hz)., MS (m/z): 453 (M+H)⁺. |
| 42 | N-tert-butyl-2-[6-chloro-2-(5-{2-[(²H₃)methyloxy]phenyl}-2-oxo-1,2-dihydro-1,6-naphthyridin-3-yl)-1H-imidazo[4,5-c]pyridin-4-yl]acetamide | ¹H-NMR (DMSO-D₆) δ: 13.19-12.79 (2H, m), 8.66 (1H, s), 8.64 (1H, d, J = 5.5 Hz), 8.12 (0.25H, s), 7.77 (0.75H, m), 7.67-7.52 (2H, m), 7.42 (1H, dd, J = 7.3, 1.8 Hz), 7.36 (1H, d, J = 5.5 Hz), 7.32-7.24 (1H, m), 7.21-7.14 (1H, m), 4.00 (0.5H, s), 3.83 (1.5H, s), 1.28 (2.25H, s), 1.19(6.75H, s)., MS (m/z): 520 (M+H)⁺. |
| 43 | 3-[6-chloro-4-(3-oxopiperazin-1-yl)-1H-imidazo[4,5-c]pyridin-2-yl]-5-{2-[(²H₃)methyloxy]phenyl}-1,6-naphthyridin-2(1H)-one | ¹H-NMR (DMSO-D₆) δ: 12.80 (1H, br s), 12.75 (1H, br s), 8.59 (1H, d, J = 6.1 Hz), 8.56 (1H, s), 8.05 (1H, s), 7.55-7.49 (1H, m), 7.38 (1H, dd, J = 7.6, 1.5 Hz), 7.30 (1H, d, J = 6.1 Hz), 7. 28 (1H, d, J = 8.5 Hz), 7.15-7.10 (1H, m), 6.96 (1H, s), 4.52 (2 H, d, J = 7.9 Hz), 4.32-4.15 (2H, m), 3.29-3.24 (2H, m)., MS (m/z): 505 (M+H)⁺. |
| 44 | 3-(6-chloro-4-{[dimethyl(oxide)-λ⁶-sulfanylidene]amino}-1H-imidazo[4,5-c]pyridin-2-yl)-5-{2-[(²H₃)methyloxy]phenyl}-1,6-naphthyridin-2(1H)-one | ¹H-NMR (DMSO-D₆) δ : 12.92-12.75 (2H, m), 8.66-8.47 (2H, m), 7.62-7.55 (1H, m), 7.41-7.10 (5H, m), 3.56-3.43 (6H, m)., MS (m/z): 498 (M+H)⁺. |
| 45 | 2-[3-(6-chloro-4-methyl-1H-imidazo[4,5-c]pyridin-2-yl)-2-oxo-1,2-dihydro-1,6-naphthyridin-5-yl]-3-fluorobenzonitrile | ¹H-NMR (DMSO-D₆) δ : 8.74 (1H, d, J = 5.5 Hz), 8.53 (1H, d, J = 2.5 Hz), 8.05 (1H, dd, J = 7.4, 1.2 Hz), 8.00-7.88 (2H, m), 7.54-7.48 (2H, m), 2.62 (3H, s)., MS (m/z): 431 (M+H)⁺. |
| 46 | 3-(6-chloro-1 H-imidazo[4,5-c]pyridin-2-yl)-5-[3-(2-hydroxypropan-2-yl)phenyl]-1,6-naphthyridin-2(1H)-one | ¹H-NMR (DMSO-D₆) δ : 13.09-12.98 (2H, m), 9.06 (1H, br s), 8.74-8.69 (1H, m), 8.64 (1H, d, J = 5.5 Hz), 7.76 (1H, s), 7.69 (1H, d, J = 7.3 Hz), 7.64 (1H, s), 7.54 (1H, t, J = 7.3 Hz), 7.48 (1H, d, J = 7.3 Hz), 7.35 (1H, d, J = 5.5 Hz), 1.50 (6H, s)., MS (m/z): 432 (M+H)⁺. |
| 47 | 3-(6-chloro-1H-imidazo[4,5-c]pyridin-2-yl)-5-[3-(3-oxa-8-azabicyclo[3.2.1]octan-8-ylmethyl)phenyl]-1,6-naphthyridin-2(1H)-one | ¹H-NMR (DMSO-D₆) δ : 12.96 (1H, br s), 9.08 (1H, s), 8.72 (1H, br s), 8.66 (1H, d, J = 6.1 Hz), 7.71 (1H, s), 7.66 (1H, s), 7.60-7.56 (3H, m), 7.37 (1H, d, J = 6.1 Hz), 3.58-3.51 (3H, m), 3.35-3.29 (4H, m), 3.12-3.09 (2H, m), 2.02-1.95 (2H, m), 1.77-1.70 (2H, m)., MS (m/z): 499 (M+H)⁺. |
| 48 | 3-{6-chloro-4-[(2,2,2-trifluoroethyl)amino]-1H-imidazo[4,5-c]pyridin-2-yl}-5-(2-methylphenyl)-1,6-naphthyridin-2(1H)-one | ¹H-NMR (DMSO-D₆) δ : 12.93-12.88 (2H, m), 8.62-8.60 (1H, m), 8.51-8.47 (1H, m), 7.59-7.31 (6H, m), 6.94 (1H, br s), 4.32 (1H, br s), 4.18 (1H, br s), 2.08 (3H, br s)., MS (m/z): 485 (M+H)⁺. |
| 49 | 3-(6-chloro-4-methyl-1H-imidazo[4,5-c]pyridin-2-yl)-5-(2-fluoro-6-methoxyphenyl)-1,6-naphthyridin-2(1H)-one | ¹H-NMR (DMSO-D₆) δ : 12.9 (1H, br s), 12.95 (1H, br s), 8.67 (1H, d, J = 6.1 Hz), 8.54 (1H, s), 7.64 (1H, dd, J = 15.9, 8.6 Hz), 7.50 (1H, s), 7.40 (1H, d, J = 6.1 Hz), 7.17 (1H, d, J = 8.6 Hz), 7.08 (1H, t, J = 8.6 Hz), 3.74 (3H, s), 3.07-2.96 (2H, m), 1.28 (3H, t, J = 7.8 Hz)., MS (m/z): 450 (M+H)⁺. |
| 50 | 3-(6-chloro-4-methyl-1H-imidazo[4,5-c]pyridin-2-yl)-5-(5-fluoro-2-methoxyphenyl)-1,6-naphthyridin-2(1H)-one | ¹H-NMR (DMSO-D₆) δ : 12.98 (1H, br s), 12.90 (1H, br s), 8.69-8.60 (2H, m), 7.49 (1H, s), 7.45 (1H, td, J = 8.5, 3.1 Hz), 7.38 (1H, d, J = 6.1 Hz), 7.36-7.28 (2H, m), 3.73 (3H, s), 2.64 (3H, s)., MS (m/z): 436 (M+H)⁺. |
| 51 | 3-(6-chloro-4-methyl-1H-imidazo[4,5-c]pyridin-2-yl)-5-[2-(difluoromethoxy)phenyl]-1,6-naphthyridin-2(1H)-one | ¹H-NMR (DMSO-D₆) δ : 12.98 (2H, br s), 8.66 (1H, d, J = 5.5 Hz), 8.65 (1H, s), 7.70 (1H, t, J = 7.9 Hz), 7.60 (1H, d, J = 7.9 Hz), 7.54-7.45 (3H, m), 7.41 (1H, d, J = 5.5 Hz), 7.16 (1H, t, J = 73.9 Hz), 2.62 (3H, s)., MS (m/z): 454 (M+H)⁺. |
| 52 | 3-(6-chloro-1H-imidazo[4,5-c]pyridin-2-yl)-5-{2-[(²H₃)methyloxy]phenyl}-1,6-naphthyridin-2(1H)-one | ¹H-NMR (DMSO-D₆) δ: 13.15-12.88 (2H, br m), 8.72 (1H, s), 8.63 (1H, br s), 8.61 (1H, d, J = 5.5 Hz), 7.70-7.64 (1H, br m), 7.57 (1H, td, J = 7.8, 1.2 Hz), 7.38 (1H, dd, J = 7.8, 1.8 Hz), 7.34 (1H, d, J = 5.5 Hz), 7.27 (1H, d, J = 7.4 Hz), 7.16 (1H, td, J = 7.4, 1.2 Hz)., MS (m/z): 407 (M+H)⁺. |
| 53 | 3-{6-chloro-4-[(3S)-tetrahydrofuran-3-ylamino]-1H-imidazo[4,5-c]pyridin-2-yl}-5-{2-[(²H₃)methyloxy]phenyl}-1,6-naphthyridin-2(1H)-one | ¹H-NMR (DMSO-D₆) δ: 12.86 (1H, br s), 12.77 (1H, br s), 8.64-8.52 (2H, m), 8.03 (1H, br s), 7.58 (1H, t, J = 8.5 Hz), 7.44-7.13 (4H, m), 6.86-6.83 (1H, m), 4.70-4.54 (1H, m), 3.96-3.53 (4H, m), 2.31-2.10 (1H, m), 2.02-1.84 (1H, m). |
| 54 | 3-(6-chloro-1H-imidazo[4,5-c]pyridin-2-yl)-5-{2-fluoro-6-[(²H₃)methyloxy]phenyl}-1,6-naphthyridin-2(1H)-one | ¹H-NMR (DMSO-D₆) δ: 12.98 (2H, br s), 8.76 (1H, s), 8.67 (1H, d, J = 5.5 Hz), 8.55 (1H, s), 7.70 (1H, br s), 7.64 (1H, dd, J = 15.9, 8.6 Hz), 7.41 (1H, d, J = 5.5 Hz), 7.15 (1H, d, J = 8.6 Hz), 7.07 (1H, t, J = 8.6 Hz)., MS (m/z): 425 (M+H)⁺. |
| 55 | 3-(6-chloro-4-ethyl-1H-imidazo[4,5-c]pyridin-2-yl)-5-{2-fluoro-6-[(²H₃)methyloxy]phenyl}-1,6-naphthyridin-2(1H)-one | ¹H-NMR (DMSO-D₆) δ: 12.99 (1H, s), 12.95 (1H, s), 8.67 (1H, d, J = 5.5 Hz), 8.54 (1H, s), 7.68-7.59 (1H, m), 7.50 (1H, s), 7.40 (1H, d, J = 5.5 Hz), 7.16 (1H, d, J = 8.5 Hz), 7.11-7.04 (1H, m), 3.07-2.95 (2H, m), 1.28 (3H, t, J = 7.3 Hz)., MS (m/z): 453 (M+H)⁺. |

### Example 56

### Production of 3-[4-(3-aminopiperidin-1-yl)-6-chloro-1H-imidazo[4,5-c]pyridin-2-yl]-5-(2-methylphenyl)-1,6-naphthyridin-2(1H)-one hydrochloride

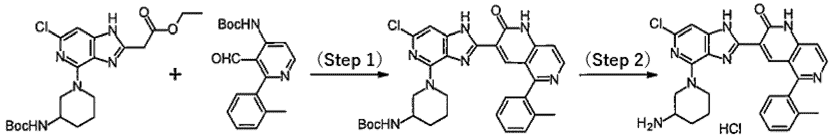

### (Step 1) tert-Butyl (1-{6-chloro-2-[5-(2-methylphenyl)-2-oxo-1,2-dihydro-1,6-naphthyridin-3-yl]-1H-imidazo[4,5-c]pyridin-4-yl}piperidin-3-yl)carbamate

A mixture of the compound (70.3 mg) obtained in Step 3 of Reference Example 8, the compound (50.2 mg) obtained in Reference Example 62, piperidine (31.8 µL), and ethanol (4.0 mL) was stirred at 90°C for 12 hours. After cooling the reaction mixture to room temperature, the solvent was evaporated under reduced pressure. The obtained residue was subjected to silica gel column chromatography (n-hexane/ethyl acetate) to obtain the crude title compound (64.1 mg).

### (Step 2) 3-[4-(3-Aminopiperidin-1-yl)-6-chloro-1H-imidazo[4,5-c]pyridin-2-yl]-5-(2-methylphenyl)-1,6-naphthyridin-2(1H)-one hydrochloride

To a mixture of the compound (64.1 mg) obtained in Step 1 above and a 4 M solution of hydrogen chloride in 1,4-dioxane (2.0 mL) was added ethanol (0.50 mL) at 80°C, and the mixture was stirred at the same temperature for 8 hours. After cooling the reaction mixture to room temperature, the mixture was concentrated under reduced pressure. Ethanol (3.0 mL) was added to the obtained residue, and the mixture was stirred at room temperature for 1 hour. The precipitated solid was collected by filtration, washed with ethanol, and then dried under reduced pressure to obtain the title compound (43.2 mg).
¹H-NMR (DMSO-D₆) δ: 13.37 (1H, br s), 12.87 (1H, s), 8.71 (1H, d, J = 5.9 Hz), 8.45 (1H, s), 8.27-8.05 (3H, m), 7.70-7.55 (2H, m), 7.54-7.39 (3H, m), 6.97 (1H, s), 5.04-4.92 (1H, m), 4.61-4.46 (1H, m), 3.48-3.34 (1H, m), 3.26-3.10 (2H, m), 2.17 (3H, s), 2.13-2.00 (1H, m), 1.78-1.43 (3H, m)., MS (m/z): 486 (M+H)⁺.

### Example 57

### Production of 3-(6-chloro-4-ethyl-1H-imidazo[4,5-c]pyridin-2-yl)-5-(2-fluoro-6-hydroxyphenyl)-1,6-naphthyridin-2(1H)-one

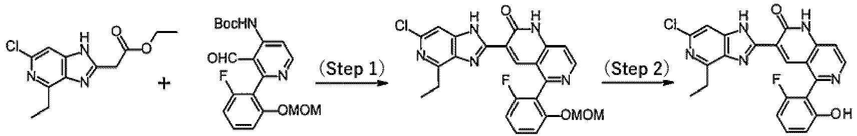

### (Step 1) 3-(6-Chloro-4-ethyl-1H-imidazo[4,5-c]pyridin-2-yl)-5-[2-fluoro-6-(methoxymethoxy)phenyl]-1,6-naphthyridin-2(1H)-one

The crude title compound was obtained by using the compound obtained in Step 3 of Reference Example 30 and the compound obtained in Reference Example 80 as production materials and performing the same operation as in Example 26. 3-(6-Chloro-4-ethyl-1H-imidazo[4,5-c]pyridin-2-yl)-5-(2-fluoro-6-hydroxyphenyl)-1,6-naphthyridin-2(1H)-one

To a suspension of the compound (124 mg) obtained in Step 1 above in 1,4-dioxane (2.0 mL) was added 6 M hydrochloric acid (0.20 mL) at room temperature, and the mixture was stirred at 80°C for 4 hours. The reaction mixture was cooled to 0°C, a 1 M aqueous solution of sodium hydroxide (1.2 mL) was added, and the precipitated solid was collected by filtration, washed with water, and then dried under reduced pressure to obtain the title compound (105 mg).
¹H-NMR (DMSO-D₆) δ: 13.00 (1H, br s), 12.93 (1H, br s), 10.27 (1H, s), 8.66 (1H, d, J = 6.1 Hz), 8.62 (1H, s), 7.50 (1H, s), 7.44 (1H, dd, J = 12.2, 8.5 Hz), 7.39 (1H, d, J = 6.1 Hz), 6.95-6.83 (2H, m), 3.02 (2H, q, J = 7.3 Hz), 1.28 (3H, t, J = 7.3 Hz)., MS (m/z): 436 (M+H)⁺.

### Example 58

### Production of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one

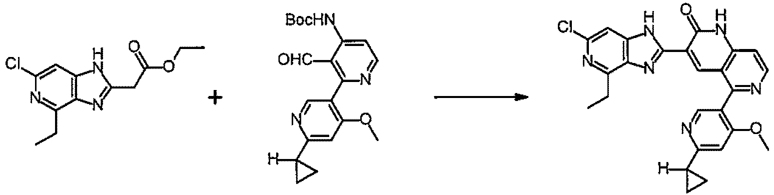

### 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one

A mixture of the compound (1.00 g) obtained in Step 3 of Reference Example 30, the compound (1.38 g) obtained in Reference Example 86, piperidine (738 µL), and ethanol (10 mL) was stirred at 90°C for 6 hours. The reaction mixture was cooled to room temperature, and then the precipitated solid was collected by filtration. The obtained solid was washed with ethanol and then dried under reduced pressure to obtain the title compound (1.53 g).
¹H-NMR (DMSO-D₆) δ: 13.01-12.78 (2H, m), 8.69-8.60 (1.9H, m), 8.52 (0.1H, s), 8.32 (0.9H, s), 8.28 (0.1H, s), 7.60 (0.1H, s), 7.50 (0.9H, s), 7.37 (1H, d, J = 5.9 Hz), 7.33 (0.9H, s), 7.29 (0.1H, s), 3.84 (2.7H, s), 3.80 (0.3H, s), 3.19-3.11 (0.2H, m), 3.06 (1.8H, q, J = 7.4 Hz), 2.32-2.20 (1H, m), 1.31 (3H, t, J = 7.4 Hz), 1.10-0.99 (4H, m)., MS (m/z): 473 (M+H)⁺.

The following compounds were synthesized by performing the same operation as in Example 58 (Table 14-1 and Table 14-2).

**Table 14-1**

| Example No. | Production Material 1 | Production Material 2 | Structure of Compound Synthesized |
|---|---|---|---|
| 59 | 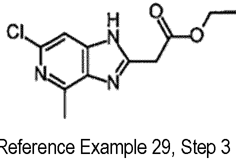 | 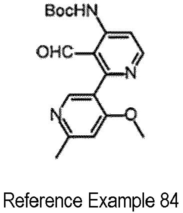 | 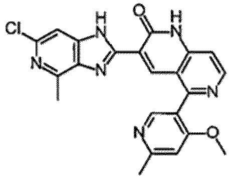 |
| 60 | 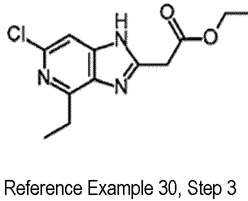 | 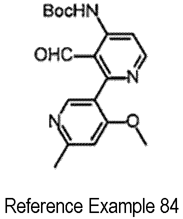 | 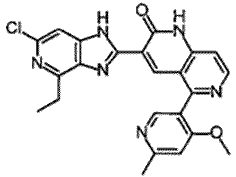 |
| 61 | 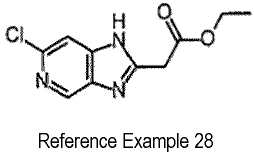 | 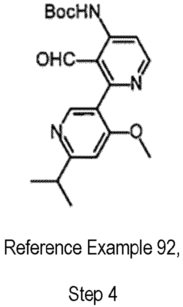 | 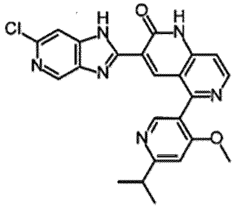 |
| 62 | 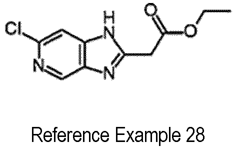 | 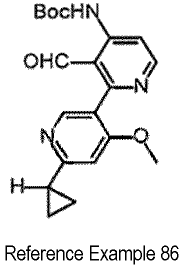 | 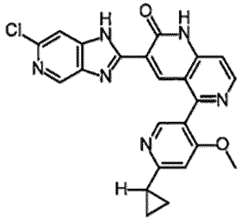 |
| 63 | 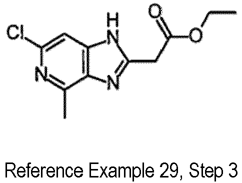 | 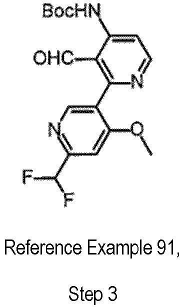 | 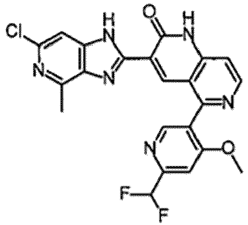 |
| 64 | 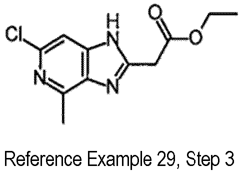 | 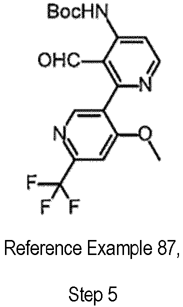 | 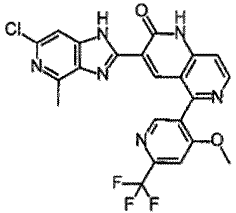 |
| 65 | 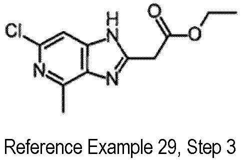 | 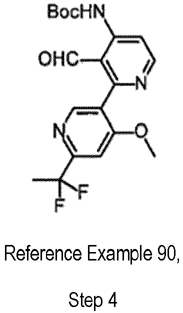 | 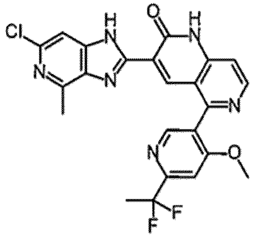 |
| 66 | 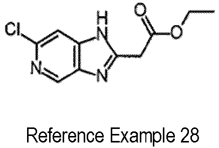 | 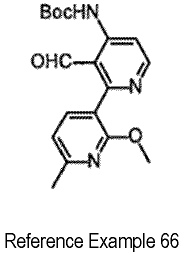 | 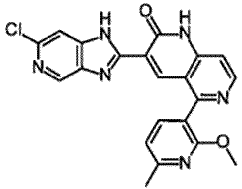 |
| 67 | 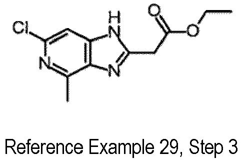 | 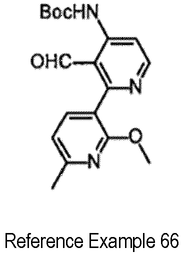 | 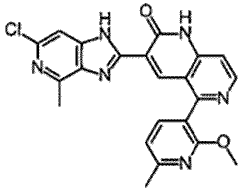 |
| 68 | 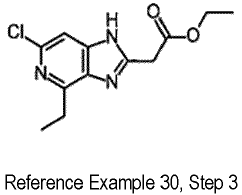 | 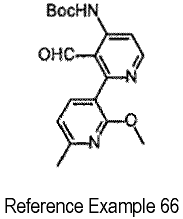 | 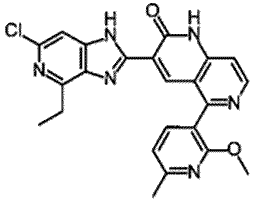 |
| 69 | 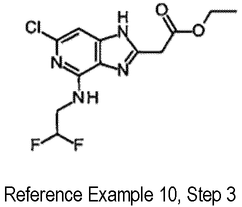 | 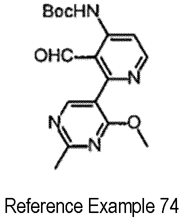 | 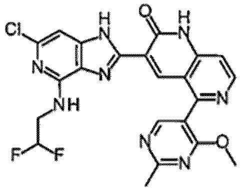 |
| 70 | 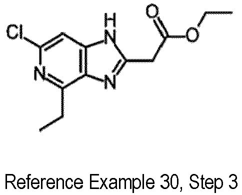 | 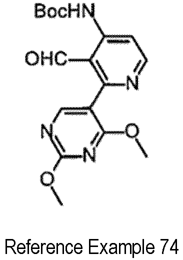 | 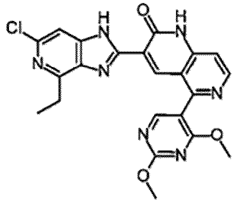 |
| 71 | 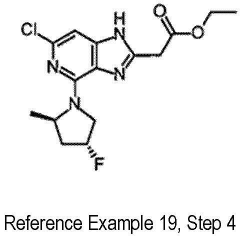 | 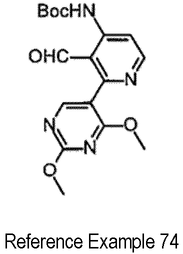 | 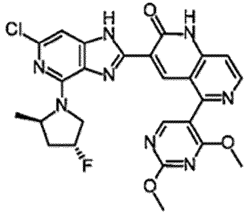 |
| 72 | 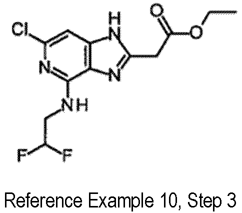 | 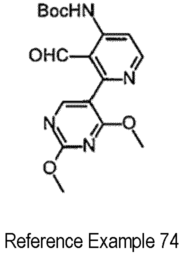 | 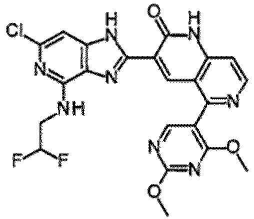 |
| 73 | 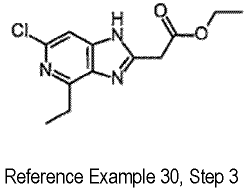 | 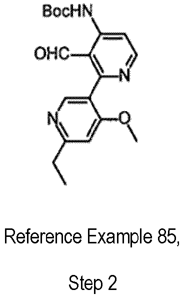 | 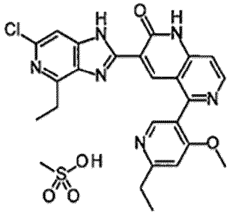 |
| 74 | 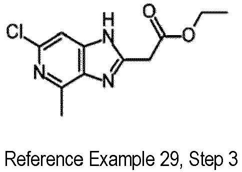 | 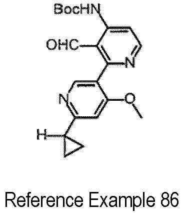 | 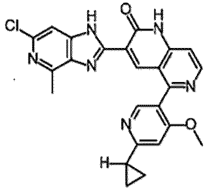 |
| 75 | 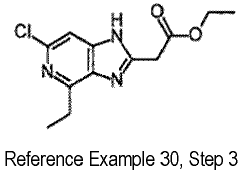 | 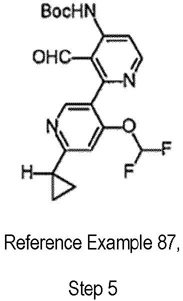 | 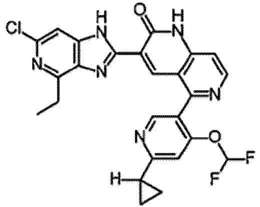 |
| 76 | 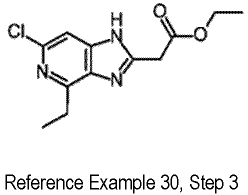 | 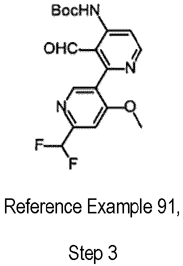 | 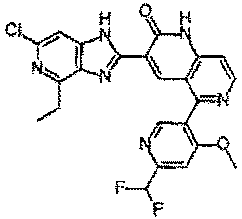 |
| 77 | 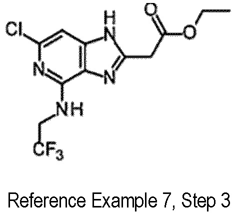 | 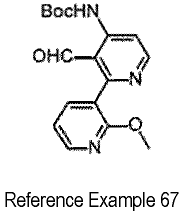 | 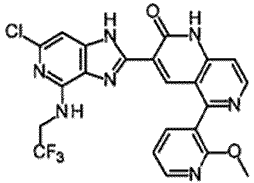 |
| 78 | 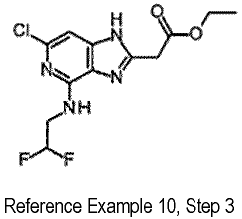 | 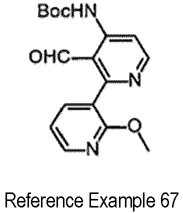 | 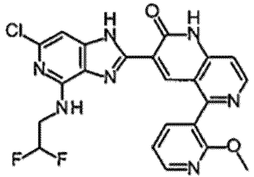 |
| 79 | 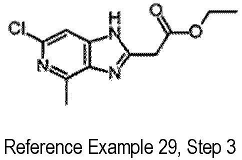 | 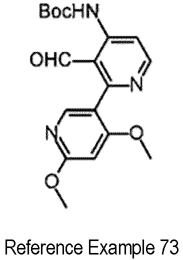 | 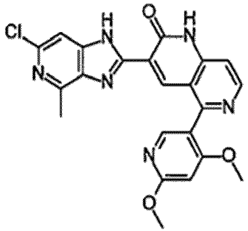 |

**Table 14-2**

| Example No. | Name of Compound Synthesized | Spectral Data |
|---|---|---|
| 59 | 3-(6-chloro-4-methyl-1H-imidazo[4,5-c]pyridin-2-yl)-5-(4-methoxy-6-methylpyridin-3-yl)-1,6-naphthyridin-2(1H)-one | ¹H-NMR (DMSO-D₆) δ : 12.98 (1H, br s), 12.90 (1H, br s), 8.74-8.59 (2H, m), 8.36 (1H, s), 7.49 (1H, s), 7.38 (1H, d, J = 5.5 Hz), 7.28 (1H, s), 3.83 (3H, s), 2.66 (3H, s), 2.60 (3H, s)., MS (m/z): 433 (M+H)⁺. |
| 60 | 3-(6-chloro-4-ethyl-1H-imidazo[4,5-c]pyridin-2-yl)-5-(4-methoxy-6-methylpyridin-3-yl)-1,6-naphthyridin-2(1H)-one | ¹H-NMR (DMSO-D₆) δ : 12.98 (1H, br s), 12.90 (1H, br s), 8.77-8.59 (2H, m), 8.37 (1H, s), 7.50 (1H, s), 7.38 (1H, d, J = 5.5 Hz), 7.28 (1H, s), 3.82 (3H, s), 3.05 (2H, q, J = 7.5 Hz), 2.61 (3H, s), 1.30 (3H, t, J = 7.5 Hz)., MS (m/z): 447 (M+H)⁺. |
| 61 | 3-(6-chloro-1H-imidazo[4,5-c]pyridin-2-yl)-5-[4-methoxy-6-(propan-2-yl)pyridin-3-yl]-1,6-naphthyridin-2(1H)-one | ¹H-NMR (DMSO-D₆) δ : 13.25-12.92 (2H, m), 8.76 (1H, s), 8.68-8.63 (2H, m), 8.39 (1H, s), 7.76-7.66 (1H, m), 7.39 (1H, d, J = 5.5 Hz), 7.23 (1H, s), 3.81 (3H, s), 3.20-3.12 (1H, m), 1.36 (6H, d, J = 6.7 Hz)., MS (m/z): 447 (M+H)⁺. |
| 62 | 3-(6-chloro-1H-imidazo[4,5-c]pyridin-2-yl)-5-(6-cyclopropyl-4-methoxypyridin-3-yl)-1,6-naphthyridin-2(1H)-one | ¹H-NMR (DMSO-D₆) δ : 12.93 (1H, br s), 8.75 (1H, s), 8.64-8.58 (2H, m), 8.26 (1H, s), 7.66 (1H, s), 7.35 (1H, d, J = 6.1 Hz), 7.29 (1H, s), 3.78 (3H, s), 2.27-2.19 (1H, m), 1.07-0.98 (4H, m)., 445 (M+H)⁺. |
| 63 | 3-(6-chloro-4-methyl-1H-imidazo[4,5-c]pyridin-2-yl)-5-[6-(difluoromethyl)-4-methoxypyridin-3-yl]-1,6-naphthyridin-2(1H)-one | ¹H-NMR (DMSO-D₆) δ : 12.97 (1H, s), 12.94 (1H, s), 8.67 (1H, d, J = 6.1 Hz), 8.61 (1H, s), 8.58 (1H, s), 7.65 (1H, s), 7.47 (1H, s), 7.41 (1H, d, J = 6.1 Hz), 7.07 (1H, t, J = 54.9 Hz), 3.91 (3H, s), 2.63 (3H, s)., MS (m/z): 469 (M+H)⁺. |
| 64 | 3-(6-chloro-4-methyl-1H-imidazo[4,5-c]pyridin-2-yl)-5-[4-methoxy-6-(trifluoromethyl)pyridin-3-yl]-1,6-naphthyridin-2(1H)-one | ¹H-NMR (DMSO-D₆) δ : 12.98 (1H, s), 8.70 (1H, s), 8.70 (1H, d, J = 5.5 Hz), 8.58 (1H, br s), 7.88 (1H, s), 7.50 (1H, s), 7.44 (1H, d, J = 5.5 Hz), 3.98 (3H, s), 2.65 (3H, s)., MS (m/z): 487 (M+H)⁺. |
| 65 | 3-(6-chloro-4-methyl-1H-imidazo[4,5-c]pyridin-2-yl)-5-[6-(1,1-difluoroethyl)-4-methoxypyridin-3-yl]-1,6-naphthyridin-2(1H)-one | ¹H-NMR (DMSO-D₆) δ : 12.99 (1H, s), 12.96 (1H, s), 8.69 (1H, d, J = 5.5 Hz), 8.61 (2H, s), 7.62 (1H, s), 7.49 (1H, s), 7.43 (1H, d, J = 5.5 Hz), 3.94 (3H, s), 2.65 (3H, s), 2.11 (3H, t, J = 19.2 Hz)., MS (m/z): 483 (M+H)⁺. |
| 66 | 3-(6-chloro-1H-imidazo[4,5-c]pyridin-2-yl)-5-(2-methoxy-6-methylpyridin-3-yl)-1,6-naphthyridin-2(1H)-one | ¹H-NMR (DMSO-D₆) δ : 13.18 (0.34H, br s), 13.06 (0.66H, br s), 12.94 (1H, br s), 8.77 (1H, s), 8.71-8.62 (2H, m), 7.79 (1H, d, J = 7.4 Hz), 7.75 (0.34H, s), 7.67 (0.66H, s), 7.38 (1H, d, J = 5.5 Hz), 7.13 (1H, d, J = 7.4 Hz), 3.80 (3H, s), 2.57 (3H, s)., MS (m/z): 419 (M+H)⁺. |
| 67 | 3-(6-chloro-4-methyl-1H-imidazo[4,5-c]pyridin-2-yl)-5-(2-methoxy-6-methylpyridin-3-yl)-1,6-naphthyridin-2(1H)-one | ¹H-NMR (DMSO-D₆) δ: 12.97 (2H, br s), 8.69 (1H, s), 8.64 (1H, d, J = 5.5 Hz), 7.80 (1H, d, J = 7.4 Hz), 7.50 (1H, s), 7.37 (1H, d, J = 5.5 Hz), 7.12 (1H, d, J = 7.4 Hz), 3.84 (3H, s), 2.66 (3H, s), 2.56 (3H, s)., MS (m/z): 433 (M+H)⁺. |
| 68 | 3-(6-chloro-4-ethyl-1H-imidazo[4,5-c]pyridin-2-yl)-5-(2-methoxy-6-methylpyridin-3-yl)-1,6-naphthyridin-2(1H)-one | ¹H-NMR (DMSO-D₆) δ: 12.98 (1H, br s), 12.89 (1H, br s), 8.69 (1H, s), 8.64 (1H, d, J = 6.1 Hz), 7.81 (1H, d, J = 7.3 Hz), 7.50 (1H, s), 7.36 (1H, d, J = 6.1 Hz), 7.12 (1H, d, J = 7.3 Hz), 3.84 (3H, s), 3.04 (2H, q, J = 7.6 Hz), 2.56 (3H, s), 1.30 (3H, t, J = 7.6 Hz)., MS (m/z): 447 (M+H)⁺. |
| 69 | 3-(6-chloro-4-[(2,2-difluoroethyl)amino]-1H-imidazo[4,5-c]pyridin-2-yl)-5-(4-methoxy-2-methylpyrimidin-5-yl)-1,6-naphthyridin-2(1H)-one | ¹H-NMR (DMSO-D₆) δ: 13.04-12.77 (2H, m), 8.67-8.53 (3H, m), 8.28-8.22 (0.25H, m), 7.51-7.45 (0.75H, m), 7.42-7.38 (1H, m), 6.93 (0.25H, s), 6.93 (0.75H, s), 6.39-6.00 (1H, m), 3.95-3.74 (5H, m), 2.70 (3H, s)., MS (m/z): 499 (M+H)⁺. |
| 70 | 3-(6-chloro-4-ethyl-1H-imidazo[4,5-c]pyridin-2-yl)-5-(2,4-dimethoxypyrimidin-5-yl)-1,6-naphthyridin-2(1H)-one | ¹H-NMR (DMSO-D₆) δ: 12.99 (2H, br s), 8.69 (1H, s), 8.64 (1H, d, J = 5.5 Hz), 8.56 (1H, s), 7.51 (1H, s), 7.38 (1H, d, J = 5.5 Hz), 4.05 (3H, s), 3.93 (3H, s), 3.07 (2H, q, J = 7.3 Hz),1.31 (3H, t, J = 7.3 Hz)., MS (m/z): 464 (M+H)⁺. |
| 71 | 3-(6-chloro-4-[(2R,4R)-4-fluoro-2-methylpyrrolidin-1-yl]-1H-imidazo[4,5-c]pyridin-2-yl)-5-(2,4-dimethoxypyrimidin-5-yl)-1,6-naphthyridin-2(1H)-one | ¹H-NMR (DMSO-D₆) δ: 12.76 (2H, br s), 8.62 (1H, d, J = 6.1 Hz), 8.58 (1H, s), 8.53 (1H, s), 7.36 (1H, d, J = 6.1 Hz), 6.88 (1H, s), 5.39 (1H, d, J = 53.7 Hz), 4.75 (1H, br s), 4.49 (1H, br s), 4.04 (3H, s), 3.92 (3H, s), 3.87-3.79 (1H, m), 2.45-2.30 (1H, m), 2.11-1.90 (1H, m), 1.29 (3H, d, J = 6.1 Hz)., MS (m/z): 537 (M+H)⁺. |
| 72 | 3-(6-chloro-4-[(2,2-difluoroethyl)amino]-1H-imidazo[4,5-c]pyridin-2-yl)-5-(2,4-dimethoxypyrimidin-5-yl)-1,6-naphthyridin-2(1H)-one | ¹H-NMR (DMSO-D₆) δ: 12.99-12.82 (2H, m), 8.66-8.57 (2H, m), 8.54 (0.7H, s), 8.52 (0.3H, s), 8.28-8.23 (0.3H, m), 7.52-7.47 (0.7H, m), 7.41-7.36 (1H, m), 6.94 (0.3H, s), 6.93 (0.7H, s), 6.22 (0.3H, tt, J = 55.8, 4.0 Hz), 6.18 (0.7H, tt, J = 55.8, 4.0 Hz), 4.04 (3H, s), 3.92-3.75 (5H, m)., MS (m/z): 515 (M+H)⁺. |
| 73 | 3-(6-chloro-4-ethyl-1H-imidazo[4,5-c]pyridin-2-yl)-5-(6-ethyl-4-methoxypyridin-3-yl)-1,6-naphthyridin-2(1H)-one | ¹H-NMR (DMSO-D₆) δ: 12.98 (1H, br s), 12.90 (1H, br s), 8.66 (1H, d, J = 5.5 Hz), 8.65 (1H, s), 8.40 (1H, s), 7.50 (1H, s), 7.38 (1H, d, J = 5.5 Hz), 7.27 (1H, s), 3.84 (3H, s), 3.04 (2H, q, J = 7.9 Hz), 2.88 (2H, q, J = 7.9 Hz), 1.34 (3H, t, J = 7.9 Hz), 1.30 (3H, t, J = 7.9 Hz). |
| 74 | 3-(6-chloro-4-methyl-1H-imidazo[4,5-c]pyridin-2-yl)-5-(6-cyclopropyl-4-methoxypyridin-3-yl)-1,6-naphthyridin-2(1H)-one | ¹H-NMR (DMSO-D₆) δ: 12.98 (1H, s), 12.89 (1H, s), 8.64 (2H, d, J = 4.9 Hz), 8.31 (1H, s), 7.49 (1H, s), 7.37 (1H, d, J = 4.9 Hz), 7.33 (1H, s), 3.85 (3H, s), 2.67 (3H, s), 2.29-2.23 (1H, m), 1.07-1.04 (4H, m)., MS (m/z): 459 (M+H)⁺. |
| 75 | 3-(6-chloro-4-ethyl-1H-imidazo[4,5-c]pyridin-2-yl)-5-[6-cyclopropyl-4-(difluoromethoxy)pyridin-3-yl]-1,6-naphthyridin-2(1H)-one | 1H-NMR (CD3OD) δ: 8.84 (1H, s), 8.62 (1H, d, J = 5.5 Hz), 8.50 (1H, s), 7.49 (1H, s), 7.41 (1H, d, J = 5.5 Hz), 7.37-6.97 (2H, m), 3.16-3.08 (2H, m), 2.35-2.23 (1H, m), 1.34 (3H, t, J = 7.4 Hz), 1.20-1.10 (4H, m). |
| 76 | 3-(6-chloro-4-ethyl-1H-imidazo[4,5-c]pyridin-2-yl)-5-[6-(difluoromethyl)-4-methoxypyridin-3-yl]-1,6-naphthyridin-2(1H)-one | ¹H-NMR (DMSO-D₆) δ: 8.66 (1H, d, J = 5.5 Hz), 8.60 (1H, s), 8.56 (1H, s), 7.65 (1H, s), 7.49 (1H, s), 7.40 (1H, d, J = 5.5 Hz), 7.07 (1H, t, J = 54.9 Hz), 3.90 (3H, s), 3.03 (2H, q, J = 8.3 Hz), 1.27 (3H, t, J = 8.3 Hz)., MS (m/z): 483 (M+H)⁺. |
| 77 | 3-{6-chloro-4-[(2,2,2-trifluoroethyl)amino]-1H-imidazo[4,5-c]pyridin-2-yl}-5-(2-methoxypyridin-3-yl)-1,6-naphthyridin-2(1H)-one | ¹H-NMR (DMSO-D₆) δ: 12.97-12.86 (2H, m), 8.67-8.52 (2H, m), 8.47-8.39 (1H, m), 7.93-7.86 (1H, m), 7.63 (1H, t, J = 6.7 Hz), 7.42-7.35 (1H, m), 7.29-7.23 (1H, m), 7.00-6.95 (1H, s), 4.42-4.31 (1H, m), 4.30-4.16 (1H, m), 3.85-3.78 (3H, m)., MS (m/z): 502 (M+H)⁺. |
| 78 | 3-{6-chloro-4-[(2,2-difluoroethyl)amino]-1H-imidazo[4,5-c]pyridin-2-yl}-5-(2-methoxypyridin-3-yl)-1,6-naphthyridin-2(1H)-one | ¹H-NMR (DMSO-D₆) δ: 13.00-12.79 (2H, m), 8.66-8.60 (1H, m), 8.57 (0.3H, s), 8.54 (0.7H, s), 8.46-8.41 (1H, m), 7.92-7.87 (1H, m), 7.43-7.36 (1.7H, m), 7.30-7.23 (1H, m), 6.93 (1H, s), 6.39-5.98 (1H, m), 3.97-3.73 (5H, m)., MS (m/z): 484 (M+H)⁺. |
| 79 | 3-(6-chloro-4-methyl-1H-imidazo[4,5-c]pyridin-2-yl)-5-(4,6-dimethoxypyridin-3-yl)-1,6-naphthyridin-2(1H)-one | ¹H-NMR (DMSO-D₆) δ: 12.97 (1H, s), 12.89 (1H, s), 8.66 (1H, s), 8.64 (1H, d, J = 6.1 Hz), 8.14 (1H, s), 7.49 (1H, s), 7.37 (1H, d, J = 6.1 Hz), 6.73 (1H, s), 3.96 (3H, s), 3.81 (3H, s), 2.66 (3H, s)., MS (m/z): 449 (M+H)⁺. |

### Example 80

### Production of 3-(6-chloro-4-ethyl-1H-imidazo[4,5-c]pyridin-2-yl)-5-[4-methoxy-6-(trifluoromethyl)pyridin-3-yl]-1,6-naphthyridin-2(1H)-one

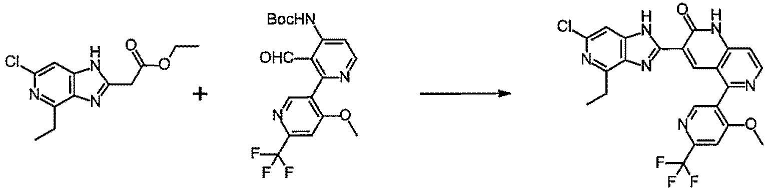

### 3-(6-Chloro-4-ethyl-1H-imidazo[4,5-c]pyridin-2-yl)-5-[4-methoxy-6-(trifluoromethyl)pyridin-3-yl]-1,6-naphthyridin-2(1H)-one

A mixture of the compound (76.0 mg) obtained in Step 3 of Reference Example 30, the compound (51.2 mg) obtained in Step 2 of Reference Example 89, piperidine (53 µL), and ethanol (2.0 mL) was stirred at 90°C for 9 hours. The reaction mixture was cooled to room temperature, and then the precipitated solid was collected by filtration. The obtained solid was washed with ethanol, and then dried under reduced pressure to obtain the title compound (40.0 mg).
¹H-NMR (DMSO-D₆) δ: 13.00 (1H, s), 12.98 (1H, s), 8.71 (1H, s), 8.71 (1H, d, J = 6.1 Hz), 8.59 (1H, s), 7.89 (1H, s), 7.50 (1H, s), 7.45 (1H, d, J = 6.1 Hz), 3.98 (3H, s), 3.04 (2H, q, J = 7.3 Hz), 1.29 (3H, t, J = 7.3 Hz)., MS (m/z): 501 (M+H)⁺.

### Example 81

### Production of 3-(6-chloro-4-ethyl-1H-imidazo[4,5-c]pyridin-2-yl)-5-[6-(difluoromethoxy)-4-methoxypyridin-3-yl]-1,6-naphthyridin-2(1H)-one

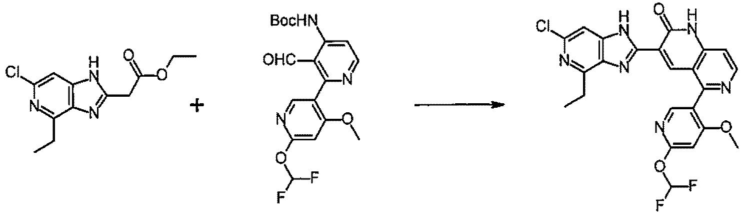

### 3-(6-Chloro-4-ethyl-1H-imidazo[4, 5-c]pyridin-2-yl)-5-[6-(difluoromethoxy)-4-methoxypyridin-3-yl]-1,6-naphthyridin-2(1H)-one

A mixture of the compound (45.3 mg) obtained in Step 3 of Reference Example 30, the compound (66.9 mg) obtained in Step 3 of Reference Example 93, piperidine (47 µL), and ethanol (1.1 mL) was stirred at 90°C for 4 hours. The reaction mixture was cooled to room temperature, and then the precipitated solid was collected by filtration. The obtained solid was washed with ethanol, and then dried under reduced pressure to obtain the title compound (43.8 mg).
¹H-NMR (DMSO-D₆) δ: 12.96 (1H, s), 12.91 (1H, s), 8.64 (1H, d, J = 5.5 Hz), 8.61 (1H, s), 8.22 (1H, s), 7.84 (1H, t, J = 72.6 Hz), 7.48 (1H, s), 7.38 (1H, d, J = 5.5 Hz), 7.08 (1H, s), 3.85 (3H, s), 3.04 (2H, q, J = 7.5 Hz), 1.28 (3H, t, J = 7.5 Hz)., MS (m/z): 499 (M+H)⁺.

### Example 82

### Production of 3-(6-chloro-4-ethyl-1H-imidazo[4,5-c]pyridin-2-yl)-5-[6-(1,1-difluoroethyl)-4-methoxypyridin-3-yl]-1,6-naphthyridin-2(1H)-one

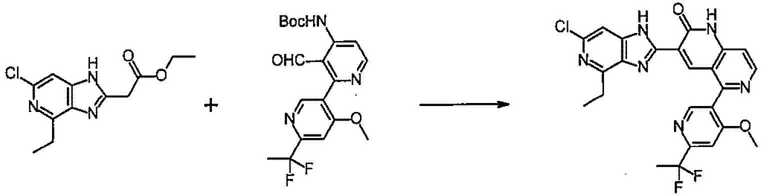

### 3-(6-Chloro-4-ethyl-1H-imidazo[4,5-c]pyridin-2-yl)-5-[6-(1,1-difluoroethyl)-4-methoxypyridin-3-yl]-1,6-naphthyridin-2(1H)-one

A mixture of the compound (60.4 mg) obtained in Step 3 of Reference Example 30, the compound (82.1 mg) obtained in Step 4 of Reference Example 90, piperidine (58 µL), and ethanol (4.2 mL) was stirred at 90°C for 5 hours. The reaction mixture was cooled to room temperature, and then the precipitated solid was collected by filtration. The obtained solid was washed with ethanol, and then dried under reduced pressure to obtain the title compound (64.6 mg).
¹H-NMR (DMSO-D₆) δ: 12.99 (1H, s), 12.96 (1H, s), 8.69 (1H, d, J = 5.5 Hz), 8.61 (2H, s), 8.60 (2H, s), 7.62 (1H, s), 7.50 (1H, s), 7.43 (1H, d, J = 5.5 Hz), 3.93 (3H, s), 3.04 (2H, q, J = 7.3 Hz), 2.11 (3H, t, J = 7.3 Hz)., MS (m/z): 497 (M+H)⁺.

### Example 83

### Production of 3-(6-chloro-4-ethyl-1H-imidazo[4,5-c]pyridin-2-yl)-5-[1-(difluoromethyl)-3,5-dimethyl-1H-pyrazol-4-yl]-1,6-naphthyridin-2(1H)-one

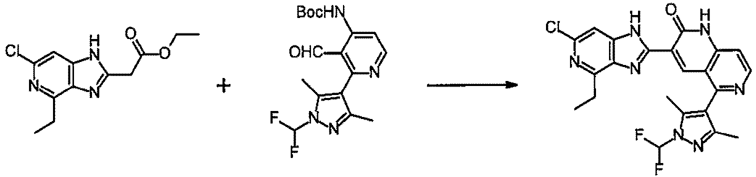

### (Step 1) 3-(6-Chloro-4-ethyl-1H-imidazo[4,5-c]pyridin-2-yl)-5-[1-(difluoromethyl)-3,5-dimethyl-1H-pyrazol-4-yl]-1,6-naphthyridin-2(1H)-one

A mixture of the compound (50 mg) obtained in Step 3 of Reference Example 30, the compound (80 mg) obtained in Step 2 of Reference Example 94, piperidine (60 µL), and ethanol (5 mL) was stirred at 90°C for 16.5 hours. After cooling the reaction mixture to room temperature, the mixture was concentrated under reduced pressure. A mixture of n-hexane/ethyl acetate was added to the obtained residue. The precipitated solid was collected by filtration, and then dried under reduced pressure to obtain the title compound (64 mg).
¹H-NMR (DMSO-D₆) δ: 12.98 (1H, br s), 12.91 (1H, br s), 8.74 (1H, s), 8.67 (1H, d, J = 5.5 Hz), 7.93 (1H, t, J = 57.7 Hz), 7.51 (1H, s), 7.36 (1H, d, J = 5.5 Hz), 3.07 (2H, q, J = 7.5 Hz), 2.35 (3H, s), 2.18 (3H, s), 1.32 (3H, t, J = 7.5 Hz)., MS (m/z): 470 (M+H)⁺.

The following compounds were synthesized by performing the same operation as in Example 83 (Table 15-1 and Table 15-2).

**Table 15-1**

| | | | |
|---|---|---|---|
| 84 | 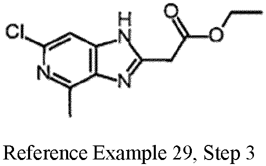 | 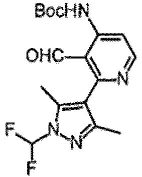 | 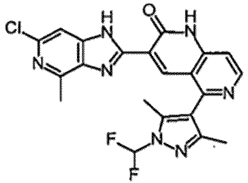 |
| | | 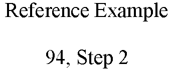 | |
| 85 | 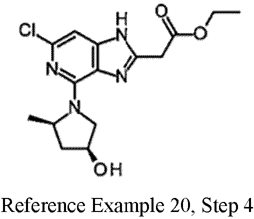 | 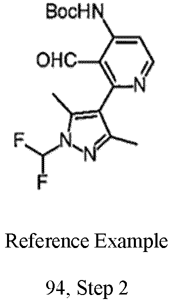 | 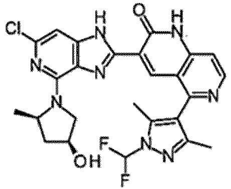 |
| 86 | 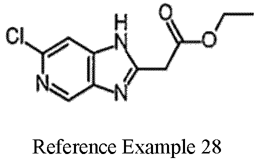 | 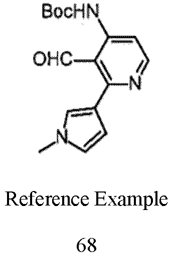 | 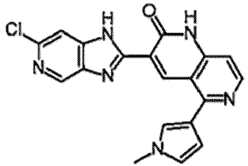 |
| 87 | 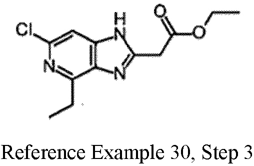 | 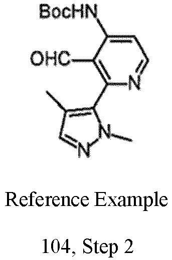 | 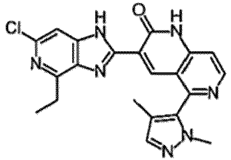 |
| 88 | 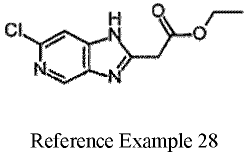 | 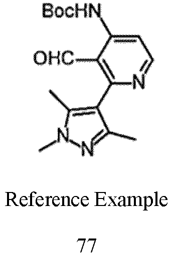 | 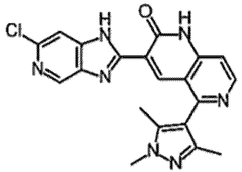 |
| 89 | 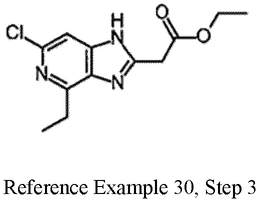 | 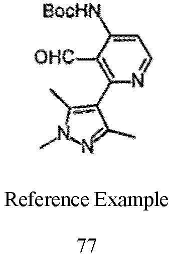 | 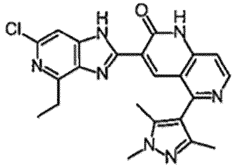 |
| 90 | 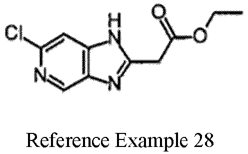 | 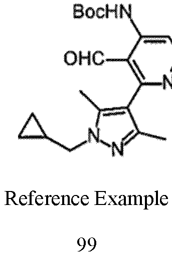 | 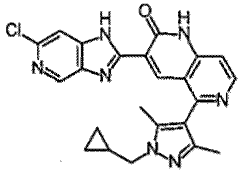 |
| 91 | 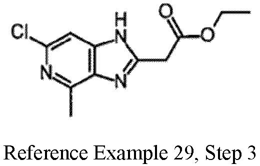 | 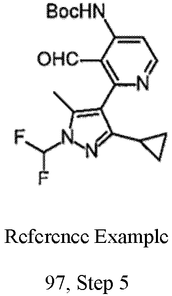 | 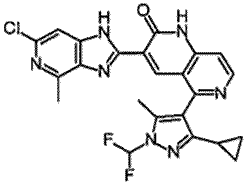 |
| 92 | 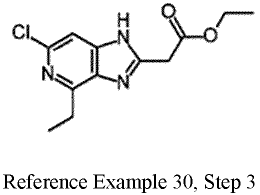 | 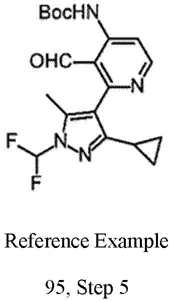 | 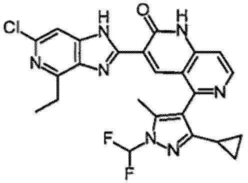 |
| 93 | 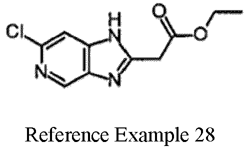 | 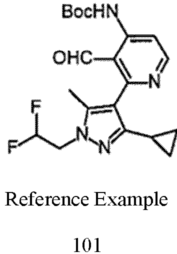 | 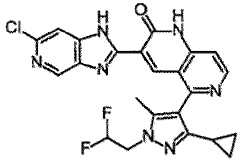 |
| 94 | 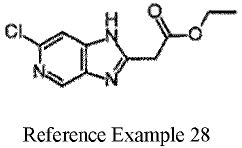 | 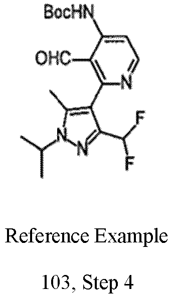 | 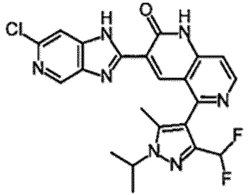 |
| 95 | 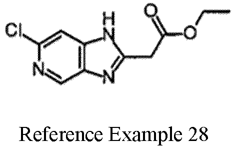 | 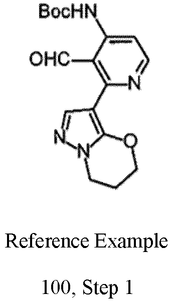 | 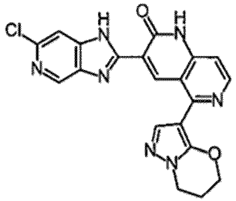 |
| 96 | 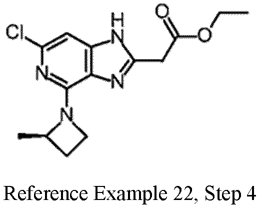 | 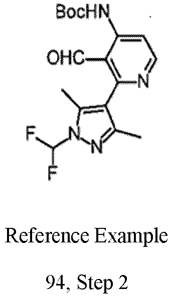 | 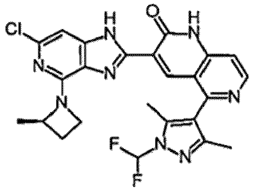 |
| 97 | 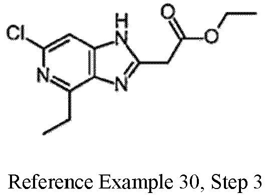 | 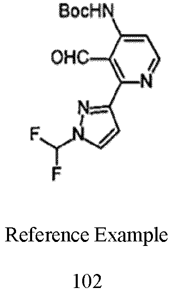 | 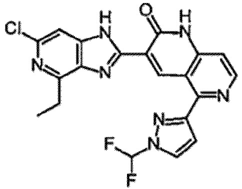 |
| 98 | 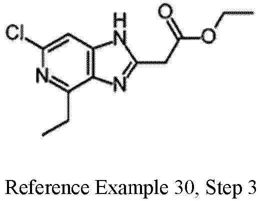 | 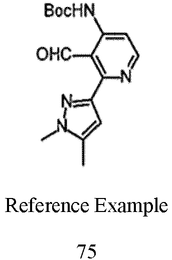 | 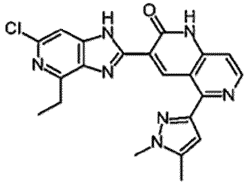 |
| 99 | 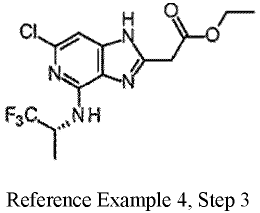 | 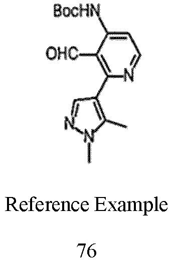 | 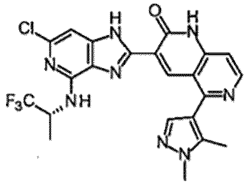 |
| 100 | 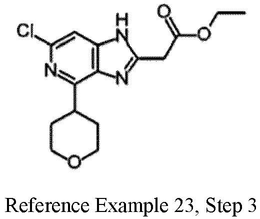 | 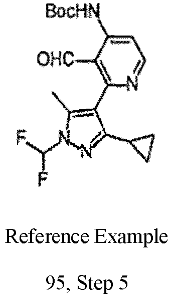 | 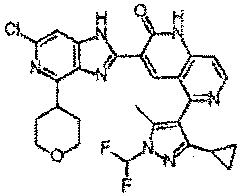 |

**Table 15-2**

| | | |
|---|---|---|
| 84 | 3-(6-chloro-4-methyl-1 H-imidazo[4,5-c]pyridin-2-yl)-5-[1-(difluoromethyl)-3,5-dimethyl-1H-pyrazol-4-yl]-1,6-naphthyridin-2(1H)-one | ¹H-NMR (DMSO-D₆) δ: 12.83 (1H, br s), 8.72 (1H, s), 8.67 (1H, d, J = 5.5 Hz), 7.93 (1H, t, J = 57.7 Hz), 7.51 (1H, s), 7.36 (1H, d, J = 5.5 Hz), 2.68 (3H, s), 2.35 (3H, s), 2.17 (3H, s)., MS (m/z): 456 (M+H)⁺. |
| 85 | 3-{6-chloro-4-[(2R,4S)-4-hydroxy-2-methylpyrrolidin-1-yl]-1H-imidazo[4,5-c]pyridin-2-yl}-5-[1-(difluoromethyl)-3,5-dimethyl-1H-pyrazol-4-yl]-1,6-naphthyridin-2(1H)-one | ¹H-NMR (DMSO-D₆) δ: 12.82 (1H, br s), 12.69 (1H, s), 8.64-8.61 (2H, m), 7.91 (1H, t, J = 57.7 Hz), 7.33 (1H, d, J = 6.1 Hz), 6.82 (1H, s), 5.03-4.81 (2H, m), 4.37 (1H, br s), 3.91-3.65 (2H, m), 2.34 (3H, s), 2.27-2.20 (1H, m), 2.18 (3H, s), 1.73 (1H, d, J = 12.8 Hz), 1.41 (3H, d, J = 6.1 Hz)., MS (m/z): 541 (M+H)⁺. |
| 86 | 3-(6-chloro-1H-imidazo[4,5-c]pyridin-2-yl)-5-(1-methyl-1H-pyrrol-3-yl)-1,6-naphthyridin-2(1H)-one | ¹H-NMR (DMSO-D₆) δ: 13.18-13.01 (1H, m), 12.76 (1H, br s), 9.55-9.50 (1H, m), 8.81-8.76 (1H, m), 8.51 (1H, d, J = 5.9 Hz), 7.76 (0.33H, br s), 7.69 (0.67H, br s), 7.38-7.34 (1H, m), 7.14 (1H, d, J = 5.9 Hz), 6.99-6.96 (1H, m), 6.56-6.53 (1H, m), 3.78 (3H, s)., MS (m/z): 377 (M+H)⁺. |
| 87 | 3-(6-chloro-4-ethyl-1H-imidazo[4,5-c]pyridin-2-yl)-5-(1,4-dimethyl-1H-pyrazol-5-yl)-1,6-naphthyridin-2(1H)-one | ¹H-NMR (DMSO-D₆) δ: 13.00 (1H, s), 12.99 (1H, s), 8.74-8.72 (2H, m), 7.55 (1H, s), 7.51 (1H, s), 7.42 (1H, d, J = 6.1 Hz), 3.78 (3H, s), 3.07 (2H, q, J = 7.2 Hz), 1.94 (3H, s), 1.31 (3H, t, J = 7.2 Hz)., MS (m/z): 420 (M+H)⁺. |
| 88 | 3-(6-chloro-1H-imidazo[4,5-c]pyridin-2-yl)-5-(1,3,5-trimethyl-1H-pyrazol-4-yl)-1,6-naphthyridin-2(1H)-one | ¹H-NMR (DMSO-D₆) δ: 13.16 (0.36H, br s), 13.05 (0.64H, br s), 12.90-12.85 (1H, m), 8.83 (0.36H, s), 8.80 (0.64H, s), 8.79 (0.64H, s), 8.77 (0.36H, s), 8.64 (0.36H, d, J = 6.1 Hz), 8.63 (0.64H, d, J = 6.1 Hz), 7.77 (0.36H, s), 7.67 (0.64H, s), 7.29 (1H, d, J = 6.1 Hz), 3.82 (3H, s), 2.21 (3H, s), 2.06 (3H, s)., MS (m/z): 406 (M+H)⁺. |
| 89 | 3-(6-chloro-4-ethyl-1H-imidazo[4,5-c]pyridin-2-yl)-5-(1,3,5-trimethyl-1H-pyrazol-4-yl)-1,6-naphthyridin-2(1H)-one | ¹H-NMR (DMSO-D₆) δ: 12.98 (1H, s), 12.84 (1H, s), 8.82 (1H, s), 8.63 (1H, d, J = 5.5 Hz), 7.51 (1H, s), 7.28 (1H, d, J = 5.5 Hz), 3.82 (3H, s), 3.09 (2H, q, J = 7.7 Hz), 2.23 (3H, s), 2.11 (3H, s), 1.33 (3H, t, J = 7.7 Hz)., MS (m/z): 434 (M+H)⁺. |
| 90 | 3-(6-chloro-1H-imidazo[4,5-c]pyridin-2-yl)-5-[1-(cyclopropylmethyl)-3,5-dimethyl-1H-pyrazol-4-yl]-1,6-naphthyridin-2(1H)-one | ¹H-NMR (DMSO-D₆) δ: 12.69 (1H, br s), 8.82 (1H, s), 8.77 (1H, s), 8.63 (1H, d, J = 5.5 Hz), 7.70 (1H, s), 7.29 (1H, d, J = 5.5 Hz), 4.01 (2H, br s), 2.22 (3H, s), 2.08 (3H, s), 1.36-1.23 (1H, m), 0.62-0.56 (2H, m), 0.45-0.41 (2H, m)., MS (m/z): 446 (M+H)⁺ |
| 91 | 3-(6-chloro-4-methyl-1H-imidazo[4,5-c]pyridin-2-yl)-5-[3-cyclopropyl-1-(difluoromethyl)-5-methyl-1H-pyrazol-4-yl]-1,6-naphthyridin-2(1H)-one | ¹H-NMR (DMSO-D₆) δ: 12.97 (2H, br s), 8.95 (1H, s), 8.69 (1H, d, J = 6.1 Hz), 7.88 (1H, t, J = 57.7 Hz), 7.51 (1H, s), 7.37 (1H, d, J = 6.1 Hz), 2.69 (3H, s), 2.37 (3H, s), 1.70-1.60 (1H, m), 1.02-0.73 (4H, m)., MS (m/z): 482 (M+H)⁺. |
| 92 | 3-(6-chloro-4-ethyl-1H-imidazo[4,5-c]pyridin-2-yl)-5-[3-cyclopropyl-1-(difluoromethyl)-5-methyl-1H-pyrazol-4-yl]-1,6-naphthyridin-2(1H)-one | ¹H-NMR (DMSO-D₆) δ: 8.95 (1H, s), 8.68 (1H, d, J = 6.1 Hz), 7.88 (1H, t, J = 57.4 Hz), 7.52 (1H, s), 7.37 (1H, d, J = 6.1 Hz), 3.16-3.02 (2H, m), 2.38 (3H, s), 1.70-1.59 (1H, m), 1.33 (3H, t, J = 7.3 Hz), 1.03-0.75 (4H, m)., MS (m/z): 496 (M+H)⁺. |
| 93 | 3-(6-chloro-1 H-imidazo[4,5-c]pyridin-2-yl)-5-[3-cyclopropyl-1-(2,2-difluoroethyl)-5-methyl-1 H-pyrazol-4-yl]-1,6-naphthyridin-2(1H)-one | ¹H-NMR (DMSO-D₆) δ: 12.92 (2H, br s), 8.97 (1H, s), 8.77 (1H, s), 8.65 (1H, d, J = 5.5 Hz), 7.70 (1H, s), 7.32 (1H, d, J = 5.5 Hz), 6.43 (1H, t, J = 54.9 Hz), 4.63 (2H, t, J = 14.3 Hz), 2.24 (3H, s), 1.64-1.53 (1H, m), 0.84-0.77 (2H, m), 0.71-0.62 (2H, m)., MS (m/z): 482 (M+H)⁺. |
| 94 | 3-(6-chloro-1H-imidazo[4,5-c]pyridin-2-yl)-5-[3-(difluoromethyl)-5-methyl-1-(propan-2-yl)-1H-pyrazol-4-yl]-1,6-naphthyridin-2(1H)-one | ¹H-NMR (DMSO-D₆) δ: 8.75 (1H, s), 8.74 (1H, s), 8.60 (1H, d, J = 6.1 Hz), 7.69 (1H, s), 7.33 (1H, d, J = 6.1 Hz), 6.93 (1H, t, J = 53.7 Hz), 4.78-4.70 (1H, m), 2.21 (3H, s), 1.51 (6H, br s)., MS (m/z): 470 (M+H)⁺. |
| 95 | 3-(6-chloro-1H-imidazo[4,5-c]pyridin-2-yl)-5-(6,7-dihydro-5H-pyrazolo[5,1-b][1,3]oxazin-3-yl)-1,6-naphthyridin-2(1H)-one | ¹H-NMR (DMSO-D₆) δ: 13.01 (1H, br s), 12.76 (1H, br s), 9.26 (1H, s), 8.75 (1H, s), 8.51 (1H, d, J = 5.5 Hz), 7.71 (1H, s), 7.66 (1H, s), 7.17 (1H, d, J = 5.5 Hz), 4.40 (2H, t, J = 4.9 Hz), 4.24 (2H, t, J = 6.1 Hz), 2.33-2.22 (2H, m)., MS (m/z): 420 (M+H)⁺. |
| 96 | 3-{6-chloro-4-[(2R)-2-methylazetidin-1-yl]-1H-imidazo[4,5-c]pyridin-2-yl}-5-[1-(difluoromethyl)-3,5-dimethyl-1H-pyrazol-4-yl]-1,6-naphthyridin-2(1H)-one | ¹H-NMR (DMSO-D₆) δ: 12.68 (1H, br s), 8.64 (1H, d, J = 6.1 Hz), 8.62 (1H, s), 7.90 (1H, t, J = 58.0 Hz), 7.34 (1H, d, J = 6.1 Hz), 6.86 (1H, s), 4.73-4.63 (1H, m), 4.22-4.05 (2H, m), 2.55-2.50 (1H, m), 2.33 (3H, s), 2.16 (3H, s), 2.06-1.96 (1H, m), 1.57 (3H, d, J = 6.1 Hz). |
| 97 | 3-(6-chloro-4-ethyl-1H-imidazo[4,5-c]pyridin-2-yl)-5-[1-(difluoromethyl)-1H-pyrazol-3-yl]-1,6-naphthyridin-2(1H)-one | ¹H-NMR (DMSO-D₆) δ: 12.98 (2H, br s), 10.36 (1H, s), 8.66 (1H, d, J = 6.1 Hz), 8.47 (1H, d, J = 2.4 Hz), 8.00 (1H, t, J = 58.9 Hz), 7.52 (1H, s), 7.38 (1H, d, J = 6.1 Hz), 7.17 (1H, d, J = 2.4 Hz), 3.14 (2H, q, J = 7.5 Hz), 1.37 (3H, t, J = 7.5 Hz). |
| 98 | 3-(6-chloro-4-ethyl-1H-imidazo[4,5-c]pyridin-2-yl)-5-(1,5-dimethyl-1H-pyrazol-3-yl)-1,6-naphthyridin-2(1H)-one | ¹H-NMR (DMSO-D₆) δ: 13.01 (1H, br s), 9.13 (1H, s), 8.59 (1H, d, J = 6.1 Hz), 7.75 (1H, s), 7.52 (1H, s), 7.24 (1H, d, J = 6.1 Hz), 3.89 (3H, s), 3.10 (2H, q, J = 7.5 Hz), 2.43 (3H, s), 1.32 (3H, t, J = 7.5 Hz). |
| 99 | 3-(6-chloro-4-{[(2R)-1,1,1-trifluoropropan-2-yl]amino}-1H-imidazo[4,5-c]pyridin-2-yl)-5-(1,5-dimethyl-1H-pyrazol-4-yl)-1,6-naphthyridin-2(1H)-one | ¹H-NMR (DMSO-D₆) δ: 9.06 (1H, br s), 8.58 (1H, d, J = 5.5 Hz), 7.73 (1H, s), 7.24 (1H, d, J = 6.1 Hz), 6.99 (1H, s), 5.21 (1H, br s), 3.89 (3H, s), 2.40 (3H, s), 1.42 (3H, d, J = 7.3 Hz). |
| 100 | 3-[6-chloro-4-(tetrahydro-2H-pyran-4-yl)-1H-imidazo[4,5-c]pyridin-2-yl]-5-[3-cyclopropyl-1-(difluoromethyl)-5-methyl-1H-pyrazol-4-yl]-1,6-naphthyridin-2(1H)-one | ¹H-NMR (DMSO-D₆) δ: 12.98 (1H, s), 12.89 (1H, s), 8.93 (1H, s), 8.67 (1H, d, J = 6.1 Hz), 7.86 (1H, t, J = 57.7 Hz), 7.50 (1H, s), 7.35 (1H, d, J = 6.1 Hz), 3.98-3.97 (2H, br m), 3.63-3.56 (1H, m), 3.47 (2H, t, J = 11.3 Hz), 2.35 (3H, s), 1.95-1.93 (2H, br m), 1.81-1.78 (2H, br m), 1.69-1.68 (1H, br m), 0.96-0.94 (1H, br m), 0.89-0.86 (1H, br m), 0.82-0.80 (2H, br m)., MS (m/z): 552 (M+H)⁺. |

### Example 101

### Production of 3-(6-chloro-4-ethyl-1H-imidazo[4,5-c]pyridin-2-yl)-5-(6-cyclopropyl-4-methoxypyridin-3-yl)-3,4-dihydropyrido[4,3-d]pyrimidin-2(1H)-one

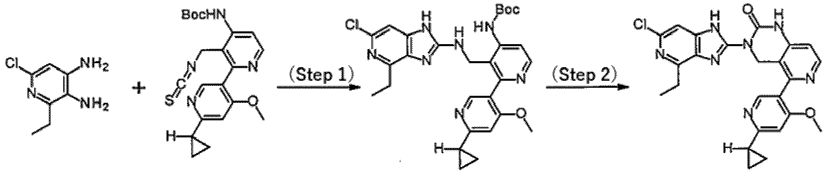

### (Step 1) tert-Butyl (3-{[(6-chloro-4-ethyl-1H-imidazo[4,5-c]pyridin-2-yl)amino]methyl}-6'-cyclopropyl-4'-methoxy[2,3'-bipyridin]-4-yl)carbamate

A mixture of the compound (45.5 mg) obtained in Step 2 of Reference Example 30, the compound (100 mg) obtained in Step 3 of Reference Example 105, imidazole (16.5 mg), and N,N-dimethylformamide (1.5 mL) was stirred at 80°C for 9 hours. Then, N,N'-diisopropylcarbodiimide (84.0 µL) was added, and the mixture was stirred at the same temperature for another 7 hours. After cooling the reaction mixture to room temperature, water was added, and the mixture was extracted three times with ethyl acetate. The obtained organic layer was washed with water and saturated sodium chloride solution, and then dried over anhydrous sodium sulfate. The obtained organic layer was filtered, and the solvent was evaporated under reduced pressure. The residue was purified by silica gel column chromatography (ethyl acetate/methanol) to obtain the title compound (68.1 mg). ¹H-NMR (DMSO-D₆) δ: 12.54 (1H, s), 10.79 (1H, s), 8.42 (1H, d, J = 4.9 Hz), 8.17 (1H, s), 7.30 (1H, s), 7.22 (1H, s), 6.95 (1H, d, J = 4.9 Hz), 5.22-4.83 (2H, m), 3.81 (3H, s), 2.93 (2H, q, J = 7.4 Hz), 2.24-2.16 (1H, m), 1.26 (3H, t, J = 7.4 Hz), 1.04-0.97 (4H, m).

### (Step 2) 3-(6-Chloro-4-ethyl-1H-imidazo[4,5-c]pyridin-2-yl)-5-(6-cyclopropyl-4-methoxypyridin-3-yl)-3,4-dihydropyrido[4,3-d]pyrimidin-2(1H)-one

To a solution of the compound (68.1 mg) obtained in Step 1 above in dichloromethane (1.0 mL) was added a 4.0 M solution of hydrogen chloride in 1,4-dioxane (330 µL) at room temperature, and the mixture was stirred at the same temperature for 17.5 hours. The reaction mixture was concentrated under reduced pressure. N,N-Dimethylformamide (1.0 mL), triethylamine (51.5 µL), and 1,1'-carbonyldiimidazole (40.0 mg) were added to the obtained residue at room temperature, and the mixture was stirred at the same temperature for 30 minutes. Then, the mixture was stirred at 70°C for another 4 hours. After cooling the reaction mixture to room temperature, water was added, and the mixture was extracted three times with ethyl acetate. The obtained organic layer was washed with water and saturated sodium chloride solution, and then dried over anhydrous sodium sulfate. The obtained organic layer was filtered, and the solvent was evaporated under reduced pressure. The obtained residue was purified by silica gel column chromatography (dichloromethane/methanol) to obtain the title compound (34.4 mg).
¹H-NMR (DMSO-D₆) δ: 12.51 (1H, br s), 10.76 (1H, br s), 8.39 (1H, d, J = 4.9 Hz), 8.14 (1H, s), 7.27 (1H, s), 7.19 (1H, s), 6.92 (1H, d, J = 4.9 Hz), 5.00 (2H, br s), 3.78 (3H, s), 2.90 (2H, q, J = 7.6 Hz), 2.21-2.13 (1H, m), 1.22 (3H, t, J = 7.6 Hz), 1.01-0.95 (4H, m)., MS (m/z): 476 (M+H)⁺.

### Example 102

### Production of 3-(6-chloro-1H-imidazo[4,5-c]pyridin-2-yl)-5-(2-chlorophenyl)-3,4-dihydropyrido[4,3-d]pyrimidin-2(1H)-one

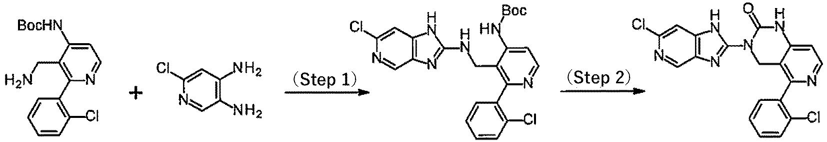

### (Step 1) tert-Butyl [3-{[(6-chloro-1H-imidazo[4,5-c]pyridin-2-yl)amino]methyl}-2-(2-chlorophenyl)pyridin-4-yl]carbamate

To a solution of the compound (150 mg) obtained in Step 3 of Reference Example 106 in acetonitrile (4 mL) was added 1,1'-thiocarbonyldiimidazole (96 mg) at room temperature, and the mixture was stirred at the same temperature for 1 hour. Then, N,N-dimethylformamide (1.5 mL) and 6-chloropyridin-3,4-diamine (77.4 mg) were added, and the mixture was stirred at 80°C for another 2 hours. Subsequently, N,N'-diisopropylcarbodiimide (84 µL) was added to the reaction mixture at 80°C, and the mixture was stirred at the same temperature for 7 hours. After cooling the reaction mixture to room temperature, water was added, and the mixture was extracted three times with ethyl acetate. The obtained organic layer was washed with saturated sodium chloride solution, and then dried over anhydrous sodium sulfate. The obtained organic layer was filtered, and the solvent was evaporated under reduced pressure. The residue was purified by silica gel column chromatography (n-hexane/ethyl acetate) to obtain the title compound (128 mg). ¹H-NMR (CDCl₃) δ: 11.06 (0.35H, s), 10.93 (0.65H, s), 10.68 (0.65H, br s), 10.43 (0.35H, br s), 8.46-8.35 (1.35H, m), 8.18 (0.35H, d, J = 6.1 Hz), 8.15 (0.65H, d, J = 6.1 Hz), 7.96 (0.65H, s), 7.42-6.90 (5H, m), 5.14 (0.65H, t, J = 6.7 Hz), 4.89 (0.35H, t, J = 6.7 Hz), 4.44 (1.30H, d, J = 6.7 Hz), 4.41 (0.70H, d, J = 6.7 Hz), 1.63 (5.85H, s), 1.59 (3.15H, s).

### (Step 2) 3-(6-Chloro-1H-imidazo[4,5-c]pyridin-2-yl)-5-(2-chlorophenyl)-3,4-dihydropyrido[4,3-d]pyrimidin-2(1H)-one

To a solution of the compound (125 mg) obtained in Step 1 above in N,N-dimethylformamide (3 mL) was added 55% sodium hydride in oil (56 mg) at room temperature, and the mixture was stirred at 70°C for 9 hours. The reaction mixture was cooled to room temperature, saturated ammonium chloride solution was added, and the mixture was extracted three times with ethyl acetate. The obtained organic layer was washed with water and saturated sodium chloride solution, and then dried over anhydrous sodium sulfate. The obtained organic layer was filtered, and the solvent was evaporated under reduced pressure. The residue was purified by silica gel column chromatography (dichloromethane/methanol) to obtain the title compound (40.5 mg).
¹H-NMR (DMSO-D₆) δ: 12.63 (1H, br s), 10.90 (1H, br s), 8.50 (1H, s), 8.43 (1H, d, J = 5.5 Hz), 7.67 (1H, dd, J = 7.9, 1.2 Hz), 7.62-7.41 (4H, m), 6.98 (1H, d, J = 5.5 Hz), 4.98 (2H, s), 3.33 (3H, s)., MS (m/z): 411 (M+H)⁺.

### Example 103

### Production of 3-(6-chloro-1H-imidazo[4,5-c]pyridin-2-yl)-5-(2-methoxyphenyl)-3,4-dihydropyrido[4,3-d]pyrimidin-2(1H)-one

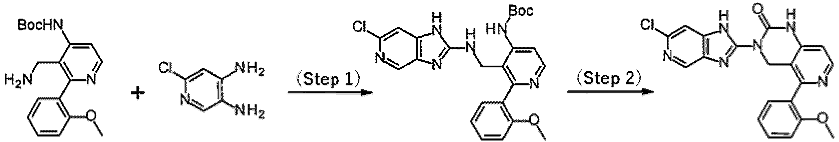

### (Steps 1 and 2) 3-(6-Chloro-1H-imidazo[4,5-c]pyridin-2-yl)-5-(2-methoxyphenyl)-3,4-dihydropyrido[4,3-d]pyrimidin-2(1H)-one

The title compound was obtained by using the compound obtained in Step 3 of Reference Example 107 as a production material and performing the same operations as in Steps 1 and 2 of Example 102.
¹H-NMR (DMSO-D₆) δ: 12.59 (1H, br s), 10.81 (1H, br s), 8.48 (1H, s), 8.39 (1H, d, J = 5.5 Hz), 7.57-7.40 (2H, m), 7.27 (1H, dd, J = 7.4, 1.8 Hz), 7.20 (1H, d, J = 8.0 Hz), 7.10 (1H, t, J = 7.4 Hz), 6.93 (1H, d, J = 5.5 Hz), 4.98 (2H, s), 3.72 (3H, s)., MS (m/z): 407 (M+H)⁺.

### Example 104

### Production of 3-(6-chloro-1H-imidazo[4,5-c]pyridin-2-yl)-5-[1-(cyclopropylsulfonyl)-1,2,5,6-tetrahydropyridin-3-yl]-1,6-naphthyridin-2(1H)-one

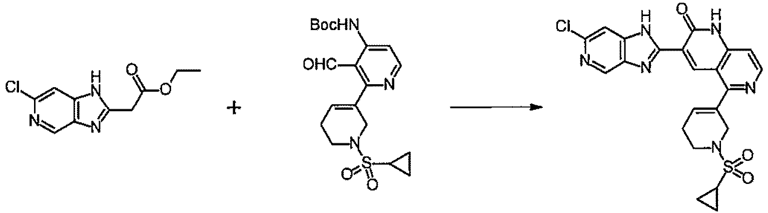

### 3-(6-Chloro-1H-imidazo[4,5-c]pyridin-2-yl)-5-[1-(cyclopropylsulfonyl)-1,2,5,6-tetrahydropyridin-3-yl]-1,6-naphthyridin-2(1H)-one

A mixture of the compound (65 mg) obtained in Reference Example 28, the compound (110 mg) obtained in Step 2 of Reference Example 109, piperidine (49 µL), and ethanol (3 mL) was stirred at 90°C for 1.5 hours. The reaction mixture was cooled to room temperature, and then the precipitated solid was collected by filtration. The obtained solid was washed with ethanol and then dried under reduced pressure to obtain the title compound (58.8 mg).
¹H-NMR (DMSO-D₆) δ: 13.20-12.77 (2H, m), 9.27 (1H, s), 8.80 (1H, s), 8.57 (1H, d, J = 5.9 Hz), 7.70 (1H, br s), 7.29 (1H, d, J = 5.9 Hz), 6.25-6.20 (1H, m), 4.35-4.29 (2H, m), 3.59-3.51 (2H, m), 2.77-2.70 (1H, m), 2.60-2.53 (2H, m), 1.08-0.98 (4H, m)., MS (m/z): 483 (M+H)⁺.

### Example 105

### Production of 3-(6-chloro-1H-imidazo[4,5-c]pyridin-2-yl)-5-[1-(cyclopropylcarbonyl)-1,2,5,6-tetrahydropyridin-3-yl]-1,6-naphthyridin-2(1H)-one

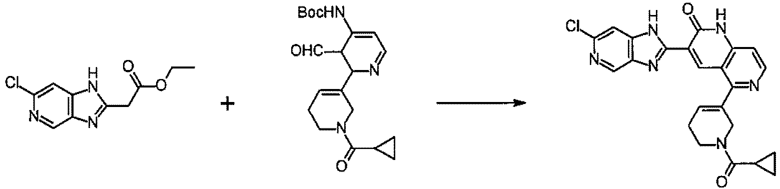

### 3-(6-Chloro-1H-imidazo[4,5-c]pyridin-2-yl)-5-[1-(cyclopropylcarbonyl)-1,2,5,6-tetrahydropyridin-3-yl]-1,6-naphthyridin-2(1H)-one

The title compound was obtained by using the compound obtained in Reference Example 28 and the compound obtained in Step 3 of Reference Example 108 as production materials and performing the same operation as in Example 104.
¹H-NMR (DMSO-D₆) δ: 9.24 (1H, s), 8.78 (1H, s), 8.54 (1H, d, J = 5.5 Hz), 7.69 (1H, s), 7.25 (1H, d, J = 5.5 Hz), 6.30-6.15 (1H, m), 4.72 (1H, s), 4.48 (1H, s), 3.99-3.88 (1H, m), 3.80-3.67 (1H, m), 2.46-2.34 (2H, m), 2.17-1.93 (1H, m), 0.82-0.67 (4H, m)., MS (m/z): 447 (M+H)⁺.

### Example 106

### Production of 3-(6-chloro-1H-imidazo[4,5-c]pyridin-2-yl)-5-[1-(phenylsulfonyl)azepan-3-yl]-1,6-naphthyridin-2(1H)-one

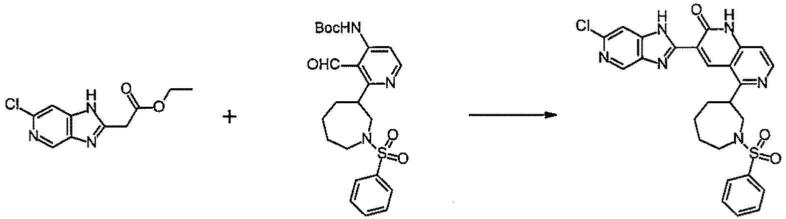

### 3-(6-Chloro-1H-imidazo[4,5-c]pyridin-2-yl)-5-[1-(phenylsulfonyl)azepan-3-yl]-1,6-naphthyridin-2(1H)-one

The title compound was obtained by using the compound obtained in Reference Example 28 and the compound obtained in Step 4 of Reference Example 110 as production materials and performing the same operation as in Example 104.
¹H-NMR (DMSO-D₆) δ: 13.21 (0.35H, s), 13.10 (0.65H, s), 12.89-12.85 (1H, m), 9.24 (0.35H, s), 9.22 (0.65H, s), 8.90 (0.65H, s), 8.81 (0.35H, d, J = 0.8 Hz), 8.51 (0.35H, d, J = 5.5 Hz), 8.50 (0.65H, d, J = 5.5 Hz), 7.87 (0.35H, s), 7.82 (2H, d, J = 7.0 Hz), 7.71 (0.65H, d, J = 1.2 Hz), 7.66-7.59 (3H, m), 7.24 (1H, d, J = 5.5 Hz), 3.84-3.61 (3H, m), 3.57-3.47 (1H, m), 3.23-3.11 (1H, m), 2.04-1.83 (3H, m), 1.80-1.58 (3H, m)., MS (m/z): 535 (M+H)⁺.

### Example 107

### Production of 7-chloro-18-fluoro-10,14-dimethyl-3,8,10,14,21,25,31-heptaazahexacyclo[18.6.2.2^{16,19}.1^{2,5}.0^{4,9}.0^{24,28}]hentriacont-1(27),2,4,6,8,16,18,20(28),21,23,29-undecaen-15,26-dione

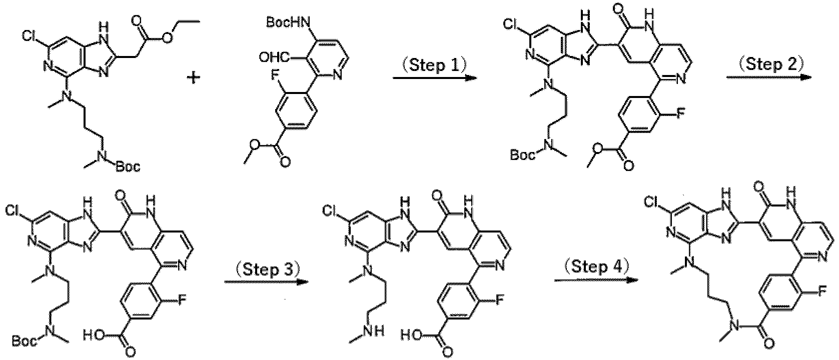

### (Step 1) Methyl 4-(3-{4-[{3-[(tert-butoxycarbonyl)(methyl)amino]propyl}(methyl)amino]-6-chloro-1H-imidazo[4,5-c]pyridin-2-yl}-2-oxo-1,2-dihydro-1,6-naphthyridin-5-yl)-3-fluorobenzoate

A mixture of the compound (170 mg) obtained in Step 3 of Reference Example 41, the compound (190 mg) obtained in Reference Example 78, piperidine (0.10 mL), and ethanol (10 mL) was stirred at 90°C for 21 hours. After cooling the reaction mixture to room temperature, the solvent was evaporated under reduced pressure. A mixture of n-hexane/ethyl acetate was added to the residue, and the precipitated solid was collected by filtration to obtain the crude title compound (118 mg).

### (Step 2) 4-(3-{4-[{3-[(tert-Butoxycarbonyl)(methyl)amino]propyl}(methyl)amino]-6-chloro-1H-imidazo[4,5-c]pyridin-2-yl}-2-oxo-1,2-dihydro-1,6-naphthyridin-5-yl)-3-fluorobenzoic acid

To a solution of the compound (118 mg) obtained in Step 1 above in methanol (2 mL)-tetrahydrofuran (2 mL) was added a 1 M aqueous solution of sodium hydroxide (2 mL) at room temperature, and the mixture was stirred at the same temperature for 24 hours. 1 M Hydrochloric acid (2.3 mL) and water were added to the reaction mixture, and the mixture was extracted three times with ethyl acetate. The obtained organic layer was washed with water and saturated sodium chloride solution, and then dried over anhydrous sodium sulfate. The obtained organic layer was filtered, and the solvent was evaporated under reduced pressure to obtain the crude title compound (110 mg).

### (Step 3) 4-[3-(6-Chloro-4-{methyl[3-(methylamino)propyl]amino}-1H-imidazo[4,5-c]pyridin-2-yl)-2-oxo-1,2-dihydro-1,6-naphthyridin-5-yl]-3-fluorobenzoic acid

To a suspension of the compound (110 mg) obtained in Step 2 above in dichloromethane (5 mL) was added trifluoroacetic acid (1 mL) at room temperature, and the mixture was stirred at the same temperature for 4 hours. The reaction mixture was concentrated under reduced pressure. The remaining trifluoroacetic acid was removed by azeotropic distillation with toluene to obtain the crude title compound (100 mg).

### (Step 4) 7-Chloro-18-fluoro-10,14-dimethyl-3,8,10,14,21,25,31-heptaazahexacyclo[18.6.2.2^{16,19}.1^{2,5}.0^{4,9}.0^{24,28}]hentriacont-1(27),2,4,6,8,16,18,20(28),21,23,29-undecaen-15,26-dione

To a solution of the compound (100 mg) obtained in Step 3 above in N,N-dimethylformamide (18 mL) were added O-(7-azabenzotriazol-1-yl)-N,N,N',N'-tetramethyluronium hexafluorophosphate (0.14 g) and N,N-diisopropylethylamine (0.16 mL) at room temperature, and the mixture was stirred at the same temperature for 13.5 hours. The reaction mixture was diluted with ethyl acetate, and water and saturated ammonium chloride solution were added. After stirring, the organic layer and the aqueous layer were separated. The aqueous layer was extracted twice with ethyl acetate. The combined organic layer was washed three times with water and once with saturated sodium chloride solution, and then dried over anhydrous sodium sulfate. The obtained organic layer was filtered, and the solvent was evaporated under reduced pressure. The residue was suspended in a mixture of n-hexane/ethyl acetate, and the precipitated solid was collected by filtration to obtain the title compound (73 mg).
¹H-NMR (DMSO-D₆) δ: 12.85 (1H, s), 12.69 (1H, s), 8.68 (1H, d, J = 5.5 Hz), 8.30 (1H, d, J = 1.8 Hz), 7.63 (1H, t, J = 7.3 Hz), 7.57 (1H, dd, J = 9.8, 1.2 Hz), 7.44-7.40 (2H, m), 6.88 (1H, s), 4.20-4.09 (1H, m), 3.73-3.65 (1H, m), 3.12-3.03 (2H, m), 3.11 (3H, s), 3.03 (3H, s), 2.06-1.87 (2H, m)., MS (m/z): 518 (M+H)⁺.

The following compounds were synthesized by performing the same operations as in Steps 1 to 4 of Example 107 (Table 15-3 and Table 15-4).

**Table 15-3**

| Example No. | Production Material 1 | Production Material 2 | Structure of Compound Synthesized |
|---|---|---|---|
| 108 | 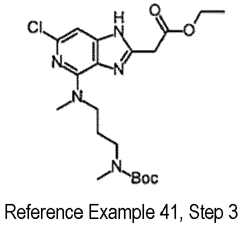 | 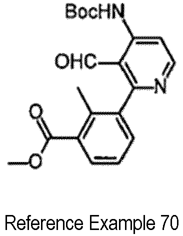 | 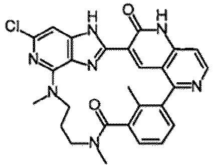 |
| 109 | 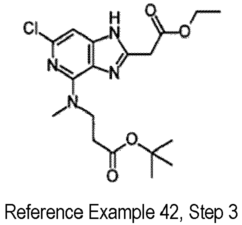 | 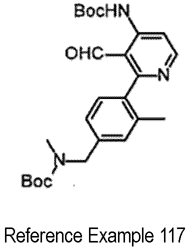 | 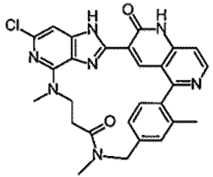 |
| 110 | 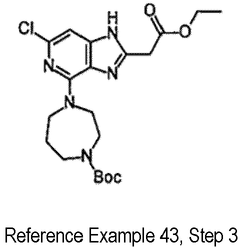 | 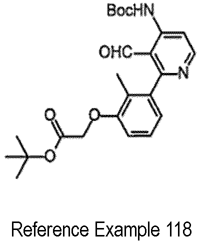 | 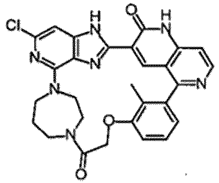 |
| 111 | 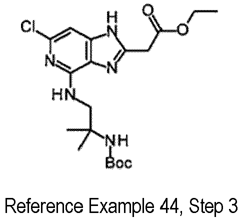 | 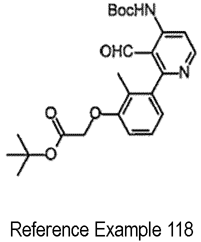 | 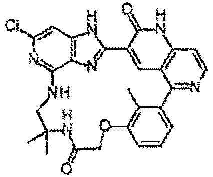 |
| 112 | 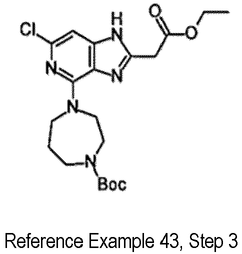 | 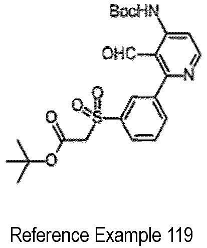 | 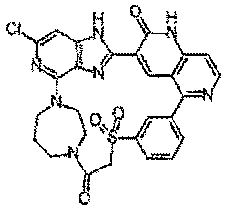 |
| 113 | 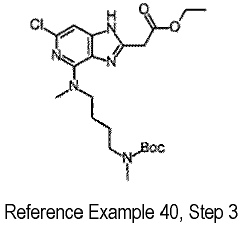 | 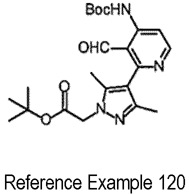 | 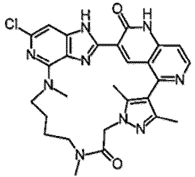 |
| 114 | 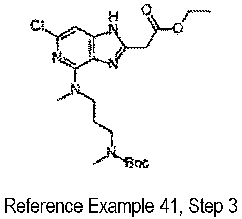 | 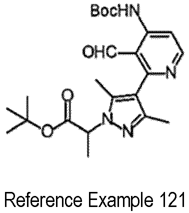 | 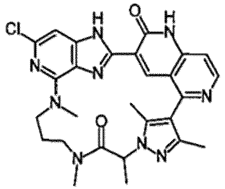 |
| 115 | 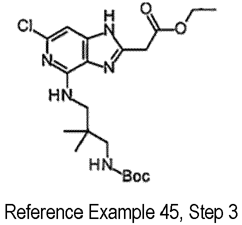 | 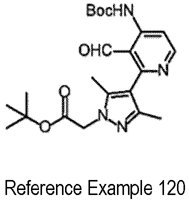 | 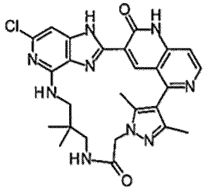 |
| 116 | 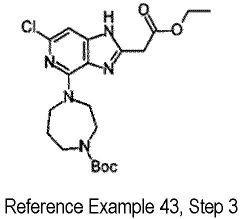 | 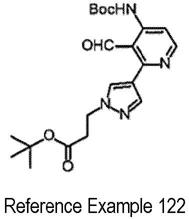 | 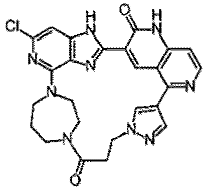 |

**Table 15-4**

| Reference Example No. | Name of Compound Synthesized | Synthesis Method | Spectral Data |
|---|---|---|---|
| 108 | 7-chloro-10,14,30-trimethyl-3,8,10, 14,22,26,31-heptaazahexacyclo [19.6.2.1^{2,5}.1^{16,20}.0^{4,9}.0^{25,29}] hentriacont-1(28),2,4,6,8,16(30),17,19,21(29),22,24-undecaen-15,27-dione | Example 107, Steps 1 to 4 | ¹H-NMR (DMSO-D₆) δ: 12.85 (1H, br s), 12.71 (1H, s), 8.66 (1H, d, J = 6.1 Hz), 8.48 (1H, s), 7.49 (1H, t, J = 7.3 Hz), 7.44 (1H, dd, J = 7.3, 1.5 Hz), 7.40-7.36 (2H, m), 6.86 (1H, s), 4.90-4.83 (1H, m), 3.34-3.27 (2H, m), 3.09 (3H, s), 3.07 (3H, s), 2.96-2.85 (1H, m), 2.09-1.96 (2H, m), 1.91 (3H, s)., MS (m/z): 514 (M+H)⁺. |
| 109 | 7-chloro-10,14,18-trimethyl-3,8,10, 14,21,25,31-heptaazahexacyclo [18.6.2.2^{16,19}.1^{2,5}.0^{4,9}.0^{24,28}] hentriacont-1(27),2,4,6,8,16,18,20(28),21,23,29-undecaen-13,26-dione | Example 107, Steps 1, 3, and 4 | ¹H-NMR (DMSO-D₆) δ: 13.83 (1H, s), 12.79 (1H, s), 8.77 (1H, dd, J = 4.3, 1.2 Hz), 8.62 (1H, d, J = 6.1 Hz), 8.54 (1H, dd, J = 8.5, 1.2 Hz), 7.54-7.50 (1H, m), 7.41-7.32 (2H, m), 6.87 (1H, s), 4.57-4.18 (2H, m),3.15 (3H, s), 3.03 (3H, s), 2.63-2.55 (2H, m), 2.02-1.97 (5H, m)., MS (m/z): 514 (M+H)⁺. |
| 110 | 7-chloro-32-methyl-17-oxa-3,8,10,14,24,28,35-heptaazaheptacyclo [21.6.2.2^{10,14}.1^{2,5}.1^{18,22}.0^{4,9}.0^{27,31}] pentatriacont-1(30),2,4,6,8,18(32),19,21,23(31),24,26-undecaen-15,29-dione | Example 107, Steps 1, 3, and 4 | ¹H-NMR (DMSO-D₆) δ: 12.83 (1H, s), 12.72 (1H, s), 8.65 (1H, d, J = 6.1 Hz), 8.58 (1H, s), 7.43 (1H, t, J = 7.9 Hz), 7.38 (1H, d, J = 6.1 Hz), 7.21 (1H, d, J = 7.9 Hz), 7.08 (1H, d, J = 7.9 Hz), 6.91 (1H, s), 5.06 (1H, d, J = 12.2 Hz), 4.73 (1H, d, J = 12.2 Hz), 4.62-4.51 (1H, m), 4.46-4.34 (1H, m), 4.10-3.95 (2H, m), 3.68-3.59 (1H, m), 3.56-3.47 (1H, m), 3.37-3.29 (1H, m), 2.84-2.74 (1H, m), 2.27-2.15 (1H, m), 1.96 (3H, s), 1.89-1.77 (1H, m)., MS (m/z): 542 (M+H)⁺. |
| 111 | 7-chloro-12,12,31-trimethyl-16-oxa-3,8,10,13,23,27,32-heptaazahexacyclo [20.6.2.1^{2,5}.1^{17,21}.0^{4,9}.0^{26,30}] dotriacont-1(29),2,4,6,8,17(31),18,20,22(30),23,25-undecaen-14,28-dione | Example 107, Steps 1, 3, and 4 | ¹H-NMR (DMSO-D₆) δ: 12.79 (1H, br s), 12.57 (1H, br s), 8.62 (1H, d, J = 5.5 Hz), 8.49 (1H, br s), 7.47-7.23 (5H, m), 6.92 (1H, d, J = 7.3 Hz), 6.76 (1H, s), 4.85 (1H, d, J = 14.6 Hz), 4.67 (1H, d, J = 14.6 Hz), 4.06-3.72 (2H, m), 2.00 (3H, s), 1.32 (3H, s), 1.22 (3H, s)., MS (m/z): 530 (M+H)⁺. |
| 112 | 7-chloro-17-thia-3,8,10,14,24,28,35-heptaazaheptacyclo [21.6.2.2^{10,14}.1^{2,5}.1^{18,22}.0^{4,9}.0^{27,31}] pentatriacont-1(30)2,4,6,8,18(32),19,21,23(31),24,26-undecaen-15,29-dione17,17-dioxide | Example 107, Steps 1, 3, and 4* | ¹H-NMR (DMSO-D₆) δ: 12.86 (1H, br s), 8.98 (1H, s), 8.69 (1H, d, J = 5.5 Hz), 8.32 (1H, s), 8.23 (1H, d, J = 7.9 Hz), 8.10 (1H, d, J = 7.9 Hz), 7.95 (1H, t, J = 7.9 Hz), 7.41 (1H, d, J = 5.5 Hz), 6.92 (1H, s), 4.77 (2H, s), 4.47-4.44 (2H, m), 3.98-3.93 (2H, m), 3.86 (2H, t, J = 6.7 Hz), 3.68-3.63 (2H, m), 1.69-1.61 (2H, m)., MS (m/z): 576 (M+H)⁺. |
| | | * | |
| | | Conducted at 70°C | |
| 113 | 7-chloro-10,15,20,31-tetramethyl-3,8,10,15,18,19,23,27,32-nonaazahexacyclo [20.6.2.1^{2,5}.1^{18,21}.0^{4,9}.0^{26,30}] dotriacont-1(29),2,4,6,8,19,21(31),22(30),23,25-decaen-16,28-dione | Example 107, Steps 1, 3, and 4 | ¹H-NMR (DMSO-D₆) δ: 12.78-12.66 (2H, m), 8.70 (0.5H, s), 8.60 (1H, d, J = 6.1 Hz), 8.53 (0.5H, s), 7.29-7.25 (1H, m), 6.85 (0.5H, s), 6.83 (0.5H, s), 5.14-4.89 (2H, m), 4.85-4.76 (0.5H, m), 4.48-4.37 (0.5H, m), 3.85-3.68 (1.5H, m), 3.68-3.57 (0.5H, m), 3.31-3.20 (1H, m), 3.16-3.04 (4.5H, m), 2.91-2.83 (1.5H, m), 2.20-1.96 (6H, m), 1.77-1.39 (3H, m), 1.28-1.12 (1H, m)., MS (m/z): 546 (M+H)⁺. |
| 114 | 7-chloro-10,14,16,19,30-pentamethyl-3,8,10,14,17,18,22,26,31-nonaazahexacyclo [19.6.2.1^{2,5}.1^{17,20}.0^{4,9}.0^{25,29}] hentriacont-1(28),2,4,6,8,18,20(30),21(29),22,24-decaen-15,27-dione | Example 107, Steps 1, 3, and 4 | ¹H-NMR (DMSO-D₆) δ: 12.73-12.70 (2H, m), 8.63-8.61 (2H, m), 7.28 (1H, d, J = 5.5 Hz), 6.90 (1H, s), 5.35-5.31 (1H, m), 4.73-4.65 (1H, m), 4.14-4.01 (1H, m), 3.73-3.64 (1H, m), 3.22-3.14 (1H, m), 3.08 (3H, s), 2.94-2.92 (1H, m), 2.91 (3H, s), 2.25 (3H, s), 2.14 (3H, s), 1.45 (3H, br s), 1.18-1.17 (1H, m)., MS (m/z): 546 (M+H)⁺. |
| 115 | 7-chloro-12,12,19,30-tetramethyl-3,8,10,14,17,18,22,26,31-nonaazahexacyclo [19.6.2.1^{2,5}.1^{17,20}.0^{4,9}.0^{25,29}] hentriacont-1(28),2,4,6,8,18,20(30),21(29),22,24-decaen-15,27-dione | Example 107, Steps 1, 3, and 4 | ¹H-NMR (DMSO-D₆) δ: 12.70 (1H, s), 12.58 (1H, s), 9.09 (1H, s), 8.60 (1H, d, J = 5.5 Hz), 7.95 (1H, br s), 7.25 (1H, d, J = 5.5 Hz), 6.84 (1H, br s), 6.80 (1H, s), 4.94 (1H, d, J = 17.8 Hz), 4.78 (1H, d, J = 17.8 Hz), 4.06-3.94 (1H, m), 3.35-3.20 (2H, m), 3.12-2.97 (1H, m), 2.24 (3H, s), 2.12 (3H, s), 0.98 (3H, s), 0.92 (3H, s)., MS (m/z): 532 (M+H)⁺. |
| 116 | 7-chloro-3,8,10,14,18,19,23,27,34-nonaazaheptacyclo [20.6.2.2^{10,14}.1^{2,5}.1^{18,21}.0^{4,9}.0^{26,30}] tetratriacont-1(29),2,4,6,8,19,21(31),22(30),23,25-decaen-15,28-dione | Example 107, Steps 1, 3, and 4* | ¹H-NMR (DMSO-D₆) δ: 12.74 (2H, br s), 9.16 (1H, s), 8.60 (1H, s), 8.55 (1H, d, J = 6.1 Hz), 7.90 (1H, s), 7.24-7.21 (1H, m), 6.91 (1H, s), 4.58-4.47 (4H, m), 4.16-4.11 (2H, m), 3.93-3.87 (2H, m), 3.65-3.59 (2H, m), 3.03 (2H, t, J = 7.9 Hz), 1.69-1.60 (2H, m)., MS (m/z): 516 (M+H)⁺. |
| | | * | |
| | | Conducted at 60°C | |

### Example 117

### Production of 3-{6-chloro-4-[(3R)-3-methylmorpholin-4-yl]-1H-imidazo[4,5-c]pyridin-2-yl}-1,6-naphthyridin-2(1H)-one p-toluenesulfonate 3-{6-Chloro-4-[(3R)-3-methylmorpholin-4-yl]-1H-imidazo[4,5-c]pyridin-2-yl}-1,6-naphthyridin-2(1H)-one p-toluenesulfonate

To a suspension of the compound (3.76 g) obtained in Example 1 in ethanol (53 mL) was added p-toluenesulfonic acid monohydrate (1.80 g) at room temperature, and the mixture was stirred at 90°C for 1.5 hours. The reaction mixture was cooled to room temperature, and the precipitated solid was collected by filtration. The obtained solid was washed with ethanol and then dried under reduced pressure to obtain the title compound (5.01 g).
¹H-NMR (DMSO-D₆) δ: 13.26 (1H, br s), 12.90 (1H, br s), 9.41 (1H, br s), 9.10 (1H, s), 8.69-8.63 (1H, m), 7.59-7.51 (1H, m), 7.47 (2H, d, J = 7.9 Hz), 7.11 (2H, d, J = 7.9 Hz), 6.99 (1H, s), 5.51-5.37 (1H, m), 4.91-4.76 (1H, m), 4.04-3.94 (1H, m), 3.82-3.72 (2H, m), 3.64-3.54 (1H, m), 3.42-3.32 (1H, m), 2.29 (3H, s), 1.28 (3H, d, J = 6.7 Hz)., MS (m/z): 397 (M+H)⁺.

### Example 118

### Production of 3-{6-chloro-4-[(2R,4R)-4-fluoro-2-methylpyrrolidin-1-yl]-1H-imidazo[4,5-c]pyridin-2-yl}-1,6-naphthyridin-2(1H)-one methanesulfonate 3-{6-Chloro-4-[(2R,4R)-4-fluoro-2-methylpyrrolidin-1-yl]-1H-imidazo[4,5-c]pyridin-2-yl}-1,6-naphthyridin-2(1H)-one methanesulfonate

To a suspension of the compound (48 mg) obtained in Example 10 in ethanol (2.0 mL) was added methanesulfonic acid (8.2 µL) at room temperature, and the mixture was stirred at the same temperature for 8 hours. The reaction mixture was concentrated under reduced pressure. Ethyl acetate was added to the residue, and the precipitated solid was collected by filtration and dried under reduced pressure to obtain the title compound (56 mg).
¹H-NMR (DMSO-D₆) δ: 13.34 (1H, s), 12.83 (1H, s), 9.45 (1H, s), 9.14 (1H, s), 8.69 (1H, d, J = 6.1 Hz), 7.60 (1H, d, J = 6.1 Hz), 6.91 (1H, s), 5.45 (1H, d, J = 53.7 Hz), 4.86-4.57 (2H, m), 4.04 (1H, dd, J = 36.6, 12.8 Hz), 2.31 (3H, s), 2.32-2.27 (1H, m), 2.09-1.93 (1H, m), 1.36 (3H, d, J = 6.1 Hz)., MS (m/z): 399 (M+H)⁺.

The following compounds were synthesized by performing the same operation as in Example 118 (Table 16).

**Table 16**

| Example No. | Production Material | Name of Compound Synthesized | Spectral Data |
|---|---|---|---|
| 119 | Example 11 | 3-{6-chloro-4-[(2R,4R)-4-fluoro-2-methylpyrrolidin-1-yl]-1H-imidazo[4,5-c]pyridin-2-yl}-5-(tetrahydro-2H-pyran-4-yl)-1,6-naphthyridin-2(1H)-one methanesulfonate | ¹H-NMR (DMSO-D₆) δ: 12.98 (1H, s), 12.80 (1H, s), 9.20 (1H, s), 8.53 (1H, d, J = 6.1 Hz), 7.34 (1H, d, J = 6.1 Hz), 6.91 (1H, s), 5.45 (1H, d, J = 54.3 Hz), 4.90-4.46 (2H, m), 4.11-3.95 (3H, m), 3.89-3.75 (1H, m), 3.61 (2H, t, J = 12.2 Hz), 2.49-2.42 (1H, m), 2.31 (3H, s), 2.09-1.90 (3H, m), 1.79 (2H, d, J = 11.6 Hz), 1.38 (3H, d, J = 6.1 Hz)., MS (m/z): 483 (M+H)⁺. |
| 120 | Example 12 | 3-{6-chloro-4-[(2R,4S)-4-hydroxy-2-methylpyrrolidin-1-yl]-1H-imidazo[4,5-c]pyridin-2-yl}-1,6-naphthyridin-2(1H)-one methanesulfonate | ¹H-NMR(DMSO-D₆) δ: 13.01 (1H, br s), 12.73 (1H, s), 9.30 (1H, br s), 9.03 (1H, s), 8.61 (1H, d, J = 6.1 Hz), 7.44 (1H, br s), 6.84 (1H, s), 4.86-4.73 (1H, m), 4.42 (1H, br s), 4.15-3.91 (2H, m), 3.54-3.47 (1H, m), 2.29 (3H, s), 2.34-2.24 (2H, m), 1.77-1.70 (1H, m), 1.46 (3H, d, J = 6.1 Hz)., MS (m/z): 397 (M+H)⁺. |
| 121 | Example 13 | 3-{6-chloro-4-[(2R,4R)-4-hydroxy-2-methylpyrrolidin-1-yl]-1H-imidazo[4,5-c]pyridin-2-yl}-5-methyl-1,6-naphthyridin-2(1H)-one methanesulfonate | ¹H-NMR (DMSO-D₆) δ: 13.25 (1H, br s), 12.78 (1H, s), 9.00 (1H, s), 8.54 (1H, d, J = 6.7 Hz), 7.45 (1H, br s), 6.85 (1H, s), 4.74 (1H, br s), 4.49-4.43 (1H, m), 4.04 (2H, br s), 2.96 (3H, s), 2.30 (3H, s), 2.13-2.05 (1H, m), 1.88-1.79 (1H, m), 1.33 (3H, d, J = 6.7 Hz)., MS (m/z): 411 (M+H)⁺. |
| 122 | Example 14 | 3-{6-chloro-4-[(2R,3S)-3-hydroxy-2-methylpyrrolidin-1-yl]-1H-imidazo[4,5-c]pyridin-2-yl}-5-ethyl-1,6-naphthyridin-2(1H)-one methanesulfonate | ¹H-NMR (DMSO-D₆) δ: 13.36 (1H, s), 12.78 (1H, s), 9.01 (1H, s), 8.58 (1H, d, J = 6.1 Hz), 7.52 (1H, d, J = 6.1 Hz), 6.81 (1H, s), 4.67 (1H, br s), 4.13-3.25 (4H, m), 2.49-2.44 (1H, m), 2.32 (3H, s), 2.19 (2H, q, J = 7.3 Hz), 1.95-1.84 (1H, m), 1.39 (3H, t, J = 7.3 Hz), 1.28 (3H, d, J = 5.5 Hz)., MS (m/z): 425 (M+H)⁺. |
| 123 | Example 15 | 3-{6-chloro-4-[(2R)-2-methylazetidin-1-yl]-1H-imidazo[4,5-c]pyridin-2-yl}-5-methyl-1,6-naphthyridin-2(1H)-one methanesulfonate | ¹H-NMR (DMSO-D₆) δ: 13.42 (1H, s), 12.82 (1H, s), 8.97 (1H, s), 8.58 (1H, d, J = 6.1 Hz), 7.53 (1H, d, J = 6.1 Hz), 6.86 (1H, s), 4.80-4.70 (1H, m), 4.37 (1H, br s), 4.22 (1H, d, J = 7.3 Hz), 2.99 (3H, s), 2.31 (3H, s), 2.34-2.30 (1H, m), 2.12-2.01 (1H, m), 1.64 (3H, d, J = 6.1 Hz)., MS (m/z): 381 (M+H)⁺. |
| 124 | Example 16 | 3-{6-chloro-4-[(3R)-3-methylmorpholin-4-yl]-1H-imidazo[4,5-c]pyridin-2-yl}-5-methyl-1,6-naphthyridin-2(1H)-one methanesulfonate | ¹H-NMR (DMSO-D₆) δ: 13.38 (1H, br s), 12.92 (1H, s), 8.96 (1H, s), 8.57 (1H, d, J = 6.3 Hz), 7.52 (1H, d, J = 6.3 Hz), 6.97 (1H, s), 5.44-5.30 (1H, m), 4.97-4.79 (1H, m), 4.04-3.95 (1H, m), 3.83-3.71 (2H, m), 3.63-3.53 (1H, m), 3.44-3.32 (1H, m), 2.32 (3H, s), 1.29 (3H, d, J = 6.7 Hz)., MS (m/z): 411 (M+H)⁺. |

### Example 125

### Production of 3-[6-chloro-4-(tetrahydro-2H-pyran-4-yl)-1H-imidazo[4,5-c]pyridin-2-yl]-5-methyl-1,6-naphthyridin-2(1H)-one methanesulfonate 3-[6-Chloro-4-(tetrahydro-2H-pyran-4-yl)-1H-imidazo[4,5-c]pyridin-2-yl]-5-methyl-1,6-naphthyridin-2(1H)-one methanesulfonate

To a suspension of the compound (273 mg) obtained in Example 17 in ethanol (7.0 mL) was added methanesulfonic acid (67.0 µL) at room temperature, and the mixture was stirred at 90°C for 4 hours. After cooling the reaction mixture to room temperature, the precipitated solid was collected by filtration. The obtained solid was washed with ethanol and then dried under reduced pressure to obtain the title compound (339 mg).
¹H-NMR (DMSO-D₆) δ: 13.47 (1H, s), 9.13 (1H, s), 8.60 (1H, d, J = 6.7 Hz), 7.58-7.50 (2H, m), 4.06-3.96 (2H, m), 3.83-3.72 (1H, m), 3.61-3.51 (2H, m), 3.06 (3H, s), 2.32 (3H, s), 2.08-1.93 (2H, m), 1.86-1.75 (2H, m)., MS (m/z): 396 (M+H)⁺.

The following compounds were synthesized by performing the same operation as in Example 125 (Table 17).

**Table 17**

| Example No. | Production Material | Name of Compound Synthesized | Spectral Data |
|---|---|---|---|
| 126 | Example 21 | 3-[6-chloro-4-(tetrahydro-2H-pyran-4-yl)-1H-imidazo[4,5-c]pyridin-2-yl]-1,6-naphthyridin-2(1H)-one methanesulfonate | ¹H-NMR (DMSO-D₆) δ: 13.21 (1H, br s), 9.41 (1H, s), 9.26 (1H, s), 8.65 (1H, d, J = 6.1 Hz), 7.55-7.47 (2H, m), 4.00 (2H, dd, J = 11.0, 4.0 Hz), 3.75-3.66 (1H, m), 3.52 (2H, t, J = 11.0 Hz), 2.27 (3H, s), 2.07-1.95 (2H, m), 1.83-1.75 (2H, m)., MS (m/z): 382 (M+H)⁺. |
| 127 | Example 22 | 3-[6-chloro-4-(tetrahydro-2H-pyran-4-yl)-1H-imidazo[4,5-c]pyridin-2-yl]-5-ethyl-1,6-naphthyridin-2(1H)-one methanesulfonate | ¹H-NMR (DMSO-D₆) δ: 13.31 (1H, br s), 9.13 (1H, s), 8.57 (1H, s), 7.54 (1H, s), 7.46 (1H, s), 3.98 (2H, dd, J = 10.4, 3.7 Hz), 3.81-3.70 (1H, m), 3.60-3.49 (2H, m), 3.39-3.28 (2H, m), 2.29-2.26 (3H, m), 2.04-1.90 (2H, m), 1.82-1.74 (2H, m), 1.36 (3H, t, J = 6.7 Hz)., MS (m/z): 410 (M+H)⁺. |
| 128 | Example 23 | 3-[6-chloro-4-(tetrahydro-2H-pyran-4-yl)-1H-imidazo[4,5,c]pyridin-2-yl]-5-(1-methylcyclopropyl)-1,6-naphthyridin-2(1H)-one methanesulfonate | ¹H-NMR (DMSO-D₆) δ: 13.30 (1H, br s), 9.43 (1H, s), 8.53 (1H, d, J = 6.7 Hz), 7.55 (1H, s), 7.51-7.45 (1H, m), 3.99 (2H, dd, J = 11.0, 3.7 Hz), 3.76-3.62 (1H, m), 3.54 (2H, t, J = 11.0 Hz), 2.28 (3H, s), 2.09-1.95 (2H, m), 1.90-1.79 (2H, m), 1.54 (3H, s), 1.20-1.13 (2H, m), 1.13-1.06 (2H, m)., MS (m/z): 436 (M+H)⁺. |
| 129 | Example 48 | 3-{6-chloro-4-[(2,2,2-trifluoroethyl)amino]-1H-imidazo[4,5-c]pyridin-2-yl}-5-(2-methylphenyl)-1,6-naphthyridin-2(1H)-one methanesulfonate | ¹H-NMR (DMSO-D₆) δ: 13.19-13.14 (1H, m), 8.64 (1H, d, J = 3.1 Hz), 8.45 (1H, s), 7.51-7.43 (5H, m), 6.95 (1H, s), 4.23 (2H, s), 2.27 (3H, s), 2.10 (3H, s)., MS (m/z): 485 (M+H)⁺. |
| 130 | Example 49 | 3-(6-chloro-4-methyl-1H-imidazo[4,5-c]pyridin-2-yl)-5-(2-fluoro-6-methoxyphenyl)-1,6-naphthyridin-2(1H)-one methanesulfonate | ¹H-NMR (DMSO-D₆) δ: 13.06 (1H, br s), 8.70 (1H, d, J = 5.5 Hz), 8.53 (1H, s), 7.67 (1H, q, J = 7.7 Hz), 7.51 (1H, s), 7.48-7.43 (1H, m), 7.19 (1H, d, J = 8.5 Hz), 7.10 (1H, t, J = 8.5 Hz), 3.75 (3H, s), 3.07-2.99 (2H, m), 2.31 (3H, s), 1.28 (3H, t, J = 7.6 Hz)., MS (m/z): 450 (M+H)⁺. |
| 131 | Example 50 | 3-(6-chloro-4-methyl-1H-imidazo[4,5-c]pyridin-2-yl)-5-(5-fluoro-2-methoxyphenyl)-1,6-naphthyridin-2(1H)-one methanesulfonate | ¹H-NMR (DMSO-D₆) δ: 13.01 (1H, br s), 8.68-8.59 (2H, m), 7.51-7.37 (3H, m), 7.37-7.30 (2H, m), 3.72 (3H, s), 2.63 (3H, s), 2.28-2.26 (3H, m)., MS (m/z): 436 (M+H)⁺. |
| 132 | Example 51 | 3-(6-chloro-4-methyl-1H-imidazo[4,5-c]pyridin-2-yl)-5-[2-(difluoromethoxy)phenyl]-1,6-naphthyridin-2(1H)-one methanesulfonate | ¹H-NMR (DMSO-D₆) δ: 13.09 (1H, s), 8.69 (1H, d, J = 6.1 Hz), 8.63 (1H, s), 7.73 (1H, t, J = 7.9 Hz), 7.64 (1H, d, J = 7.9 Hz), 7.56-7.45 (4H, m), 7.17 (1H, t, J = 73.2 Hz), 2.64 (3H, s), 2.31 (3H, s)., MS (m/z): 454 (M+H)⁺. |
| 133 | Example 52 | 3-(6-chloro-1H-imidazo[4,5-c]pyridin-2-yl)-5-{2-[(²H₃)methyloxy]phenyl}-1,6-naphthyridin-2(1H)-one methanesulfonate | ¹H-NMR (DMSO-D₆) δ: 13.29 (1H, s), 8.77 (1H, s), 8.71 (1H, d, J = 6.1 Hz), 8.66 (1H, s), 7.74-7.65 (2H, m), 7.57-7.47 (2H, m), 7.39-7.34 (1H, m), 7.29-7.22 (1H, m), 2.31 (3H, s)., MS (m/z): 407 (M+H)⁺. |
| 134 | Example 53 | 3-{6-chloro-4-[(3S)-tetrahydrofuran-3-ylamino]-1H-imidazo[4,5-c]pyridin-2-yl}-5-(2-[(²H₃)methyloxy]phenyl)-1,6-naphthyridin-2(1H)-one methanesulfonate | ¹H-NMR (DMSO-D₆) δ: 13.27 (1H, br s), 8.69 (1H, d, J = 5.5 Hz), 8.57 (1H, s), 7.70 (1H, t, J = 8.5 Hz), 7.59-7.49 (2H, m), 7.36 (1H, d, J = 8.5 Hz), 7.25 (1H, t, J = 8.5 Hz), 6.85 (1H, s), 4.61 (1H, br s), 3.99-3.69 (4H, m), 2.30 (3H, s), 2.26-2.15 (1H, m), 1.99-1.88 (1H, m)., MS (m/z): 492 (M+H)⁺. |
| 135 | Example 54 | 3-(6-chloro-1H-imidazo[4,5-c]pyridin-2-yl)-5-{2-fluoro-6-[(²H₃)methyloxy]phenyl}-1,6-naphthyridin-2(1H)-one methanesulfonate | ¹H-NMR (DMSO-D₆) δ: 13.07 (1H, s), 8.74 (1H, s), 8.67 (1H, d, J = 5.5 Hz), 8.53 (1H, s), 7.68-7.61 (2H, m), 7.44 (1H, d, J = 5.5 Hz), 7.15 (1H, d, J = 8.5 Hz), 7.08 (1H, t, J = 8.5 Hz), 2.28 (3H, s)., MS (m/z): 425 (M+H)⁺. |
| 136 | Example 55 | 3-(6-chloro-4-ethyl-1H-imidazo[4,5-c]pyridin-2-yl)-5-{2-fluoro-6-[(²H₃)methyloxy]phenyl}-1,6-naphthyridin-2(1H)-one methanesulfonate | ¹H-NMR (DMSO-D₆) δ: 13.10 (1H, s), 8.71 (1H, d, J = 6.1 Hz), 8.53 (1H, s), 7.72-7.64 (1H, m), 7.51 (1H, s), 7.48 (1H, d, J = 6.1 Hz), 7.19 (1H, d, J = 8.5 Hz), 7.11 (1H, t, J = 8.5 Hz), 3.09-2.98 (2H, m), 2.31 (2H, s), 1.28 (3H, t, J = 7.3 Hz)., MS (m/z): 453 (M+H)⁺. |
| 137 | Example 57 | 3-(6-chloro-4-ethyl-1H-imidazo[4,5-c]pyridin-2-yl)-5-(2-fluoro-6-hydroxyphenyl)-1,6-naphthyridin-2(1H)-one methanesulfonate | ¹H-NMR (DMSO-D₆) δ: 13.19 (1H, br s), 10.60 (1H, br s), 8.75-8.66 (1H, m), 8.58 (1H, s), 7.57-7.42 (3H, m), 7.02-6.86 (2H, m), 3.01 (2H, q, J = 7.3 Hz), 2.29 (3H, s), 1.25 (3H, t, J = 7.3 Hz)., MS (m/z): 436 (M+H)⁺. |

### Example 138

### Production of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one methanesulfonate 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one methanesulfonate

To the compound (30.32 mg) obtained in Example 58 were added 1.002 mol/L methanesulfonic acid solution (66 µL) and water (234 µL) at room temperature, and the mixture was stirred at 40°C for 24 hours. The precipitated solid was collected by filtration and dried at room temperature for 17 hours to obtain the title compound (35.13 mg) as crystals (values measured by elemental analysis: C: 48.34%, H: 5.18%, N: 12.95%, Cl: 5.50%, S: 4.97%). ¹H-NMR (DMSO-D₆) δ: 13.05 (1H, br s), 8.70 (1H, d, J = 5.9 Hz), 8.67 (1H, br s), 8.60 (1H, s), 7.52 (1H, s), 7.48 (1H, d, J = 5.9 Hz), 7.42 (1H, s), 4.00 (3H, s), 3.08 (2H, q, J = 7.4 Hz), 2.46-2.36 (1H, m), 2.32 (3H, s), 1.43-1.24 (7H, m), MS (m/z): 473 (M+H)⁺.

The results of measuring the obtained solid with a powder X-ray diffractometer (SmartLab, manufactured by Rigaku Corporation) are shown in Fig. 1. In Fig. 1 showing the diffraction pattern of powder X-ray diffraction (CuKα, λ = 1.54 angstroms, scan speed = 20°/min), the characteristic peaks of this crystal are shown in Table 18, and all peaks are shown in Table 19. In Table 19, the peaks marked with * represent the top 10 peaks with the highest relative intensity among all peaks.

**Table 18**

| Peak No. | 2θ | d Value | Relative Intensity | Peak No. | 2θ | d Value | Relative Intensity |
|---|---|---|---|---|---|---|---|
| 1 | 4.65 | 18.99 | 39 | 6 | 19.51 | 4.54 | 25 |
| 2 | 9.33 | 9.47 | 100 | 7 | 21.73 | 4.08 | 24 |
| 3 | 10.13 | 8.72 | 26 | 8 | 23.92 | 3.72 | 24 |
| 4 | 12.73 | 6.94 | 43 | 9 | 26.02 | 3.42 | 24 |
| 5 | 18.76 | 4.72 | 28 | 10 | 27.11 | 3.29 | 27 |

**Table 19**

| No. | Position (2θ) | Position (θ) | d Value | Intensity | Relative Intensity |
|---|---|---|---|---|---|
| 3* | 4.65 | 2.32 | 18.99 | 48582 | 39 |
| 1* | 9.33 | 4.66 | 9.47 | 123714 | 100 |
| 6* | 10.13 | 5.06 | 8.72 | 31700 | 26 |
| 33 | 10.70 | 5.35 | 8.26 | 6975 | 6 |
| 2* | 12.73 | 6.37 | 6.94 | 53196 | 43 |
| 11 | 13.29 | 6.64 | 6.65 | 29137 | 24 |
| 44 | 13.68 | 6.84 | 6.46 | 4856 | 4 |
| 19 | 14.01 | 7.00 | 6.31 | 18941 | 15 |
| 18 | 16.02 | 8.01 | 5.53 | 19970 | 16 |
| 12 | 16.30 | 8.15 | 5.43 | 27579 | 22 |
| 22 | 17.30 | 8.65 | 5.12 | 11923 | 10 |
| 23 | 18.37 | 9.19 | 4.82 | 11767 | 10 |
| 4* | 18.76 | 9.38 | 4.72 | 34684 | 28 |
| 13 | 19.01 | 9.50 | 4.66 | 24879 | 20 |
| 7* | 19.51 | 9.76 | 4.54 | 30476 | 25 |
| 14 | 19.64 | 9.82 | 4.51 | 24216 | 20 |
| 26 | 19.85 | 9.92 | 4.47 | 8776 | 7 |
| 24 | 20.08 | 10.04 | 4.42 | 9228 | 7 |
| 16 | 20.38 | 10.19 | 4.35 | 22529 | 18 |
| 9* | 21.73 | 10.87 | 4.08 | 29908 | 24 |
| 17 | 22.03 | 11.02 | 4.03 | 22204 | 18 |
| 36 | 23.00 | 11.50 | 3.86 | 6286 | 5 |
| 40 | 23.43 | 11.72 | 3.79 | 5961 | 5 |
| 10* | 23.92 | 11.96 | 3.72 | 29523 | 24 |
| 15 | 24.01 | 12.00 | 3.70 | 23067 | 19 |
| 34 | 24.20 | 12.10 | 3.67 | 6581 | 5 |
| 25 | 24.65 | 12.33 | 3.61 | 8920 | 7 |
| 35 | 25.26 | 12.63 | 3.52 | 6564 | 5 |
| 20 | 25.76 | 12.88 | 3.45 | 14663 | 12 |
| 8* | 26.02 | 13.01 | 3.42 | 29987 | 24 |
| 27 | 26.36 | 13.18 | 3.38 | 8335 | 7 |
| 41 | 26.67 | 13.34 | 3.34 | 5581 | 5 |
| 39 | 26.78 | 13.39 | 3.32 | 6140 | 5 |
| 5* | 27.11 | 13.55 | 3.29 | 33762 | 27 |
| 38 | 28.30 | 14.15 | 3.15 | 6155 | 5 |
| 42 | 29.05 | 14.52 | 3.07 | 5309 | 4 |
| 28 | 29.39 | 14.69 | 3.04 | 8297 | 7 |
| 45 | 29.73 | 14.87 | 3.00 | 4621 | 4 |
| 51 | 30.19 | 15.10 | 2.96 | 4068 | 3 |
| 32 | 30.35 | 15.18 | 2.94 | 7521 | 6 |
| 29 | 30.57 | 15.29 | 2.92 | 8079 | 7 |
| 31 | 30.81 | 15.41 | 2.90 | 7635 | 6 |
| 37 | 31.21 | 15.61 | 2.86 | 6179 | 5 |
| 21 | 32.09 | 16.05 | 2.79 | 13186 | 11 |
| 43 | 33.01 | 16.51 | 2.71 | 4970 | 4 |
| 53 | 33.51 | 16.76 | 2.67 | 3672 | 3 |
| 50 | 33.68 | 16.84 | 2.66 | 4083 | 3 |
| 48 | 35.08 | 17.54 | 2.55 | 4156 | 3 |
| 52 | 35.45 | 17.72 | 2.53 | 3846 | 3 |
| 30 | 37.19 | 18.60 | 2.41 | 7957 | 6 |
| 54 | 37.82 | 18.91 | 2.38 | 3466 | 3 |
| 47 | 38.07 | 19.03 | 2.36 | 4261 | 3 |
| 46 | 38.65 | 19.33 | 2.33 | 4323 | 3 |
| 49 | 38.94 | 19.47 | 2.31 | 4102 | 3 |

The following compounds were synthesized by performing the same operation as in Example 138 (Table 20).

**Table 20**

| Example No. | Production Material | Name of Compound Synthesized | Spectral Data |
|---|---|---|---|
| 139 | Example 73 | 3-(6-chloro-4-ethyl-1H-imidazo[4,5-c]pyridin-2-yl)-5-(6-ethyl-4-methoxypyridin-3-yl)-1,6-naphthyridin-2(1H)-one methanesulfonate | ¹H-NMR (DMSO-D₆) δ: 13.02 (1H, br s), 8.83 (1H, s), 8.69 (1H, d, J = 5.5 Hz), 8.57 (1H, s), 7.82 (1H, s), 7.49 (1H, s), 7.46 (1H, d, J = 5.5 Hz), 4.03 (3H, s), 3.06-3.03 (2H, m), 2.27 (3H, s), 1.40 (3H, t, J = 7.3 Hz), 1.28 (3H, t, J = 7.3 Hz)., MS (m/z): 461 (M+H)⁺. |
| 140 | Example 74 | 3-(6-chloro-4-methyl-1H-imidazo[4,5-c]pyridin-2-yl)-5-(6-cyclopropyl-4-methoxypyridin-3-yl)-1,6-naphthyridin-2(1H)-one methanesulfonate | ¹H-NMR (DMSO-D₆) δ: 13.01 (1H, s), 8.69 (1H, d, J = 6.1 Hz), 8.62 (1H, s), 8.58 (1H, br s), 7.51 (1H, s), 7.45 (1H, d, J = 6.1 Hz), 7.39 (1H, s), 3.97 (3H, s), 2.69 (3H, s), 2.41-2.31 (1H, m), 2.30 (3H, s), 1.28-1.25 (4H, m)., MS (m/z): 459 (M+H)⁺. |
| 141 | Example 76 | 3-(6-chloro-4-ethyl-1H-imidazo[4,5-c]pyridin-2-yl)-5-[6-(difluoromethyl)-4-methoxypyridin-3-yl]-1,6-naphthyridin-2(1H)-one methanesulfonate | ¹H-NMR (DMSO-D₆) δ: 13.01 (1H, s), 8.69 (1H, d, J = 6.1 Hz), 8.62 (1H, s), 8.57 (1H, s), 7.67 (1H, s), 7.49 (1H, s), 7.44 (1H, d, J = 6.1 Hz), 7.08 (1H, t, J = 54.9 Hz), 3.91 (3H, s), 3.03 (2H, q, J = 7.5 Hz), 2.29 (3H, s), 1.27 (3H, t, J = 7.5 Hz)., MS (m/z): 483 (M+H)⁺. |
| 142 | Example 77 | 3-{6-chloro-4-[(2,2,2-trifluoroethyl)amino]-1H-imidazo[4,5-c]pyridin-2-yl}-5-(2-methoxypyridin-3-yl)-1,6-naphthyridin-2(1H)-one methanesulfonate | ¹H-NMR (DMSO-D₆) δ: 12.98 (1H, s), 8.65 (1H, d, J = 5.5 Hz), 8.56 (1H, s), 8.46 (1H, dd, J = 5.2, 2.1 Hz), 7.92 (1H, dd, J = 7.3, 2.1 Hz), 7.42 (1H, d, J = 5.5 Hz), 7.28 (1H, dd, J = 7.3, 5.2 Hz), 6.98 (1H, s), 4.28 (2H, br s), 3.83 (3H, s), 2.30 (3H, s)., MS (m/z): 502 (M+H)⁺. |
| 143 | Example 78 | 3-{6-chloro-4-[(2,2-difluoroethyl)amino]-1H-imidazo[4,5-c]pyridin-2-yl}-5-(2-methoxypyridin-3-yl)-1,6-naphthyridin-2(1H)-one methanesulfonate | ¹H-NMR (DMSO-D₆) δ: 13.10 (1H, s), 8.67 (1H, d, J = 5.5 Hz), 8.55 (1H, s), 8.49 (1H, dd, J = 4.9, 1.8 Hz), 7.95 (1H, dd, J = 7.3, 1.8 Hz), 7.48 (1H, d, J = 5.5 Hz), 7.31 (1H, dd, J = 7.3, 4.9 Hz), 6.93 (1H, s), 6.18 (1H, tt, J = 56.5, 4.3 Hz), 4.83-4.39 (1H, m), 3.92-3.76 (5H, m), 2.31 (3H, s)., MS (m/z): 484 (M+H)⁺. |
| 144 | Example 79 | 3-(6-chloro-4-methyl-1H-imidazo[4,5-c]pyridin-2-yl)-5-(4,6-dimethoxypyridin-3-yl)-1,6-naphthyridin-2(1H)-one methanesulfonate | ¹H-NMR (DMSO-D₆) δ: 13.01 (1H, s), 8.69-8.64 (2H, m), 8.17 (1H, s), 7.51 (1H, s), 7.42 (1H, d, J = 6.1 Hz), 6.75 (1H, s), 3.97 (3H, s), 3.82 (3H, s), 2.68 (3H, s), 2.30 (3H, s)., MS (m/z): 449 (M+H)⁺. |

### Example 145

### Production of 3-(6-chloro-4-ethyl-1H-imidazo[4,5-c]pyridin-2-yl)-5-[4-methoxy-6-(trifluoromethyl)pyridin-3-yl]-1,6-naphthyridin-2(1H)-one methanesulfonate 3-(6-Chloro-4-ethyl-1H-imidazo[4,5-c]pyridin-2-yl)-5-[4-methoxy-6-(trifluoromethyl)pyridin-3-yl]-1,6-naphthyridin-2(1H)-one methanesulfonate

To a suspension of the compound (40.0 mg) obtained in Example 80 in acetonitrile (2.6 mL) was added methanesulfonic acid (5.1 µL) at room temperature, and the mixture was stirred at the same temperature for 2.5 hours. The precipitated solid was collected by filtration, washed with acetonitrile, and then dried under reduced pressure to obtain the title compound (46.3 mg).
¹H-NMR (DMSO-D₆) δ: 13.01 (1H, s), 8.72 (1H, s), 8.71 (1H, d, J = 6.1 Hz), 8.58 (1H, s), 7.89 (1H, s), 7.51 (1H, s), 7.46 (1H, d, J = 6.1 Hz), 3.97 (3H, s), 3.05 (2H, q, J = 7.5 Hz), 2.31 (3H, s), 1.29 (3H, t, J = 7.5 Hz)., MS (m/z): 501 (M+H)⁺.

### Example 146

### Production of 3-(6-chloro-4-ethyl-1H-imidazo[4,5-c]pyridin-2-yl)-5-[6-(difluoromethoxy)-4-methoxypyridin-3-yl]-1,6-naphthyridin-2(1H)-one methanesulfonate 3-(6-Chloro-4-ethyl-1H-imidazo[4,5-c]pyridin-2-yl)-5-[6-(difluoromethoxy)-4-methoxypyridin-3-yl]-1,6-naphthyridin-2(1H)-one methanesulfonate

The title compound was obtained by using the compound obtained in Example 81 as a production material and performing the same operation as in Example 145.
¹H-NMR (DMSO-D₆) δ: 13.01 (1H, s), 8.72 (1H, s), 8.71 (1H, d, J = 6.1 Hz), 8.58 (1H, s), 7.89 (1H, s), 7.51 (1H, s), 7.46 (1H, d, J = 6.1 Hz), 3.97 (3H, s), 3.05 (2H, q, J = 7.5 Hz), 2.31 (3H, s), 1.29 (3H, t, J = 7.5 Hz)., MS (m/z): 499 (M+H)⁺.

### Example 147

### Production of 3-(6-chloro-4-ethyl-1H-imidazo[4,5-c]pyridin-2-yl)-5-[6-(1,1-difluoroethyl)-4-methoxypyridin-3-yl]-1,6-naphthyridin-2(1H)-one methanesulfonate 3-(6-Chloro-4-ethyl-1H-imidazo[4,5-c]pyridin-2-yl)-5-[6-(1,1-difluoroethyl)-4-methoxypyridin-3-yl]-1,6-naphthyridin-2(1H)-one methanesulfonate

The title compound was obtained by using the compound obtained in Example 82 as a production material and performing the same operation as in Example 145.
¹H-NMR (DMSO-D₆) δ: 13.04 (1H, s), 8.71 (1H, d, J = 5.5 Hz), 8.63 (1H, s), 8.59 (1H, s), 7.63 (1H, s), 7.51 (1H, s), 7.47 (1H, d, J = 5.5 Hz), 3.94 (3H, s), 3.05 (2H, q, J = 7.5 Hz), 2.32 (3H, s), 2.11 (3H, t, J = 19.2 Hz), 1.29 (3H, t, J = 7.5 Hz)., MS (m/z): 497 (M+H)⁺.

### Example 148

### Production of 3-(6-chloro-4-ethyl-1H-imidazo[4,5-c]pyridin-2-yl)-5-[1-(difluoromethyl)-3,5-dimethyl-1H-pyrazol-4-yl]-1,6-naphthyridin-2(1H)-one methanesulfonate 3-(6-Chloro-4-ethyl-1H-imidazo[4,5-c]pyridin-2-yl)-5-[1-(difluoromethyl)-3,5-dimethyl-1H-pyrazol-4-yl]-1,6-naphthyridin-2(1H)-one methanesulfonate

To a suspension of the compound (35 mg) obtained in Example 83 in ethanol (2 mL) was added methanesulfonic acid (5.0 µL) at room temperature, and the mixture was stirred at 90°C for 3.5 hours and then at room temperature for 1.5 hours. The reaction mixture was concentrated under reduced pressure. Ethyl acetate was added to the residue, and the precipitated solid was collected by filtration and dried under reduced pressure to obtain the title compound (32 mg).
¹H-NMR (DMSO-D₆) δ: 13.13 (1H, s), 8.69-8.72 (2H, m), 7.95 (1H, t, J = 57.7 Hz), 7.52 (1H, s), 7.46 (1H, d, J = 6.1 Hz), 2.38 (3H, s), 2.31 (3H, s), 2.19 (3H, s)., MS (m/z): 470 (M+H)⁺.

The following compounds were synthesized by performing the same operation as in Example 148 (Table 21).

**Table 21**

| Example No. | Production Material | Name of Compound Synthesized | Spectral Data |
|---|---|---|---|
| 149 | Example 96 | 3-{6-chloro-4-[(2R)-2-methylazetidin-1-yl]-1H-imidazo[4,5-c]pyridin-2-yl}-5-[1-(difluoromethyl)-3,5-dimethyl-1H-pyrazol-4-yl]-1,6-naphthyridin-2(1H)-one methanesulfonate | ¹H-NMR (DMSO-D₆) δ: 13.02 (1H, s), 12.74 (1H, s), 8.67 (1H, d, J = 6.1 Hz), 8.59 (1H, s), 7.93 (1H, t, J = 58.0 Hz), 7.43 (1H, d, J = 6.1 Hz), 6.86 (1H, s), 4.74-4.64 (1H, m), 4.24-4.05 (2H, m), 2.35 (3H, s), 2.37-2.32 (1H, m), 2.31 (3H, s), 2.18 (3H, s), 2.07-1.97 (1H, m), 1.57 (3H, d, J = 6.1 Hz)., MS (m/z): 511 (M+H)⁺. |
| 150 | Example 97 | 3-(6-chloro-4-ethyl-1H-imidazo[4,5-c]pyridin-2-yl)-5-[1-(difluoromethyl)-1H-pyrazol-3-yl]-1,6-naphthyridin-2(1H)-one methanesulfonate | ¹H-NMR (DMSO-D₆) δ: 12.97 (1H, s), 10.33 (1H, s), 8.67 (1H, d, J = 6.1 Hz), 8.49 (1H, d, J = 2.4 Hz), 8.01 (1H, t, J = 58.6 Hz), 7.53 (1H, s), 7.42 (1H, d, J = 6.1 Hz), 7.19 (1H, d, J = 2.4 Hz), 3.14 (2H, q, J = 7.5 Hz), 2.35 (3H, s), 1.37 (3H, t, J = 7.5 Hz)., MS (m/z): 442 (M+H)⁺. |
| 151 | Example 98 | 3-(6-chloro-4-ethyl-1H-imidazo[4,5-c]pyridin-2-yl)-5-(1,5-dimethyl-1H-pyrazol-3-yl)-1,6-naphthyridin-2(1H)-one methanesulfonate | ¹H-NMR (DMSO-D₆) δ: 13.34 (1H, s), 8.99 (1H, s), 8.66 (1H, d, J = 6.1 Hz), 7.90 (1H, s), 7.52 (1H, s), 7.49 (1H, d, J = 6.1 Hz), 3.94 (3H, s), 3.11 (2H, q, J = 7.3 Hz), 2.42 (3H, s), 2.32 (3H, s), 1.33 (3H, t, J = 7.3 Hz)., MS (m/z): 420 (M+H)⁺. |
| 152 | Example 99 | 3-(6-chloro-4-{[(2R)-1,1,1-trifluoropropan-2-yl]amino}-1H-imidazo[4,5-c]pyridin-2-yl)-5-(1,5-dimethyl-1H-pyrazol-4-yl)-1,6-naphthyridin-2(1H)-one methanesulfonate | ¹H-NMR (DMSO-D₆) δ: 13.20 (1H, br s), 8.97 (1H, s), 8.63 (1H, d, J = 6.1 Hz), 7.98 (1H, br s), 7.85 (1H, s), 7.43 (1H, br s), 6.99 (1H, s), 5.22 (1H, br s), 3.92 (3H, s), 2.40 (3H, s), 2.30 (3H, s), 1.42 (3H, d, J = 6.7 Hz)., MS (m/z): 503 (M+H)⁺. |
| 153 | Example 100 | 3-[6-chloro-4-(tetrahydro-2H-pyran-4-yl)-1H-imidazo[4,5-c]pyridin-2-yl]-5-[3-cyclopropyl-1-(difluoromethyl)-5-methyl-1H-pyrazol-4-yl]-1,6-naphthyridin-2(1H)-one methanesulfonate | ¹H-NMR (DMSO-D₆) δ: 13.00 (1H, s), 8.95 (1H, s), 8.70 (1H, d, J = 6.1 Hz), 7.90 (1H, t, J = 57.7 Hz), 7.53 (1H, s), 7.41 (1H, d, J = 6.1 Hz), 4.00 (2H, dd, J = 10.7, 4.0 Hz), 3.71-3.56 (1H, m), 3.54-3.47 (2H, m), 2.39 (3H, s), 2.30 (3H, s), 1.95 (2H, br s), 1.89-1.78 (2H, m), 1.73-1.70 (1H, m), 0.99 (1H, br s), 0.86-0.83 (3H, m)., MS (m/z): 552 (M+H)⁺. |

### Example 154

### Production of 3-(6-chloro-4-ethyl-1H-imidazo[4,5-c]pyridin-2-yl)-5-(6-cyclopropyl-4-methoxypyridin-3-yl)-3,4-dihydropyrido[4,3-d]pyrimidin-2(1H)-one methanesulfonate 3-(6-Chloro-4-ethyl-1H-imidazo[4,5-c]pyridin-2-yl)-5-(6-cyclopropyl-4-methoxypyridin-3-yl)-3,4-dihydropyrido[4,3-d]pyrimidin-2(1H)-one methanesulfonate

To a suspension of the compound (23.5 mg) obtained in Example 101 in ethanol (3.0 mL) was added a 10% solution of methanesulfonic acid in ethanol (47.6 µL) at room temperature, and the mixture was stirred at the same temperature for 6 hours. The precipitated solid was collected by filtration, washed with ethanol, and then dried under reduced pressure to obtain the title compound (14.2 mg).
¹H-NMR (DMSO-D₆) δ: 12.58 (1H, br s), 11.13 (1H, br s), 8.51 (1H, d, J = 5.9 Hz), 8.39 (1H, s), 7.38-7.35 (2H, m), 7.09 (1H, d, J = 5.9 Hz), 5.07 (2H, br s), 3.91 (3H, s), 3.03-2.85 (2H, m), 2.35-2.24 (3H, m), 1.25 (3H, t, J = 7.4 Hz), 1.21-1.10 (4H, m)., MS (m/z): 476 (M+H)⁺.

### Example 155

### Production of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one 1,2-ethanedisulfonate 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one 1,2-ethanedisulfonate

To the compound (30.30 mg) obtained in Example 58 were added 1.000 mol/L 1,2-ethanedisulfonic acid solution (33 µL) and water (267 µL) at room temperature, and the mixture was stirred at 40°C for 24 hours. The precipitated solid was collected by filtration and dried at room temperature for 17 hours to obtain the title compound (29.91 mg) as crystals (values measured by elemental analysis: C: 51.53%, H: 4.80%, N: 13.79%, Cl: 5.84%, S: 5.26%). ¹H-NMR (DMSO-D₆) δ: 13.05 (1H, br s), 8.70 (1H, d, J = 5.9 Hz), 8.66 (1H, br s), 8.60 (1H, s), 7.51 (1H, s), 7.48 (1H, d, J = 5.9 Hz), 7.42 (1H, s), 4.00 (3H, s), 3.08 (2H, q, J = 7.4 Hz), 2.65-2.60 (2H, m), 2.44-2.35 (1H, m), 1.40-1.25 (7H, m), MS (m/z): 473 (M+H)⁺.

The results of measuring the obtained solid with a powder X-ray diffractometer (SmartLab, manufactured by Rigaku Corporation) are shown in Fig. 2. In Fig. 2 showing the diffraction pattern of powder X-ray diffraction (CuKα, λ = 1.54 angstroms, scan speed = 20°/min), the characteristic peaks of this crystal are shown in Table 22, and all peaks are shown in Table 23. In Table 23, the peaks marked with * represent the top 10 peaks with the highest relative intensity among all peaks.

**Table 22**

| Peak No. | 2θ | d Value | Relative Intensity | Peak No. | 2θ | d Value | Relative Intensity |
|---|---|---|---|---|---|---|---|
| 1 | 6.33 | 13.95 | 74 | 5 | 18.36 | 4.83 | 41 |
| 2 | 8.40 | 10.51 | 17 | 6 | 20.73 | 4.28 | 25 |
| 3 | 12.68 | 6.97 | 100 | 7 | 22.39 | 3.97 | 18 |
| 4 | 16.87 | 5.25 | 30 | 8 | 24.33 | 3.65 | 17 |
| | | | | 9 | 25.55 | 3.48 | 10 |

**Table 23**

| No. | Position (2θ) | Position (θ) | d Value | Intensity | Relative Intensity |
|---|---|---|---|---|---|
| 2* | 6.33 | 3.16 | 13.95 | 85738 | 74 |
| 18 | 7.70 | 3.85 | 11.47 | 17783 | 15 |
| 15 | 8.40 | 4.20 | 10.51 | 19734 | 17 |
| 41 | 11.93 | 5.97 | 7.41 | 6328 | 5 |
| 1* | 12.68 | 6.34 | 6.97 | 116635 | 100 |
| 5* | 13.21 | 6.60 | 6.70 | 45443 | 39 |
| 11 | 13.33 | 6.66 | 6.63 | 21155 | 18 |
| 35 | 13.47 | 6.74 | 6.57 | 8550 | 7 |
| 19 | 14.87 | 7.43 | 5.95 | 16811 | 14 |
| 50 | 15.07 | 7.54 | 5.87 | 3808 | 3 |
| 20 | 15.77 | 7.88 | 5.61 | 16606 | 14 |
| 26 | 15.94 | 7.97 | 5.55 | 11506 | 10 |
| 7* | 16.87 | 8.43 | 5.25 | 34919 | 30 |
| 22 | 17.65 | 8.83 | 5.02 | 14167 | 12 |
| 4* | 18.36 | 9.18 | 4.83 | 47331 | 41 |
| 28 | 18.69 | 9.35 | 4.74 | 11238 | 10 |
| 21 | 19.05 | 9.52 | 4.65 | 14687 | 13 |
| 45 | 19.51 | 9.75 | 4.54 | 5614 | 5 |
| 29 | 20.31 | 10.15 | 4.37 | 10881 | 9 |
| 8* | 20.73 | 10.37 | 4.28 | 28722 | 25 |
| 30 | 21.87 | 10.94 | 4.06 | 10210 | 9 |
| 23 | 22.08 | 11.04 | 4.02 | 13856 | 12 |
| 17 | 22.22 | 11.11 | 4.00 | 18794 | 16 |
| 12 | 22.39 | 11.20 | 3.97 | 21007 | 18 |
| 33 | 22.62 | 11.31 | 3.93 | 9355 | 8 |
| 36 | 22.76 | 11.38 | 3.90 | 8126 | 7 |
| 31 | 23.86 | 11.93 | 3.72 | 9939 | 9 |
| 40 | 24.10 | 12.05 | 3.69 | 6469 | 6 |
| 16 | 24.33 | 12.17 | 3.65 | 19507 | 17 |
| 27 | 24.91 | 12.45 | 3.57 | 11350 | 10 |
| 32 | 25.09 | 12.55 | 3.54 | 9661 | 8 |
| 25 | 25.55 | 12.77 | 3.48 | 11578 | 10 |
| 10* | 25.93 | 12.96 | 3.43 | 26567 | 23 |
| 9* | 26.09 | 13.04 | 3.41 | 27894 | 24 |
| 6* | 26.23 | 13.11 | 3.39 | 40287 | 35 |
| 14 | 26.62 | 13.31 | 3.34 | 19925 | 17 |
| 3* | 26.84 | 13.42 | 3.32 | 83829 | 72 |
| 13 | 27.14 | 13.57 | 3.28 | 20735 | 18 |
| 43 | 27.89 | 13.95 | 3.19 | 5935 | 5 |
| 46 | 28.42 | 14.21 | 3.14 | 5554 | 5 |
| 24 | 28.95 | 14.48 | 3.08 | 13457 | 12 |
| 39 | 29.20 | 14.60 | 3.05 | 6723 | 6 |
| 37 | 29.93 | 14.97 | 2.98 | 8010 | 7 |
| 34 | 31.25 | 15.63 | 2.86 | 8841 | 8 |
| 38 | 32.07 | 16.04 | 2.79 | 7302 | 6 |
| 42 | 33.85 | 16.92 | 2.65 | 5952 | 5 |
| 48 | 34.27 | 17.14 | 2.61 | 4854 | 4 |
| 53 | 35.26 | 17.63 | 2.54 | 3304 | 3 |
| 44 | 35.95 | 17.98 | 2.50 | 5747 | 5 |
| 54 | 36.62 | 18.31 | 2.45 | 3248 | 3 |
| 49 | 37.12 | 18.56 | 2.42 | 3835 | 3 |
| 52 | 38.26 | 19.13 | 2.35 | 3602 | 3 |
| 51 | 38.74 | 19.37 | 2.32 | 3660 | 3 |
| 47 | 39.40 | 19.70 | 2.28 | 5026 | 4 |

### Example 156

### Production of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one benzenesulfonate 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one benzenesulfonate

To the compound (30.82 mg) obtained in Example 58 were added 1.000 mol/L benzenesulfonic acid solution (67 µL) and water (233 µL) at room temperature, and the mixture was stirred at 40°C for 24 hours. The precipitated solid was collected by filtration and dried at room temperature for 17 hours to obtain the title compound (35.92 mg) as crystals (values measured by elemental analysis: C: 55.17%, H: 4.75%, N: 12.24%, Cl: 5.24%, S: 4.77%). ¹H-NMR (DMSO-D₆) δ: 13.05 (1H, br s), 8.70 (1H, d, J = 5.9 Hz), 8.67 (1H, br s), 8.60 (1H, s), 7.61-7.57 (2H, m), 7.52 (1H, s), 7.48 (1H, d, J = 5.9 Hz), 7.42 (1H, s), 7.35-7.27 (3H, m), 4.00 (3H, s), 3.08 (2H, q, J = 7.4 Hz), 2.45-2.37 (1H, m), 1.45-1.26 (7H, m)., MS (m/z): 473 (M+H)⁺.

The results of measuring the obtained solid with a powder X-ray diffractometer (SmartLab, manufactured by Rigaku Corporation) are shown in Fig. 3. In Fig. 3 showing the diffraction pattern of powder X-ray diffraction (CuKα, λ = 1.54 angstroms, scan speed = 20°/min), the characteristic peaks of this crystal are shown in Table 24, and all peaks are shown in Table 25. In Table 25, the peaks marked with * represent the top 10 peaks with the highest relative intensity among all peaks.

**Table 24**

| Peak No. | 20 | d Value | Relative Intensity | Peak No. | 20 | d Value | Relative Intensity |
|---|---|---|---|---|---|---|---|
| 1 | 5.75 | 15.35 | 48 | 6 | 16.04 | 5.52 | 21 |
| 2 | 7.08 | 12.47 | 75 | 7 | 17.18 | 5.16 | 27 |
| 3 | 11.55 | 7.65 | 100 | 8 | 22.91 | 3.88 | 30 |
| 4 | 12.76 | 6.93 | 23 | 9 | 25.41 | 3.50 | 47 |
| 5 | 13.34 | 6.63 | 38 | 10 | 25.73 | 3.46 | 24 |

**Table 25**

| No. | Position (2θ) | Position (θ) | d Value | Intensity | Relative Intensity |
|---|---|---|---|---|---|
| 3* | 5.75 | 2.87 | 15.35 | 51915 | 48 |
| 2* | 7.08 | 3.54 | 12.47 | 82037 | 75 |
| 19 | 8.51 | 4.25 | 10.38 | 12064 | 11 |
| 15 | 9.71 | 4.86 | 9.10 | 16664 | 15 |
| 1* | 11.55 | 5.77 | 7.65 | 109159 | 100 |
| 34 | 12.11 | 6.05 | 7.30 | 6928 | 6 |
| 20 | 12.36 | 6.18 | 7.15 | 11487 | 11 |
| 9* | 12.76 | 6.38 | 6.93 | 25283 | 23 |
| 5* | 13.34 | 6.67 | 6.63 | 41882 | 38 |
| 25 | 14.04 | 7.02 | 6.30 | 9453 | 9 |
| 51 | 14.16 | 7.08 | 6.25 | 4299 | 4 |
| 31 | 14.35 | 7.18 | 6.16 | 7704 | 7 |
| 38 | 14.61 | 7.30 | 6.06 | 6309 | 6 |
| 36 | 15.64 | 7.82 | 5.66 | 6360 | 6 |
| 10* | 16.04 | 8.02 | 5.52 | 23269 | 21 |
| 43 | 16.55 | 8.28 | 5.35 | 5392 | 5 |
| 7* | 17.18 | 8.59 | 5.16 | 29359 | 27 |
| 27 | 17.64 | 8.82 | 5.02 | 9069 | 8 |
| 23 | 17.93 | 8.97 | 4.94 | 10897 | 10 |
| 49 | 18.46 | 9.23 | 4.80 | 5012 | 5 |
| 28 | 18.75 | 9.37 | 4.73 | 8953 | 8 |
| 11 | 19.84 | 9.92 | 4.47 | 22797 | 21 |
| 29 | 20.04 | 10.02 | 4.42 | 8513 | 8 |
| 40 | 20.30 | 10.15 | 4.37 | 6136 | 6 |
| 17 | 20.73 | 10.36 | 4.28 | 15882 | 15 |
| 14 | 21.03 | 10.51 | 4.22 | 16811 | 15 |
| 18 | 21.41 | 10.71 | 4.14 | 14651 | 13 |
| 41 | 21.66 | 10.83 | 4.10 | 5892 | 5 |
| 44 | 22.04 | 11.02 | 4.03 | 5333 | 5 |
| 22 | 22.27 | 11.13 | 3.99 | 11140 | 10 |
| 33 | 22.39 | 11.20 | 3.97 | 7135 | 7 |
| 6* | 22.91 | 11.46 | 3.88 | 32710 | 30 |
| 24 | 23.26 | 11.63 | 3.82 | 9978 | 9 |
| 30 | 23.97 | 11.98 | 3.71 | 8242 | 8 |
| 37 | 24.40 | 12.20 | 3.64 | 6320 | 6 |
| 26 | 24.67 | 12.34 | 3.60 | 9308 | 9 |
| 16 | 25.05 | 12.53 | 3.55 | 16659 | 15 |
| 4* | 25.41 | 12.71 | 3.50 | 51004 | 47 |
| 8* | 25.73 | 12.87 | 3.46 | 26535 | 24 |
| 13 | 25.99 | 13.00 | 3.42 | 19846 | 18 |
| 12 | 27.10 | 13.55 | 3.29 | 21486 | 20 |
| 32 | 27.60 | 13.80 | 3.23 | 7561 | 7 |
| 35 | 28.38 | 14.19 | 3.14 | 6391 | 6 |
| 21 | 28.68 | 14.34 | 3.11 | 11381 | 10 |
| 39 | 29.84 | 14.92 | 2.99 | 6244 | 6 |
| 42 | 30.37 | 15.19 | 2.94 | 5600 | 5 |
| 46 | 31.60 | 15.80 | 2.83 | 5197 | 5 |
| 48 | 32.17 | 16.08 | 2.78 | 5054 | 5 |
| 52 | 34.23 | 17.12 | 2.62 | 3708 | 3 |
| 47 | 35.08 | 17.54 | 2.55 | 5088 | 5 |
| 50 | 36.37 | 18.19 | 2.47 | 4689 | 4 |
| 45 | 38.21 | 19.10 | 2.35 | 5281 | 5 |

### Example 157

### Production of 3-(6-chloro-4-ethyl-1H-imidazo[4,5-c]pyridin-2-yl)-5-(6-cyclopropyl-4-methoxypyridin-3-yl)-1,6-naphthyridin-2(1H)-one adipate 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one adipate

To the compound (29.52 mg) obtained in Example 58 were added 1.000 mol/L benzenesulfonic acid solution (67 µL) and water (233 µL) at room temperature, and the mixture was stirred at 40°C for 24 hours. The precipitated solid was collected by filtration and dried at room temperature for 17 hours to obtain the title compound (29.96 mg) as crystals (values measured by elemental analysis: C: 60.89%, H: 4.91%, N: 14.98%, Cl: 6.39%, S: 0.00%). ¹H-NMR (DMSO-D₆) δ: 12.97 (1H, s), 12.86 (0.6H, br s), 12.04 (0.4H, br s), 8.67-8.60 (2H, m), 8.32 (1H, s), 7.50 (1H, s), 7.37 (1H, d, J = 5.9 Hz), 7.33 (1H, s), 4.84 (3H, s), 3.06 (2H, q, J = 7.4 Hz), 2.31-2.15 (3H, m), 1.54-1.45 (2H, m), 1.31 (3H, t, J = 7.4 Hz), 1.10-0.98 (4H, m)., MS (m/z): 473 (M+H)⁺.

The results of measuring the obtained solid with a powder X-ray diffractometer (SmartLab, manufactured by Rigaku Corporation) are shown in Fig. 4. In Fig. 4 showing the diffraction pattern of powder X-ray diffraction (CuKα, λ = 1.54 angstroms, scan speed = 20°/min), the characteristic peaks of this crystal are shown in Table 26, and all peaks are shown in Table 27. In Table 27, the peaks marked with * represent the top 10 peaks with the highest relative intensity among all peaks.

**Table 26**

| Peak No. | 20 | d Value | Relative Intensity | Peak No. | 20 | d Value | Relative Intensity |
|---|---|---|---|---|---|---|---|
| 1 | 6.76 | 13.07 | 100 | 6 | 16.75 | 5.29 | 9 |
| 2 | 8.11 | 10.89 | 15 | 7 | 18.88 | 4.70 | 6 |
| 3 | 12.23 | 7.23 | 11 | 8 | 19.71 | 4.50 | 8 |
| 4 | 13.54 | 6.53 | 30 | 9 | 21.35 | 4.16 | 12 |
| 5 | 15.88 | 5.57 | 7 | 10 | 25.76 | 3.45 | 15 |

**Table 27**

| No. | Position (2θ) | Position (θ) | d Value | Intensity | Relative Intensity |
|---|---|---|---|---|---|
| 1* | 6.76 | 3.38 | 13.07 | 423970 | 100 |
| 4* | 8.11 | 4.06 | 10.89 | 63061 | 15 |
| 17 | 8.60 | 4.30 | 10.27 | 17715 | 4 |
| 13 | 10.21 | 5.11 | 8.65 | 23117 | 5 |
| 6* | 12.23 | 6.11 | 7.23 | 48650 | 11 |
| 20 | 12.96 | 6.48 | 6.83 | 12350 | 3 |
| 28 | 13.20 | 6.60 | 6.70 | 8708 | 2 |
| 2* | 13.54 | 6.77 | 6.53 | 126783 | 30 |
| 42 | 14.01 | 7.01 | 6.31 | 5115 | 1 |
| 49 | 15.15 | 7.58 | 5.84 | 4239 | 1 |
| 18 | 15.34 | 7.67 | 5.77 | 14192 | 3 |
| 9* | 15.88 | 7.94 | 5.57 | 29925 | 7 |
| 7* | 16.75 | 8.37 | 5.29 | 36038 | 9 |
| 21 | 17.27 | 8.63 | 5.13 | 12055 | 3 |
| 31 | 17.54 | 8.77 | 5.05 | 7187 | 2 |
| 14 | 18.06 | 9.03 | 4.90 | 20000 | 5 |
| 29 | 18.55 | 9.28 | 4.78 | 7927 | 2 |
| 10* | 18.88 | 9.44 | 4.70 | 24180 | 6 |
| 19 | 19.18 | 9.59 | 4.62 | 13153 | 3 |
| 8* | 19.71 | 9.85 | 4.50 | 34270 | 8 |
| 26 | 20.54 | 10.27 | 4.32 | 9683 | 2 |
| 47 | 20.77 | 10.38 | 4.27 | 4567 | 1 |
| 5* | 21.35 | 10.67 | 4.16 | 51906 | 12 |
| 15 | 21.79 | 10.90 | 4.07 | 19445 | 5 |
| 41 | 22.48 | 11.24 | 3.95 | 5352 | 1 |
| 11 | 23.01 | 11.50 | 3.86 | 23977 | 6 |
| 30 | 23.31 | 11.66 | 3.81 | 7337 | 2 |
| 39 | 23.69 | 11.84 | 3.75 | 5740 | 1 |
| 12 | 24.47 | 12.23 | 3.63 | 23487 | 6 |
| 22 | 25.40 | 12.70 | 3.50 | 11447 | 3 |
| 3* | 25.76 | 12.88 | 3.45 | 63608 | 15 |
| 25 | 26.13 | 13.06 | 3.41 | 9926 | 2 |
| 27 | 26.52 | 13.26 | 3.36 | 9140 | 2 |
| 16 | 27.41 | 13.70 | 3.25 | 19027 | 4 |
| 24 | 27.72 | 13.86 | 3.21 | 10409 | 2 |
| 45 | 28.62 | 14.31 | 3.12 | 4810 | 1 |
| 36 | 29.07 | 14.53 | 3.07 | 5992 | 1 |
| 23 | 29.92 | 14.96 | 2.98 | 11420 | 3 |
| 34 | 30.90 | 15.45 | 2.89 | 6116 | 1 |
| 32 | 31.17 | 15.59 | 2.87 | 6399 | 2 |
| 48 | 31.65 | 15.83 | 2.82 | 4395 | 1 |
| 43 | 32.08 | 16.04 | 2.79 | 5089 | 1 |
| 35 | 32.46 | 16.23 | 2.76 | 6018 | 1 |
| 53 | 32.83 | 16.41 | 2.72 | 3148 | 1 |
| 38 | 33.51 | 16.75 | 2.67 | 5791 | 1 |
| 52 | 34.12 | 17.06 | 2.62 | 3236 | 1 |
| 51 | 34.73 | 17.37 | 2.58 | 3256 | 1 |
| 40 | 35.32 | 17.66 | 2.54 | 5495 | 1 |
| 50 | 35.55 | 17.78 | 2.52 | 3853 | 1 |
| 37 | 37.33 | 18.67 | 2.41 | 5807 | 1 |
| 44 | 37.77 | 18.88 | 2.38 | 5034 | 1 |
| 33 | 38.13 | 19.06 | 2.36 | 6172 | 1 |
| 46 | 39.33 | 19.66 | 2.29 | 4704 | 1 |

### (Test Examples)

The pharmacological activity of the compounds of the present invention was confirmed by the following tests.

### <Test Example 1: Measurement of SMG1 kinase activity>

The reaction was performed by the ADP-Glo Kinase Assay (Promega, catalog number V9102) using a 384-well plate under the conditions of an assay buffer (100 mM HEPES (pH 7.5), 50 mM NaCl, 10 mM MnCl₂, 0.01% BSA, 0.1% Tween 20, 5 mM TCEP). After incubating a test substance (DMSO solution; final DMSO concentration: 1%) and purified human SMG1 enzyme (Genes Dev. 2011 Jan 15; 25(2): 153-164, produced by Daiichi Sankyo RD Novare Co., Ltd.; final concentration: 5 nM) at room temperature for 15 minutes, a substrate, Upfl peptide (Biotin-(PEG8) QIDVALSQDSTYQG, produced by Eurofins genomics; final concentration: 8 µg/mL), and ATP (Promega: 4 µM) were added, and the mixture was reacted at room temperature for 60 minutes (see Non-Patent Literature R. Melero, et al., Structure Volume 22, Issue 8, 5 August 2014, Pages 1105-1119 for the Upfl peptide). ADP-Glo Reagent was added, and the mixture was reacted at room temperature for 40 minutes. Kinase Detection Reagent was added, and the mixture was reacted for another 30 minutes. The luminescence intensity was measured using EnVision (PerkinElmer).

The activity value (%) of the test substance-added well was calculated from the following formula, where the average luminescence intensity of the DMSO-added well was 0% and the average luminescence intensity of the SMG1 enzyme-free well was -100%. Activity value (%) of test substance-added well = [(luminescence intensity of each well - average luminescence intensity of SMG1-free well) / (average luminescence intensity of DMSO-added well - average luminescence intensity of SMG1-free well)] × 100 - 100

The qAC50 (compound concentration showing 50% inhibition, synonymous with IC50) of the test substance was calculated using the activity value (%) at each concentration by Smart fit of Screener (Genedata). Smart fit is a general 4-parameter logistic regression model. The IC50 values are shown in Table 28.

### <Test Example 2: Measurement of NMD inhibitory activity in cells>

NMD inhibitory activity was measured by treating EMT6 murine breast cancer cell line (ATCC) with a test substance and quantifying the increase in the transcript of *Snhgl,* an NMD target gene (see F. Usui, et al., Scientific Reports volume 9, Article number: 1279 (2019) for the NMD target gene). That is, EMT6 cells were suspended in Waymouth's medium (Gibco, 15% FBS) and seeded in a 96-well plate at 2 × 10⁴ cells/well. The cells were cultured for 1 day. The next day, serially diluted test substance (DMSO solution, final DMSO concentration: 2%) was added. After culturing for 6 hours, the supernatant was removed, and RNA was extracted using the RNeasy 96 kit (QIAGEN, 74182). After synthesizing cDNA using the High Capacity cDNA Reverse Transcription kit (Applied Biosystems, 4368813), the cDNA was mixed with PrimeTime Gene Expression Master Mix (Integrated DNA Technologies) and various probes, and gene expression was quantified using QuantStudio 6 Flex (Thermo Fisher Scientific Inc.). For the probe for the *Snhg1* gene, which is an NMD target, Mm.PT.58.44039547 (FAM) (Integrated DNA Technologies) was used, and for the probe for the Tbp gene, which is an endogenous control, Mm00446973_m1 Tbp (VIC-PL) (Applied Biosystems) was used. The relative quantification of *Snhg1* transcripts was calculated by the ΔΔCT method, using Tbp as an endogenous control and setting the expression level of *Snhg1* in untreated wells to 1.

A known NMD inhibitory compound (pyrimidine derivative Example 11i, Bioorg Med Chem Lett. 2012, 22(21), 6636-41) was used as a positive control drug for NMD inhibition. The NMD inhibitory activity of the test substance-added well was calculated as follows: the maximum fold change in Snghl expression in the positive control drug-added well relative to that in the untreated well was defined as the maximum NMD inhibitory activity, and the EC50 (compound concentration showing 50% activity) of the test substance showing a 1/2-fold change was calculated by the GROWTH function of Microsoft Excel. The calculated EC50 values of each test substance are shown in Table 28.

**Table 28**

| Example | Test Example 1 | Test Example 2 | Example | Test Example 1 | Test Example 2 |
|---|---|---|---|---|---|
| | ADP-Glo IC₅₀ (nM) | Cell-based Snhg1 EC₅₀ (nM) | | ADP-Glo IC₅₀ (nM) | Cell-based Snhg1 EC₅₀ (nM) |
| 2 | 4.9 | 94 | 91 | 1.8 | 34 |
| 3 | 4.6 | 161 | 92 | 1.9 | 20 |
| 4 | 2.4 | 41 | 93 | 1.2 | 81 |
| 5 | 1.4 | 30 | 94 | 1.5 | 60 |
| 6 | 1.4 | 8.6 | 95 | 1.0 | 82 |
| 7 | 6.0 | 95 | 102 | 2.8 | 76 |
| 8 | 1.8 | 36 | 103 | 3.3 | 71 |
| 9 | 4.0 | 122 | 104 | 0.79 | 75 |
| 18 | 0.57 | 43 | 105 | 1.5 | 59 |
| 19 | 2.4 | 111 | 106 | 2.5 | 87 |
| 20 | 2.0 | 13 | 107 | 0.64 | 30 |
| 24 | 2.2 | 76 | 108 | 1.0 | 2.0 |
| 25 | 1.8 | 39 | 109 | 2.3 | 165 |
| 26 | 0.99 | 28 | 110 | 1.3 | 1.1 |
| 27 | 1.0 | 2.8 | 111 | 1.6 | 8.4 |
| 28 | 2.0 | 78 | 112 | 1.3 | 510 |
| 29 | 2.0 | 56 | 113 | 0.38 | 8.3 |
| 30 | 1.9 | 40 | 114 | 1.1 | 3.2 |
| 31 | 1.3 | 34 | 115 | 0.64 | 58 |
| 32 | 2.4 | 77 | 116 | 1.2 | 38 |
| 33 | 3.0 | 48 | 117 | 5.1 | 80 |
| 34 | 2.2 | 25 | 118 | 1.8 | 34 |
| 35 | 1.4 | 40 | 119 | 3.4 | 24 |
| 36 | 1.2 | 11 | 120 | 0.96 | 37 |
| 37 | 0.36 | 9.4 | 121 | 1.4 | 46 |
| 38 | 1.7 | 10 | 122 | 2.2 | 28 |
| 39 | 1.9 | 19 | 123 | 1.6 | 46 |
| 40 | 0.53 | 11 | 124 | 3.2 | 55 |
| 41 | 1.1 | 4.8 | 125 | 0.72 | 23 |
| 42 | 2.6 | 124 | 126 | 1.6 | 29 |
| 43 | 0.97 | 24 | 127 | 0.96 | 34 |
| 44 | 0.50 | 54 | 128 | 4.5 | 56 |
| 45 | 0.80 | 11 | 129 | 3.5 | 17 |
| 46 | 1.8 | 115 | 130 | 1.6 | 11 |
| 47 | 2.1 | 117 | 131 | 0.78 | 22 |
| 56 | 0.52 | 1.2 | 132 | 3.5 | 50 |
| 59 | 0.52 | 41 | 133 | 2.4 | 44 |
| 60 | 0.71 | 5.7 | 134 | 1.1 | 18 |
| 61 | 1.4 | 26 | 135 | 2.2 | 39 |
| 62 | 0.95 | 16 | 136 | 0.63 | 5.3 |
| 63 | 0.68 | 14 | 137 | 1.2 | 10 |
| 64 | 0.84 | 15 | 138 | 0.36 | 1.8 |
| 65 | 0.69 | 15 | 139 | 0.31 | 4.3 |
| 66 | 2.2 | 67 | 140 | 1.0 | 8.8 |
| 67 | 1.0 | 32 | 141 | 0.37 | 3.1 |
| 68 | 0.62 | 10 | 142 | 0.63 | 3.7 |
| 69 | 0.91 | 6.8 | 143 | 0.85 | 12 |
| 70 | 0.56 | 15 | 144 | 0.95 | 19 |
| 71 | 1.3 | 2.3 | 145 | 1.2 | 3.1 |
| 72 | 0.81 | 4.5 | 146 | 0.49 | 3.8 |
| 75 | 1.2 | 51 | 147 | 0.67 | 3.9 |
| 84 | 1.0 | 42 | 148 | 0.73 | 12 |
| 85 | 0.39 | 0.47 | 149 | 0.67 | 0.61 |
| 86 | 1.8 | 43 | 150 | 3.6 | 66 |
| 87 | 1.3 | 59 | 151 | 1.5 | 16 |
| 88 | 1.1 | 46 | 152 | 1.1 | 4.0 |
| 89 | 0.59 | 14 | 153 | 0.96 | 5.1 |
| 90 | 1.5 | 74 | 154 | 0.53 | 0.50 |

### <Test Example 3: Evaluation of NMD inhibitory activity in vivo>

In this test, NMD inhibitory activity in tumor tissues was evaluated when a test substance was orally administered to mice subcutaneously implanted with EMT6 murine breast cancer cell line. As an indicator of NMD inhibitory activity, the increase in the transcript of *Snhgl,* an NMD target gene, was quantified. First, BALB/c mice were subcutaneously implanted with EMT6 cells (ATCC), and engraftment was confirmed 7 days later. A 0.5% methylcellulose suspension of the test substance was prepared and orally administered at the doses shown in Table 22. Six hours after administration of the test substance, mice were euthanized, and the collected tumor tissues were cryopreserved. RNA was extracted from the tumor tissues using the illustra RNAspin Mini RNA Isolation kit (GE Healthcare Life Sciences). After synthesizing cDNA using the High-Capacity cDNA Reverse Transcription Kits (Applied Biosystems, 4368813), the cDNA was mixed with PrimeTime Gene Expression Master Mix (Integrated DNA Technologies) and various probes, and gene expression was quantified by QuantStudio 6 Flex (Thermo Fisher Scientific Inc.). For the probe for the *Snhg1* gene, which is an NMD target, Mm.PT.58.44039547 (FAM) (Integrated DNA Technologies) was used, and for the probe for the Gapdh gene, which is an endogenous control, Mm99999915_g2 (VIC-PL) (Applied Biosystems) was used. The relative quantification of *Snhg1* transcripts in each sample was calculated by the ΔΔCT method using Gapdh as an endogenous control, and the expression level of *Snhg1* in each test substance administration group was calculated by setting the expression level of the Vehicle group (0.5% methylcellulose administration group) to 1. The results are shown in Table 29.

**Table 29**

| Example | Dose (mg/kg) | Snhg1 fold change | Example | Dose (mg/kg) | Snhg1 fold change |
|---|---|---|---|---|---|
| 8 | 30 | 4.1 | 127 | 10 | 6.2 |
| 19 | 100 | 7.5 | 128 | 30 | 6.2 |
| 26 | 10 | 12 | 129 | 10 | 9.0 |
| 28 | 25 | 8.8 | 130 | 10 | 9.2 |
| 29 | 30 | 4.6 | 131 | 10 | 7.1 |
| 31 | 10 | 5.5 | 132 | 10 | 7.4 |
| 32 | 50 | 12 | 133 | 12.5 | 8.2 |
| 34 | 25 | 6.2 | 135 | 12.5 | 10 |
| 35 | 10 | 4.6 | 136 | 10 | 4.8 |
| 36 | 10 | 7.5 | 137 | 25 | 5.1 |
| 37 | 10 | 8.4 | 138 | 10 | 12 |
| 38 | 30 | 9.2 | 139 | 10 | 7.5 |
| 61 | 100 | 4.3 | 140 | 10 | 6.3 |
| 68 | 30 | 8.3 | 141 | 10 | 8.4 |
| 70 | 10 | 3.6 | 142 | 10 | 11 |
| 72 | 30 | 4.3 | 143 | 10 | 8.5 |
| 84 | 10 | 4.6 | 144 | 30 | 4.8 |
| 85 | 10 | 6.9 | 145 | 10 | 8.9 |
| 91 | 10 | 5.9 | 146 | 10 | 10 |
| 92 | 10 | 6.5 | 147 | 10 | 9.5 |
| 117 | 10 | 5.1 | 148 | 10 | 5.8 |
| 118 | 10 | 9.1 | 149 | 10 | 12 |
| 123 | 30 | 4.4 | 152 | 10 | 7.9 |
| 124 | 10 | 4.6 | 153 | 10 | 6.9 |
| 125 | 10 | 6.5 | 154 | 10 | 10 |
| 126 | 30 | 4.1 | | | |

### <Test Example 4: Antitumor test in a syngeneic model>

In this test, the antitumor effect of a test substance alone and in combination with an immune checkpoint inhibitor was examined using mice subcutaneously implanted with MC38 mouse colorectal cancer cell line. C57BL6/J mice (CLEA Japan, Inc.) were subcutaneously implanted with MC38 mouse colorectal cancer cell line (NCI) at 3 × 10⁵ cells/head. The test substance was pulverized in an agate mortar, a suspension was prepared using a 0.5% methylcellulose solution (FUJIFILM Wako Pure Chemical Corporation, #133-17815), and the suspension was orally administered once a day from the day after subcutaneous implantation at the doses shown in Figs. 5 to 9. Anti-mouse PD-1 antibody (Bio X cell, BE0146, clone: RMP1-14) or anti-mouse PD-L1 antibody (Bio X cell, BE0101, clone: 10F.9G2) was diluted with PBS to a predetermined concentration, and intraperitoneally administered at 10 mg/kg for a total of 4 times every 3 to 4 days starting from 3 days after subcutaneous implantation or for a total of 3 times every 3 to 4 days starting from 7 days after subcutaneous implantation. The tumor diameter in each group was measured over time using calipers (Mitutoyo, CD-15AX), and the tumor volume was calculated by the following formula using the SMAD2 system (JMACS Software Co., Ltd.). Tumor volume (mm³) = major axis (mm) × [minor axis (mm)]² × 1/2

The antitumor effect was evaluated by comparing the tumor volume in each group with that in the Vehicle group (0.5% methylcellulose administration group). The results are shown in Figs. 5 to 9.

### Industrial Applicability

The compound of the present invention, or a pharmaceutically acceptable salt thereof, exhibits SMG1 and NMD inhibitory activity, an anticancer effect as a single agent, and an anticancer effect in combination with an immune checkpoint inhibitor.

## Claims

1. A compound represented by formula (1), or a pharmaceutically acceptable salt thereof: 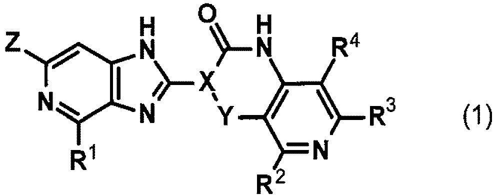 where in formula (1),
R¹ represents a hydrogen atom, a C1-C6 alkyl group, where the C1-C6 alkyl group may be substituted with a hydroxyl group or a halogen atom, a C3-C6 cycloalkyl group, a phenyl group, where the phenyl group may be substituted with an amino group or a halogen atom, a 2-tert butylamino-2-oxoethyl group, a [dimethyl(oxido)-λ6-sulfanylidene]amino group, a group represented by the following formula (2): 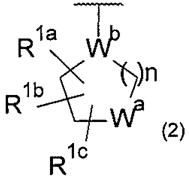
where in formula (2), W^{a} represents CH₂, an oxygen atom, or N-R^{1f}, W^{b} represents CH, a nitrogen atom, or C-OH, R^{1a}, R^{1b}, and R^{1c} each independently represent the same or different hydrogen atom, halogen atom, hydroxyl group, C1-C6 alkyl group optionally substituted with a halogen atom, amino group, or oxo group, and n represents 0, 1, or 2,
or -NR^{1d}R^{1e};
R^{1d} and R^{1e} each independently represent the same or different hydrogen atom, C1-C6 alkyl group, where the C1-C6 alkyl group may be substituted with a halogen atom or a 4- to 7-membered heterocyclic group, a 4- to 7-membered heterocyclic group, or a C3-C6 cycloalkyl group optionally substituted with a hydroxyl group;
R^{1f} represents a hydrogen atom, a C1-C6 alkyl group, or a C3-C6 cycloalkyl group;
R² represents a hydrogen atom, a C1-C6 alkyl group, a C3-C6 cycloalkyl group optionally substituted with a C1-C6 alkyl group, a 2,4-dimethylpyrazolyl group, or any group selected from the following formula (3): 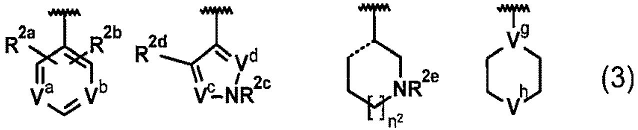
where in formula (3), the broken line represents a single bond or a double bond, V^{a} represents C-R^{2f} or a nitrogen atom, V^{b} represents C-R^{2g} or a nitrogen atom, V^{c} represents C-R^{2j} or a nitrogen atom, V^{d} represents C-R^{2k} or a nitrogen atom, V^{g} represents CH or a nitrogen atom, V^{h} represents CHN(CH₃)₂, N-R²¹, or an oxygen atom, and n² represents 1 or 2,
R^{2a} and R^{2b} each independently represent the same or different hydrogen atom, halogen atom, hydroxyl group, cyano group, C1-C6 alkyl group, where the C1-C6 alkyl group may be substituted with a hydroxyl group, a halogen atom, or a 4- to 7-membered heterocyclic group, a C1-C6 alkoxy group, where the C1-C6 alkoxy group may be substituted with a halogen atom or deuterium, or a C3-C6 cycloalkyl group,
R^{2c} represents a C3-C6 cycloalkyl group or a C1-C6 alkyl group, where the C1-C6 alkyl group may be substituted with a C3-C6 cycloalkyl group or a halogen atom,
R^{2d} represents a hydrogen atom, a C1-C6 alkyl group optionally substituted with a halogen atom, a C1-C6 alkoxy group, or a C3-C6 cycloalkyl group,
R^{2e} represents a C1-C6 alkylsulfonyl group, a C3-C6 cycloalkylsulfonyl group, a benzenesulfonyl group, where the benzenesulfonyl group may be substituted with a C1-C6 alkyl group or a halogen atom, a C3-C6 cycloalkylcarbonyl group, a C1-C6 alkylcarbonyl group, or a benzoyl group, where the benzoyl group may be substituted with a C1-C6 alkyl group or a halogen atom,
R^{2f} and R^{2g} each independently represent the same or different hydrogen atom, halogen atom, C1-C6 alkoxy group optionally substituted with a halogen atom, or C1-C6 alkyl group optionally substituted with a hydroxyl group, or the following formula (4) 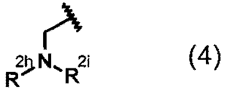
where in formula (4), R^{2h} and R²ⁱ each independently represent the same or different C1-C6 alkyl group, or R^{2h} and R²ⁱ may be bonded to each other to form a 4- to 7-membered heterocyclic group optionally substituted with a C1-C6 alkyl group,
R^{2j} and R^{2k} each independently represent the same or different hydrogen atom, C1-C6 alkyl group optionally substituted with a halogen atom, or C3-C6 cycloalkyl group, or
R^{2c} and R^{2k} may be bonded to each other to form a group shown below, 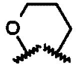
R²¹ represents a C1-C6 alkyl group or a C3-C6 cycloalkyl group;
or alternatively, R¹ and R² may be bonded to each other to form a group represented by the following formula (5),
where the leftmost bond of each group represents the bond between the aromatic ring and R¹ of the compound represented by formula (1), and the rightmost bond represents the bond between the aromatic ring and R² of the compound represented by formula (1), 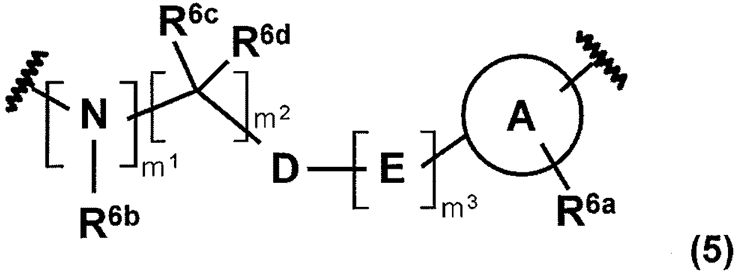
where in formula (5), A represents an aryl group, a heteroaryl group, or a 4- to 7-membered heterocyclic group, R^{6a} represents a hydrogen atom, a halogen atom, a C1-C6 alkyl group, a C3-C6 cycloalkyl group, or a C1-C6 alkoxy group, D represents -NR^{6e}CO- or -CONR^{6f}-, E represents -CR^{6g}R^{6h}-, -NR⁶ⁱ- -O-, -S-, -SO-, or -SO₂-, and when there are a plurality of Es, the plurality of Es may be the same or different, R^{6b}, R^{6c}, R^{6d}, R^{6e}, R^{6f}, R^{6g}, R^{6h}, and R⁶ⁱ each independently represent a hydrogen atom, a C1-C6 alkyl group, or a C3-C6 cycloalkyl group, or R^{6b} and R^{6c}, R^{6b} and R^{6e}, or R^{6c} and R^{6d} may be bonded to each other to form a ring, m¹ represents 0 or 1, m² represents 1, 2, 3, 4, or 5, and m³ represents 0, 1, 2, or 3;
R³ represents a hydrogen atom, a halogen atom, a C1-C6 alkyl group, or a 1-methyl-3,6-dihydro-2H-pyrazin-5-yl group;
R⁴ represents a hydrogen atom or a halogen atom;
X-Y represents C=CH or N-CH₂; and
Z represents a hydrogen atom, a halogen atom, a C1-C6 alkyl group optionally substituted with a halogen atom, a C1-C6 alkoxy group, a cyano group, or a C1-C6 alkoxycarbonyl group.

2. The compound according to claim 1, or a pharmaceutically acceptable salt thereof, wherein R¹ represents a hydrogen atom, a C1-C6 alkyl group, -NR^{11d}R^{11e}, or any group selected from the following formula (6): 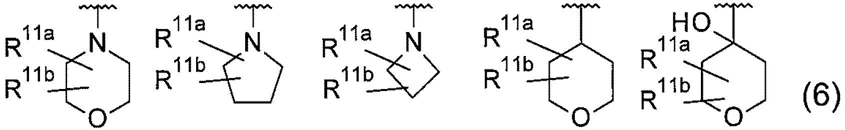 where in formula (6),
R^{11a} and R^{11b} each independently represent the same or different hydrogen atom, halogen atom, hydroxyl group, or C1-C6 alkyl group; and
R^{11d} and R^{11e} each independently represent the same or different hydrogen atom or C1-C6 alkyl group optionally substituted with a halogen atom.

3. The compound according to claim 1 or 2, or a pharmaceutically acceptable salt thereof, wherein R² represents a hydrogen atom, a C1-C6 alkyl group, a C3-C6 cycloalkyl group optionally substituted with a C1-C6 alkyl group, a 4-(dimethylamino)piperidin-1-yl group, or any group selected from the following formula (7): 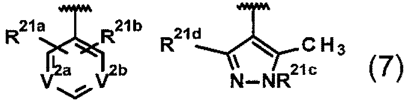 where in formula (7),
V^{2a} represents C-R^{21e} or a nitrogen atom;
V^{2b} represents C-R^{21f} or a nitrogen atom;
R^{21a} and R^{21b} each independently represent the same or different hydrogen atom, halogen atom, hydroxyl group, C1-C6 alkyl group optionally substituted with a halogen atom, C1-C6 alkoxy group, where the C1-C6 alkoxy group may be substituted with a halogen atom or deuterium, or C3-C6 cycloalkyl group;
R^{21c} represents a C1-C6 alkyl group optionally substituted with a halogen atom;
R^{21d} represents a hydrogen atom or a C3-C6 cycloalkyl group;
R^{21e} represents a hydrogen atom or a halogen atom; and
R^{21f} represents a hydrogen atom or a halogen atom.

4. The compound according to any one of claims 1 to 3, or a pharmaceutically acceptable salt thereof, wherein
R³ represents a hydrogen atom;
R⁴ represents a hydrogen atom;
X-Y represents C=CH or N-CH₂; and
Z represents a halogen atom.

5. The compound according to claim 1, or a pharmaceutically acceptable salt thereof, wherein
R¹ represents any group selected from an ethyl group, a 2,2-difluoroethylamino group, or a [(2R)-1,1,1-trifluoropropan-2-yl]amino group;
R² represents a 2-methoxypyridin-3-yl group, a 1,5-dimethyl-1H-pyrazol-4-yl group, or a group represented by the following formula (8): 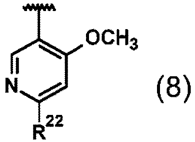
where in formula (8), R²² represents a C1-C6 alkyl group optionally substituted with a halogen atom, a C1-C6 alkoxy group optionally substituted with a halogen atom, or a C3-C6 cycloalkyl group;
R³ represents a hydrogen atom;
R⁴ represents a hydrogen atom;
X-Y represents C=CH or N-CH₂; and
Z represents a halogen atom.

6. The compound according to claim 1, or a pharmaceutically acceptable salt thereof, which is selected from the following compounds:
3-(6-chloro-4-ethyl-1H-imidazo[4,5-c]pyridin-2-yl)-5-(6-cyclopropyl-4-methoxypyridin-3-yl)-1,6-naphthyridin-2(1H)-one,
3-(6-chloro-4-ethyl-1H-imidazo[4,5-c]pyridin-2-yl)-5-[4-methoxy-6-(trifluoromethyl)pyridin-3-yl]-1,6-naphthyridin-2(1H)-one,
3-(6-chloro-4-{[(2R)-1,1,1-trifluoropropan-2-yl]amino}-1H-imidazo[4,5-c]pyridin-2-yl)-5-(1,5-dimethyl-1H-pyrazol-4-yl)-1,6-naphthyridin-2(1H)-one,
3-(6-chloro-4-ethyl-1H-imidazo[4,5-c]pyridin-2-yl)-5-[6-(1,1-difluoroethyl)-4-methoxypyridin-3-yl]-1,6-naphthyridin-2(1H)-one,
3-(6-chloro-4-ethyl-1H-imidazo[4,5-c]pyridin-2-yl)-5-[6-(difluoromethoxy)-4-methoxypyridin-3-yl]-1,6-naphthyridin-2(1H)-one,
3-{6-chloro-4-[(2,2-difluoroethyl)amino]-1H-imidazo[4,5-c]pyridin-2-yl}-5-(2-methoxypyridin-3-yl)-1,6-naphthyridin-2(1H)-one, or
3-(6-chloro-4-ethyl-1H-imidazo[4,5-c]pyridin-2-yl)-5-(6-cyclopropyl-4-methoxypyridin-3-yl)-3,4-dihydropyrido[4,3-d]pyrimidin-2(1H)-one.

7. 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one, or a pharmaceutically acceptable salt thereof.

8. 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one 1,2-ethanedisulfonate.

9. 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one methanesulfonate.

10. 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one benzenesulfonate.

11. 3-(6-Chloro-4-ethyl-1H-imidazo[4,5-c]pyridin-2-yl)-5-(6-cyclopropyl-4-methoxypyridin-3-yl)-1,6-naphthyridin-2(1H)-one adipate.

12. An SMG1 inhibitor comprising the compound according to any one of claims 1 to 11, or a pharmaceutically acceptable salt thereof, as an active ingredient.

13. An NMD inhibitor comprising the compound according to any one of claims 1 to 11, or a pharmaceutically acceptable salt thereof, as an active ingredient.

14. A pharmaceutical composition comprising the compound according to any one of claims 1 to 11, or a pharmaceutically acceptable salt thereof, as an active ingredient, and optionally comprising a pharmaceutically acceptable carrier.

15. The pharmaceutical composition according to claim 14 for treating cancer.

16. The pharmaceutical composition according to claim 15, wherein the compound according to any one of claims 1 to 11, or a pharmaceutically acceptable salt thereof, is administered in combination with an immune checkpoint inhibitor.

17. The pharmaceutical composition according to claim 16, wherein the compound according to any one of claims 1 to 11, or a pharmaceutically acceptable salt thereof, and the immune checkpoint inhibitor are each contained as an active ingredient in different pharmaceutical formulations, and are administered simultaneously or at different times.

18. The pharmaceutical composition according to claim 16, wherein the compound according to any one of claims 1 to 11, or a pharmaceutically acceptable salt thereof, and the immune checkpoint inhibitor are contained as active ingredients in a single pharmaceutical formulation.

19. The pharmaceutical composition according to any one of claims 16 to 18, wherein the immune checkpoint inhibitor is an anti-PD-1 antibody, an anti-PD-L1 antibody, or an anti-CTLA-4 antibody.

20. The pharmaceutical composition according to any one of claims 15 to 19, wherein the cancer is hematological cancer selected from the group consisting of leukemia, lymphoma, and multiple myeloma; sarcoma selected from the group consisting of osteosarcoma, chordoma, chondrosarcoma, and Ewing sarcoma; or solid cancer selected from the group consisting of gastric cancer, lung cancer, colorectal cancer, esophageal cancer, head and neck cancer, breast cancer, liver cancer, kidney cancer, tongue cancer, thyroid cancer, uterine cancer, ovarian cancer, prostate cancer, cervical cancer, pancreatic cancer, skin cancer, adrenal cancer, biliary tract cancer, thymic cancer, mesothelioma, bladder cancer, and brain tumor.

21. An anticancer agent comprising the compound according to any one of claims 1 to 11, or a pharmaceutically acceptable salt thereof, as an active ingredient.

22. A method for treating cancer, comprising administering an effective amount of the compound according to any one of claims 1 to 11, or a pharmaceutically acceptable salt thereof.

23. A method for treating cancer, wherein the compound according to any one of claims 1 to 11, or a pharmaceutically acceptable salt thereof, is administered in combination with an immune checkpoint inhibitor.

24. The treatment method according to claim 23, wherein the compound according to any one of claims 1 to 11, or a pharmaceutically acceptable salt thereof, and the immune checkpoint inhibitor are each contained as an active ingredient in different pharmaceutical formulations, and are administered simultaneously or at different times.

25. The treatment method according to claim 23, wherein the compound according to any one of claims 1 to 11, or a pharmaceutically acceptable salt thereof, and the immune checkpoint inhibitor are contained as active ingredients in a single pharmaceutical formulation and administered.

26. The treatment method according to any one of claims 23 to 25, wherein the immune checkpoint inhibitor is an anti-PD-1 antibody, an anti-PD-L1 antibody, or an anti-CTLA-4 antibody.

27. The treatment method according to any one of claims 22 to 26, wherein the cancer is hematological cancer selected from the group consisting of leukemia, lymphoma, and multiple myeloma; sarcoma selected from the group consisting of osteosarcoma, chordoma, chondrosarcoma, and Ewing sarcoma; or solid cancer selected from the group consisting of gastric cancer, lung cancer, colorectal cancer, esophageal cancer, head and neck cancer, breast cancer, liver cancer, kidney cancer, tongue cancer, thyroid cancer, uterine cancer, ovarian cancer, prostate cancer, cervical cancer, pancreatic cancer, skin cancer, adrenal cancer, biliary tract cancer, thymic cancer, mesothelioma, bladder cancer, and brain tumor.

28. The compound according to any one of claims 1 to 11, or a pharmaceutically acceptable salt thereof, for use in treating cancer.

29. The compound according to any one of claims 1 to 11, or a pharmaceutically acceptable salt thereof, for use in treating cancer, which is administered in combination with an immune checkpoint inhibitor.

30. The compound according to claim 29, or a pharmaceutically acceptable salt thereof, wherein the compound according to any one of claims 1 to 11, or a pharmaceutically acceptable salt thereof, and the immune checkpoint inhibitor are each contained as an active ingredient in different pharmaceutical formulations, and are administered simultaneously or at different times.

31. The compound according to claim 29, or a pharmaceutically acceptable salt thereof, wherein the compound according to any one of claims 1 to 11, or a pharmaceutically acceptable salt thereof, and the immune checkpoint inhibitor are contained as active ingredients in a single pharmaceutical formulation and are administered.

32. The compound according to any one of claims 29 to 31, or a pharmaceutically acceptable salt thereof, wherein the immune checkpoint inhibitor is an anti-PD-1 antibody, an anti-PD-L1 antibody, or an anti-CTLA-4 antibody.

33. The compound according to any one of claims 28 to 32, or a pharmaceutically acceptable salt thereof, wherein the cancer is hematological cancer selected from the group consisting of leukemia, lymphoma, and multiple myeloma; sarcoma selected from the group consisting of osteosarcoma, chordoma, chondrosarcoma, and Ewing sarcoma; or solid cancer selected from the group consisting of gastric cancer, lung cancer, colorectal cancer, esophageal cancer, head and neck cancer, breast cancer, liver cancer, kidney cancer, tongue cancer, thyroid cancer, uterine cancer, ovarian cancer, prostate cancer, cervical cancer, pancreatic cancer, skin cancer, adrenal cancer, biliary tract cancer, thymic cancer, mesothelioma, bladder cancer, and brain tumor.

34. Use of the compound according to any one of claims 1 to 11, or a pharmaceutically acceptable salt thereof, in the manufacture of a medicament for the treatment of cancer.

35. The use according to claim 34, wherein the compound according to any one of claims 1 to 11, or a pharmaceutically acceptable salt thereof, is administered in combination with an immune checkpoint inhibitor.

36. The use according to claim 35, wherein the compound according to any one of claims 1 to 11, or a pharmaceutically acceptable salt thereof, and the immune checkpoint inhibitor are each contained as an active ingredient in different pharmaceutical formulations, and are administered simultaneously or at different times.

37. The use according to claim 35, wherein the compound according to any one of claims 1 to 11, or a pharmaceutically acceptable salt thereof, and the immune checkpoint inhibitor are contained as active ingredients in a single pharmaceutical formulation and are administered.

38. The use according to any one of claims 35 to 37, wherein the immune checkpoint inhibitor is an anti-PD-1 antibody, an anti-PD-L1 antibody, or an anti-CTLA-4 antibody.

39. The use according to any one of claims 34 to 38, wherein the cancer is hematological cancer selected from the group consisting of leukemia, lymphoma, and multiple myeloma; sarcoma selected from the group consisting of osteosarcoma, chordoma, chondrosarcoma, and Ewing sarcoma; or solid cancer selected from the group consisting of gastric cancer, lung cancer, colorectal cancer, esophageal cancer, head and neck cancer, breast cancer, liver cancer, kidney cancer, tongue cancer, thyroid cancer, uterine cancer, ovarian cancer, prostate cancer, cervical cancer, pancreatic cancer, skin cancer, adrenal cancer, biliary tract cancer, thymic cancer, mesothelioma, bladder cancer, and brain tumor.
